(19) Europäisches Patentamt
European Patent Office
Office européen des brevets

(11) **EP 4 501 910 A1**

(12) **EUROPEAN PATENT APPLICATION**
published in accordance with Art. 153(4) EPC

(43) Date of publication:
**05.02.2025 Bulletin 2025/06**

(21) Application number: **23781323.3**

(22) Date of filing: **28.03.2023**

(51) International Patent Classification (IPC):
***C07D 211/60*** *(2006.01)* ***C07D 211/16*** *(2006.01)*
***C07D 207/16*** *(2006.01)* ***C07D 207/08*** *(2006.01)*
***C07D 401/06*** *(2006.01)* ***C07D 409/06*** *(2006.01)*
***C07D 405/06*** *(2006.01)* ***A61K 31/445*** *(2006.01)*
***A61K 31/40*** *(2006.01)* ***A61P 25/00*** *(2006.01)*

(52) Cooperative Patent Classification (CPC):
**A61K 31/40; A61K 31/401; A61K 31/445; A61K 31/4525; A61K 31/4535; A61K 31/4545; A61K 31/506; A61P 25/00; A61P 25/28; C07D 207/08; C07D 207/16; C07D 211/16; C07D 211/60; C07D 401/06; C07D 401/10;** (Cont.)

(86) International application number:
**PCT/KR2023/004138**

(87) International publication number:
**WO 2023/191468 (05.10.2023 Gazette 2023/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.03.2022 US 202263324221 P**

(71) Applicant: **BNH Research Co., Ltd.**
**Goyang-si, Gyeonggi-do 10594 (KR)**

(72) Inventors:
- **CHUNG, Seung Soo**
  **Goyang-si, Gyeonggi-do 10596 (KR)**
- **KIM, Young Hwan**
  **Goyang-si, Gyeonggi-do 10218 (KR)**
- **JEONG, Ji Hyun**
  **Seoul 03974 (KR)**

(74) Representative: **Ullrich & Naumann PartG mbB**
**Schneidmühlstrasse 21**
**69115 Heidelberg (DE)**

Remarks:
The complete document including Reference Table(s) and the Sequence Listing(s) can be downloaded from the EPO website

# (54) NITROGEN-CONTAINING HETEROCYCLIC COMPOUNDS AND USES THEREOF

(57) The present invention relates to nitrogen-containing heterocyclic compound derivatives and uses thereof. The compounds according to the present invention can exert therapeutic effects on various neurological disorders, such as neurodegenerative diseases, neurodegenerative pathologies, neuropsychiatric disorders, memory loss, cognitive dysfunction/impairment, and impairment of extinction learning, by effectively enhancing synaptic plasticity without side effects.

[FIG. 3C]

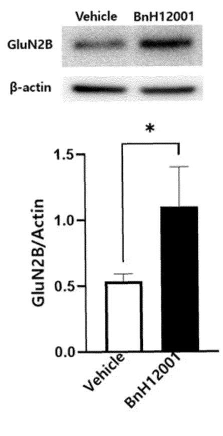


EP 4 501 910 A1

(52) Cooperative Patent Classification (CPC): (Cont.)
**C07D 403/10; C07D 405/06; C07D 405/10; C07D 409/06; C07D 409/10; C07D 413/10; G01N 33/487**

## Description

### Technical Field

The present disclosure relates to nitrogen-containing heterocyclic compounds and uses thereof. More specifically, the present disclosure relates to compounds, compositions or methods for preventing or treating a neurological disorder or a neurodegenerative disorder.

### Background Art

Synaptic plasticity and critical period

A neural network is a network made of neurons. A synapse is a node in the neural network through which neurons exchange information with each other. Within a network, synapses are "plastic," because their numbers and strengths change continuously throughout life, as synaptic inputs are added, removed, or refined in response to ongoing neural activities. Synaptic plasticity refers to these neural activity-dependent modifications of the synapses, which is manifested by changes in the strength and/or efficiency of synaptic transmission.

Higher-order functions, such as learning, cognition, synthesis, judgment, art, or creativity, depend on the ability to form neural networks in the cerebral cortex (called the cortical neural network). Known as the critical period, synaptic plasticity of the cortical neural network peaks during the early days after birth. After the critical period, the cortical synaptic plasticity decreases gradually with age, eventually leading to a state in which experiences no longer results in active changes in the cerebral cortex. Interestingly, though, recent studies have demonstrated that under certain conditions, synaptic plasticity could be restored to heightened levels even after the critical period had been closed: in an animal model, peripheral nerve injury reactivated synaptic plasticity in the somatosensory thalamocortical circuits that transmits peripheral sensory signals to the cerebral cortex (Yu et al., 2012, Neuron. 74:731-42; Petrus et al., 2014, Neuron. 81:664-73; Gao et al., 2014, J Neurosci. 34:10770-9).

Reduced synaptic plasticity is also related to degeneration of the brain function in adults. In particular, the synaptic plasticity of the thalamic cortical circuit is significantly limited with age. If reopening the critical period is possible, as mentioned above, reactivating or increasing cortical synaptic plasticity via therapeutical means may be useful for treating conditions caused by reduced synaptic plasticity.

Histone deacetylase inhibitors

The bulk of the DNA in the cell exists as packed in a denser and more compacted form known as chromatin. The compaction occurs by winding the DNA around a group of protein called histones. The degree of chromatin compaction varies according to the level of acetylation of histone. In general, histone acetyltransferase (HAT) performs acetylation, which opens the access to the DNA, and histone deacetylase (HDAC) performs deacetylation, which closes the access to the DNA. Since transcription can only occur when the access is open, chromatin compaction works as a regulatory mechanism for gene expression (Verdin and Ott, 2015, Nat Rev Mol Cell Biol 16:258-264).

Eleven types of mammalian HDACs have been identified to date. These are largely classified into four classes based on the degree of homology with yeast HDAC. Class I includes HDACs 1, 2, 3 and 8. Class II HDACs are divided into Class IIa (HDACs 4, 5, 7, and 9) and Class IIb (HDACs 6 and 10) based on structural similarity. Class III HDACs includes SIRT1-7, and Class IV includes HDAC11.

While class I HDACs are mainly confined in the nucleus, Class II HDACs shuttle between the nucleus and the cytoplasm through the phosphorylation of specific serine residues. The N-terminal domains of HDACs interact with several tissue-specific transcription factors, and act as a transcriptional repressor. The C-terminal domains of HDACs possess the deacetylase activity. Class IIa HDACs, however, do not possess deacetylase activity because they carry a loss-of-function mutation in which their tyrosine (Tyr) residues in the C-terminal catalytic domains are substituted with histidine (His).

HDAC4

HDAC4 is expressed throughout the body. HDAC4 is particularly highly expressed in the brain, heart and skeletal muscle (Grozinger et al., 1999, Proc. Natl. Acad. Sci. USA 96, 4868-4873; Darcy et al., 2010, J. Comp. Neurol. 518, 722-740). In the brain, HDAC4 level peaks immediately after birth (Sando et al., 2012, Cell 151, 821-834). HDAC4 is detected in the corticotropin-releasing hormone (CRH) neurons, (100±0%), the oxytocin neurons (98±2%), the vasopressin neurons (100±0%), orexin neurons (100±0%), the AgRP neurons (100±0%), the POMC neurons (92±4%), the histamine neurons (85±7%), the dopamine neurons (100±0%), the serotonin neurons (93±2%), and the noradrenaline neurons (100±0%) (Takase et al., 2013, PloS ONE 8:e58473).

Cytoplasmic HDAC4 is rapidly translocated to the nucleus in response to a low potassium or excitotoxic glutamate condition that induces apoptosis. Ectopic overexpression of HDAC4 in the nucleus promoted neuronal cell death and inhibited the transcriptional activities of survival factors, such as MEF2 and CREB (Bolger and Yao, 2005, J Neurosci. 2005; 25 :9544-9553). Neurons containing HDAC4 are uniform in size and are widely distributed in neuropils including paraventricular nucleus (PVN), lateral hypothalamus (LHA), hypothalamic arcuate nucleus (ARC), tuberous mammary nucleus (TMN), dorsal raphe (DR) and locus coeruleus (LC). In particular, HDAC4 is co-localized with PSD95 (Mielcarek et al., 2013, PLoS Biol. 11:e1001717; Takase et al., 2013, PloS ONE 8:e58473), suggesting a role for HDAC4 in synaptic plasticity (Sando et al., 2012, Cell 151, 821-834; Mielcarek et al., 2013, PLoS Biol. 11:e1001717).

In animal experimental models, tissue-specific deletion of HDAC4 in rat hippocampus abolished long-lasting, stressinduced behavioral changes (Sailaja et al., 2012, Proc. Natl. Acad. Sci. USA 109, E3687-E3695). HDAC4 overexpression accelerated the death of cerebellar granule neurons by increasing susceptibility to $H_2O_2$ damage through inhibition of the activity of peroxisome proliferator-activated receptor $\alpha$ (PPAR$\alpha$) (Bolger and Yao, 2005, J. Neurosci. 25, 9544-9553; Li et al., 2012, Nat. Med. 18, 783-790; Sando et al., 2012; Cell 151, 821-834; Yang et al., 2011, FEBS Lett. 585, 761-766). HDAC4 overexpression in the rat hippocampus induced depression-like behaviors (Sarkar et al., 2014, Neuropsychopharmacology 39, 2221-2232).

In the Drosophila model, the overexpression of HDAC4 in adult mushroom body neurons, which are structures important for memory formation, caused damage to long-term courtship memory, but had no effect on short-term memory (Fitzsimons et al., 2013, PloS ONE 8:e83903). A subsequent study examining the relationship with the subcellular distribution of HDAC4 showed that mushroom body morphogenesis, eye development, and deficits in long-term memory were associated with nuclear HDAC4 abundance (Main et al., 2021, Front. Mol. Neurosci. 14:616-642).

In humans, an increase in HDAC4 levels was confirmed in the post-mortem HD frontotemporal lobar degeneration (FTLD) brain (Yeh et al., 2013, Brain Res. 1504, 16-24; Whitehouse et al., 2014, Neuropathol. Appl. Neurobiol. 41, 245257). HDAC4 was identified as a component of Lewy bodies in Parkinson's disease brains and of intranuclear inclusions in the neuronal intranuclear inclusion disease (Takahashi-Fujigasaki and Fujigasaki, 2006, Neuropathol. Appl. Neurobiol. 32, 562-566). HDAC4 was observed to be significantly overexpressed in the specific cortical regions of autistic patients (Nardone et al., 2014, Transl. Psychiatry 4:e433).

HDAC inhibitors and synaptic plasticity

Histone modification have been proposed as a potential method to restore cortical plasticity in adulthood. Valproic acid is known as a histone deacetylase (HDAC) inhibitor. In a clinical study that participants took valproic acid capsules, the critical period was reopened and absolute pitch learning, which was only possible during the critical period, became possible even in adults (Gervain et al., 2013, Front Syst Neurosci. 7:102). When adult rats were exposed to an enriching environment as compared to a normal cage condition, the total amount of H3 acetylation of the visual cortex was increased compared to the control group, and ocular dominance plasticity, which appears only during the critical period, was also observed after the critical period (Baroncelli L et al., 2016, J Neurosci. 36:3430-40).

Treatment with suberoylanilide hydroxamic acid (SAHA), which is an HDAC inhibitor, increased H3 acetylation in the visual cortex to the level typically observed in young rats, and in particular, the H3 acetylation of the P3 promotor of brain-derived neurotrophic factor (BNDF), which is known for its importance in developmental synaptic plasticity and longterm memory in the visual cortex, was also increased (Baroncelli L et al., 2016, J Neurosci. 36:3430-40). The increased BDNF expression was also observed in neurospheres as well (Bagheri et al., 2019, Int. J. Mol. Sci. 20: 1109).

SAHA or other HDAC inhibitors have been found to have an effect of improving symptoms in a mouse model of Huntington's disease (HD) (Ferrante *et al.*, 2003; Hockly et al., 2003, J Biol. Chem. 278, 16059-16072; Gardian et al., 2005, J. Biol. Chem. 280, 556-563). HDAC4 is correlated with the length of polyglutamine expressed in Huntington's disease. It is co-localized with cytoplasmic inclusions, and a wide range of peripheral organ pathologies such as skeletal muscle atrophy and heart failure that appear in Huntington's disease (Zielonka et al., 2014, Front. Physiol. 5:380; Mielcarek et al., 2014, PLoS Genet. 10:e1004550; Mielcarek et al., 2014, PloS ONE 9:e108961; Zielonka et al., 2014, Exp. Clin. Cardiol. 20, 25472554). Reducing HDAC4 levels in R6/2, which is a mouse model of Huntington's disease, delayed cytoplasmic aggregate formation in various regions of the brain, improved motor co-ordination and neurological phenotype, and had a lifespan extension effect (Mielcarek et al., 2013, PLoS Biol. 11:e1001717).

Interestingly, SAHA treatment in R6/2 mice restored cortical transcript levels of BDNF (Mielcarek et al., 2011, PloS ONE 6:e27746). Since HDAC4 and HDAC5 can inhibit specific BDNF transcripts and this effect has been shown to be reversed by SAHA treatment, memory enhancing and neuroprotective effects by HDAC inhibitors may be associated with increased BDNF expression (Koppel and Timmusk, 2013, Neuropharmacology 75, 106-11).

HDAC4 may play a central role in brain physiology and could be a useful target for treating several neurodegenerative disorders. However, currently available HDAC inhibitors are broad spectrum HDAC inhibition, rendering them unsuitable for inhibiting a specific HDAC. Although SAHA increases the synaptic plasticity, it causes severe side effects including fatigue, nausea, diarrhea, or thrombocytopenia that renders it unsuitable for human use (Meghan E. et al., 2011,

Bone. 48: 1117-1126).

Therefore, it would be useful to develop selective HDAC inhibitors for targeted therapy.

## Disclosure of Invention

### Technical Problem

The object of the present disclosure is to solve all of the above-mentioned problems.

An object of the present disclosure is to provide a nitrogen-containing heterocyclic compound, a stereoisomer thereof, or a pharmaceutically acceptable salt thereof for improving synaptic plasticity.

Another object of the present disclosure is to provide a pharmaceutical composition for treating or preventing a neurological disorder or a neurodegenerative disorder, comprising, as an active ingredient, a nitrogen-containing heterocyclic compound, a stereoisomer thereof, or a pharmaceutically acceptable salt thereof for improving synaptic plasticity.

Yet another object of the present disclosure is to provide a method for treating or preventing a neurological disorder or a neurodegenerative disorder, comprising administering to a subject a nitrogen-containing heterocyclic compound, a stereoisomer thereof, or a pharmaceutically acceptable salt thereof for improving synaptic plasticity.

Still yet another object of the present disclosure is to provide a pharmaceutical composition for combined administration, comprising a nitrogen-containing heterocyclic compound, a stereoisomer thereof, or a pharmaceutically acceptable salt thereof for improving synaptic plasticity, and a second therapeutic agent, and a combined administration method using the same.

The object of the present disclosure is not limited to the above-mentioned objects. The objects of the present disclosure will become clearer from the following description and may be realized by means and combinations thereof as set forth in the claims.

### Solution to Problem

Representative configurations of the present disclosure to achieve the above-mentioned objects are as follows.

According to an aspect of the present disclosure, there is provided a compound represented by Formula (1), a stereoisomer thereof, or a pharmaceutically acceptable salt thereof:

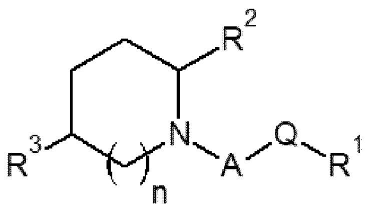

(1)

$R^1$ is $C_{1-6}$ alkyl, $C_{3-12}$ cycloalkyl, aryl, or heteroaryl, wherein at least one hydrogen atom in these groups is unsubstituted or each independently substituted with a halogen atom, $C_{1-6}$ alkyl, $C_{1-6}$ alkoxy, nitro, amino optionally substituted with at least one $C_{1-6}$ alkyl, or hydroxy,

A is C=O or a bond,

Q is $-(CR^4R^5)_m-$ or a bond, wherein $R^4$ and $R^5$ are each independently hydrogen or $C_{1-6}$ alkyl, and m is an integer of 1 to 6,

$R^2$ is hydrogen, $C_{1-6}$ alkyl, or aryl,

$R^3$ is hydrogen, carboxyl, N-hydroxy carboxamide, or carboxamide, wherein at least one hydrogen in these groups is unsubstituted or substituted with $C_{1-6}$ alkyl, and

n is 0 or 1.

In an embodiment, $R^1$ is $C_{1-6}$ alkyl, $C_{3-12}$ cycloalkyl, phenyl, pyridinyl, pyridazinyl, pyrimidinyl, pyrazinyl, pyrrole, furanyl, or thiophenyl, wherein at least one hydrogen atom in these groups may be unsubstituted or each independently substituted with a halogen atom, $C_{1-6}$ alkyl, $C_{1-6}$ alkoxy, nitro, amino optionally substituted with at least one C1-6 alkyl, or hydroxy.

In an embodiment, $R^1$ may be methyl, cyclohexyl, phenyl, 4-methoxyphenyl, 4-(tert-butyl)phenyl, 2-chlorophenyl, 3-chlorophenyl, 4-chlorophenyl, 4-fluorophenyl, 4-bromophenyl, 3,4-dichlorophenyl, 2-chloro-4-fluorophenyl, p-tolyl, m-tolyl, pyridin-4-yl, thiophen-2-yl, furan-2-yl, pyridin-3-yl, pyridin-2-yl, pyrimidin-5-yl, pyrimidin-2-yl, 4-nitrophenyl, 4-aminophenyl, 3-nitrophenyl, 3-aminophenyl, 4-methoxy-pyridin-3-yl, 6-methoxy-pyridin-3-yl, 4-(dimethylamino)phenyl,

or 4-hydroxy-phenyl.

In an embodiment, $R^2$ may be H or $C_{1-6}$ alkyl.

In an embodiment, $R^3$ may be H, carboxyl, methyl carboxyl, ethyl carboxyl, N-hydroxycarboxamide, carboxamide, or sodium carboxylate.

According to another aspect of the present disclosure, there is provided a compound represented by Formula (2-A) or (2-B), a stereoisomer thereof, or a pharmaceutically acceptable salt thereof:

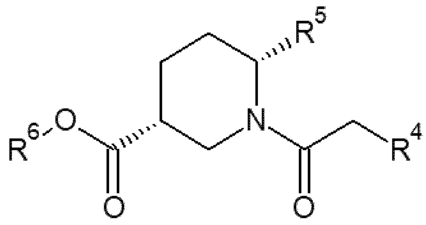
(2-A) or
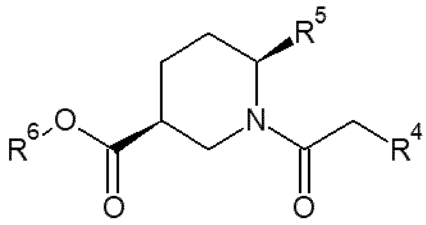
(2-B)

in the formula,

$R^4$ is $C_{1-6}$ alkyl, $C_{3-12}$ cycloalkyl, phenyl, pyridinyl, pyridazinyl, pyrimidinyl, pyrazinyl, pyrrole, furanyl, or thiophenyl, wherein at least one hydrogen atom in these groups is unsubstituted or each independently substituted with a halogen atom, $C_{1-6}$ alkyl, $C_{1-6}$ alkoxy, nitro, amino optionally substituted with at least one $C_{1-6}$ alkyl, or hydroxy, and
$R^5$ and $R^6$ are each independently hydrogen or $C_{1-6}$ alkyl.

According to yet another aspect of the present disclosure, there is provided a compound represented by Formula (4), a stereoisomer thereof, or a pharmaceutically acceptable salt thereof:

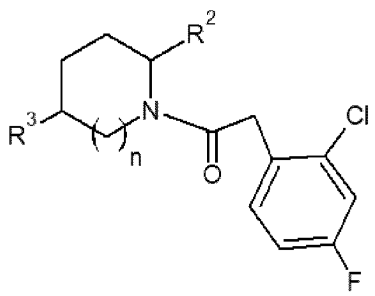
(4)

in the formula,

$R^2$ is hydrogen, $C_{1-6}$ alkyl, or aryl,
$R^3$ is hydrogen, carboxyl, N-hydroxycarboxamide, or carboxamide, wherein at least one hydrogen in these groups is unsubstituted or substituted with $C_{1-6}$ alkyl, and
n is 0 or 1.

According to still yet another aspect of the present disclosure, there is provided a compound represented by Formula (5), a stereoisomer thereof, or a pharmaceutically acceptable salt thereof:

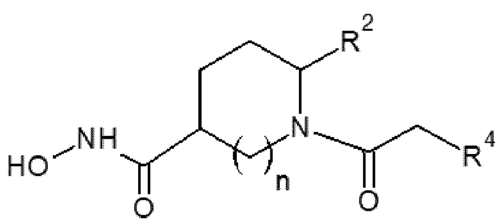
(5)

in the formula,

$R^2$ is hydrogen, $C_{1-6}$ alkyl, or aryl,
$R^4$ is phenyl or phenyl substituted with at least one halogen, and

n is 0 or 1.

In an embodiment, the compound may be a compound selected from the compounds as set forth in Tables 2 to 4, a stereoisomer thereof, or a pharmaceutically acceptable salt thereof.
In an embodiment, the compound may be BnH12001 or an enantiomer thereof.
In an embodiment, the stereoisomer may be an optical isomer.
In an embodiment, the compound may be an inhibitor of HDAC. In another embodiment, the HDAC may be HDAC4.
In an embodiment, the compound may increase synaptic plasticity.
According to still yet another aspect of the present disclosure, there is provided a pharmaceutical composition comprising, as an active ingredient, the compound, a stereoisomer thereof, or a pharmaceutically acceptable salt thereof.
In an embodiment, the composition may be for prevention or treatment of a neurological disorder or a neurodegenerative disorder.
In another embodiment, the compound, a stereoisomer thereof, or a pharmaceutically acceptable salt thereof may be delivered to the brain at a concentration of about 0.1 to about 3 μM.
In another embodiment, the disorder may be Alzheimer's disease.
In another embodiment, the disorder may be PTSD.
In an embodiment, the composition may further comprise a second therapeutic agent.
In another embodiment, the second therapeutic agent may be aducanumab.
According to still yet another aspect of the present disclosure, there is provided a method for preventing or treating a neurological disorder or a neurodegenerative disorder, comprising administering, to a subject in need thereof, the compound, a stereoisomer thereof, or a pharmaceutically acceptable salt thereof.
According to still yet another aspect of the present disclosure, there is provided a use of the compound, a stereoisomer thereof or a pharmaceutically acceptable salt thereof as a medicament or for manufacture of a medicament.
According to an embodiment, the use may be for reactivating or increasing brain synaptic plasticity.
According to another embodiment, the use may be for prevention or treatment of a neurological disorder or a neurodegenerative disorder.

## Advantageous Effects of Invention

The compound according to the present disclosure can exert a therapeutic effect on various neurological disorders or neurodegenerative disorders by effectively enhancing or reactivating synaptic plasticity without any adverse effects.

## Brief Description of Drawings

FIG. 1A illustrates a measurement of cell viability in response to the administration of BnH12001 (0, 0.1, 1, 10, 100 μM) in human cell line SH-SY5Y.

FIG. 1B illustrates a measurement of cell viability in response to the administration of BnH12001 (0, 0.1, 1, 10, 100 μM) in human cell line 293T.

FIG. 2 illustrates a measurement of the expressions of BDNF and GluN2B in 293T cells, after treatment with the compound BnH12001.

FIGS. 3A to 3C illustrate measurements of the expressions of HDAC4, BDNF, and GluN2B in SH-SY5Y cells, after treatment with the compound BnH12001.

FIG. 4A illustrates an osmotic pump (Os pump) injection site in a mouse experiment..

FIG. 4B illustrates a patch recording site in a mouse experiment.

FIG. 4C illustrates a measurement of excitatory/inhibitory balance in the mouse barrel cortex (layer 4) in response to the administration of BnH12001 (Os pump, ventricle injection, 3 μM).

FIG. 4D illustrates changes in thalamocortical (TC) excitatory postsynaptic currents (EPSC) potency in the mouse

barrel cortex (layer 4) in response to the in response to the administration of BnH12001 (Os pump, ventricle injection, 3 μM).

FIG. 5A illustrates a measurement of excitation/inhibition balance in the mouse barrel cortex (layer 4) in response to the oral administration of BnH12001 (1 mg/kg).

FIG. 5B illustrates changes in TC EPSC potency in the mouse barrel cortex (layer 4) in response to the oral administration of BnH12001 (1 mg/kg).

FIG. 5C illustrates changes in TC EPSC potency in the mouse barrel cortex (layer 4) in response to the administration of BnH12001 by each dose (0.03, 0.1, 0.3, 1, 3, 10 mg/kg).

FIG. 6A illustrates the dosing schedule to analyze the efficacy of BnH12001 enantiomer (3 S,6R, 98%<), which is administered alone or in combination with aducanumab in improving ex vivo synaptic plasticity in an Alzheimer's animal model.

FIG. 6B illustrates a measurment of LTP of field excitatory postsynaptic potentials in the hippocampus of an Alzheimer's mouse model in response to BnH12001 enantiomer (3S,6R, 98%<) administered alone or in combination with aducanumab.

FIG. 6C illustrates a measurement of excitation/inhibition balance (right) and changes in TC EPSC potency (left) in the sensory cortex of an Alzheimer's mouse model in response to BnH12001 enantiomer (3S,6R, 98%<) administered alone or in combination with aducanumab.

FIG. 7 illustrates the binding properties of BnH12001 to HDAC4.

FIGS. 8A and 8B illustrate HDAC4 shRNA's knockdown effect on HDAC4, BDNF, and GluN2B expressions in human cell line SH-SY5Y

FIGS. 9A to 9C illustrate the effect of BnH12001 on the expression of BDNF and GluN2B.

FIGS. 10A and 10B illustrate the time course of BnH12001's effects on HDAC4 expression.

FIGS. 11A and 11B illustrate BnH12001's effects on thalamocortical EPSC potency.

FIG. 12A illustrates changes in TC EPSC potency in the mouse barrel cortex (layer 4) in response to the oral administration (1 mg/kg) of BnH12001 enantiomer (3R,6S, 98%<) and BnH12001 enantiomer (3S,6R, 98%<).

FIG. 12B illustrates a measurement of excitation/inhibition balance in the mouse barrel cortex (layer 4) in response to the oral administration (1 mg/kg) of BnH12001 enantiomer (3R,6S, 98%<) and BnH12001 enantiomer (3S,6R, 98%<).

FIG. 13Aillustrates the effect of BnH12001 enantiomer (3 S,6R, 98%<) on the expressions of BDNF and GluN2B in primary cultured cortical neurons.

FIG. 13B illustrates the effect of BnH12001 enantiomer (3 S,6R, 98%<) on the expressions of HDAC4, BDNF, and GluN2B in 8-week-old C57BL/6J mice.

FIG. 13C illustrates the effect of BnH12001 enantiomer (3S,6R, 98%<) on the expression of GluN2B in 13-month-old C57BL/6J mice.

**Modes for Carrying out Invention**

The detailed description of the present disclosure set forth below will be described with reference to specific drawings with respect to specific embodiments in which the present disclosure may be practiced; however, the present disclosure is not limited thereto and, if properly described, is limited only by the appended claims, along with the full scope of equivalents to which such claims are entitled. Unless otherwise indicated, terms used in describing the present disclosure are to be understood in their ordinary meaning and apply to the same terms used herein as well as to the aspect or embodiment of the disclosure for which the term is defined.

Throughout the description, where compositions and kits are described as having, including, or comprising specific components, it is contemplated that, additionally, there are compositions and kits of the present disclosure that consist essentially of, or consist of, the recited components.

In the application, where an element or component is said to be included in and/or selected from a list of recited elements or components, it should be understood that the element or component can be any one of the recited elements or components, or the element or component can be selected from a group consisting of two or more of the recited elements or components.

The order of steps or order for performing certain actions is immaterial so long as the present teachings remain operable. Moreover, two or more steps or actions may be conducted simultaneously.

At various places in the present specification, variable or parameters are disclosed in groups or in ranges. It is specifically intended that the description include each and every individual subcombination of the members of such groups and ranges.

The use of any and all examples, or exemplary language herein, for example, "such as" or "including," is intended merely to illustrate better the present disclosure and does not pose a limitation on the scope of the disclosure unless claimed. No language in the specification should be construed as indicating any non-claimed element as essential to the practice of the present disclosure.

**I. Definition**

The articles "a" and "an" are used in this disclosure to refer to one or more than one (that is, at least one), unless the context is inappropriate.

"And/or" is used in this disclosure to mean either "and" or "or" unless indicated otherwise.

"At least one" includes individually each of the recited objects after the expression and the various combinations of two or more of the recited objects unless otherwise understood from the context and use.

"Comprise," "comprises," "comprising," "Include," "includes," "including," "have," "has," "having," "contain," "contains," or "containing," including grammatical equivalents thereof, should be understood generally as open-ended and non-limiting, for example, not excluding additional unrecited elements or steps, unless otherwise specifically stated or understood from the context.

"About" means approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%.

"Individual," "patient," and "subject" are used interchangeably and include any animal, including mammals, for example, mice, rats, other rodents, rabbits, dogs, cats, swine, cattle, sheep, horses, or primates, including humans.

"Treat", "treating," or "treatment" includes any effect, for example, lessening, reducing, modulating, ameliorating, alleviating or eliminating, that results in the improvement of the condition, disease, disorder, and the like, or ameliorating a symptom thereof. Treating can be curing, improving, or at least partially ameliorating the disorder. In certain embodiments, treating is curing the disease.

"Active agent" is used to indicate a compound or a pharmaceutically acceptable salt thereof which has biological activity. In some embodiments, the "active agent" is a compound or pharmaceutically acceptable salt thereof having pharmaceutical utility. For example, an active agent may be an anti-neurodegenerative therapeutic.

"Compound" means a chemical having a structure represented by chemical formula described herein, a stereoisomer thereof including a mixture of the stereoisomer, or a pharmaceutically acceptable salt thereof,.

"Pharmaceutically acceptable" includes molecular entities and formulations that do not produce an adverse, allergic or other untoward reaction when administered to an animal, or a human, as appropriate.

"Pharmaceutically acceptable salt" refers to those salts which retain the biological effects and properties of the free base or free acid. For example, pharmaceutically acceptable salts may be prepared by the addition of an inorganic base or an organic base to the free acid. Salts derived from inorganic bases include, but are not limited to, sodium, potassium, lithium, ammonium, calcium, magnesium salts, and the like. Salts derived from organic bases include, but are not limited to, primary, secondary, and tertiary amines, substituted amines including naturally occurring substituted amines, cyclic amines and basic ion exchange resins such as isopropylamine, trimethylamine, diethylamine, triethylamine, tripropylamine, ethanolamine, diethylamine, lysine, arginine, N-ethylpiperidine, piperidine, piperazine, and the like.

"Pharmaceutically acceptable excipient" and "pharmaceutically acceptable carrier" refer to a substance that aids the administration of an active agent to and absorption by a subject and can be included in the compositions of the present disclosure without causing a significant adverse toxicological effect on the patient. Non-limiting examples of pharmaceutically acceptable excipients include water, NaCl, normal saline solutions, lactated Ringer's, normal sucrose, normal glucose, binders, fillers, disintegrants, lubricants, coatings, sweeteners, flavors, salt solutions (such as Ringer's solution), alcohols, oils, gelatins, carbohydrates such as lactose, amylose or starch, fatty acid esters, hydroxymethylcellulose,

polyvinyl pyrrolidine, and colors, and the like. Such preparations can be sterilized and, if desired, mixed with auxiliary agents such as lubricants, preservatives, stabilizers, wetting agents, emulsifiers, salts for influencing osmotic pressure, buffers, coloring, and/or aromatic substances and the like that do not deleteriously react with the compounds of the disclosure. One of skill in the art will recognize that other pharmaceutical excipients are useful in the present disclosure.

Exemplary pharmaceutically acceptable carriers or components thereof are sugars, such as lactose, glucose and sucrose; starches, such as corn starch and potato starch; cellulose and its derivatives, such as sodium carboxymethyl cellulose, ethyl cellulose, and methyl cellulose; powdered tragacanth; malt; gelatin; talc; solid lubricants, such as stearic acid and magnesium stearate; calcium sulfate; synthetic oils; vegetable oils, such as peanut oil, cottonseed oil, sesame oil, olive oil, and corn oil; polyols such as propylene glycol, glycerin, sorbitol, mannitol, and polyethylene glycol; alginic acid; phosphate buffer solutions; emulsifiers, such as the TWEENs®; wetting agents, such sodium lauryl sulfate; coloring agents; flavoring agents; tableting agents; stabilizers; antioxidants; preservatives; pyrogen-free water; isotonic saline; and phosphate buffer solutions.

"Pharmaceutical composition" or "pharmaceutical formulation" refers to the combination of an active agent with a carrier, inert or active.

"Effective amount" refers to the amount of a compound or composition (for example, a compound or composition of the present disclosure) sufficient to effect beneficial or desired results. An effective amount may be administered in one or more administrations, applications or dosages and is not intended to be limited to a particular formulation or administration route.

"Administering" means oral administration, administration as a suppository, topical contact, intravenous, parenteral, intraperitoneal, intramuscular, intralesional, intrathecal, intracranial, intracerebroventricular, intranasal or subcutaneous administration, or the implantation of a slow-release device, for example, a mini-Os pump, to a subject. Administration is by any route, including parenteral and transmucosal (for example, buccal, sublingual, palatal, gingival, nasal, vaginal, rectal, or transdermal). Parenteral administration includes, for example, intravenous, intramuscular, intraarterial, intradermal, subcutaneous, intraperitoneal, intraventricular, and intracranial. Other modes of delivery include, but are not limited to, the use of liposomal formulations, intravenous infusion, transdermal patches, and the like. By "co-administer" it is meant that a composition described herein is administered at the same time, just prior to, or just after the administration of one or more additional therapies (for example, anticancer agent, chemotherapeutic, or treatment for a neurodegenerative disease). The compounds of the disclosure can be administered alone or can be co-administered to the patient. Coadministration is meant to include simultaneous or sequential administration of the compounds individually or in combination (more than one compound or agent). Thus, the preparations can also be combined, when desired, with other active substances (for example, to reduce metabolic degradation).

The pharmaceutical formulations of the disclosure may be administered to a mammal, such as a human, but may also be administered to other mammals such as an animal in need of veterinary treatment, for example, domestic animals (for example, dogs, cats, and the like), farm animals (for example, cows, sheep, pigs, horses, and the like), and laboratory animals (for example, rats, mice, guinea pigs, and the like).

"Cognitive function" of a subject may be defined as an intellectual activity or process. Examples of intellectual activities or processes include, but are not limited to, attention, processing speed, learning and memory, executive function, verbal fluency and working memory. For example, the composition of the present disclosure may improve cognitive function if it improves one or more intellectual activities or processes in a subject with impaired cognitive function.

"Histone deacetylase" or "HDAC" are intended to refer to any one of a family of enzymes that remove Nε-acetyl groups from the ε-amino groups of lysine residues of a protein (for example, a histone, or tubulin). Unless otherwise indicated by context, the term "histone" is meant to refer to any histone protein, including H1, H2A, H2B, H3, H4, and H5, from any species.

"A condition or disorder mediated by HDAC" or "a condition or disorder mediated by histone deacetylase" refers to a condition or disorder in which HDAC and/or the action of HDAC is important or necessary, for example, for the onset, progress, or expression.

"Inhibiting HDAC enzymatic activity" or "inhibiting HDAC" means reducing the HDAC's catalytic activity or reducing the HDAC's activities not related to its catalytic activity.

"Neurological disorder" includes neurodegenerative disease, neuropsychiatric disorder, memory loss, cognitive function disorders/impairment, extinction learning disorders.

"Neurodegenerative disease" implies any disorder that might be reversed, deterred, managed, treated, improved, or eliminated with agents that stimulate the generation of new neurons.

"Memory" refers to the ability to recover information about past events or knowledge.

"Age related memory loss" refers to any of a continuum of conditions characterized by a deterioration of neurological functioning that does not rise to the level of a dementia, as further defined herein and/or as defined by the Diagnostic and Statistical Manual of Mental Disorders: 4th Edition of the American Psychiatric Association (DSM-IV, 1994).

"Injury related memory loss" refers to a loss of memory wherein there is damage to the brain, and there may have

also been neurological damage.

"Impaired cognitive function" refers to cognitive function that is not as robust as that observed in an age-matched normal subject and includes states in which cognitive function is reduced.

"Alkyl", as used herein, unless otherwise specified, refers to a saturated straight, branched, or cyclic, primary, secondary, or tertiary hydrocarbon of typically $C_1$ to $C_{10}$, and specifically includes methyl, $CF_3$, $CCl_3$, $CFCl_2$, $CF_2Cl$, ethyl, $CH_2CF_3$, $CF_2CF_3$, propyl, isopropyl, cyclopropyl, butyl, isobutyl, sec-butyl, tert-butyl, pentyl, cyclopentyl, isopentyl, neopentyl, hexyl, isohexyl, cyclohexyl, cyclohexylmethyl, 3-methylpentyl, 2,2-dimethylbutyl, and 2,3-dimethylbutyl. The term includes both substituted and unsubstituted alkyl groups, and particularly includes halogenated alkyl groups, and even more particularly fluorinated alkyl groups. Non-limiting examples of moieties with which the alkyl group can be substituted are selected from the group consisting of halogen (fluoro, chloro, bromo, or iodo), hydroxyl, amino, alkylamino, arylamino, alkoxy, aryloxy, thiol, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991, hereby incorporated by reference.

As used herein, unless otherwise specified, "aryl" refers to an aromatic monocyclic or polycyclic system containing from 6 to 14 carbon atoms, and specifically includes phenyl, biphenyl, or naphthyl, preferably phenyl. The term includes both substituted and unsubstituted moieties. Aryl groups may be substituted with any described moiety, including, but not limited to, one or more moieties selected from the group consisting of halogen (fluoro, chloro, bromo, or iodo), hydroxyl, amino, alkylamino, dialkylamino, arylamino, alkoxy, aryloxy, thiol, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991, hereby incorporated by reference.

As used herein, "heteroaryl", unless otherwise specified, refers to an aromatic monocyclic or polycyclic system having 5 to 14 ring atoms, wherein 1 to 4 of the ring atoms are independently O, N, or S and the remaining ring atoms are carbon atoms, preferably may be an aromatic monocyclic system having 5 or 6 ring atoms, wherein 1 to 4 of the ring atoms are independently O, N or S and the remaining ring atoms are carbon atoms, and specifically includes pyridinyl, pyridazinyl, pyrimidinyl, pyrazinyl, pyrrole, furanyl, thiophenyl, imidazolyl, or oxazolyl. Heteroaryl groups may be substituted with any described moiety, including, but not limited to, one or more moieties selected from the group consisting of halogen (fluoro, chloro, bromo, or iodo), hydroxyl, amino, alkylamino, dialkylamino, arylamino, alkoxy, aryloxy, thiol, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991, hereby incorporated by reference.

## II. Active compounds

In an aspect of the present disclosure, there is provided a compound of Formula (1), a stereoisomer thereof, or a pharmaceutically acceptable salt thereof:

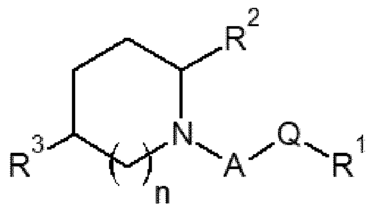

(1)

in the formula,

$R^1$ is $C_{1-6}$ alkyl, $C_{3-12}$ cycloalkyl, aryl, or heteroaryl, wherein at least one hydrogen atom in these groups may be unsubstituted or each independently substituted with a halogen atom, $C_{1-6}$ alkyl, $C_{1-6}$ alkoxy, nitro, amino optionally substituted with at least one $C_{1-6}$ alkyl, or hydroxy,

A is C=O or a bond,

Q is $-(CR^4R^5)_m-$ or a bond, wherein $R^4$ and $R^5$ are each independently hydrogen or $C_{1-6}$ alkyl, and m is an integer of 1 to 6,

$R^2$ is hydrogen, $C_{1-6}$ alkyl, or aryl,

$R^3$ is hydrogen, carboxyl, N-hydroxycarboxamide, or carboxamide, wherein at least one hydrogen in these groups may be unsubstituted or substituted with $C_{1-6}$ alkyl, and

n is 0 or 1.

In an embodiment, the stereoisomer may be an optical isomer. Preferably, the compound may be in the form of (3R,6S)-enantiomer or (3S,6R)-enantiomer.

In an embodiment, $R^1$ may be $C_{1-6}$ alkyl, $C_{3-12}$ cycloalkyl, phenyl, pyridinyl, pyridazinyl, pyrimidinyl, pyrazinyl, pyrrole, furanyl, or thiophenyl. Here, at least one hydrogen atom in the $R^1$ group may be each independently substituted with a halogen atom, $C_{1-6}$ alkyl, $C_{1-6}$ alkoxy, nitro, amino optionally substituted with at least one $C_{1-6}$ alkyl, or hydroxy. Preferably, the at least one hydrogen atom in the $R^1$ group may be each independently substituted with a halogen atom, $C_{1-6}$ alkyl, $C_{1-6}$ alkoxy, or amino optionally substituted with at least one $C_{1-6}$ alkyl.

In an embodiment, $R^1$ may be $C_{1-6}$ alkyl, $C_{3-12}$ cycloalkyl, phenyl, phenyl substituted with at least one halogen, phenyl substituted with at least one $C_{1-6}$ alkyl, phenyl substituted with at least one halogen and at least one $C_{1-6}$ alkyl, phenyl substituted with at least one $C_{1-6}$ alkoxy, phenyl substituted with at least one amino optionally substituted with at least one $C_{1-6}$ alkyl, phenyl substituted with at least one nitro, pyridinyl, pyridinyl substituted with at least one $C_{1-6}$ alkoxy, pyrimidinyl, pyrazinyl, pyrrole, furanyl, or thiophenyl.

In another embodiment, $R^1$ may be methyl, cyclohexyl, phenyl, 4-methoxyphenyl, 4-(tert-butyl)phenyl, 2-chlorophenyl, 3-chlorophenyl, 4-chlorophenyl, 4-fluorophenyl, 4-bromophenyl, 3,4-dichlorophenyl, 2-chloro-4-fluorophenyl, p-tolyl, m-tolyl, pyridin-4-yl, thiophen-2-yl, furan-2-yl, pyridin-3-yl, pyridin-2-yl, pyrimidin-5-yl, pyrimidin-2-yl, 4-nitrophenyl, 4-aminophenyl, 3-nitrophenyl, 3-aminophenyl, 4-methoxy-pyridin-3-yl, 6-methoxy-pyridin-3-yl, 4-(dimethylamino)phenyl, or 4-hydroxy-phenyl.

In an embodiment, $R^2$ may be H or $C_{1-6}$ alkyl.

In an embodiment, $R^3$ may be H, carboxyl, methyl carboxyl, ethyl carboxyl, N-hydroxycarboxamide, carboxamide, or sodium carboxylate.

In an embodiment, A and Q may not be a bond at the same time.

In an embodiment, m may be an integer of 1 to 6, an integer of 1 to 3, or an integer of 1 to 2.

In an embodiment, there may be provided a compound of Formula (2-A) or (2-B):

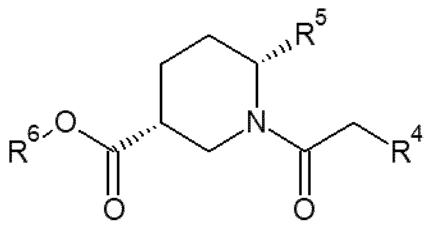 (2-A) or 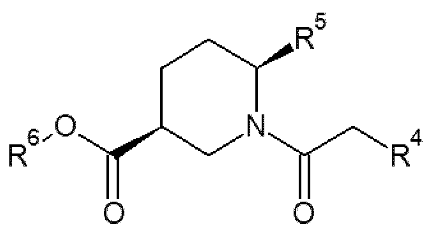 (2-B)

in the formula,

$R^4$ is $C_{1-6}$ alkyl, $C_{3-12}$ cycloalkyl, aryl, or heteroaryl, wherein at least one hydrogen atom in these groups may be unsubstituted or each independently substituted with a halogen atom, $C_{1-6}$ alkyl, $C_{1-6}$ alkoxy, nitro, amino optionally substituted with at least one $C_{1-6}$ alkyl, or hydroxy, and $R^5$ and $R^6$ are each independently hydrogen or $C_{1-6}$ alkyl.

In an embodiment, $R^4$ may be $C_{1-6}$ alkyl, $C_{3-12}$ cycloalkyl, phenyl, pyridinyl, pyridazinyl, pyrimidinyl, pyrazinyl, pyrrole, furanyl, or thiophenyl. Here, at least one hydrogen atom in the $R^4$ group may be each independently substituted with a halogen atom, $C_{1-6}$ alkyl, $C_{1-6}$ alkoxy, nitro, amino optionally substituted with at least one $C_{1-6}$ alkyl, or hydroxy.

In an embodiment, $R^4$ may be $C_{1-6}$ alkyl, $C_{3-12}$ cycloalkyl, phenyl, phenyl substituted with at least one halogen, phenyl substituted with at least one $C_{1-6}$ alkyl, phenyl substituted with at least one halogen and at least one $C_{1-6}$ alkyl, phenyl substituted with at least one $C_{1-6}$ alkoxy, phenyl substituted with at least one amino optionally substituted with at least one $C_{1-6}$ alkyl, phenyl substituted with at least one nitro, pyridinyl, pyridinyl substituted with at least one $C_{1-6}$ alkoxy, pyrimidinyl, pyrazinyl, pyrrole, furanyl, or thiophenyl.

In another embodiment, $R^4$ may be methyl, cyclohexyl, phenyl, 4-methoxyphenyl, 4-(tert-butyl)phenyl, 2-chlorophenyl, 3-chlorophenyl, 4-chlorophenyl, 4-fluorophenyl, 4-bromophenyl, 3,4-dichlorophenyl, 2-chloro-4-fluorophenyl, p-tolyl, m-tolyl, pyridin-4-yl, thiophen-2-yl, furan-2-yl, pyridin-3-yl, pyridin-2-yl, pyrimidin-5-yl, pyrimidin-2-yl, 4-nitrophenyl, 4-aminophenyl, 3-nitrophenyl, 3-aminophenyl, 4-methoxy-pyridin-3-yl, 6-methoxy-pyridin-3-yl, 4-(dimethylamino)phenyl, or 4-hydroxy-phenyl.

In an embodiment, there may be provided a compound of Formula (4):

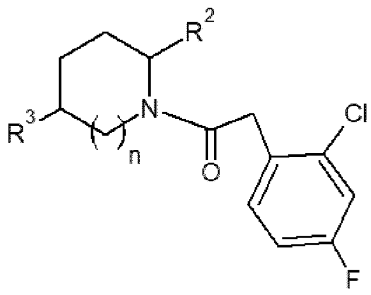

(4)

in the formula,

$R^2$ is hydrogen, $C_{1-6}$ alkyl, or aryl,
$R^3$ is hydrogen, carboxyl, N-hydroxycarboxamide, or carboxamide, wherein at least one hydrogen in these groups may be unsubstituted or substituted with $C_{1-6}$ alkyl, and
n is 0 or 1.

Preferably, $R^2$ may be hydrogen or $C_{1-6}$ alkyl.

In an embodiment, there may be provided a compound of Formula (5):

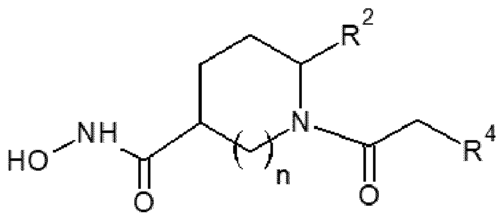

(5)

in the formula,

$R^2$ is hydrogen, $C_{1-6}$ alkyl, or aryl,
$R^4$ is phenyl or phenyl substituted with at least one halogen, preferably 2-chloro-4-fluorophenyl, and
n is 0 or 1.

Preferably, $R^2$ may be hydrogen or $C_{1-6}$ alkyl.

In an embodiment, the compound of Formula (1) may be provided in the form of a pharmaceutically acceptable salt thereof. Preferably, the salt may be a sodium salt.

In an embodiment, the compound of Formula (1) includes, but is not limited to, the compounds set forth in Table 1. BnH12001, as shown in Table 1, is used interchangeably herein with "BnH12001 (racemate)" or "12-004."

[Table 1]

| Name | Structure | Name | Structure |
|---|---|---|---|
| BnH12001 | 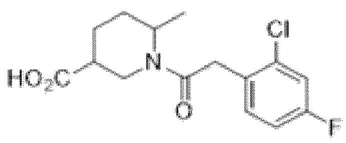 | BnH12001 enantiomer( 3R,6S, 98%<) | 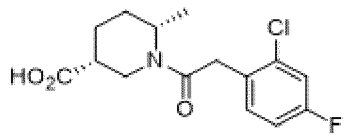 |
| BnH12001 enantiomer (3S,6R, 98%<) | 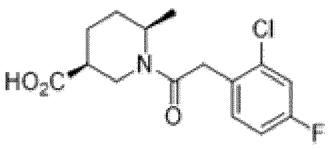 | BnH12004 | 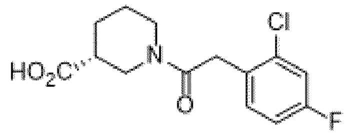 |
| BnH12005 | 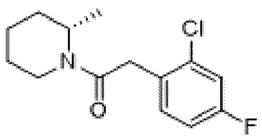 | BnH12006 | 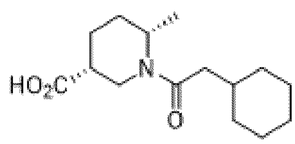 |
| BnH12007 | 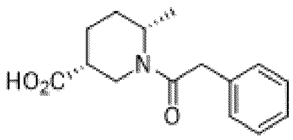 | BnH12008 | 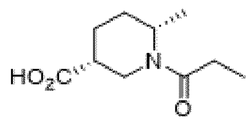 |
| BnH12009 | 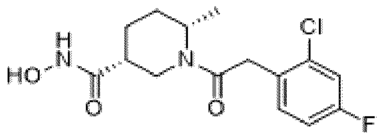 | BnH12010 | 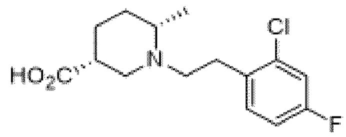 |
| BnH12011 | 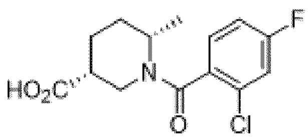 | BnH12012 | 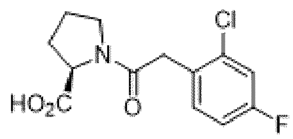 |
| BnH12013 | 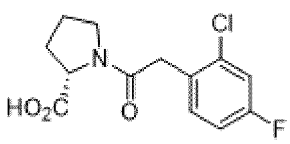 | BnH12014 | 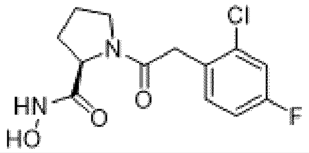 |

| | | | |
|---|---|---|---|
| BnH12016 | 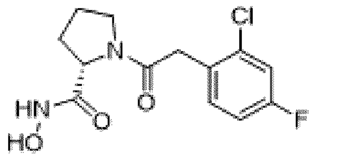 | BnH12018 | 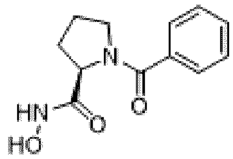 |
| BnH12019 | 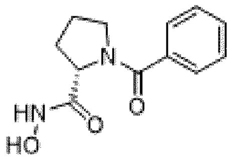 | BnH12020 | 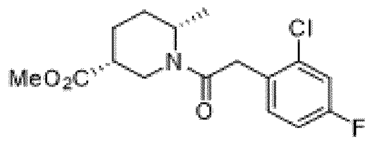 |
| BnH12021 | 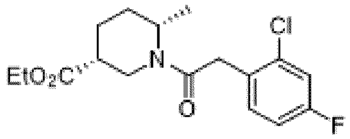 | BnH12022 | 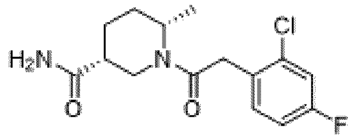 |
| BnH12023 | 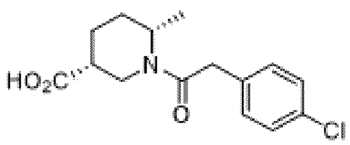 | BnH12024 | 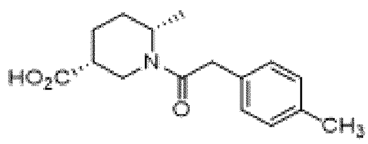 |
| BnH12025 | 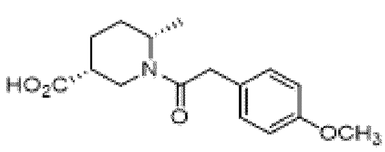 | BnH12026 | 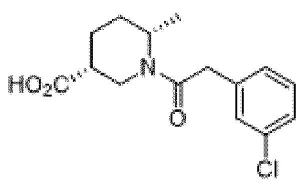 |
| BnH12027 | 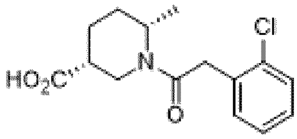 | BnH12028 | 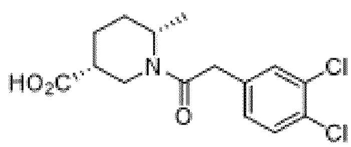 |
| BnH12029 | 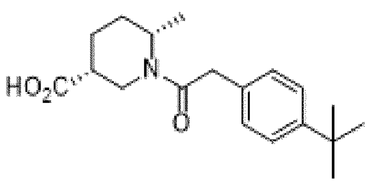 | BnH12030 | 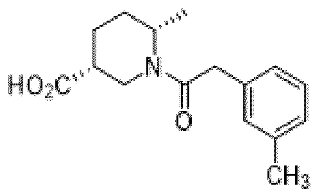 |
| BnH12031 | 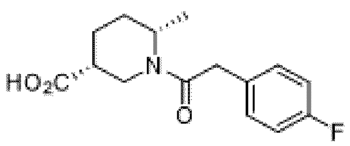 | BnH12032 | 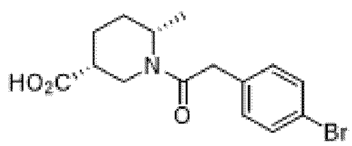 |
| BnH12033 | 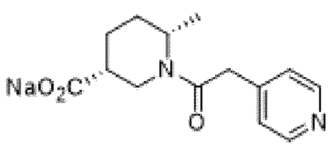 | BnH12034 | 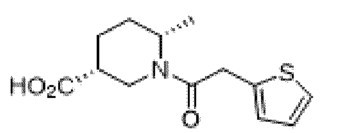 |

| | | | |
|---|---|---|---|
| BnH12035 | 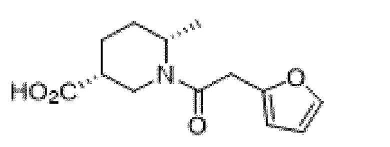 | BnH12038 | 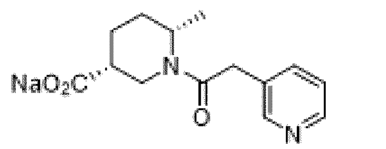 |
| BnH12039 | 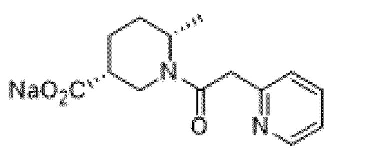 | BnH12040 | 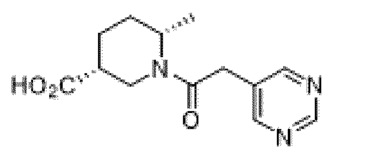 |
| BnH12041 | 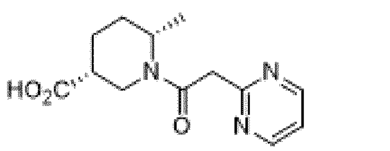 | BnH12060 | 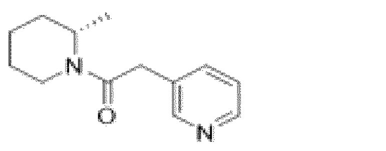 |
| BnH12061 | 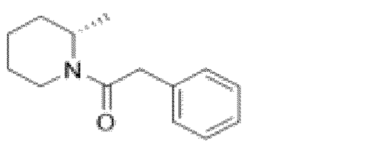 | BnH12062 | 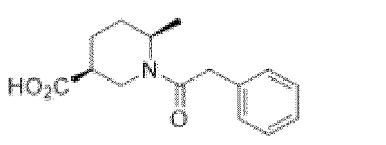 |
| BnH12063 | 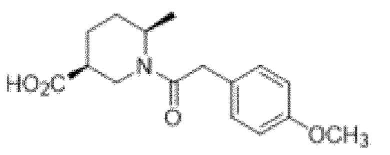 | BnH12065 | 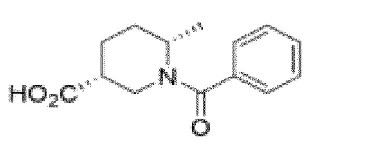 |
| BnH12068 | 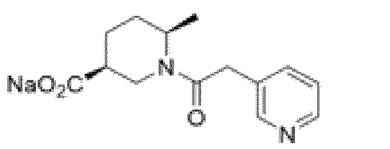 | BnH12069 | 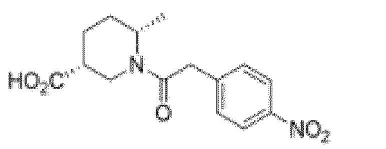 |
| BnH12070 | 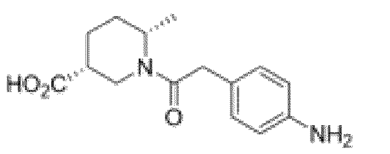 | BnH12073 | 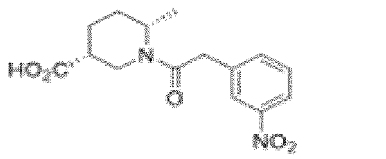 |
| BnH12074 | 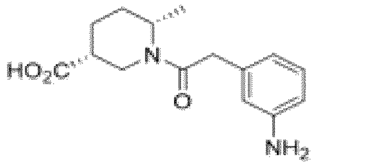 | BnH12075 | 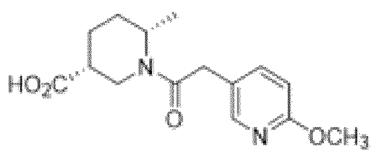 |
| BnH12076 | 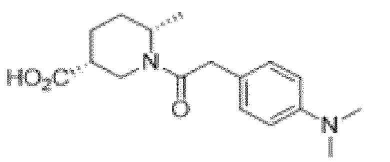 | BnH12079 | 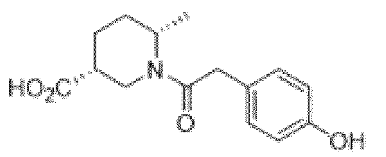 |
| BnH12094 | 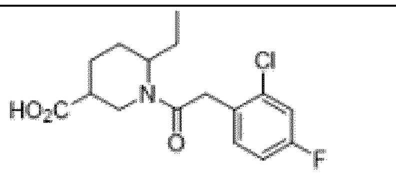 | BnH12096 | 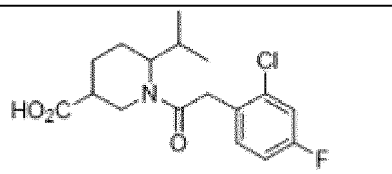 |

In an aspect, the compounds and compositions described herein may be inhibitors of HDAC. Preferably, the HDAC is HDAC4.

In another aspect, the compounds and compositions described herein are synaptic plasticity modulators. Preferably, the synaptic plasticity is synaptic plasticity in the barrel cortex (for example, layer 4) of the brain.

### III. Pharmaceutical composition

In general, the compounds described herein may be formulated into pharmaceutical compositions using techniques well known to those skilled in the art. The concentration of active compound in the pharmaceutical composition will vary depending on absorption, inactivation and excretion rates of the drug as well as other factors known to those of skill in the art. It is to be noted that dosage values will also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed composition. The active ingredient may be administered at once or may be divided into a number of smaller doses to be administered at varying intervals of time.

The concentration of inhibitor which reduces the activity of an HDAC to 50% of that of the uninhibited HDAC is determined as the $IC_{50}$ value. In some embodiments, such reduction of HDAC is at least 50%, such as at least about 75%, for example, at least about 90%. In some embodiments, HDAC activity is reduced by at least 95%, such as by at least 99%. In some embodiments, the compounds described herein have an $IC_{50}$ value less than 100 nanomolar. In some embodiments, the compounds described herein have an $IC_{50}$ value from 100 nanomolar to 1 micromolar. In some embodiments, the compounds described herein have an $IC_{50}$ value from 1 to 25 micromolar.

In some embodiments, such inhibition is specific, that is, the histone deacetylase inhibitor reduces the ability of a histone deacetylase to remove an acetyl group from a protein at a concentration that is lower than the concentration of the inhibitor that is required to produce another, unrelated biological effect. In some embodiments, the concentration of the inhibitor required for histone deacetylase inhibitory activity is at least 2-fold lower, such as at least 5-fold lower, for example, at least 10-fold lower, such as at least 20-fold lower than the concentration required to produce an unrelated biological effect.

In some embodiments, the pharmaceutical composition comprises about 25 mg, about 50 mg, about 75 mg, about 100 mg, about 150 mg, about 200 mg, about 250 mg, about 300 mg, about 350 mg, about 400 mg, about 450 mg, about 500 mg, about 600 mg, about 700 mg, about 800 mg, about 900 mg, about 1000 mg, about 1250 mg, about 1500 mg, about 1750 mg, about 2000 mg, about 2250 mg, about 2500 mg, about 2750 mg, or about 3000 mg of the compound.

In some embodiments, the pharmaceutical composition comprises about 0.01 mg/kg to about 125 mg/kg and preferably from about 1 mg/kg to about 50 mg/kg of subject body weight per day, one or more times a day, to obtain the desired therapeutic effect.

In certain embodiments, the composition comprises, for example, 0.0001 to 99.9 wt %, preferably 0.001 to 50 wt % of the compound based on the total composition weight. The content ratio is a value based on the dry amount from which the solvent is removed.

In certain embodiments, the composition comprises a 5% (w/v), 10% (w/v), 15% (w/v), 20% (w/v), 25% (w/v), 30% (w/v), 35% (w/v) or 40% (w/v) aqueous solution of one or more compounds. In some embodiments, the effective amount of the composition comprises a 25% (w/v) solution of one or more compounds.

In certain embodiments, the pharmaceutical compositions of the present disclosure further comprise one or more pharmaceutically acceptable excipients. In some embodiments, the one or more pharmaceutically acceptable excipients are selected from the group comprising a diluent, a buffering agent, a preservative, a stabilizer, a solubilizing agent or any combination thereof.

In certain embodiments, the pharmaceutical composition may be formulated for administration as a liquid dosage form suitable for intracavitary, intradermal, intramuscular, intrathecal, intravenous, subcutaneous, or intracerebroventricular administration.

In certain embodiments, a liquid dosage form of a pharmaceutical composition as described herein further comprises a diluent. In some embodiments, the insert diluent is a saline solution.

In certain embodiments, a liquid dosage form of a pharmaceutical composition as described herein further comprises a buffering agent. For example, suitable buffering agents for use with the present disclosure include, but are not limited to, both organic and inorganic acids and salts thereof, such as citrate buffers (for example, monosodium citrate-disodium citrate mixture, citric acid-trisodium citrate mixture, citric acid-monosodium citrate mixture), succinate buffers (for example, succinic acid-monosodium succinate mixture, succinic acid-sodium hydroxide mixture, succinic acid-disodium succinate mixture), tartrate buffers (for example, tartaric acid-sodium tartrate mixture, tartaric acid-potassium tartrate mixture, tartaric acid-sodium hydroxide mixture), fumarate buffers (for example, fumaric acid-monosodium fumarate mixture, fumaric acid-disodium fumarate mixture, monosodium fumarate-disodium fumarate mixture), gluconate buffers (for example, gluconic acid-sodium gluconate mixture, gluconic acid-sodium hydroxide mixture, gluconic acid-potassium gluconate mixture), oxalate buffer (for example, oxalic acid-sodium oxalate mixture, oxalic acid-sodium

hydroxide mixture, oxalic acid-potassium oxalate mixture, and the like), lactate buffers (for example, lactic acid-sodium lactate mixture, lactic acid-sodium hydroxide mixture, lactic acid-potassium lactate mixture, and the like), and acetate buffers (for example, acetic acid-sodium acetate mixture, acetic acid-sodium hydroxide mixture, and the like). Additionally, phosphate buffers, histidine buffers and trimethylamine salts such as Tris may be used.

In certain embodiments, a liquid dosage form of a pharmaceutical composition as described herein further comprises a preservative. For example, suitable preservatives for use with the present disclosure include, but are not limited to phenol, benzyl alcohol, meta-cresol, methyl paraben, propyl paraben, octadecyldimethylbenzyl ammonium chloride, benzalconium halides (for example, chloride, bromide, and iodide), hexamethonium chloride, and alkyl parabens (for example, methyl or propyl paraben, catechol, resorcinol, cyclohexanol, and 3-pentanol).

In certain embodiments, a liquid dosage form of a pharmaceutical composition as described herein further comprises a stabilizer. For example, suitable stabilizers include, but are not limited to, polyhydric sugar alcohols, trihydric or higher sugar alcohols, amino acids, organic sugars or sugar alcohols, polyvinylpyrrolidone monosaccharides, trisaccacharides, polysaccharides, proteins, sulfur containing reducing agents, amino acid polymers, and polyethylene glycol.

In certain embodiments, a liquid dosage form of a pharmaceutical composition as described herein further comprises a solubilizing agent. In some embodiments, the solubilizing agent is an ionic surfactant. Examples of non-ionic surfactants include, but are not limited to, polysorbates, polyoxamers, pluronic polyols, and polyoxyethylene sorbitan monoethers.

In certain embodiments, pharmaceutical compositions disclosed herein may be prepared for storage as lyophilized formulations or aqueous solutions by mixing the composition with optional pharmaceutically acceptable carriers, excipients or stabilizers typically employed in the art (for example, buffering agents, stabilizing agents, preservatives, isotonifiers, non-ionic detergents, antioxidants, and other miscellaneous additives).

In a case where the compound is administered orally, it may be formulated into pills, capsules, cachets, tablets or the like, together with pharmaceutically acceptable excipients.

Compressed tablets may be prepared by compressing in a suitable machine the active ingredient in a free-flowing form such as a powder or granules, optionally mixed with a binder, lubricant, inert diluent, preservative, surface active or dispersing agent. Molded tablets may be made by molding in a suitable machine a mixture of the powdered active ingredient moistened with an inert liquid diluent. The tablets may optionally be coated or scored and optionally are formulated so as to provide slow or controlled release of the active ingredient therefrom. Tablets, troches, lozenges, aqueous or oil suspensions, dispersible powders or granules, emulsions, hard or soft capsules, for example, gelatin capsules, syrups or elixirs may be prepared for oral use. Formulations of the compounds of the present disclosure intended for oral use may be prepared according to any method known to the art for the manufacture of pharmaceutical compositions and such compositions may contain one or more agents including sweetening agents, flavoring agents, coloring agents and preserving agents, in order to provide a palatable preparation. Tablets containing the active ingredient are in admixture with nontoxic pharmaceutically acceptable excipient which are suitable for manufacture of tablets. These excipients may be, for example, inert diluents, such as calcium or sodium carbonate, lactose, calcium or sodium phosphate; granulating and disintegrating agents, such as maize starch, or alginic acid; binding agents, such as starch, gelatin or acacia; and lubricating agents, such as magnesium stearate, stearic acid, or talc. Tablets may be uncoated or may be coated by known techniques including microencapsulation to delay disintegration and adsorption in the gastrointestinal tract and thereby provide a sustained action over a longer period. For example, a time delay material such as glyceryl monostearate or glyceryl distearate alone or with a wax may be employed.

In certain embodiments, the pharmaceutical compositions of the present disclosure are administered to the subject by intracavitary, intradermal, intramuscular, intrathecal, intravenous, subcutaneous, or intracerebroventricular administration.

## IV. Biological assay

Described herein are methods of selecting compounds useful for the present disclosure.

In an aspect, compounds for the present disclosure are selected for their ability to interact with an HDAC. In an embodiment, the HDAC is HDAC4. In another embodiment, binding of the compounds to the HDAC is measured by surface plasmon resonance assay. In an embodiment, the compound is BnH12001 or an enantiomer thereof.

In an aspect, compounds are selected for their ability to increase the expression of BDNF and/or GluN2B in the targeted neurons. In an aspect, compounds are selected for their ability to increase the expression of BDNF in the targeted neurons. In another embodiment, compounds are selected for their ability to increase the expression of GluN2B in the targeted neurons. In an embodiment, RT-PCR is used to measure the level of expressions. In an embodiment, the compound is selected from the group consisting of BnH12001 or an enantiomer thereof, BnH12005, BnH12006, BnH12007, BnH12009, BnH12010, BnH12016, BnH12018, BnH12025, BnH12038, BnH12039, BnH12060, BnH12061, BnH12063, BnH12065, BnH12068, BnH12069, BnH12070, BnH12073, BnH12074, BnH12079, and

BnH12096. Preferably, the compound is BnH12001 or an enantiomer thereof.

In an aspect, compounds for the present disclosure are selected for their non-toxicity toward human cells. In an embodiment, compounds of the present disclosure are selected for not significantly affecting the cell viability of human cell line SH-SY5Y In another embodiment, compounds for the present disclosure are selected for not significantly affecting the cell viability of human cell line 293T. In an embodiment, the compounds do not lower cell viabilities of SH-SY5Y or 293 at a concentration of about 0.1, 1, 10, 100 μM. In an embodiment, the compound may be one selected from the compounds described in Table 5. Preferably, the compound is BnH12001 or an enantiomer thereof.

In an aspect, compounds for the present disclosure are selected for their ability to increase the potency, when measured via TC EPSC potency in the rodent barrel cortex (layer 4). In an embodiment, the compounds increase the measured potency at a concentration of about 3 μM, when delivered by Os pump through ventricle injection. In another embodiment, the concentration may be about 0.1 to about 3 μM. In another embodiment, the concentration may be about 3 μM. In another embodiment, the concentration may be about 0.03, 0.1, 0.3, 1, 3, or 10 mg/kg. In an embodiment, the compound is BnH12001 or an enantiomer thereof.

Described herein are detailed examples of methods useful for selecting the compounds of the present disclosure.

## V. Therapeutic uses of compounds

The compounds of the present disclosure may be useful for inhibiting at least one type of HDAC. In an aspect, the compounds described herein may be therapeutically useful, as certain HDACs are known to be involved in biological pathways governing higher-order brain functions. The compounds described herein may therefore be useful when brain pathologies are associated with or mediated by HDACs.

Recent reports have detailed the importance of histone acetylation in central nervous system (CNS) functions such as neuronal differentiation, memory formation, drug addiction, and depression (Citrome, Psychopharmacol. Bull. 2003, 37, Suppl. 2, 74-88; Johannessen, CNS Drug Rev. 2003, 9, 199-216; Tsankova et al, 2006, Nat. Neurosci. 9, 519-525).

A neurological disorder is a condition having as a component a central or peripheral nervous system malfunction. Neurological disorders may cause a disturbance in the structure or function of the nervous system resulting from developmental abnormalities, disease, genetic defects, injury or toxin. These disorders may affect the central nervous system (for example, the brain, brainstem and cerebellum), the peripheral nervous system (for example, the cranial nerves, spinal nerves, and sympathetic and parasympathetic nervous systems) and/or the autonomic nervous system (for example, the part of the nervous system that regulates involuntary action and that is divided into the sympathetic and parasympathetic nervous systems).

Accordingly, provided herein is a method of treating or preventing a neurological disorder or neurodegenerative disease mediated by HDAC in a subject in need of such a treatment or prevention, comprising administering to the subject a therapeutically effective amount of at least one compound described herein. In some embodiments, the compounds described herein may be useful for modulating the activities of Class IIa HDACs. In some embodiments, the compound is an inhibitor of HDAC4.

Most neurodegenerative disorders are caused by the degeneration of the neurons in the brain. Neurons containing HDAC4 are widely distributed in the brain (Mielcarek et al., 2013, PLoS Biol. 11:e1001717; Takase et al., 2013, PloS ONE 8:e58473). Treatments as the present disclosure that modulate the activities of HDAC4 in the brain could, therefore, be useful for any neurodegenerative disease of the brain since the mechanism of intervention contemplated by the present disclosure can target HDAC4 in the neuron regardless of the observed pathology of the brain. There can be no neurodegenerative disease that the compounds of the present disclosure would have no utility. The compounds of the present disclosure are not bound by the current understanding of the molecular mechanisms involving HDAC4 in the neurons of the brain. As such, the compounds of the present disclosure may be useful for any chronic or acute neurodegenerative disorders, so long as the neurons affected by the pathological manifestation of the disease express HDAC4.

Examples of neurodegenerative disorders include: chronic neurodegenerative diseases such as familial and sporadic amyotrophic lateral sclerosis (FALS and ALS, respectively), familial and sporadic Parkinson's disease, Huntington's disease, familial and sporadic Alzheimer's disease, multiple sclerosis, muscular dystrophy, olivopontocerebellar atrophy, multiple system atrophy, Wilson's disease, progressive supranuclear palsy, diffuse Lewy body disease, corticodentatonigral degeneration, progressive familial myoclonic epilepsy, striatonigral degeneration, torsion dystonia, familial tremor, Down's Syndrome, Gilles de la Tourette syndrome, Hallervorden-Spatz disease, diabetic peripheral neuropathy, dementia pugilistica, AIDS dementia, age related dementia, age associated memory impairment, and amyloidosis-related neurodegenerative diseases such as those caused by the prion protein (PrP) which is associated with transmissible spongiform encephalopathy (Creutzfeldt-Jakob disease, Gerstmann-Straussler-Scheinker syndrome, scrapie, and kuru), and those caused by excess cystatin C accumulation (hereditary cystatin C angiopathy). Examples of acute neurodegenerative disorders include: traumatic brain injury (for example, surgery- related brain injury), cerebral

edema, peripheral nerve damage, spinal cord injury, Leigh's disease, Guillain-Barre syndrome, lysosomal storage disorders such as lipofuscinosis, Alper's disease, restless leg syndrome, vertigo as result of CNS degeneration; pathologies arising with chronic alcohol or drug abuse including, for example, the degeneration of neurons in locus coeruleus and cerebellum, drug-induced movement disorders; pathologies arising with aging including degeneration of cerebellar neurons and cortical neurons leading to cognitive and motor impairments; and pathologies arising with chronic amphetamine abuse to including degeneration of basal ganglia neurons leading to motor impairments; pathological changes resulting from focal trauma such as stroke, focal ischemia, vascular insufficiency, hypoxic-ischemic encephalopathy, hyperglycemia, hypoglycemia or direct trauma; pathologies arising as a negative side-effect of therapeutic drugs and treatments (for example, degeneration of cingulate and entorhinal cortex neurons in response to anticonvulsant doses of antagonists of the NMD A class of glutamate receptor) and Wernicke-Korsakoffs related dementia.

In addition to acute or chronic degenerative disorders, the compounds of the present disclosure may be useful for treating other types of diseases of the brain, so long as the affected neurons are regulated in part by HDAC4. Examples of these conditions include: neuropsychiatric disorders related to cognition, dementia, social behavior, anxiety, or other personality disorders, for example, schizophrenia, delirium, attention deficit disorder (ADD), bipolar disorders, obsessive-compulsive disorders, or eating disorders; fear and anxiety disorders, for example, panic disorder, various phobias, post-traumatic stress disorder (PTSD), psychogenic erectile dysfunction, or insomnia; various forms of addictions; and mood disorders such as depression.

The types of diseases that could benefit from the use of the compounds described herein are not limited to the diseases of the brain. Any neurological diseases in which the affected neurons are regulated by HDAC4 could potentially benefit from the compounds of the present disclosure. As described herein, Alzheimer's disease and PTSD, though vastly different in their pathology and the known causes of the diseases, benefited from the treatment of compounds described herein. In the case of Alzheimer's disease, BnH12 family compounds reduced the symptoms of the disease, and improved the retention of memory. Examples of neurological diseases that can benefit from the compounds of the present disclosure include: diseases of the peripheral nervous system due to degeneration or injury, for example, ataxia or trauma; and an acute or chronic degenerative disease of the eye, for example, glaucoma.

Memory loss or reduced cognitive function can also benefit from a treatment utilizing compounds of the present disclosure, so long as the affected neuron's plasticity is regulated in part by HDAC4. Examples of these disorders include: conditions related to cognitive functions such as memory loss/impairment, acts of creativity, problem-solving, and possibly intuition.

Memories include short-term memory (also referred to as working or recent memory) and long-term memory. Short-term memories involve recent events, while long-term memories relate to the recall of events of the more distant past. Methods of assessing the ability to recall a memory are known to those of skill in the art and may involve routine cognitive tests.

Age related memory loss is characterized by objective loss of memory in an older subject compared to his or her younger years, but cognitive test performance that is within normal limits for the subject's age. Age related memory loss subjects score within a normal range on standardized diagnostic tests for dementias, as set forth by the DSM-IV. Moreover, the DSM-IV provides separate diagnostic criteria for a condition termed Age-Related Cognitive Decline. Age-related memory loss may include decreased brain weight, gyral atrophy, ventricular dilation, and selective loss of neurons within different brain regions. For purposes of some embodiments of the present disclosure, more progressive forms of memory loss are also included under the definition of age-related memory disorder. Thus, persons having greater than age-normal memory loss and cognitive impairment, yet scoring below the diagnostic threshold for frank dementia, may be referred to as having a mild neurocognitive disorder, mild cognitive impairment, late-life forgetfulness, benign senescent forgetfulness, incipient dementia, provisional dementia, and the like. Such subjects may be slightly more susceptible to developing frank dementia in later life (see also US patent application 2006/008517).

Symptoms associated with age-related memory loss include but are not limited to alterations in biochemical markers associated with the aging brain, such as IL-1 beta, IFN-gamma, p-JNK, p-ERK, reduction in synaptic activity or function, such as synaptic plasticity, evidenced by reduction in long term potentiation, diminution of memory and learning. In some embodiments, the compounds of the present disclosure modulate the activities of IL-1 beta, IFN-gamma, p-JNK, or p-ERK.

Methods for enhancing memories may include reestablishing access to memories as well as recapturing memories. The term reestablishing access as used herein refers to increasing retrieval of a memory. While not bound by a mechanism of action, it is believed that the compounds of the disclosure are effective in increasing retrieval of memories by re-establishing a synaptic network. The process of reestablishing a synaptic network may include an increase in the number of active brain synapses and or a reversal of neuronal loss.

In some instances, learning is referred to as memory. Learning, unlike memory enhancement, refers to the ability to create new memories that had not previously existed. In some embodiments, in order to test the ability of a compound to influence the ability of a subject to learn rather than recall old memories, at least one compound of the present disclosure is administered prior to or at the same time as the memory is created. In some embodiments, to test the ability of a compound

to affect recall of a previously created memory, at least one of the compounds described herein is administered after the memory is created and preferably after the memory is lost.

Also provided is a method of enhancing learning and memory formation in a subject suffering from memory loss comprising administering to the subject an effective amount of at least one compound described herein. The method may also be equally useful for enhancing learning and memory formation in a normal subject.

Neurogenesis, or the birth of new neuronal cells, was thought to occur only in developing organisms. However, recent research has demonstrated that neurogenesis continues into and throughout adult life. On-going neurogenesis is thought to be an important mechanism underlying neuronal plasticity, enabling organisms to adapt to environmental changes and influencing learning and memory throughout life. In an aspect, the disclosure includes a method of increasing synaptic density in a subject comprising administering to the subject in need of such increase a compound of the disclosure or a pharmaceutically acceptable salt, hydrate, solvate, or prodrug thereof. In an aspect, the disclosure includes a method of increasing synaptic plasticity in a subject comprising administering to the subject in need of such increase a compound of the disclosure or a pharmaceutically acceptable salt, hydrate, solvate, or prodrug thereof. In an aspect, the disclosure includes a method of increasing dendritic density in neurons in a subject comprising administering to the subject in need of such increase a compound of the disclosure or a pharmaceutically acceptable salt, hydrate, solvate, or prodrug thereof.

The present disclosure provides methods for enhancing memory in a subject having a memory disorder. Examples of types of memory disorders include Alzheimer's disease, absent-minded professor, absent-mindedness, amnesia, anterograde amnesia, blackout (alcohol-related amnesia), bromism, childhood amnesia, false memory syndrome, fugue state, hyperthymesia, Korsakoff s syndrome, lacunar amnesia, memory distrust syndrome, memory loss, post-traumatic amnesia, prosopamnesia, psychogenic amnesia, repressed memory, retrograde amnesia, Ribot' s Law, selective memory loss, source amnesia, sourcemonitoring error, the seven sins of memory, tip of the tongue, transient epileptic amnesia, transient global amnesia, and twilight sleep.

In an embodiment, the memory disorder is Alzheimer's disease. Such methods optionally involve administering the inhibitor and monitoring the subject to identify recapture of a memory that was previously lost. Subjects may be monitored by routine tests known in the art.

In other embodiments the Alzheimer's subject is one that has late-stage Alzheimer's disease. Many of the compounds suggested for treating Alzheimer's disease are designed to treat the early stages of the disease by preventing plaque buildup. The compounds of the disclosure are useful for treating both early stages and late stages of dementia because they improve memory and cognition rather than preventing only plaque accumulation.

In an embodiment, a condition or disorder mediated by HDAC is cognitive function disorders/impairments. Accordingly, also provided is a method of promoting cognitive function in a subject suffering from cognitive function disorders/impairments comprising administering to the subject an effective amount of at least one compound described herein. The present disclosure also provides a method of promoting cognitive function in a normal subject.

In some cases, cognitive function is reduced by about 5%, about 10%, about 30%, or more, compared to cognitive function measured in an age-matched normal subject. Cognitive function may be promoted to any detectable degree, but in humans preferably is promoted sufficiently to allow an impaired subject to carry out daily activities of normal life.

Cognitive function may be assessed, and thus optionally defined, via one or more tests or assays for cognitive function. Non-limiting examples of a test or assay for cognitive function include CANTAB (see for example Fray et al. "CANTAB battery: proposed utility in neurotoxicology. "Neurotoxicol Teratol 1996; 18(4):499-504), Stroop Test, Trail Making, Wechsler Digit Span, or the CogState computerized cognitive test (see also Dehaene et al. "Reward-dependent learning in neuronal networks for planning and decision making. " Brain Res. 2000; 126:21729; Iverson et al. "Interpreting change on the WAIS-III/ WMS-I11 in clinical samples." Arch Clin Neuropsychol. 2001; 16(2):183- 91; and Weaver et al. "Mild memory impairment in healthy older adults is distinct from normal aging." Cogn. 2006;60(2): 146-55). The methods of the disclosure may be used to promote cognitive function in a normal subject or to treat, alleviate and/or prevent a subject from having a cognitive dysfunction. A normal subject, as used herein, is a subject that has not been diagnosed with a disorder associated with impaired cognitive function.

In some embodiments, the cognitive function disorders or impairments are associated with, but not limited to, Alzheimer's disease, Huntington's disease, seizure induced memory loss, schizophrenia, Rubinstein Taybi syndrome, Rett Syndrome, Fragile X, Lewy body dementia, vascular dementia, bipolar disorder and social, cognitive and learning disorders associated with autism spectrum disorders (ASD), attention deficit hyperactivity disorder (ADHD), dyslexia, learning disorders, traumatic head injury, stroke induced cognitive and motor impairment, traumatic brain injury, neurodegeneration and neuronal loss mediated cognitive impairment, and attention deficit disorder.

In some embodiments, the cognitive function disorders or impairments are associated with, but not limited to, anxiety disorders, conditioned fear response, panic disorders, obsessive compulsive disorders, post-traumatic stress disorder, phobias, social anxiety disorders, substance dependence recovery or Age Associated Memory Impairment (AAMI), and Age Related Cognitive Decline (ARCD).

It has been demonstrated that administration of the HDAC inhibitors sodium butyrate or Trichostatin A facilitates

fear extinction in mice and this enhancement mirrors that caused by commonly used behavioral manipulation and is consistent with other studies demonstrating a role for the hippocampus in the extinction of contextual fear (Lattal, et al., 2007, Behav. Neurosci. 121, 5, 1125-1131). In an embodiment, a condition or disorder mediated by HDAC is extinction learning disorders, for example, a fear extinction deficit. Accordingly, also provided is a method of treating or preventing extinction learning disorders in a subject suffering from extinction learning disorders comprising administering to the subject an effective amount of at least one compound described herein.

The compounds of the disclosure may be used to facilitate the psychological process of extinction learning and thus are useful for treating, alleviating, and/or preventing neuropsychiatric disorders and other related disorders. Unlike traditional anti-anxiety drugs that are administered on a chronic basis and address physiological symptoms of anxiety, the compounds of the disclosure may be used on a chronic or acute basis in conjunction with a second therapy, for example, psychotherapy.

Although not thoroughly explored in the present disclosure, HDAC4 mediates gene expression in other cell types, including the immune cells. The compounds of the present disclosure are not bound by the current understanding of the molecular mechanisms involving HDAC4 in immune cells, or in other types of cells found in cancer. As such, the compounds of the present disclosure may be useful for any diseases including: hyperproliferative diseases, for example, malignant tumors of various origins; diseases of the immune system, for example, autoimmune disease, inflammation, or allergies; infectious disease; graft rejection; and fibrotic disease.

In some embodiments, the condition or disorder mediated by HDAC is an immune disorder that may be co-treated with TNF$\alpha$ or other immune modulators, upon administering to the subject a therapeutically effective amount of at least one compound as described herein. Accordingly, also provided is a method of treating an immune disorder that is mediated by HDAC in a subject in need of such a treatment, comprising administering to the subject a therapeutically effective amount of at least one compound described herein, either before, during, or after a treatment with TNF$\alpha$ or other immune modulators.

In some embodiments, the condition or disorder mediated by HDAC comprises a disorder that may also be treated by progenitor/stem cell based therapies such as: disorders related to diabetes (organ failure, cirrhosis, and hepatitis); central nervous system (CNS) disorders associated with dysregulation of progenitor cells in the brain (for example, PTSD); tumors (for example, retinoblastomas); disorders affecting oligodendrocyte progenitor cells (for example, astrocytomas and ependymal cell tumors); multiple sclerosis; demyelinating disorders such as leukodystrophies; neuropathies associated with white matter loss; cerebellar disorders such as ataxia; and olfactory progenitor disorders (for example, anosmic conditions). Accordingly, also provided is a method of treating a disorder that is mediated by HDAC in a subject in need of such a treatment, comprising administering to the subject a therapeutically effective amount of at least one compound described herein, either before, during, or after a treatment with progenitor/stem cell based therapies.

In some embodiments, the condition or disorder mediated by HDAC comprises a blood pressure disorder related to nitric oxide (NO) regulation (for example, hypertension, erectile dysfunction, asthma; and ocular disorders as glaucoma). Accordingly, also provided is a method of treating a blood pressure disorder related to nitric oxide (NO) regulation that is mediated by HDAC in a subject in need of such a treatment, comprising administering to the subject a therapeutically effective amount of at least one compound described herein.

In some embodiments, the condition or disorder is a cardiac hypertrophic disorder. Accordingly, also provided is a method of treating a cardiac hypertrophic disorder that is mediated by HDAC in a subject in need of such a treatment, comprising administering to the subject a therapeutically effective amount of at least one compound described herein.

In some embodiments, the condition or disorder mediated by HDAC comprises a hematological disorder such as thalassemia, anemia, and sickle cell anemia. Accordingly, also provided is a method of treating a hematological disorder mediated by HDAC in a subject in need of such a treatment, comprising administering to the subject a therapeutically effective amount of at least one compound described herein.

In some embodiments, the condition or disorder mediated by HDAC comprises a metabolic disease such as prediabetes or diabetes (type I or II). Accordingly, also provided is a method of treating a metabolic disease, such as prediabetes or diabetes (type I or II), mediated by HDAC in a subject in need of such a treatment, comprising administering to the subject a therapeutically effective amount of at least one compound described herein.

In an aspect, the present disclosure provides pharmaceutical compositions further comprising a second therapeutic agent for the treatment of condition or disorder mediated by HDAC in a subject suffering from the condition or disorder mediated by HDAC. In some embodiments, the second therapeutic agent is indicated to treat condition or disorder mediated by HDAC, preferably HDAC4.

Also provided are methods of treatment of condition or disorder mediated by HDAC in a subject suffering from the condition or disorder mediated by HDAC in which at least one compound, or pharmaceutically acceptable salt thereof described herein is given to the subject in combination with one or more additional active agents.

In some embodiments, the second therapeutic agent is selected from the group consisting of donepezil, rivastigmine, galantamine, memantine, verubecestat, solanezumab, bapineuzumab, aducanumab, lecanemab, tideglusib, epothilone D, and ABBV-8E12.

In some embodiments, the second therapeutic agent is selected from the group consisting of a cholinesterase inhibitor, an NMDA receptor antagonist, a humanized antibody which targets tau protein, a humanized antibody which targets amyloid beta protein, and a BACE inhibitor.

Typical treatments encompassed by the present disclosure include combination therapies. For instance, the combination therapy may be a pharmacotherapy (that is, a compound of the disclosure) and a behavioral therapy. Behavioral therapy comprises, but is not limited to, electroconvulsive seizure therapy, exercise, group therapy, talk therapy, or conditioning. In another embodiment, the behavioral therapy is cognitive-behavioral therapy. Examples of behavioral therapy that may be used in the ongoing methods are described, for example, in Cognitive-Behavioral Therapies by K. Dobson, ed., Guilford Publications, Inc., 2002; The new Handbook of Cognitive Therapy: Basics and Beyond by Judith S.S. Beck, Guilford Publications, Inc. 1995, which are herein incorporated by reference in their entireties. Any pharmaceutical active ingredient that is recognized by the skilled artisan as being a pharmacologic agent that enhances learning or conditioning can be used in the methods of the present disclosure. For example, one such class of pharmaceutical active ingredients contemplated herein comprises compounds that increase the level of norepinephrine in the brain. Such compounds include those acting as norepinephrine reuptake inhibitors, for example tomoxetine, reboxetine, duloxetine, venlafaxine, and milnacipran, and those compounds that cause release of norepinephrine, for example amphetamine, dextroamphetamine, pemoline, and methylphenidate. Another class of such pharmaceutical active ingredients is those compounds that increase the level of acetylcholine in the brain, including, for example, compounds that block its breakdown. Examples of such compounds include, but are not limited to, donepezil HC1 or Aricept™ and tacrine, which inhibit cholinesterase activity.

The methods for combination therapy may comprise subjecting the subject to one or more sessions of a combination therapy protocol, where the combination therapy protocol comprises an acute administration of a therapeutically effective amount of a compound of the disclosure that enhances learning or conditioning in combination with a session of psychotherapy. In an aspect, the exposure to the compound occurs within about 24 hours prior to initiating the session of psychotherapy, preferably within about 12 hours, and more preferably within about 6 hours prior to initiating the session of psychotherapy. A full course of treatment for the neuropsychiatric disorder entails at least one session of this combination therapy protocol.

Hereinafter, the present disclosure will be described in more detail by the following examples. The following examples are presented to aid in the understanding of the present disclosure, and are not intended to limit the scope thereof in any way, and should not be construed as limiting.

Methods for obtaining the compounds described herein or pharmaceutically acceptable salts thereof will be apparent to those skilled in the art, and suitable procedures are described, for example, in the preparation examples below and in the references cited herein.

## VI. Method for producing active compound

Methods of synthesizing the compounds of the present disclosure are provided herein. The names and structures of the specific compounds illustrated are presented in Tables 2 to 4.

[Table 2]

| No. | Compound name |
|---|---|
| (1) BnH12001 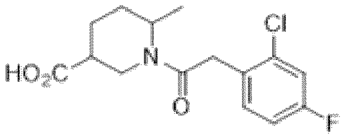 | 1-(2-(2-chloro-4-fluorophenyl)acetyl)-6-methylpiperidine-3-carboxylic acid |
| (2) BnH12001 enantiomer (3R,6S, 98%<) 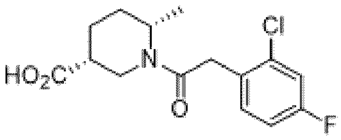 | (3R,6S)-1-(2-(2-chloro-4-fluorophenyl)acetyl)-6-methylpiperidine-3-carboxylic acid |
| (3) BnH12001 enantiomer (3S,6R, 98%<) 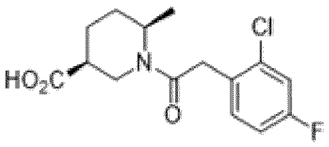 | (3S,6R)-1-(2-(2-chloro-4-fluorophenyl)acetyl)-6-methylpiperidine-3-carboxylic acid |
| (4) BnH12004 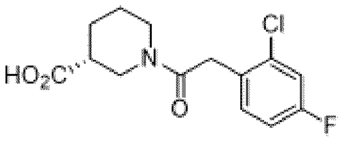 | (R)-1-(2-(2-chloro-4-fluorophenyl)acetyl)piperidine-3-carboxylic acid |
| (5) BnH12005 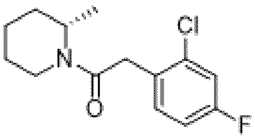 | (S)-2-(2-chloro-4-fluorophenyl)-1-(2-methylpiperidine-1-yl)ethan-1-one |
| (6) BnH12006 | (3R,6S)-1-(2-cyclohexylacetyl)-6-methylpiperidine-3-carboxylic |

| | |
|---|---|
| 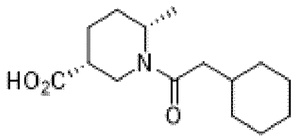 | acid |
| (7) BnH12007 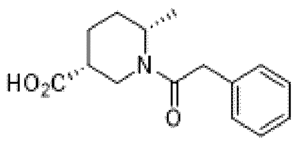 | (3R,6S)-6-methyl-1-(2-phenylacetyl)piperidine-3-carboxylic acid |
| (8) BnH12008 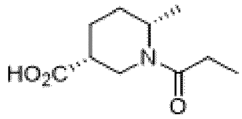 | (3R,6S)-6-methyl-1-propionylpiperidine-3-carboxylic acid |
| (9) BnH12009 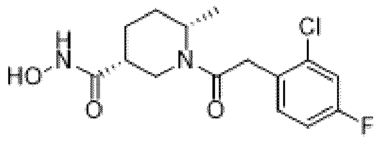 | (3R,6S)-1-(2-(2-chloro-4-fluorophenyl)acetyl)-N-hydroxy-6-methylpiperidine-3-carboxamide |
| (10) BnH12010 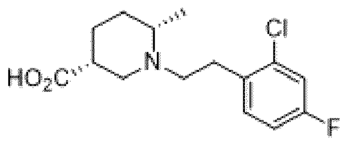 | (3R,6S)-1-(2-chloro-4-fluorophenethyl)-6-methylpiperidine-3-carboxylic acid |
| (11) BnH12011 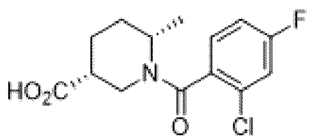 | (3R,6S)-1-(2-chloro-4-fluorobenzoyl)-6-methylpiperidine-3-carboxylic acid |
| (12) BnH12012 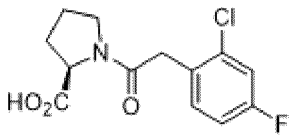 | (2-(2-chloro-4-fluorophenyl)acetyl)-D-proline |

| | |
|---|---|
| (13) BnH12013 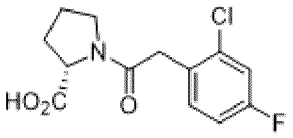 | (2-(2-chloro-4-fluorophenyl)acetyl)-L-proline |
| (14) BnH12014 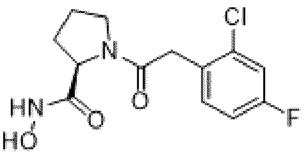 | (R)-1-(2-(2-chloro-4-fluorophenyl)acetyl)-N-hydroxypyrrolidine-2-carboxamide |
| (15) BnH12016 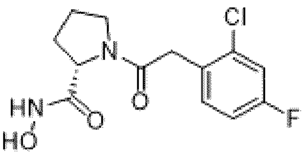 | (S)-1-(2-(2-chloro-4-fluorophenyl)acetyl)-N-hydroxypyrrolidine-2-carboxamide |
| (16) BnH12018 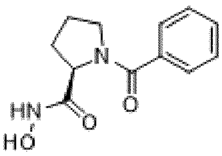 | (R)-1-benzoyl-N-hydroxypyrrolidine-2-carboxamide |
| (17) BnH12019 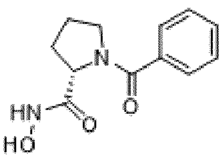 | (S)-1-benzoyl-N-hydroxypyrrolidine-2-carboxamide |
| (18) BnH12020 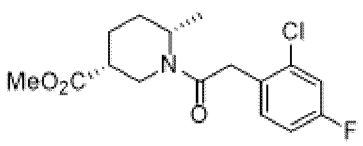 | Methyl (3R,6S)-1-(2-(2-chloro-4-fluorophenyl)acetyl)-6-methylpiperidine-3-carboxylate |
| (19) BnH12021 | Ethyl (3R,6S)-1-(2-(2-chloro-4-fluorophenyl)acetyl)-6-methylpiperidine-3-carboxylate |

| | |
|---|---|
| 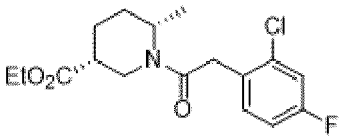 | |
| (20) BnH12022 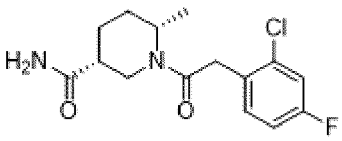 | (3R,6S)-1-(2-(2-chloro-4-fluorophenyl)acetyl)-6-methylpiperidine-3-carboxamide |

[Table 3]

| | |
|---|---|
| (21) BnH12023 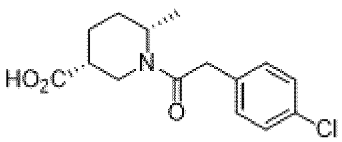 | (3R,6S)-1-(2-(4-chlorophenyl)acetyl)-6-methylpiperidine-3-carboxylic acid |
| (22) BnH12024 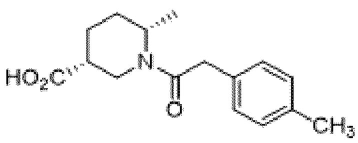 | (3R,6S)-6-methyl-1-(2-(p-tolyl)acetyl)piperidine-3-carboxylic acid |
| (23) BnH12025 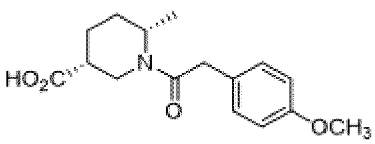 | (3R,6S)-1-(2-(4-methoxyphenyl)acetyl)-6-methylpiperidine-3-carboxylic acid |
| (24) BnH12026 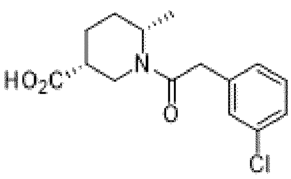 | (3R,6S)-1-(2-(3-chlorophenyl)acetyl)-6-methylpiperidine-3-carboxylic acid |

| | |
|---|---|
| (25) BnH12027 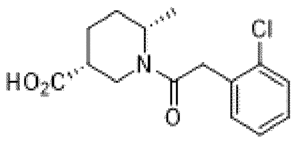 | (3R,6S)-1-(2-(2-chlorophenyl)acetyl)-6-methylpiperidine-3-carboxylic acid |
| (26) BnH12028 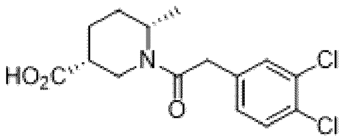 | (3R,6S)-1-(2-(3,4- dichlorophenyl)acetyl)-6-methylpiperidine-3-carboxylic acid |
| (27) BnH12029 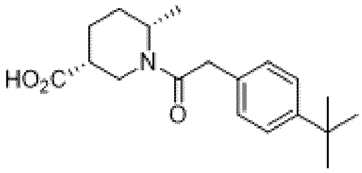 | (3R,6S)-1-(2-(4-(tert-butyl)phenyl)acetyl)-6-methylpiperidine-3-carboxylic acid |
| (28) BnH12030 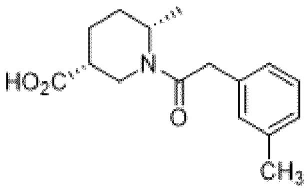 | (3R,6S)-6-methyl-1-(2-(m-tolyl)acetyl)piperidine-3-carboxylic acid |
| (29) BnH12031 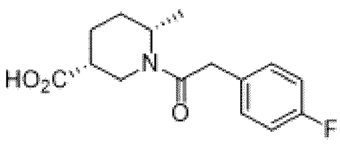 | (3R,6S)-1-(2-(4-fluorophenyl)acetyl)-6-methylpiperidine-3-carboxylic acid |
| (30) BnH12032 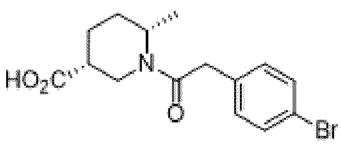 | (3R,6S)-1-(2-(4-bromophenyl)acetyl)-6-methylpiperidine-3-carboxylic acid |

| | |
|---|---|
| (31) BnH12033<br>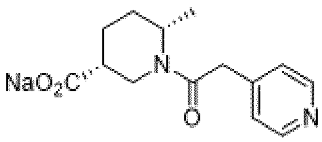 | Sodium (3R,6S)-6-methyl-1-(2-(pyridin-4-yl)acetyl)piperidine-3-carboxylate |
| (32) BnH12034<br>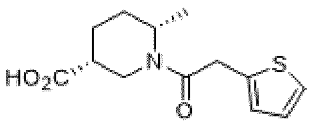 | (3R,6S)-6-methyl-1-(2-(thiophen-2-yl)acetyl)piperidine-3-carboxylic acid |
| (33) BnH12035<br>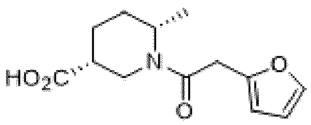 | (3R,6S)-1-(2-(furan-2-yl)acetyl)-6-methylpiperidine-3-carboxylic acid |
| (34) BnH12038<br>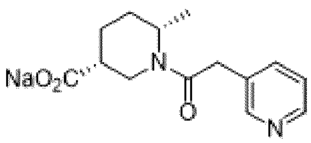 | Sodium (3R,6S)-6-methyl-1-(2-(pyridin-3-yl)acetyl)piperidine-3-carboxylate |
| (35) BnH12039<br>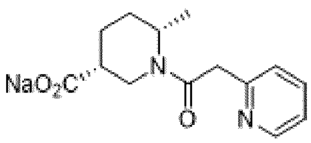 | Sodium (3R,6S)-6-methyl-1-(2-(pyridin-2-yl)acetyl)piperidine-3-carboxylate |

[Table 4]

| | |
|---|---|
| (36) BnH12040 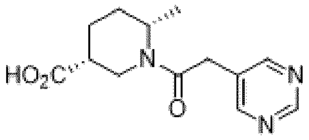 | (3R,6S)-6-methyl-1-(2-(pyrimidin-5-yl)acetyl)piperidine-3-carboxylic acid |
| (37) BnH12041 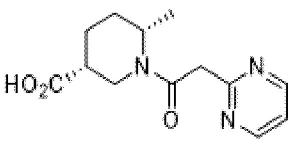 | (3R,6S)-6-methyl-1-(2-(pyrimidin-2-yl)acetyl)piperidine-3-carboxylic acid |
| (38) BnH12060 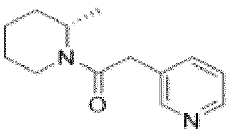 | (S)-1-(2-methylpiperidine-1-yl)-2-(pyridin-3-yl)ethan-1-one |
| (39) BnH12061 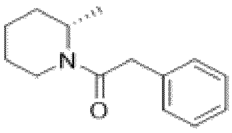 | (S)-1-(2-methylpiperidine-1-yl)-2-phenylethan-1-one |
| (40) BnH12062 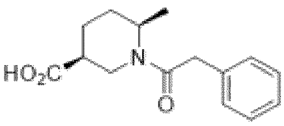 | (3S,6R)-6-methyl-1-(2-phenylacetyl)piperidine-3-carboxylic acid |
| (41) BnH12063 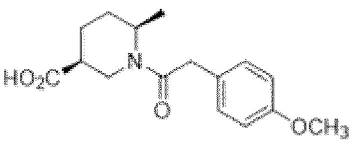 | (3S,6R)-1-(2-(4-methoxyphenyl)acetyl)-6-methylpiperidine-3-carboxylic acid |
| (42) BnH12065 | (3R,6S)-1-benzoyl-6-methylpiperidine-3-carboxylic acid |

| | |
|---|---|
| 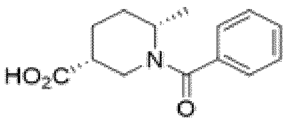 | |
| (43) BnH12068<br>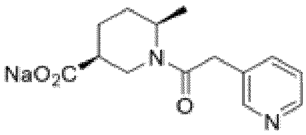 | Sodium (3S,6R)-6-methyl-1-(2-(pyridin-3-yl)acetyl)piperidine-3-carboxylate |
| (44) BnH12069<br>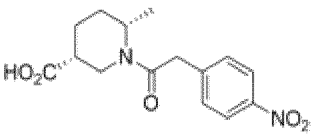 | (3R,6S)-6-methyl-1-(2-(4-nitrophenyl)acetyl))piperidine-3-carboxylic acid |
| (45) BnH12070<br>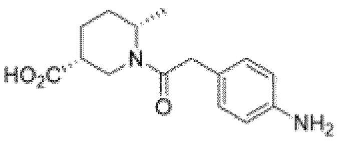 | (3R,6S)-1-(2-(4-aminophenyl)acetyl)-6-methylpiperidine-3-carboxylic acid |
| (46) BnH12073<br>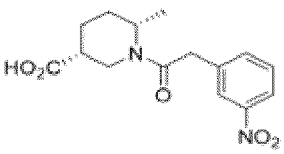 | (3R,6S)-6-methyl-1-(2-(3-nitrophenyl)acetyl))piperidine-3-carboxylic acid |
| (47) BnH12074<br>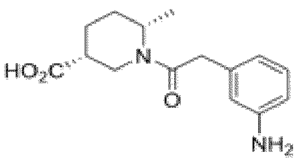 | (3R,6S)-1-(2-(3-aminophenyl)acetyl)-6-methylpiperidine-3-carboxylic acid |
| (48) BnH12075<br>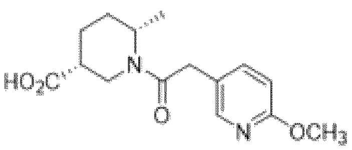 | (3R,6S)-1-(2-(6-methoxypyridin-3-yl)acetyl))-6-methylpiperidine-3-carboxylic acid |

| | |
|---|---|
| (49) BnH12076 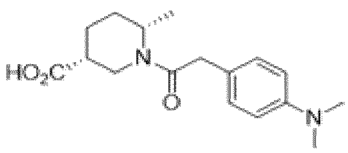 | (3R,6S)-1-(2-(4-(dimethylamino)phenyl)acetyl)-6-methylpiperidine-3-carboxylic acid |
| (50) BnH12079 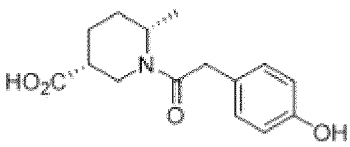 | (3R,6S)-1-(2-(4-hydroxy-phenyl)acetyl)-6-methylpiperidine-3-carboxylic acid |
| (51) BnH12094 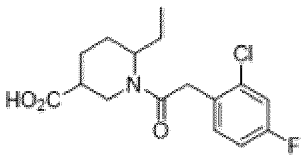 | 1-(2-(2-chloro-4-fluorophenyl)acetyl))-6-ethylpiperidine-3-carboxylic acid |
| (52) BnH12096 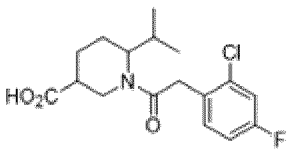 | 1-(2-(2-chloro-4-fluorophenyl)acetyl))-6-isopropylpiperidine-3-carboxylic acid |

Methods for obtaining the compounds described herein, stereoisomers thereof, or pharmaceutically acceptable salts thereof will be apparent to those skilled in the art, and suitable procedures are described, for example, in the following preparation examples and examples as well as in the references cited herein.

<u>Preparation Example</u>

**Preparation Example 1. Preparation of active compounds according to representative reaction scheme 1**

[Reaction scheme 1]

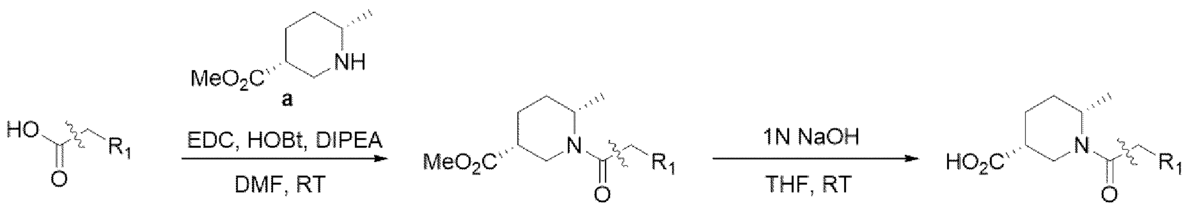


**step 1** **step 2** **Compounds**

| Compound | $R_1$ | Compound | $R_1$ |
|---|---|---|---|
| BnH12001 enantiomer (3R,6S, 98%<) | 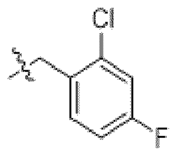 | BnH12006 | 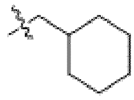 |
| BnH12007 | 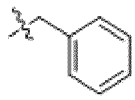 | BnH12008 | 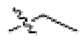 |
| BnH12011 | 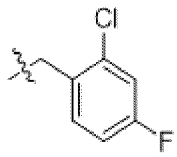 | BnH12023 | 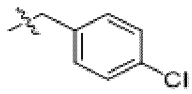 |
| BnH12024 | 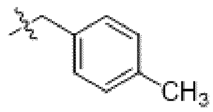 | BnH12025 | 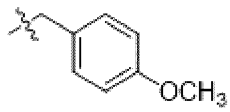 |
| BnH12026 | 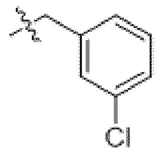 | BnH12027 | 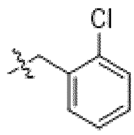 |
| BnH12028 | 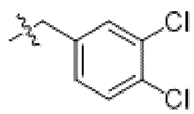 | BnH12029 | 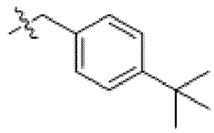 |
| BnH12030 | 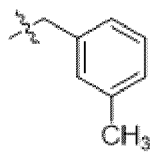 | BnH12031 | 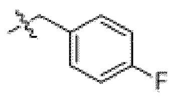 |

| | | | |
|---|---|---|---|
| BnH12032 | 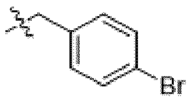 | BnH12033 | 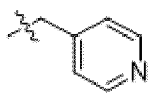 |
| BnH12034 | 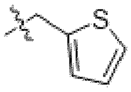 | BnH12035 | 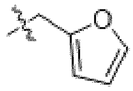 |
| BnH12038 | 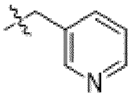 | BnH12039 | 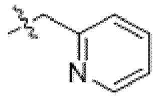 |
| BnH12040 | 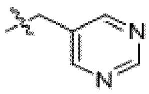 | BnH12041 | 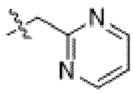 |
| BnH12065 | 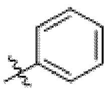 | BnH12069 | 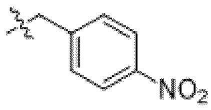 |
| BnH12070 | 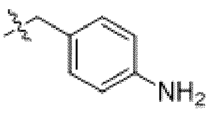 | BnH12073 | 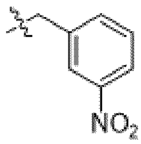 |
| BnH12074 | 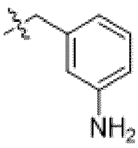 | BnH12075 | 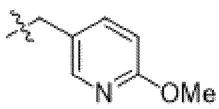 |
| BnH12076 | 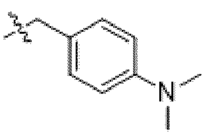 | BnH12079 | 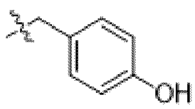 |

**(1) Synthesis of compound BnH12001 enantiomer (3R,6S, 98%<)**

(3R,6S)-1-(2-(2-chloro-4-fluorophenyl)acetyl)-6-methylpiperidine-3-carboxylic acid

**Step 1:** Synthesis of methyl (3R,6S)-1-(2-(2-chloro-4-fluorophenyl)acetyl)-6-methylpiperidine-3-carboxylate **(compound BnH12020)**

To a solution of 2-chloro-4-fluorophenylacetic acid (2.22 g, 11.8 mmol) in DMF (40.0 mL) were added methyl (3R,6S)-rel-6-methyl-3-piperidinecarboxylate (1.94 g, 12.4 mmol), DIPEA (3.08 mL, 17.7 mmol), HOBt (1.91 g, 14.1 mmol), and EDC·HCl (2.19 g, 14.1 mmol). The mixture was stirred at room temperature for 3 hours and 30 minutes. The

mixture was diluted with EtOAc (40.0 mL) and washed with saturated aqueous $NH_4Cl$ (40.0 mL) and brine (40.0 ml). The organic layer was dried over $MgSO_4$, filtered, and concentrated under reduced pressure. The residue was purified by column chromatography on $SiO_2$ (n-hexane:EtOAc = 3:1 to 2:1) to afford the desired compound (3.28 g, 85%) as a colorless oil.

$^1H$ NMR (400 MHz, Chloroform-d) δ 7.14 (dd, *J* = 8.5, 2.6 Hz, 1H), 6.96 (td, *J* = 8.3, 2.6 Hz, 1H), 4.97 (s, 0.5H), 4.80 (d, *J* = 13.8 Hz, 0.5H), 4.16 (s, 0.5H), 3.90 (d, *J* = 13.9 Hz, 0.5H), 3.82 - 3.71 (m, 2H), 3.24 (t, *J* = 13.1 Hz, 0.5H), 2.81 (t, *J* = 12.9 Hz, 0.5H), 2.38 (d, *J* = 35.9 Hz, 1H), 1.98 (d, *J* = 13.2 Hz, 1H), 1.90 - 1.75 (m, 1H), 1.64 (s, 2H), 1.28 - 1.16 (m, 3H).

**Step 2:** Synthesis of compound BnH12001 enantiomer (3S,6R)

To a solution of methyl (3R,6S)-1-(2-(2-chloro-4-fluorophenyl)acetyl)-6-methylpiperidine-3-carboxylate (3.20 g, 9.76 mmol) in THF (33.7 mL) was added 1 N NaOH (14.6 mL, 14.6 mmol). The mixture was stirred at room temperature for 2 hours. The mixture was diluted with $H_2O$ (15.0 mL) and washed twice with DCM (30.0 mL × 2). The aqueous layer was acidified with 1 N HCl until pH 1 to 2 at 0°C and extracted with EtOAc (30.0 mL). The organic layer was washed with brine (30.0 mL), dried over $MgSO_4$, filtered, and concentrated under reduced pressure. $Et_2O$ (30.0 mL) was added to the residue, and the mixture was stirred at room temperature for 1 hour. The precipitate was filtered and washed with $Et_2O$ (10.0 mL) to afford compound BnH12001 enantiomer (3S,6R) (2.75 g, 89%) as a white solid.

$^1H$ NMR (400 MHz, DMSO-d6) δ 12.44 (s, 1H), 7.40 (dd, J = 8.9, 2.6 Hz, 1H), 7.34 (dt, J = 15.2, 7.6 Hz, 1H), 7.16 (td, J = 8.5, 2.7 Hz, 1H), 4.70 (s, 0.5H), 4.49 (d, J = 13.5 Hz, 0.5H), 4.27 (s, 0.5H), 3.97 - 3.81 (m, 1H), 3.81 - 3.68 (m, 1.5H), 3.14 (t, J = 12.8 Hz, 0.5H), 2.64 (t, J = 12.6 Hz, 0.5H), 2.40 - 2.31 (m, 0.5H), 2.21 (s, 0.5H), 1.84 (d, J = 12.2 Hz, 1H), 1.76 - 1.60 (m, 2H), 1.55 (s, 1H), 1.14 (dd, J = 56.8, 6.9 Hz, 3H).

**(2) Synthesis of compound BnH12006**

(3R,6S)-1-(2-cyclohexylacetyl)-6-methylpiperidine-3-carboxylic acid

**Step 1:** Synthesis of methyl (3R,6S)-1-(2-cyclohexylacetyl)-6-methylpiperidine-3-carboxylate

To a solution of cyclohexylacetic acid (100 mg, 0.703 mmol) in DMF (2.34 mL) were added methyl (3R,6S)-rel-6-methyl-3-piperidinecarboxylate (116 mg, 0.738 mmol), DIPEA (0.184 mL, 1.06 mmol), HOBt (114 mg, 0.844 mmol), and EDC·HCl (131 mg, 0.844 mmol). The mixture was stirred at room temperature for 18 hours. The mixture was diluted with EtOAc (4.00 mL) and washed with saturated aqueous $NH_4Cl$ (4.00 mL) and brine (4.00 ml). The organic layer was dried over $MgSO_4$, filtered, and concentrated under reduced pressure. The residue was purified by column chromatography on $SiO_2$ (n-hexane:EtOAc = 3:1 to 2:1) to afford the desired compound (147 mg, 74%) as a colorless oil.

**Step 2:** Synthesis of compound BnH12006

To a solution of methyl (3R,6S)-1-(2-cyclohexylacetyl)-6-methylpiperidine-3-carboxylate (147 mg, 0.522 mmol) in THF (1.80 mL) was added 1 N NaOH (0.784 mL, 0.784 mmol). The mixture was stirred at room temperature for 2 hours. The mixture was diluted with $H_2O$ (2.00 mL) and washed twice with DCM (3.00 mL × 2). The aqueous layer was acidified with 1 N HCl until pH 1 to 2 at 0°C and extracted with EtOAc (3.00 mL). The organic layer was washed with brine (3.00 mL), dried over $MgSO_4$, filtered, and concentrated under reduced pressure. The residue was purified by column chromatography on $SiO_2$ (DCM:MeOH = 95:5 to 90:10) to afford compound BnH12006 (102 mg, 73%) as a white solid.

$^1H$ NMR (400 MHz, Chloroform-d) δ 4.99 (s, 0.5H), 4.81 (s, 0.5H), 4.19 (s, 0.5H), 3.91 (s, 0.5H), 3.22 (s, 0.5H), 2.75 (s, 0.5H), 2.48 - 2.36 (m, 1H), 2.22 (s, 2H), 1.98 (s, 1H), 1.90 - 1.58 (m, 11H), 1.34 - 1.05 (m, 8H), 0.96 (d, J = 12.0 Hz, 2H).

**(3) Synthesis of compound BnH12007**

(3R,6S)-6-methyl-1-(2-phenylacetyl)piperidine-3-carboxylic acid

**Step 1:** Synthesis of methyl (3R,6S)-6-methyl-1-(2-phenylacetyl)piperidine-3-carboxylate

To a solution of phenylacetic acid (100 mg, 0.734 mmol) in DMF (2.45 mL) were added methyl (3R,6S)-rel-6-methyl-3-piperidinecarboxylate (121 mg, 0.771 mmol), DIPEA (0.192 mL, 1.10 mmol), HOBt (119 mg, 0.881 mmol), and EDC·HCl (137 mg, 0.881 mmol). The mixture was stirred at room temperature for 18 hours. The mixture was diluted with EtOAc (4.00 mL) and washed with saturated aqueous $NH_4Cl$ (4.00 mL) and brine (4.00 ml). The organic layer was dried over $MgSO_4$, filtered, and concentrated under reduced pressure. The residue was purified by column chromatography on

$SiO_2$ (n-hexane:EtOAc = 3:1 to 2:1) to afford the desired compound (135 mg, 66%) as a colorless oil.

**Step 2:** Synthesis of compound BnH12007

To a solution of methyl (3R,6S)-6-methyl-1-(2-phenylacetyl)piperidine-3-carboxylate (135 mg, 0.490 mmol) in THF (1.69 mL) was added 1 NNaOH (0.735 mL, 0.735 mmol). The mixture was stirred at room temperature for 2 hours. The mixture was diluted with $H_2O$ (2.00 mL) and washed twice with DCM (3.00 mL × 2). The aqueous layer was acidified with 1 N HCl until pH 1 to 2 at 0°C and extracted with EtOAc (3.00 mL). The organic layer was washed with brine (3.00 mL), dried over $MgSO_4$, filtered, and concentrated under reduced pressure. The residue was purified by column chromatography on $SiO_2$ (DCM:MeOH = 95:5 to 90:10) to afford compound BnH12007 (105 mg, 82%) as a white solid.

$^1$H NMR (400 MHz, Chloroform-d) δ 7.32 (t, J = 7.5 Hz, 2H), 7.28 - 7.20 (m, 3H), 4.97 (s, 0.5H), 4.82 (d, J = 13.7 Hz, 0.5H), 4.17 (s, 0.5H), 3.91 (d, J = 13.7 Hz, 0.5H), 3.76 (s, 2H), 3.12 (t, J = 12.9 Hz, 0.5H), 2.77 (t, J = 12.7 Hz, 0.5H), 2.39 (s, 0.5H), 2.05 (s, 0.5H), 1.90 (d, J = 13.2 Hz, 1H), 1.77 (d, J = 14.3 Hz, 1H), 1.66 - 1.36 (m, 2H), 1.17 - 1.06 (m, 3H).

**(4) Synthesis of compound BnH12008**

(3R,6S)-6-methyl-1-propionylpiperidine-3-carboxylic acid

**Step 1:** Synthesis of methyl (3R,6S)-6-methyl-1-propionylpiperidine-3-carboxylate

To a solution of propionic acid (0.051 mL, 0.675 mmol) in DMF (2.25 mL) were added methyl (3R,6S)-rel-6-methyl-3-piperidinecarboxylate (111 mg, 0.709 mmol), DIPEA (0.178 mL, 1.01 mmol), HOBt (109 mg, 0.810 mmol), and EDC·HCl (126 mg, 0.810 mmol). The mixture was stirred at room temperature for 18 hours. The mixture was diluted with EtOAc (4.00 mL) and washed with saturated aqueous $NH_4Cl$ (4.00 mL) and brine (4.00 ml). The organic layer was dried over $MgSO_4$, filtered, and concentrated under reduced pressure. The residue was purified by column chromatography on $SiO_2$ (n-hexane:EtOAc = 3:1 to 2:1) to afford the desired compound (83 mg, 57%) as a colorless oil.

Step 2: Synthesis of compound BnH12008

To a solution of methyl (3R,6S)-6-methyl-1-propionylpiperidine-3-carboxylate (80.0 mg, 0.375 mmol) in THF (1.29 mL) was added 1 N NaOH (0.563 mL, 0.563 mmol). The mixture was stirred at room temperature for 2 hours. The mixture was diluted with $H_2O$ (2.00 mL) and washed twice with DCM (3.00 mL × 2). The aqueous layer was acidified with 1 N HCl until pH 1 to 2 at 0°C and extracted with EtOAc (3.00 mL). The organic layer was washed with brine (3.00 mL), dried over $MgSO_4$, filtered, and concentrated under reduced pressure. The residue was purified by column chromatography on $SiO_2$ (DCM:MeOH = 95:5 to 90:10) to afford compound BnH12008 (43.0 mg, 57%) as a white solid.

$^1$H NMR (400 MHz, Chloroform-d) δ 4.96 (s, 0.5H), 4.81 (s, 0.5H), 4.17 (s, 0.5H), 3.89 (s, 0.5H), 3.22 (s, 0.5H), 2.77 (s, 0.5H), 2.50 - 2.28 (m, 4H), 2.00 (d, J = 13.6 Hz, 1H), 1.84 (qd, J = 12.1, 5.5 Hz, 2H), 1.67 (s, 3H), 1.30 - 1.06 (m, 9H).

**(5) Synthesis of compound BnH12011**

(3R,6S)-1-(2-chloro-4-fluorobenzoyl)-6-methylpiperidine-3-carboxylic acid

**Step 1:** Synthesis of methyl (3R,6S)-1-(2-chloro-4-fluorobenzoyl)-6-methylpiperidine-3-carboxylate

To a solution of 2-chloro-4-fluorobenzoic acid (100 mg, 0.573 mmol) in DMF (1.91 mL) were added methyl (3R,6S)-rel-6-methyl-3-piperidinecarboxylate (94.6 mg, 0.602 mmol), DIPEA (0.150 mL, 0.859 mmol), HOBt (92.9 mg, 0.687 mmol), and EDC·HCl (107 mg, 0.687 mmol). The mixture was stirred at room temperature for 18 hours. The mixture was diluted with EtOAc (3.00 mL) and washed with saturated aqueous $NH_4Cl$ (3.00 mL) and brine (3.00 ml). The organic layer was dried over $MgSO_4$, filtered, and concentrated under reduced pressure. The residue was purified by column chromatography on $SiO_2$ (n-hexane:EtOAc = 2:1) to afford the desired compound (123 mg, 71%) as a colorless oil.

**Step 2:** Synthesis of compound BnH12011

To a solution of methyl (3R,6S)-1-(2-chloro-4-fluorobenzoyl)-6-methylpiperidine-3-carboxylate (123.0 mg, 0.392 mmol) in THF (1.35 mL) was added 1 NNaOH (0.588 mL, 0.588 mmol). The mixture was stirred at room temperature for 2 hours. The mixture was diluted with $H_2O$ (2.00 mL) and washed twice with DCM (3.00 mL × 2). The aqueous layer was acidified with 1 N HCl until pH 1 to 2 at 0°C and extracted with EtOAc (3.00 mL). The organic layer was washed with brine (3.00 mL), dried over $MgSO_4$, filtered, and concentrated under reduced pressure to afford compound BnH12011 (87.0 mg,

74%) as a white solid.

$^1H$ NMR (400 MHz, Chloroform-d) δ 7.22 (s, 1H), 7.20 - 7.11 (m, 1H), 7.04 (td, J = 8.3, 2.5 Hz, 1H), 5.10 (s, 0.5H), 4.92 (d, J = 12.9 Hz, 0.5H), 3.69 (s, 0.5H), 3.44 - 3.28 (m, 1H), 3.17 (s, 0.5H), 2.99 (dt, J = 26.2, 12.9 Hz, 0.5H), 2.71 - 2.52 (m, 1H), 2.35 (d, J = 6.7 Hz, 0.5H), 2.04 (d, J = 11.9 Hz, 1H), 1.93 - 1.69 (m, 2H), 1.60 (d, J = 11.8 Hz, 1H), 1.32 - 1.14 (m, 3H).

**(6) Synthesis of compound BnH12023**

(3R,6S)-1-(2-(4-chlorophenyl)acetyl)-6-methylpiperidine-3-carboxylic acid

**Step 1:** Synthesis of methyl (3R,6S)-1-(2-(4-chlorophenyl)acetyl)-6-methylpiperidine-3-carboxylate

To a solution of 2-(4-chlorophenyl)acetic acid (100 mg, 0.586 mmol) in DMF (3.00 mL) were added methyl (3R,6S)-rel-6-methyl-3-piperidinecarboxylate (96.8 mg, 0.616 mmol), DIPEA (0.153 mL, 0.879 mmol), HOBt (95.1 mg, 0.703 mmol), and EDC·HCl (109 mg, 0.703 mmol). The mixture was stirred at room temperature for 3 hours and 30 minutes. The mixture was diluted with EtOAc (5.00 mL) and washed with saturated aqueous $NH_4Cl$ (5.00 mL) and brine (5.00 mL). The organic layer was dried over $MgSO_4$, filtered, and concentrated under reduced pressure. The residue was purified by column chromatography on $SiO_2$ (n-hexane:EtOAc = 2:1) to afford the desired compound (81.5 mg, 44%) as a pale yellow oil.

**Step 2:** Synthesis of compound BnH12023

To a solution of methyl (3R,6S)-1-(2-(4-chlorophenyl)acetyl)-6-methylpiperidine-3-carboxylate (70.0 mg, 0.226 mmol) in THF (1.00 mL) was added 1N NaOH (0.339 mL, 0.339 mmol). The mixture was stirred at room temperature for 2 hours. The mixture was diluted with $H_2O$ (5.00 mL) and washed twice with DCM (5.00 mL × 2). The aqueous layer was acidified with 1 N HCl until pH 1 to 2 at 0°C and extracted with EtOAc (5.00 mL). The organic layer was washed with brine (5.00 mL), dried over $MgSO_4$, filtered, and concentrated under reduced pressure to afford compound BnH12023 (57.0 mg, 85%) as a white solid.

$^1H$ NMR (400 MHz, DMSO-$d_6$) δ 12.38 (s, 1H), 7.36 (d, *J* = 7.9 Hz, 2H), 7.23 (dd, *J* = 18.1, 8.1 Hz, 2H), 4.72 (s, 0.5H), 4.51 (dd, *J* = 13.3, 4.2 Hz, 0.5H), 4.23 (s, 0.5H), 3.87 (d, *J* = 13.6 Hz, 0.5H), 3.74 (q, J = 12.6, 9.6 Hz, 2H), 3.02 (t, *J* = 12.9 Hz, 0.5H), 2.60 (t, J = 12.8 Hz, 0.5H), 2.17 (tt, *J* = 11.9, 4.3 Hz, 1H), 1.81 - 1.77 (m, 1H), 1.70 - 1.60 (m, 3H), 1.51 - 1.48 (m, 2H), 1.05 (dd, *J* = 12.5, 6.8 Hz, 3H).

**(7) Synthesis of compound BnH12024**

(3R,6S)-6-methyl-1-(2-(p-tolyl)acetyl)piperidine-3-carboxylic acid

**Step 1:** Synthesis of methyl (3R,6S)-6-methyl-1-(2-(p-tolyl)acetyl)piperidine-3-carboxylate

To a solution of p-tolylacetic acid (100 mg, 0.666 mmol) in DMF (2.22 mL) were added methyl (3R,6S)-rel-6-methyl-3-piperidinecarboxylate (110 mg, 0.699 mmol), DIPEA (0.174 mL, 0.999 mmol), HOBt (108 mg, 0.799 mmol), and EDC·HCl (124 mg, 0.799 mmol). The mixture was stirred at room temperature for 18 hours. The mixture was diluted with EtOAc (4.00 mL) and washed with saturated aqueous $NH_4Cl$ (4.00 mL) and brine (4.00 ml). The organic layer was dried over $MgSO_4$, filtered, and concentrated under reduced pressure. The residue was purified by column chromatography on $SiO_2$ (n-hexane:EtOAc = 2:1 to 1:1) to afford the desired compound (156 mg, 85%) as a colorless oil.

**Step 2:** Synthesis of compound BnH12024

To a solution of methyl (3R,6S)-6-methyl-1-(2-(p-tolyl)acetyl)piperidine-3-carboxylate (156 mg, 0.539 mmol) in THF (1.86 mL) was added 1 NNaOH (0.809 mL, 0.809 mmol). The mixture was stirred at room temperature for 2 hours. The mixture was diluted with $H_2O$ (1.50 mL) and washed twice with DCM (3.00 mL × 2). The aqueous layer was acidified with 1 N HCl until pH 1 to 2 at 0°C and extracted with EtOAc (3.00 mL). The organic layer was washed with brine (3.00 mL), dried over $MgSO_4$, filtered, and concentrated under reduced pressure to afford compound BnH12024 (135 mg, 90%) as a white solid.

$^1H$ NMR (400 MHz, Chloroform-d) δ 7.26 (s, 1H), 7.12 (s, 2H), 4.96 (s, 0.5H), 4.81 (d, J = 13.7 Hz, 0.5H), 4.17 (s, 0.5H), 3.91 (d, J = 14.0 Hz, 0.5H), 3.71 (s, 2H), 3.11 (t, J = 12.9 Hz, 0.5H), 2.76 (t, J = 12.8 Hz, 0.5H), 2.38 (s, 0.5H), 2.32 (s, 3H), 2.09 (d, J = 9.0 Hz, 0.5H), 1.90 (dd, J = 13.2, 4.0 Hz, 1H), 1.77 (d, J = 13.5 Hz, 1H), 1.67 - 1.34 (m, 2H), 1.14 - 1.03 (m, 3H).

**(8) Synthesis of compound BnH12025**

(3R,6S)-1-(2-(4-methoxyphenyl)acetyl)-6-methylpiperidine-3-carboxylic acid

**Step 1:** Synthesis of methyl (3R,6S)-1-(2-(4-methoxyphenyl)acetyl)-6-methylpiperidine-3-carboxylate

To a solution of 4-methoxyphenylacetic acid (100 mg, 0.602 mmol) in DMF (2.01 mL) were added methyl (3R,6S)-rel-6-methyl-3-piperidinecarboxylate (99.3 mg, 0.632 mmol), DIPEA (0.157 mL, 0.903 mmol), HOBt (97.6 mg, 0.722 mmol), and EDC·HCl (112 mg, 0.722 mmol). The mixture was stirred at room temperature for 18 hours. The mixture was diluted with EtOAc (4.00 mL) and washed with saturated aqueous $NH_4Cl$ (4.00 mL) and brine (4.00 ml). The organic layer was dried over $MgSO_4$, filtered, and concentrated under reduced pressure. The residue was purified by column chromatography on $SiO_2$ (n-hexane:EtOAc = 2:1 to 1:1) to afford the desired compound (147 mg, 80%) as a colorless oil.

**Step 2:** Synthesis of compound BnH12025

To a solution of methyl (3R,6S)-1-(2-(4-methoxyphenyl)acetyl)-6-methylpiperidine-3-carboxylate (145 mg, 0.475 mmol) in THF (1.64 mL) was added 1 N NaOH (0.712 mL, 0.712 mmol). The mixture was stirred at room temperature for 2 hours. The mixture was diluted with $H_2O$ (1.50 mL) and washed twice with DCM (3.00 mL × 2). The aqueous layer was acidified with 1 N HCl until pH 1 to 2 at 0°C and extracted with EtOAc (3.00 mL). The organic layer was washed with brine (3.00 mL), dried over $MgSO_4$, filtered, and concentrated under reduced pressure to afford compound BnH12025 (114 mg, 82%) as a white solid.

$^1$H NMR (400 MHz, Chloroform-d) δ 7.19 - 7.12 (m, 2H), 6.85 (d, J = 8.3 Hz, 2H), 4.96 (s, 0.5H), 4.80 (d, J = 13.7 Hz, 0.5H), 4.17 (s, 0.5H), 3.92 (d, J = 14.0 Hz, 0.5H), 3.79 (s, 3H), 3.69 (s, 2H), 3.12 (t, J = 12.9 Hz, 0.5H), 2.77 (t, J = 12.8 Hz, 0.5H), 2.36 (d, J = 13.1 Hz, 0.5H), 2.09 (d, J = 7.1 Hz, 0.5H), 1.90 (dd, J = 13.5, 3.9 Hz, 1H), 1.77 (q, J = 12.5 Hz, 1H), 1.64 - 1.38 (m, 2H), 1.12 (t, J = 8.2 Hz, 3H).

**(9) Synthesis of compound BnH12026**

(3R,6S)-1-(2-(3-chlorophenyl)acetyl)-6-methylpiperidine-3-carboxylic acid

**Step 1:** Synthesis of methyl (3R,6S)-1-(2-(3-chlorophenyl)acetyl)-6-methylpiperidine-3-carboxylate

To a solution of 2-(3-chlorophenyl)acetic acid (100 mg, 0.586 mmol) in DMF (3.00 mL) were added methyl (3R,6S)-rel-6-methyl-3-piperidinecarboxylate (96.8 mg, 0.616 mmol), DIPEA (0.153 mL, 0.879 mmol), HOBt (95.1 mg, 0.703 mmol), and EDC·HCl (109 mg, 0.703 mmol). The mixture was stirred at room temperature for 3 hours and 30 minutes. The mixture was diluted with EtOAc (5.00 mL) and washed with saturated aqueous $NH_4Cl$ (5.00 mL) and brine (5.00 mL). The organic layer was dried over $MgSO_4$, filtered, and concentrated under reduced pressure. The residue was purified by column chromatography on $SiO_2$ (n-hexane:EtOAc = 2:1) to afford the desired compound (123 mg, 67%) as a pale yellow oil.

**Step 2:** Synthesis of compound BnH12026

To a solution of methyl (3R,6S)-1-(2-(3-chlorophenyl)acetyl)-6-methylpiperidine-3-carboxylate (100 mg, 0.323 mmol) in THF (1.50 mL) was added 1N NaOH (0.484 mL, 0.484 mmol). The mixture was stirred at room temperature for 2 hours. The mixture was diluted with $H_2O$ (5.00 mL) and washed twice with DCM (5.00 mL × 2). The aqueous layer was acidified with 1 N HCl until pH 1 to 2 at 0°C and extracted with EtOAc (5.00 mL). The organic layer was washed with brine (5.00 mL), dried over $MgSO_4$, filtered, and concentrated under reduced pressure to afford compound BnH12026 (73.8 mg, 77%) as a white solid.

$^1$H NMR (400 MHz, DMSO-$d_6$) δ 12.42 (s, 1H), 7.32 (dt, *J* = 17.1, 8.8 Hz, 3H), 7.18 (dd, *J* = 17.6, 7.3 Hz, 1H), 4.73 (s, 0.5H), 4.52 (d, *J* = 13.4 Hz, 0.5H), 4.25 (s, 0.5H), 3.90 (d, *J* = 14.0 Hz, 0.5H), 3.77 (q, *J* = 13.0, 10.3 Hz, 2H), 3.04 (t, *J* = 13.1 Hz, 0.5H), 2.62 (t, *J* = 12.8 Hz, 0.5H), 2.18 (t, *J*= 13.1 Hz, 1H), 1.81 (d, *J*= 12.7 Hz, 1H), 1.67 (d, *J*= 12.8 Hz, 1H), 1.53 (s, 2H), 1.07 (dd, *J* = 15.6, 7.1 Hz, 3H).

**(10) Synthesis of compound BnH12027**

(3R,6S)-1-(2-(2-chlorophenyl)acetyl)-6-methylpiperidine-3-carboxylic acid

**Step 1:** Synthesis of methyl (3R,6S)-1-(2-(2-chlorophenyl)acetyl)-6-methylpiperidine-3-carboxylate

To a solution of 2-chlorophenylacetic acid (100 mg, 0.586 mmol) in DMF (1.95 mL) were added methyl (3R,6S)-rel-6-methyl-3-piperidinecarboxylate (96.8 mg, 0.616 mmol), DIPEA (0.153 mL, 0.879 mmol), HOBt (95.1 mg, 0.703 mmol), and EDC·HCl (109 mg, 0.703 mmol). The mixture was stirred at room temperature for 18 hours. The mixture was diluted with EtOAc (3.00 mL) and washed with saturated aqueous $NH_4Cl$ (3.00 mL) and brine (3.00 ml). The organic layer was dried over $MgSO_4$, filtered, and concentrated under reduced pressure. The residue was purified by column chromatography on $SiO_2$ (n-hexane:EtOAc = 2:1 to 1:1) to afford the desired compound (148 mg, 85%) as a colorless oil.

**Step 2:** Synthesis of compound BnH12027

To a solution of methyl (3R,6S)-1-(2-(2-chlorophenyl)acetyl)-6-methylpiperidine-3-carboxylate (145 mg, 0.468 mmol) in THF (1.61 mL) was added 1 N NaOH (0.702 mL, 0.702 mmol). The mixture was stirred at room temperature for 2 hours. The mixture was diluted with $H_2O$ (1.50 mL) and washed twice with DCM (3.00 mL × 2). The aqueous layer was acidified with 1 N HCl until pH 1 to 2 at 0°C and extracted with EtOAc (3.00 mL). The organic layer was washed with brine (3.00 mL), dried over $MgSO_4$, filtered, and concentrated under reduced pressure to afford compound BnH12027 (122 mg, 88%) as a white solid.

$^1H$ NMR (400 MHz, Chloroform-*d*) δ 7.38 (dd, *J* = 7.0, 2.3 Hz, 1H), 7.29 (s, 1H), 7.21 (ddd, *J* = 9.7, 6.7, 2.4 Hz, 2H), 4.98 (s, 0.5H), 4.82 (d, *J* = 13.8 Hz, 0.5H), 4.13 (s, 0.5H), 3.86 (dd, *J* = 20.1, 13.7 Hz, 2.5H), 3.21 (t, *J* = 12.9 Hz, 0.5H), 2.81 (t, *J* = 12.8 Hz, 0.5H), 2.42 (s, 0.5H), 2.28 (s, 0.5H), 1.99 - 1.91 (m, 1H), 1.82 (dd, *J* = 13.1, 6.6 Hz, 1H), 1.72 - 1.51 (m, 2H), 1.18 (t, *J* = 6.7 Hz, 3H).

**(11) Synthesis of compound BnH12028**

(3R,6S)-1-(2-(3,4-dichlorophenyl)acetyl)-6-methylpiperidine-3-carboxylic acid

**Step 1:** Synthesis of methyl (3R,6S)-1-(2-(3,4-dichlorophenyl)acetyl)-6-methylpiperidine-3-carboxylate

To a solution of 2-(3,4-dichlorophenyl)acetic acid (100 mg, 0.488 mmol) in DMF (3.00 mL) were added methyl (3R,6S)-rel-6-methyl-3-piperidinecarboxylate (80.5 mg, 0.512 mmol), DIPEA (0.127 mL, 0.732 mmol), HOBt (79.1 mg, 0.585 mmol), and EDC·HCl (90.9 mg, 0.585 mmol). The mixture was stirred at room temperature overnight. The mixture was diluted with EtOAc (5.00 mL) and washed with saturated aqueous $NH_4Cl$ (5.00 mL) and brine (5.00 mL). The organic layer was dried over $MgSO_4$, filtered, and concentrated under reduced pressure. The residue was purified by column chromatography on $SiO_2$ (n-hexane:EtOAc = 2:1) to afford the desired compound (120 mg, 71%) as a gray oil.

**Step 2:** Synthesis of compound BnH12028

To a solution of methyl (3R,6S)-1-(2-(3,4-dichlorophenyl)acetyl)-6-methylpiperidine-3-carboxylate (110 mg, 0.320 mmol) in THF (2.00 mL) was added 1 N NaOH (0.479 mL, 0.479 mmol). The mixture was stirred at room temperature for 2 hours. The mixture was diluted with $H_2O$ (5.00 mL) and washed twice with DCM (5.00 mL × 2). The aqueous layer was acidified with 1 N HCl until pH 1 to 2 at 0°C and extracted with EtOAc (5.00 mL). The organic layer was washed with brine (5.00 mL), dried over $MgSO_4$, filtered, and concentrated under reduced pressure to afford compound BnH12028 (103 mg, 97%) as a white solid.

$^1H$ NMR (400 MHz, DMSO-$d_6$) δ 12.41 (s, 1H), 7.56 - 7.46 (m, 2H), 7.23 - 7.19 (m, 1H), 4.71 (s, 0.5H), 4.50 (dd, *J* = 13.5, 4.3 Hz, 0.5H), 4.25 (s, 0.5H), 3.89 (dd, *J* = 14.4, 4.1 Hz, 0.5H), 3.84 - 3.69 (m, 2H), 3.06 (t, *J* = 12.9 Hz, 0.5H), 2.62 (t, *J* = 12.8 Hz, 0.5H), 2.28 - 2.16 (m, 1H), 1.83 - 1.78 (m, 1H), 1.72 - 1.62 (m, 1H) 1.57 - 1.50 (m, 2H), 1.09 (dd, *J* = 28.9, 6.9 Hz, 3H).

**(12) Synthesis of compound BnH12029**

(3R,6S)-1-(2-(4-(tert-butyl)phenyl)acetyl)-6-methylpiperidine-3-carboxylic acid

**Step 1:** Synthesis of methyl (3R,6S)-1-(2-(4-(tert-butyl)phenyl)acetyl)-6-methylpiperidine-3-carboxylate

To a solution of 2-(4-(tert-butyl)phenyl)acetic acid (100 mg, 0.520 mmol) in DMF (3.00 mL) were added methyl (3R,6S)-rel-6-methyl-3-piperidinecarboxylate (85.9 mg, 0.546 mmol), DIPEA (0.136 mL, 0.780 mmol), HOBt (84.3 mg, 0.624 mmol), and EDC·HCl (96.9 mg, 0.624 mmol). The mixture was stirred at room temperature for 3 hours and 30 minutes. The mixture was diluted with EtOAc (5.00 mL) and washed with saturated aqueous $NH_4Cl$ (5.00 mL) and brine (5.00 mL). The organic layer was dried over $MgSO_4$, filtered, and concentrated under reduced pressure. The residue was purified by column chromatography on $SiO_2$ (n-hexane:EtOAc = 2:1) to afford the desired compound (114 mg, 66%) as a yellow oil.

**Step 2:** Synthesis of compound BnH12029

To a solution of methyl (3R,6S)-1-(2-(4-(tert-butyl)phenyl)acetyl)-6-methylpiperidine-3-carboxylate (95.0 mg, 0.287 mmol) in THF (2.00 mL) was added 1 N NaOH (0.430 mL, 0.430 mmol). The mixture was stirred at room temperature for 2 hours. The mixture was diluted with $H_2O$ (5.00 mL) and washed twice with DCM (5.00 mL × 2). The aqueous layer was acidified with 1 N HCl until pH 1 to 2 at 0°C and extracted with EtOAc (5.00 mL). The organic layer was washed with brine (5.00 mL), dried over $MgSO_4$, filtered, and concentrated under reduced pressure to afford compound BnH12029 (78.2 mg, 86%) as a white solid.

$^1$H NMR (400 MHz, DMSO-$d_6$) δ 12.35 (s, 1H), 7.32 (d, *J* = 7.8 Hz, 2H), 7.12 (dd, *J* = 17.8, 7.9 Hz, 2H), 4.73 (s, 0.5H), 4.55 - 4.50 (m, 0.5H), 4.24 (s, 1H), 3.89 - 3.85 (m, 0.5H), 3.67 (q, *J* = 15.4 Hz, 2H), 3.00 (t, *J* = 12.8 Hz, 0.5H), 2.59 (t, *J* = 12.7 Hz, 0.5H), 2.18 - 2.13 (m, 1H), 1.80 - 1.76 (m, 1H), 1.69 - 1.60 (m, 1H), 1.51 - 1.49 (m, 2H), 1.26 (s, 9H), 1.04 (d, *J* = 6.9 Hz, 3H).

**(13) Synthesis of compound BnH12030**

(3R,6S)-6-methyl-1-(2-(m-tolyl)acetyl)piperidine-3-carboxylic acid

**Step 1:** Synthesis of methyl (3R,6S)-6-methyl-1-(2-(m-tolyl)acetyl)piperidine-3-carboxylate

To a solution of 2-(m-tolyl)acetic acid (100 mg, 0.666 mmol) in DMF (3.00 mL) were added methyl (3R,6S)-rel-6-methyl-3-piperidinecarboxylate (110 mg, 0.699 mmol), DIPEA (0.174 mL, 0.999 mmol), HOBt (108 mg, 0.799 mmol), and EDC·HCl (124 mg, 0.799 mmol). The mixture was stirred at room temperature for 3 hours and 30 minutes. The mixture was diluted with EtOAc (5.00 mL) and washed with saturated aqueous $NH_4Cl$ (5.00 mL) and brine (5.00 mL). The organic layer was dried over $MgSO_4$, filtered, and concentrated under reduced pressure. The residue was purified by column chromatography on $SiO_2$ (n-hexane:EtOAc = 2:1) to afford the desired compound (88.0 mg, 45%) as a colorless oil.

**Step 2:** Synthesis of compound BnH12030

To a solution of methyl (3R,6S)-6-methyl-1-(2-(m-tolyl)acetyl)piperidine-3-carboxylate (70.0 mg, 0.242 mmol) in THF (1.00 mL) was added 1 N NaOH (0.363 mL, 0.363 mmol). The mixture was stirred at room temperature for 2 hours. The mixture was diluted with $H_2O$ (5.00 mL) and washed twice with DCM (5.00 mL × 2). The aqueous layer was acidified with 1 N HCl until pH 1 to 2 at 0°C and extracted with EtOAc (5.00 mL). The organic layer was washed with brine (5.00 mL), dried over $MgSO_4$, filtered, and concentrated under reduced pressure to afford compound BnH12030 (26.7 mg, 40%) as a white solid.

$^1$H NMR (400 MHz, DMSO-$d_6$) δ 12.37 (s, 1H), 7.18 (t, *J* = 7.6 Hz, 1H), 7.04 - 6.96 (m, 3H), 4.73 (s, 0.5H), 4.53 (dd, *J* = 13.1, 4.1 Hz, 0.5H), 4.22 (s, 0.5H), 3.88 (dd, *J* = 9.0, 3.9 Hz, 0.5H), 3.73 - 3.62 (m, 2H), 2.98 (t, *J* = 12.8 Hz, 0.5H), 2.59 (t, *J* = 12.7 Hz, 0.5H), 2.27 (s, 3H), 2.19 - 2.13 (m, 0.5H), 2.09 - 1.99 (m, 0.5H), 1.77 (d, *J* = 12.6 Hz, 1H), 1.69 - 1.58 (m, 1H), 1.49 - 1.44 (m, 2H), 1.04 (d, *J* = 6.9 Hz, 3H).

**(14) Synthesis of compound BnH12031**

(3R,6S)-1-(2-(4-fluorophenyl)acetyl)-6-methylpiperidine-3-carboxylic acid

**Step 1:** Synthesis of methyl (3R,6S)-1-(2-(4-fluorophenyl)acetyl)-6-methylpiperidine-3-carboxylate

To a solution of 2-(4-fluorophenyl)acetic acid (100 mg, 0.649 mmol) in DMF (3.00 mL) were added methyl (3R,6S)-rel-6-methyl-3-piperidinecarboxylate (107 mg, 0.681 mmol), DIPEA (0.170 mL, 0.973 mmol), HOBt (105 mg, 0.779 mmol), and EDC·HCl(121 mg, 0.779 mmol). The mixture was stirred at room temperature for 3 hours and 30 minutes. The mixture was diluted with EtOAc (5.00 mL) and washed with saturated aqueous $NH_4Cl$ (5.00 mL) and brine (5.00 mL). The organic layer was dried over $MgSO_4$, filtered, and concentrated under reduced pressure. The residue was purified by column chromatography on $SiO_2$ (n-hexane:EtOAc = 2:1) to afford the desired compound (112 mg, 59%) as a yellow oil.

**Step 2:** Synthesis of compound BnH12031

To a solution of methyl (3R,6S)-1-(2-(4-fluorophenyl)acetyl)-6-methylpiperidine-3-carboxylate (100 mg, 0.341 mmol) in THF (2.00 mL) was added 1N NaOH (0.511 mL, 0.511 mmol). The mixture was stirred at room temperature for 2 hours. The mixture was diluted with $H_2O$ (5.00 mL) and washed twice with DCM (5.00 mL × 2). The aqueous layer was acidified with 1 N HCl until pH 1 to 2 at 0°C and extracted with EtOAc (5.00 mL). The organic layer was washed with brine (5.00 mL), dried over $MgSO_4$, filtered, and concentrated under reduced pressure to afford compound BnH12031 (33.1 mg, 34%) as a white solid.

$^1H$ NMR (400 MHz, DMSO-$d_6$) δ 12.38 (s, 1H), 7.24 (dt, *J* = 18.2, 6.8 Hz, 2H), 7.12 (t, *J* = 8.7 Hz, 2H), 4.72 (s, 0.5H), 4.52 (dd, *J* = 15.1, 3.7 Hz, 0.5H), 4.24 (s, 0.5H), 3.88 (dd, *J* = 14.8,3.9 Hz, 0.5H), 3.77 - 3.66 (m, 2H), 3.01 (t, *J*= 12.8 Hz, 0.5H), 2.60 (*t*, *J* = 12.7 Hz, 0.5H), 2.16 (tt, *J* = 12.8, 5.0 Hz, 1H), 1.81 - 1.76 (m, 1H), 1.70 - 1.60 (m, 1H), 1.51 - 1.47 (s, 2H), 1.05 (t, *J* = 7.6 Hz, 3H).

**(15) Synthesis of compound BnH12032**

(3R,6S)-1-(2-(4-bromophenyl)acetyl)-6-methylpiperidine-3-carboxylic acid

**Step 1:** Synthesis of methyl (3R,6S)-1-(2-(4-bromophenyl)acetyl)-6-methylpiperidine-3-carboxylate

To a solution of 2-(4-bromophenyl)acetic acid (100 mg, 0.465 mmol) in DMF (2.50 mL) were added methyl (3R,6S)-rel-6-methyl-3-piperidinecarboxylate (76.8 mg, 0.488 mmol), DIPEA (0.122 mL, 0.698 mmol), HOBt (75.4 mg, 0.558 mmol), and EDC·HCl (86.6 mg, 0.558 mmol). The mixture was stirred at room temperature overnight. The mixture was diluted with EtOAc (5.00 mL) and washed with saturated aqueous $NH_4Cl$ (5.00 mL) and brine (5.00 mL). The organic layer was dried over $MgSO_4$, filtered, and concentrated under reduced pressure. The residue was purified by column chromatography on $SiO_2$ (n-hexane:EtOAc = 2:1) to afford the desired compound (110 mg, 66%) as a gray oil.

**Step 2:** Synthesis of compound BnH12032

To a solution of methyl (3R,6S)-1-(2-(4-bromophenyl)acetyl)-6-methylpiperidine-3-carboxylate (105 mg, 0.296 mmol) in THF (2.00 mL) was added 1N NaOH (0.445 mL, 0.445 mmol). The mixture was stirred at room temperature for 2 hours. The mixture was diluted with $H_2O$ (5.00 mL) and washed twice with DCM (5.00 mL × 2). The aqueous layer was acidified with 1 N HCl until pH 1 to 2 at 0°C and extracted with EtOAc (5.00 mL). The organic layer was washed with brine (5.00 mL), dried over $MgSO_4$, filtered, and concentrated under reduced pressure to afford compound BnH12032 (36.0 mg, 35%) as a white solid.

$^1H$ NMR (400 MHz, DMSO-$d_6$) δ 12.38 (s, 1H), 7.49 (d, *J* = 8.1 Hz, 2H), 7.17 (dd, *J*= 17.8, 8.0 Hz, 2H), 4.72 (s, 0.5H), 4.51 (dd, *J* = 13.3, 4.3 Hz, 0.5H), 4.23 (s, 0.5H), 3.87 (dd, *J* = 14.5, 4.2 Hz, 0.5H), 3.89 - 3.64 (m, 2H), 3.02 (t, *J* = 12.8 Hz, 0.5H), 2.60 (t, *J* = 12.7 Hz, 0.5H), 2.21 - 2.14 (m, 1H), 1.82 - 1.76 (m, 1H), 1.66 (qd, *J* = 11.9, 4.5 Hz, 1H), 1.52 - 1.48 (m, 2H), 1.06 (dd, *J* = 13.8, 6.9 Hz, 3H).

**(16) Synthesis of compound BnH12033**

Sodium (3R,6S)-6-methyl-1-(2-(pyridin-4-yl)acetyl)piperidine-3-carboxylate

**Step 1:** Synthesis of methyl (3R,6S)-6-methyl-1-(2-(pyridin-4-yl)acetyl)piperidine-3-carboxylate

To a solution of 2-(pyridin-4-yl)acetic acid hydrochloride (100 mg, 0.576 mmol) in DMF (3.00 mL) were added methyl (3R,6S)-rel-6-methyl-3-piperidinecarboxylate (95.1 mg, 0.605 mmol), DIPEA(0.301 mL, 1.73 mmol), HOBt (93.4 mg, 0.691 mmol), and EDC·HCl (107 mg, 0.691 mmol). The mixture was stirred at room temperature overnight. The mixture was diluted with EtOAc (5.00 mL) and washed with saturated aqueous $NH_4Cl$ (5.00 mL) and brine (5.00 mL). The organic layer was dried over $MgSO_4$, filtered, and concentrated under reduced pressure. The residue was purified by column chromatography on $SiO_2$ (n-hexane:EtOAc = 1:1) to afford the desired compound (145 mg, 84%) as a yellow oil.

**Step 2:** Synthesis of compound BnH12033

To a solution of methyl (3R,6S)-1-(2-(furan-2-yl)acetyl)-6-methylpiperidine-3-carboxylate (140 mg, 0.528 mmol) in THF (2.50 mL) was added 1N NaOH (0.792 mL, 0.792 mmol). The mixture was stirred at room temperature for 2 hours. The mixture was diluted with $H_2O$ (5.00 mL) and washed twice with DCM (5.00 mL $\times$ 2). The aqueous layer was concentrated under reduced pressure, and the residue was purified by column chromatography on C18 silica ($H_2O$:MeOH = 0 to 50%) to afford compound BnH12033 (16.0 mg, 28%) as a white solid.

$^1$H NMR (400 MHz, DMSO-*d*$_6$) δ 8.47 (d, *J* = 5.7 Hz, 2H), 7.23 (dd, *J* = 18.2, 5.0 Hz, 2H), 4.69 (s, 0.5H), 4.43 (d, *J* = 13.6 Hz, 0.5H), 4.14 (s, 0.5H), 3.82 - 3.66 (m, 3H), 2.95 (t, *J* = 13.0 Hz, 0.5H), 1.83 - 1.72 (m, 2H), 1.56 - 1.47 (m, 3H), 1.06 (dd, *J* = 19.3, 6.8 Hz, 3H).

**(17) Synthesis of compound BnH12034**

(3R,6S)-6-methyl-1-(2-(thiophen-2-yl)acetyl)piperidine-3-carboxylic acid

**Step 1:** Synthesis of methyl (3R,6S)-6-methyl-1-(2-(thiophen-2-yl)acetyl)piperidine-3-carboxylate

To a solution of 2-(thiophen-2-yl)acetic acid (100 mg, 0.703 mmol) in DMF (3.50 mL) were added methyl (3R,6S)-rel-6-methyl-3-piperidinecarboxylate (116 mg, 0.739 mmol), DIPEA (0.184 mL, 1.06 mmol), HOBt (114 mg, 0.844 mmol), and EDC·HCl (131 mg, 0.84 mmol). The mixture was stirred at room temperature overnight. The mixture was diluted with EtOAc (5.00 mL) and washed with saturated aqueous $NH_4Cl$ (5.00 mL) and brine (5.00 mL). The organic layer was dried over $MgSO_4$, filtered, and concentrated under reduced pressure. The residue was purified by column chromatography on $SiO_2$ (n-hexane:EtOAc = 2:1) to afford the desired compound (152 mg, 76%) as a yellow oil.

**Step 2:** Synthesis of compound BnH12034

To a solution of methyl (3R,6S)-6-methyl-1-(2-(thiophen-2-yl)acetyl)piperidine-3-carboxylate (148 mg, 0.526 mmol) in THF (3.00 mL) was added 1 N NaOH (0.789 mL, 0.789 mmol). The mixture was stirred at room temperature for 2 hours. The mixture was diluted with $H_2O$ (5.00 mL) and washed twice with DCM (5.00 mL $\times$ 2). The aqueous layer was acidified with 1 N HCl until pH 1 to 2 at 0°C and extracted with EtOAc (5.00 mL). The organic layer was washed with brine (5.00 mL), dried over $MgSO_4$, filtered, and concentrated under reduced pressure to afford compound BnH12034 (90.3 mg, 64%) as a white solid.

$^1$H NMR (400 MHz, DMSO-*d*$_6$) δ 12.40 (s, 1H), 7.37 (dd, *J* = 5.0, 1.4 Hz, 1H), 6.96 - 6.88 (m, 2H), 4.72 (s, 0.5H), 4.50 (dd, *J* = 14.3, 4.1 Hz, 0.5H), 4.28 (s, 0.5H), 4.01 - 3.89 (m, 2.5H), 3.05 (t, *J* = 12.9 Hz, 0.5H), 2.62 (t, *J* = 12.7 Hz, 0.5H), 2.25 - 2.13 (m, 1H), 1.82 - 1.77 (m, 1H), 1.71 - 1.61 (m, 1H), 1.55 - 1.50 (m, 2H), 1.07 (dd, *J* = 20.3, 6.9 Hz, 3H).

**(18) Synthesis of compound BnH12035**

(3R,6S)-1-(2-(furan-2-yl)acetyl)-6-methylpiperidine-3-carboxylic acid

**Step 1:** Synthesis of methyl (3R,6S)-1-(2-(furan-2-yl)acetyl)-6-methylpiperidine-3-carboxylate

To a solution of 2-(furan-2-yl)acetic acid (100 mg, 0.793 mmol) in DMF (4.00 mL) were added methyl (3R,6S)-rel-6-methyl-3-piperidinecarboxylate (131 mg, 0.833 mmol), DIPEA (0.207 mL, 1.19 mmol), HOBt (129 mg, 0.952 mmol), and EDC·HCl (148 mg, 0.95 mmol). The mixture was stirred at room temperature overnight. The mixture was

diluted with EtOAc (5.00 mL) and washed with saturated aqueous $NH_4Cl$ (5.00 mL) and brine (5.00 mL). The organic layer was dried over $MgSO_4$, filtered, and concentrated under reduced pressure. The residue was purified by column chromatography on $SiO_2$ (n-hexane:EtOAc = 2:1) to afford the desired compound (153 mg, 72%) as a yellow oil.

**Step 2:** Synthesis of compound BnH12035

To a solution of methyl (3R,6S)-1-(2-(furan-2-yl)acetyl)-6-methylpiperidine-3-carboxylate (140 mg, 0.528 mmol) in THF (2.50 mL) was added 1 N NaOH (0.792 mL, 0.792 mmol). The mixture was stirred at room temperature for 2 hours. The mixture was diluted with $H_2O$ (5.00 mL) and washed twice with DCM (5.00 mL × 2). The aqueous layer was acidified with 1 N HCl until pH 1 to 2 at 0°C and extracted with EtOAc (5.00 mL). The organic layer was washed with brine (5.00 mL), dried over $MgSO_4$, filtered, and concentrated under reduced pressure to afford compound BnH12035 (100 mg, 75%) as a white solid.

$^1H$ NMR (400 MHz, DMSO-$d_6$) δ 12.41 (s, 1H), 7.54 (d, *J* = 1.8 Hz, 1H), 6.38 (t, *J* = 2.5 Hz, 1H), 6.18 (dd, *J* = 24.1, 3.2 Hz, 1H), 4.70 (t, *J* = 5.6 Hz, 0.5H), 4.49 (dd, *J* = 13.4, 4.3 Hz, 0.5H), 4.25 (dd, *J* = 8.4, 4.5 Hz, 0.5H), 3.89 (dd, *J* = 13.9, 4.2 Hz, 0.5H), 3.83 - 3.73 (m, 2H), 3.10 - 3.04 (m, 0.5H), 2.61 (t, *J* = 12.7 Hz, 0.5H), 2.28 - 2.14 (m, 1H), 1.84 - 1.78 (m, 1H), 1.72 - 1.49 (m, 2H), 1.07 (dd, *J*= 19.2, 6.9 Hz, 3H).

**(19) Synthesis of compound BnH12038**

Sodium (3R,6S)-6-methyl-1-(2-(pyridin-3-yl)acetyl)piperidine-3-carboxylate

**Step 1:** Synthesis of methyl (3R,6S)-6-methyl-1-(2-(pyridin-3-yl)acetyl)piperidine-3-carboxylate

To a solution of 2-(pyridin-3-yl)acetic acid hydrochloride (100 mg, 0.576 mmol) in DMF (3.00 mL) were added methyl (3R,6S)-rel-6-methyl-3-piperidinecarboxylate (95.1 mg, 0.605 mmol), DIPEA(0.301 mL, 1.73 mmol), HOBt (93.4 mg, 0.691 mmol), and EDC·HCl (107 mg, 0.691 mmol). The mixture was stirred at room temperature overnight. The mixture was diluted with EtOAc (5.00 mL) and washed with saturated aqueous $NH_4Cl$ (5.00 mL) and brine (5.00 mL). The organic layer was dried over $MgSO_4$, filtered, and concentrated under reduced pressure. The residue was purified by column chromatography on $SiO_2$ (DCM: MeOH = 0 to 5%) to afford the desired compound (76.2 mg, 47%) as a yellow oil.

**Step 2:** Synthesis of compound BnH12038

To a solution of methyl (3R,6S)-6-methyl-1-(2-(pyridin-3-yl)acetyl)piperidine-3-carboxylate (75.0 mg, 0.271 mmol) in THF (2.00 mL) was added 1N NaOH (0.407 mL, 0.407 mmol). The mixture was stirred at room temperature for 2 hours. The mixture was diluted with $H_2O$ (5.00 mL) and washed twice with DCM (5.00 mL × 2). The aqueous layer was concentrated under reduced pressure, and the residue was purified by column chromatography on C18 silica ($H_2O$:MeOH = 0 to 50%) to afford compound BnH12038 (26.7 mg, 34%) as a pale yellow solid.

$^1H$ NMR (400 MHz, DMSO-$d_6$) δ 8.43 - 8.38 (m, 2H), 7.60 (ddt, *J* = 14.5, 7.8, 2.0 Hz, 1H), 7.31 (dt, *J* = 8.1, 4.4 Hz, 1H), 4.68 (t, *J*= 5.8 Hz, 0.5H), 4.42 (dd, *J* = 14.2, 3.9 Hz, 0.5H), 4.21 (t, *J* = 6.3 Hz, 0.5H), 3.85 - 3.64 (m, 2.5H), 2.97 (dd, *J* = 13.9, 11.9 Hz, 0.5H), 2.54 - 2.48 (m, 0.5H), 1.86 - 1.69 (m, 2H), 1.61 - 1.42 (m, 3H), 1.06 (dd, *J* = 30.0, 6.9 Hz, 3H).

**(20) Synthesis of compound BnH12039**

Sodium (3R,6S)-6-methyl-1-(2-(pyridin-2-yl)acetyl)piperidine-3-carboxylate

**Step 1:** Synthesis of methyl (3R,6S)-6-methyl-1-(2-(pyridin-2-yl)acetyl)piperidine-3-carboxylate

To a solution of 2-(pyridin-2-yl)acetic acid hydrochloride (100 mg, 0.576 mmol) in DMF (3.00 mL) were added methyl (3R,6S)-rel-6-methyl-3-piperidinecarboxylate (95.1 mg, 0.605 mmol), DIPEA(0.301 mL, 1.73 mmol), HOBt (93.4 mg, 0.691 mmol), and EDC·HCl (107 mg, 0.691 mmol). The mixture was stirred at room temperature overnight. The mixture was diluted with EtOAc (5.00 mL) and washed with saturated aqueous $NH_4Cl$ (5.00 mL) and brine (5.00 mL). The organic layer was dried over $MgSO_4$, filtered, and concentrated under reduced pressure. The residue was purified by column chromatography on $SiO_2$ (DCM: MeOH = 0 to 5%) to afford the desired compound (71.2 mg, 44%) as a yellow oil.

**Step 2:** Synthesis of compound BnH12039

To a solution of methyl (3R,6S)-6-methyl-1-(2-(pyridin-3-yl)acetyl)piperidine-3-carboxylate (75.0 mg, 0.271 mmol) in THF (2.00 mL) was added 1N NaOH (0.407 mL, 0.407 mmol). The mixture was stirred at room temperature

for 2 hours. The mixture was diluted with $H_2O$ (5.00 mL) and washed twice with DCM (5.00 mL × 2). The aqueous layer was concentrated under reduced pressure, and the residue was purified by column chromatography on C18 silica ($H_2O$:MeOH = 0 to 50%) to afford compound BnH12039 (66.1 mg, 98%) as a white solid.

$^1$H NMR (400 MHz, DMSO-$d_6$) δ 8.43 - 8.38 (m, 2H), 7.60 (ddt, *J* = 14.5, 7.8, 2.0 Hz, 1H), 7.31 (dt, *J* = 8.1, 4.4 Hz, 1H), 4.68 (t, *J*= 5.8 Hz, 0.5H), 4.42 (dd, *J* = 14.2, 3.9 Hz, 0.5H), 4.21 (t, *J* = 6.3 Hz, 0.5H), 3.85 - 3.64 (m, 2.5H), 2.97 (dd, *J* = 13.9, 12.0 Hz, 0.5H), 2.54 - 2.48 (m, 0.5H), 1.86 - 1.69 (m, 2H), 1.61 - 1.42 (m, 3H), 1.06 (dd, *J* = 30.0, 6.9 Hz, 3H).

**(21) Synthesis of compound BnH12040**

(3R,6S)-6-methyl-1-(2-(pyrimidin-5-yl)acetyl)piperidine-3-carboxylic acid

**Step 1:** Synthesis of methyl (3R,6S)-6-methyl-1-(2-(pyrimidin-5-yl)acetyl)piperidine-3-carboxylate

To a solution of 2-(pyrimidin-5-yl)acetic acid (100 mg, 0.724 mmol) in DMF (3.50 mL) were added methyl (3R,6S)-rel-6-methyl-3-piperidinecarboxylate (120 mg, 0.760 mL), DIPEA (0.378 mL, 2.17 mmol), HOBt (117 mg, 0.869 mmol), and EDC·HCl (135 mg, 0.869 mmol). The mixture was stirred at room temperature overnight. The mixture was diluted with EtOAc (5.00 mL) and washed with saturated aqueous $NH_4Cl$ (5.00 mL) and brine (5.00 mL). The organic layer was dried over $MgSO_4$, filtered, and concentrated under reduced pressure. The residue was purified by column chromatography on $SiO_2$ (DCM:MeOH = 0-5%) to afford the desired compound (72.8 mg, 36%) as a yellow oil.

**Step 2:** Synthesis of compound BnH12040

To a solution of methyl (3R,6S)-6-methyl-1-(2-(pyrimidin-5-yl)acetyl)piperidine-3-carboxylate (65.0 mg, 0.234 mmol) in THF (1.00 mL) was added 1 NNaOH (0.352 mL, 0.352 mmol). The mixture was stirred at room temperature for 2 hours. The mixture was diluted with $H_2O$ (5.00 mL) and washed twice with DCM (5.00 mL × 2). The aqueous layer was acidified with 1 N HCl until pH 4 at 0°C and extracted with EtOAc (5.00 mL). The organic layer was washed with brine (5.00 mL), dried over $MgSO_4$, filtered, and concentrated under reduced pressure to afford compound BnH12040 (35.1 mg, 56%) as a white solid.

$^1$H NMR (400 MHz, DMSO-$d_6$) δ 12.45 (s, 1H), 9.04 (s, 1H), 8.62 (d, *J* = 12.7 Hz, 2H), 4.70 (s, 0.5H), 4.52 - 4.47 (m, 0.5H), 4.32 (s, 0.5H), 3.99 - 3.69 (m, 2.5H), 3.15 (t, *J* = 12.9 Hz, 0.5H), 2.65 (t, *J* = 12.8 Hz, 0.5H), 2.45 - 2.38 (m, 0.5H), 2.26 - 2.18 (m, 0.5H), 1.86 - 1.83 (m, 1H), 1.73 - 1.54 (m, 3H), 1.14 (dd, *J* = 58.2, 6.9 Hz, 3H).

**(22) Synthesis of compound BnH12041**

(3R,6S)-6-methyl-1-(2-(pyrimidin-2-yl)acetyl)piperidine-3-carboxylic acid

**Step 1:** Synthesis of methyl (3R,6S)-6-methyl-1-(2-(pyrimidin-2-yl)acetyl)piperidine-3-carboxylate

To a solution of 2-(pyrimidin-2-yl)acetic acid (100 mg, 0.724 mmol) in DMF (3.50 mL) were added methyl (3R,6S)-rel-6-methyl-3-piperidinecarboxylate (120 mg, 0.760 mL), DIPEA (0.378 mL, 2.17 mmol), HOBt (117 mg, 0.869 mmol), and EDC·HCl (135 mg, 0.869 mmol). The mixture was stirred at room temperature overnight. The mixture was diluted with EtOAc (5.00 mL) and washed with saturated aqueous $NH_4Cl$ (5.00 mL) and brine (5.00 mL). The organic layer was dried over $MgSO_4$, filtered, and concentrated under reduced pressure. The residue was purified by column chromatography on $SiO_2$ (DCM:MeOH = 0 to 5%) to afford the desired compound (50.0 mg, 24%) as a pale yellow oil.

**Step 2:** Synthesis of compound BnH12041

To a solution of methyl (3R,6S)-6-methyl-1-(2-(pyrimidin-2-yl)acetyl)piperidine-3-carboxylate (45.0 mg, 0.162 mmol) in THF (1.00 mL) was added 1 N NaOH (0.243 mL, 0.243 mmol). The mixture was stirred at room temperature for 2 hours. The mixture was diluted with $H_2O$ (5.00 mL) and washed twice with DCM (5.00 mL × 2). The aqueous layer was acidified with 1 N HCl until pH 4 at 0°C and extracted with EtOAc (5.00 mL). The organic layer was washed with brine (5.00 mL), dried over $MgSO_4$, filtered, and concentrated under reduced pressure to afford compound BnH12041 (22.1 mg, 51%) as a yellow viscous solid.

$^1$H NMR (400 MHz, DMSO-$d_6$) δ 12.38 (s, 1H), 8.75 (t, *J*= 4.7 Hz, 2H), 7.39 (q, *J* = 4.6 Hz, 1H), 4.71 (s, 0.5H), 4.52 - 4.48 (m, 0.5H), 4.26 (s, 0.5H), 4.10 - 3.86 (m, 2H), 3.88 (d, *J* = 14.8 Hz, 0.5H), 3.07 (t, *J* = 12.8 Hz, 0.5H), 2.68 - 2.60 (m, 0.5H), 1.84 - 1.80 (m, 1H), 1.73 - 1.61 (m, 4H), 1.09 (dd, *J* = 11.9, 6.9 Hz, 3H).

**(23) Synthesis of compound BnH12065**

(3R,6S)-1-benzoyl-6-methylpiperidine-3-carboxylic acid

**Step 1:** Synthesis of methyl (3R,6S)-1-benzoyl-6-methylpiperidine-3-carboxylate

To a solution of benzoic acid (100 mg, 0.819 mmol) in DMF (2.73 mL) were added methyl (3R,6S)-6-methylpiperidine-3-carboxylate (135 mg, 0.860 mmol), DIPEA (0.215 mL, 1.23 mmol), HOBt (133 mg, 0.983 mmol), and EDC·HCl (153 mg, 0.983 mmol). The mixture was stirred at room temperature for 2 hours. The mixture was diluted with EtOAc (5.00 mL) and washed with saturated aqueous $NH_4Cl$ (5.00 mL) and brine (5.00 mL). The organic layer was dried over $MgSO_4$, filtered, and concentrated under reduced pressure. The residue was purified by column chromatography on $SiO_2$ (n-hexane:EtOAc = 3:1 to 2:1) to afford the desired compound (150 mg, 70.1%) as a colorless oil.

**Step 2:** Synthesis of compound BnH12065

To a solution of methyl (3R,6S)-1-benzoyl-6-methylpiperidine-3-carboxylate (150 mg, 0.574 mmol) in THF (1.98 mL) was added 1 N NaOH (0.861 mL, 0.861 mmol). The mixture was stirred at room temperature for 2 hours. The mixture was diluted with $H_2O$ (2.00 mL) and washed twice with DCM (3.00 mL × 2). The aqueous layer was acidified with 1 N HCl until pH 1 to 2 at 0°C and extracted with EtOAc (3.00 mL). The organic layer was washed with brine (3.00 mL), dried over $MgSO_4$, filtered, and concentrated under reduced pressure to afford compound BnH12065 (121 mg, 80.7%) as a white solid.

$^1H$ NMR (400 MHz, DMSO-$d_6$) δ 12.46 (s, 1H), 7.45-7.43 (q, *J* = 3.2 Hz, 3H), 7.37-7.34 (q, *J* = 3.2 Hz, 2H), 2.95 (s, 1H), 2.44-2.36 (q, *J* = 11.2 Hz, 2H), 1.89-1.54 (m, 5H), 1.17-1.11 (m, 3H).

**(24) Synthesis of compound BnH12069**

(3R,6S)-6-methyl-1-(2-(4-nitrophenyl)acetyl)piperidine-3-carboxylic acid

**Step 1:** Synthesis of methyl (3R,6S)-6-methyl-1-(2-(4-nitrophenyl)acetyl)piperidine-3-carboxylate

To a solution of 2-(4-nitrophenyl)acetic acid (200 mg, 1.10 mmol) in DMF (3.68 mL) were added methyl (3R,6S)-6-methylpiperidine-3-carboxylate (182 mg, 1.16 mmol), DIPEA (0.289 mL, 1.66 mmol), HOBt (179 mg, 1.33 mmol), and EDC·HCl (206 mg, 1.33 mmol). The mixture was stirred at room temperature for 2 hours. The mixture was diluted with EtOAc (8.00 mL) and washed with saturated aqueous $NH_4Cl$ (8.00 mL) and brine (8.00 mL). The organic layer was dried over $MgSO_4$, filtered, and concentrated under reduced pressure. The residue was purified by column chromatography on $SiO_2$ (n-hexane:EtOAc = 2:1 to 1:1) to afford the desired compound (180 mg, 50.9%) as a pale yellow oil.

**Step 2:** Synthesis of compound BnH12069

To a solution of methyl (3R,6S)-6-methyl-1-(2-(4-nitrophenyl)acetyl)piperidine-3-carboxylate (180 mg, 0.562 mmol) in THF (1.94 mL) was added 1 N NaOH (0.843 mL, 0.843 mmol). The mixture was stirred at room temperature for 2 hours. The mixture was diluted with $H_2O$ (5.00 mL) and washed twice with DCM (6.00 mL × 2). The aqueous layer was acidified with 1 N HCl until pH 1 to 2 at 0°C and extracted with EtOAc (3.00 mL). The organic layer was washed with brine (5.00 mL), dried over $MgSO_4$, filtered, and concentrated under reduced pressure to afford compound BnH12069 (166 mg, 96.4%) as a white solid.

$^1H$ NMR (400 MHz, DMSO-$d_6$) δ 12.44 (s, 1H), 8.19-8.17 (d, *J* = 8.5 Hz, 2H), 7.53-7.47 (dd, *J* = 17.2, 8.3 Hz, 2H), 4.72 (s, 0.5H), 4.54 - 4.50 (d, 0.5H), 4.26 (s, 0.5H), 4.00-3.84 (q, *J* = 15.9, 15.4 Hz, 2.5H), 3.10-3.04 (t, *J* = 12.9 Hz, 0.5H), 2.67-2.60 (t, *J* = 12.8 Hz, 0.5H), 2.30-2.19 (m, 1H), 1.84-1.78 (td, *J* = 13.9, 3.6 Hz, 1H), 1.71-1.62 (m, 1H), 1.55 (s, 2H), 1.14-1.05 (dd, *J* = 26.7, 6.9 Hz, 3H).

**(25) Synthesis of compound BnH12070**

(3R,6S)-1-(2-(4-aminophenyl)acetyl)-6-methylpiperidine-3-carboxylic acid

**Step 1:** Synthesis of compound BnH12070

To a solution of BnH12068 (92.0 mg, 0.300 mmol) in MeOH (2.00 mL) was added 10 wt% palladium on carbon (9.20 mg) in an ice-water bath under $N_2$ gas at 0°C. The mixture was degassed and purged with $H_2$ gas. After removing the

cooling bath, the mixture was stirred vigorously at room temperature under $H_2$ gas for 1 hour and 30 minutes. The reaction mixture was filtered through a pad of Celite and washed with MeOH (5.00 mL). The filtrate was concentrated under reduced pressure. The residue was purified by column chromatography on $SiO_2$ (DCM: MeOH = 0 to 10%) to afford compound BnH12070 (46.0 mg, 55.4%) as a pale yellow solid.

$^1$H NMR (400 MHz, DMSO-$d_6$) δ 12.26 (s, 1H), 6.88-6.81 (dd, *J* = 17.1, 7.9 Hz, 2H), 6.50-6.47 (m, 2H), 4.91 (s, 2H), 4.71 (s, 0.5H), 4.52-4.48 (m, 0.5H), 4.19 (s, 0.5H), 3.86-3.83 (d, *J* = 13.9 Hz, 0.5H), 3.54-3.46 (m, *J* = 5.4 Hz, 2H), 2.97-2.90 (t, *J* = 12.9 Hz, 0.5H), 2.58-2.55 (d, *J* = 12.7 Hz, 0.5H), 2.14-2.00 (m, 1H), 1.76-1.72 (m, 1H), 1.63-1.60 (d, *J* = 13.2 Hz, 1H), 1.48-1.41 (d, *J*= 16.0 Hz, 2H), 1.02-0.99 (t, *J* = 6.1 Hz, 3H).

**(26) Synthesis of compound BnH12073**

(3R,6S)-6-methyl-1-(2-(3-nitrophenyl)acetyl)piperidine-3-carboxylic acid

**Step 1:** Synthesis of methyl (3R,6S)-6-methyl-1-(2-(3-nitrophenyl)acetyl)piperidine-3-carboxylate

To a solution of 2-(3-nitrophenyl)acetic acid (200 mg, 1.10 mmol) in DMF (3.68 mL) were added methyl (3R,6S)-6-methylpiperidine-3-carboxylate (182 mg, 1.16 mmol), DIPEA (0.289 mL, 1.66 mmol), HOBt (179 mg, 1.33 mmol), and EDC·HCl (206 mg, 1.33 mmol). The mixture was stirred at room temperature for 2 hours. The mixture was diluted with EtOAc (8.00 mL) and washed with saturated aqueous $NH_4Cl$ (8.00 mL) and brine (8.00 mL). The organic layer was dried over $MgSO_4$, filtered, and concentrated under reduced pressure. The residue was purified by column chromatography on $SiO_2$ (n-hexane:EtOAc = 2:1 to 1:1) to afford the desired compound (210 mg, 59.4%) as a pale yellow oil.

**Step 2:** Synthesis of compound BnH12073

To a solution of methyl (3R,6S)-6-methyl-1-(2-(3-nitrophenyl)acetyl)piperidine-3-carboxylate (210 mg, 0.656 mmol) in THF (2.26 mL) was added 1 N NaOH (0.983 mL, 0.983 mmol). The mixture was stirred at room temperature for 2 hours. The mixture was diluted with $H_2O$ (5.00 mL) and washed twice with DCM (6.00 mL × 2). The aqueous layer was acidified with 1 N HCl until pH 1 to 2 at 0°C and extracted with EtOAc (5.00 mL). The organic layer was washed with brine (5.00 mL), dried over $MgSO_4$, filtered, and concentrated under reduced pressure to afford compound BnH12073 (173 mg, 86.2%) as a white solid.

$^1$H NMR (400 MHz, DMSO-$d_6$) δ 12.45 (s, 1H), 8.13-8.09 (m, 2H), 7.70-7.58 (m, 2H), 4.72 (s, 0.5H), 4.53-4.49 (m, 0.5H), 4.30 (s, 0.5H), 4.01-3.84 (m, 2.5H), 3.12-3.05 (t, *J* = 12.8 Hz, 0.5H), 2.67-2.60 (m, 0.5H), 2.33-2.20 (m, 1H), 1.83-1.80 (d, *J* = 11.9 Hz, 1H), 1.69-1.63 (d, *J* = 11.2 Hz, 1H), 1.57-1.53 (d, *J* = 16.0 Hz, 2H), 1.16-1.05 (dd, *J* = 34.1, 6.9 Hz, 3H).

**(27) Synthesis of compound BnH12074**

(3R,6S)-1-(2-(4-aminophenyl)acetyl)-6-methylpiperidine-3-carboxylic acid

**Step 1:** Synthesis of compound BnH12074

To a solution of BnH12073 (98.0 mg, 0.320 mmol) in MeOH:THF (1:1, 2.14 mL) was added 10 wt% palladium on carbon (9.80 mg) in an ice-water bath under $N_2$ gas at 0°C. The mixture was degassed and purged with $H_2$ gas. After removing the cooling bath, the mixture was stirred vigorously at room temperature under $H_2$ gas for 2 hours. The reaction mixture was filtered through a pad of Celite and washed with MeOH (5.00 mL). The filtrate was concentrated under reduced pressure. The residue was purified by column chromatography on $SiO_2$ (DCM: MeOH = 0 to 10%) to afford compound BnH12074 (52.0 mg, 58.8%) as a pale yellow solid.

$^1$H NMR (400 MHz, DMSO-$d_6$) δ 12.37 (s, 1H), 6.95-6.90 (td, *J* = 7.8, 4.1 Hz, 1H), 6.43-6.35 (m, 3H), 5.02 (s, 2H), 4.74-4.72 (s, 0.5H), 4.55-4.51 (d, 0.5H), 4.15 (s, 0.5H), 3.83-3.79 (d, *J* = 14.4 Hz, 0.5H), 3.58-3.48 (m, 2H), 2.98-2.92 (t, *J* = 12.9 Hz, 0.5H), 2.60-2.54 (t, *J* = 12.7 Hz, 0.5H), 2.18-2.09 (m, 1H), 1.78-1.74 (d, *J* = 13.0 Hz, 1H), 1.67-1.62 (m, *J* = 13.6 Hz, 1H), 1.50-1.44 (d, *J* = 27.9 Hz, 2H), 1.04-1.00 (dd, *J* = 10.0, 6.8 Hz, 3H).

**(28) Synthesis of compound BnH12075**

(3R,6S)-1-(2-(6-methoxypyridin-3-yl)acetyl)-6-methylpiperidine-3-carboxylic acid

**Step 1:** Synthesis of methyl (3R,6S)-1-(2-(6-methoxypyridin-3-yl)acetyl)-6-methylpiperidine-3-carboxylate

To a solution of 2-(6-methoxypyridin-3-yl)acetic acid (100 mg, 0.598 mmol) in DMF (1.99 mL) were added methyl (3R,6S)-6-methylpiperidine-3-carboxylate (98.8 mg, 0.628 mmol), DIPEA (0.157 mL, 0.897 mmol), HOBt (97.0 mg, 0.718 mmol), and EDC·HCl (111 mg, 0.718 mmol). The mixture was stirred at room temperature for 3 hours. The mixture was diluted with EtOAc (5.00 mL) and washed with saturated aqueous $NH_4Cl$ (5.00 mL) and brine (5.00 mL). The organic layer was dried over $MgSO_4$, filtered, and concentrated under reduced pressure. The residue was purified by column chromatography on $SiO_2$ (n-hexane:EtOAc = 1:1) to afford the desired compound (87.0 mg, 47.5%) as a colorless oil.

**Step 2:** Synthesis of compound BnH12075

To a solution of methyl (3R,6S)-1-(2-(6-methoxypyridin-3-yl)acetyl)-6-methylpiperidine-3-carboxylate (87.0 mg, 0.284 mmol) in THF (0.979 mL) was added 1 N NaOH (0.426 mL, 0.426 mmol). The mixture was stirred at room temperature for 2 hours. The mixture was diluted with $H_2O$ (5.00 mL) and washed twice with DCM (5.00 mL × 2). The aqueous layer was acidified with 1 N HCl until pH 3 to 4 at 0°C and extracted with EtOAc (5.00 mL). The organic layer was washed with brine (5.00 mL), dried over $MgSO_4$, filtered, and concentrated under reduced pressure to afford compound BnH12075 (51.4 mg, 61.9%) as a white solid.

$^1H$ NMR (400 MHz, DMSO-$d_6$) δ 12.43 (s, 1H), 7.99-7.95 (d, *J* = 16.8 Hz, 1H), 7.55-7.49 (dt, *J* = 9.9, 5.5 Hz, 1H), 6.76-6.74 (d, *J* = 8.5 Hz, 1H), 4.71 (s, 0.5H), 4.52-4.49 (d, *J* = 13.6 Hz, 0.5H), 4.28 (s, 0.5H), 3.94-3.91 (d, *J* = 13.9 Hz, 0.5H), 3.74-3.59 (m, 2H), 3.09-3.02 (t, *J* = 12.9 Hz, 0.5H), 2.64-2.57 (t, *J* = 12.8 Hz, 0.5H), 2.26-2.17 (d, *J* = 33.1 Hz, 1H), 1.82-1.79 (d, *J* = 13.2 Hz, 1H), 1.67-1.61 (d, *J* = 12.6 Hz, 1H), 1.56-1.53 (m, 2H), 1.13-1.03 (dd, *J* = 32.5, 6.9 Hz, 3H).

**(29) Synthesis of compound BnH12076**

(3R,6S)-1-(2-(4-(dimethylamino)phenyl)acetyl)-6-methylpiperidine-3-carboxylic acid

**Step 1:** Synthesis of methyl (3R,6S)-1-(2-(4-(dimethylamino)phenyl)acetyl)-6-methylpiperidine-3-carboxylate

To a solution of 2-(4-(dimethylamino)phenyl)acetic acid (100 mg, 0.558 mmol) in DMF (1.86 mL) were added methyl (3R,6S)-6-methylpiperidine-3-carboxylate (92.1 mg, 0.586 mmol), DIPEA (0.146 mL, 0.837 mmol), HOBt (90.5 mg, 0.670 mmol), and EDC·HCl (104 mg, 0.670 mmol). The mixture was stirred at room temperature for 2 hours. The mixture was diluted with EtOAc (5.00 mL) and washed with saturated aqueous $NH_4Cl$ (5.00 mL) and brine (5.00 mL). The organic layer was dried over $MgSO_4$, filtered and concentrated under reduced pressure. The residue was purified by column chromatography on $SiO_2$ (n-hexane:EtOAc = 2:1 to 1:1) to afford the desired compound (142 mg, 79.9%) as a pale yellow oil.

**Step 2:** Synthesis of compound BnH12076

To a solution of methyl (3R,6S)-1-(2-(4-(dimethylamino)phenyl)acetyl)-6-methylpiperidine-3-carboxylate (142 mg, 0.446 mmol) in THF (1.54 mL) was added 1 N NaOH (0.669 mL, 0.669 mmol). The mixture was stirred at room temperature for 2 hours. The mixture was diluted with $H_2O$ (5.00 mL) and washed twice with DCM (5.00 mL × 2). The aqueous layer was acidified with 1 N HCl until pH 3 to 4 at 0°C and extracted with EtOAc (5.00 mL). The organic layer was washed with brine (5.00 mL), dried over $MgSO_4$, filtered, and concentrated under reduced pressure to afford compound BnH12076 (69.0 mg, 50.8%) as a white solid.

$^1H$ NMR (400 MHz, DMSO-$d_6$) δ 12.42 (s, 1H), 7.05-6.99 (dd, *J* = 18.3, 8.1 Hz, 2H), 6.68-6.66 (d, *J* = 8.2 Hz, 2H), 4.72 (s, 0.5H), 4.54-4.50 (m, 0.5H), 4.23-4.20 (d, *J* = 9.0 Hz, 0.5H), 3.88-3.85 (d, *J* = 13.9 Hz, 0.5H), 3.62-3.52 (m, 2H), 2.98-2.91 (t, *J* = 12.7 Hz, 0.5H), 2.85 (s, 6H), 2.60-2.53 (t, *J* = 12.6 Hz, 0.5H), 2.13-2.09 (m, 1H), 1.78-1.74 (d, *J* = 12.7 Hz, 1H), 1.68-1.58 (q, *J* = 13.0 Hz, 1H), 1.50-1.40 (d, *J* = 16.3 Hz, 2H), 1.03-1.00 (dd, *J* = 7.2, 2.9 Hz, 3H).

**(30) Synthesis of compound BnH12079**

(3R,6S)-1-(2-(4-hydroxyphenyl)acetyl)-6-methylpiperidine-3-carboxylic acid

**Step 1:** Synthesis of 2-(4-hydroxyphenyl)acetic acid

To a suspension of 2-(4-hydroxyphenyl)acetic acid (500 mg, 3.01 mmol) in DCM (5.02 mL) was added 1 M $BBr_3$ solution (6.02 mL, 6.02 mmol) under $N_2$ gas at -78°C, and the mixture stirred for 5 hours while heating from -78°C to -15°C. The reaction mixture was quenched with $H_2O$ (7.50 mL), and the resulting solid was filtered and washed with DCM (5.00 mL) to afford the desired compound (128 mg, 28.0%) as a pale pink solid.

**Step 2:** Synthesis of methyl (3R,6S)-1-(2-(4-hydroxyphenyl)acetyl)-6-methylpiperidine-3-carboxylate

To a solution of 2-(4-hydroxyphenyl)acetic acid (72.0 mg, 0.473 mmol) in DMF (1.58 mL) were added methyl (3R,6S)-6-methylpiperidine-3-carboxylate (78.1 mg, 0.497 mmol), DIPEA (0.124 mL, 0.710 mmol), HOBt (76.7 mg, 0.568 mmol), and EDC·HCl (88.2 mg, 0.568 mmol). The mixture was stirred at room temperature for 2 hours. The mixture was diluted with EtOAc (4.00 mL) and washed with saturated aqueous $NH_4Cl$ (4.00 mL) and brine (4.00 ml). The organic layer was dried over $MgSO_4$, filtered, and concentrated under reduced pressure. The residue was purified by column chromatography on $SiO_2$ (DCM: MeOH = 0 to 2%) to afford the desired compound (78.0 mg, 56.6%) as a pale yellow viscous solid.

**Step 3:** Synthesis of compound BnH12079

To a solution of methyl (3R,6S)-1-(2-(4-hydroxyphenyl)acetyl)-6-methylpiperidine-3-carboxylate (78.0 mg, 0.268 mmol) in THF (0.923 mL) was added 1 N NaOH (0.402 mL, 0.402 mmol). The mixture was stirred at room temperature for 2 hours. The mixture was diluted with $H_2O$ (4.00 mL) and washed three times with DCM (4.00 mL × 3). The aqueous layer was acidified with 1 N HCl until pH 1 to 2 at 0°C and extracted with EtOAc (4.00 mL). The organic layer was washed with brine (4.00 mL), dried over $MgSO_4$, filtered, and concentrated under reduced pressure to afford compound BnH12079 (69.0 mg, 50.8%) as a white solid.

$^1H$ NMR (400 MHz, DMSO-$d_6$) δ 12.37 (s, 1H), 9.25 (s, 1H), 7.05-6.97 (dd, *J* = 17.6, 7.9 Hz, 2H), 6.70-6.68 (d, *J* = 7.9 Hz, 2H), 4.73 (s, 0.5H), 4.55-4.50 (m, 0.5H), 4.21 (s, 0.5H), 3.88-3.84 (d, *J* = 13.8 Hz, 0.5H), 3.64-3.54 (d, *J* = 6.2 Hz, 2H), 3.00-2.94 (t, *J* = 12.9 Hz, 0.5H), 2.61-2.55 (d, *J* = 12.5 Hz, 0.5H), 2.16-2.07 (m, 1H), 1.79-1.75 (d, *J* = 12.9 Hz, 1H), 1.65-1.62 (d, *J* = 13.2 Hz, 1H), 1.50-1.47 (d, *J* = 15.1 Hz, 2H), 1.04-1.01 (t, *J* = 5.5 Hz, 3H).

**Preparation Example 2. Preparation of active compounds according to representative reaction scheme 2**

[Reaction scheme 2]

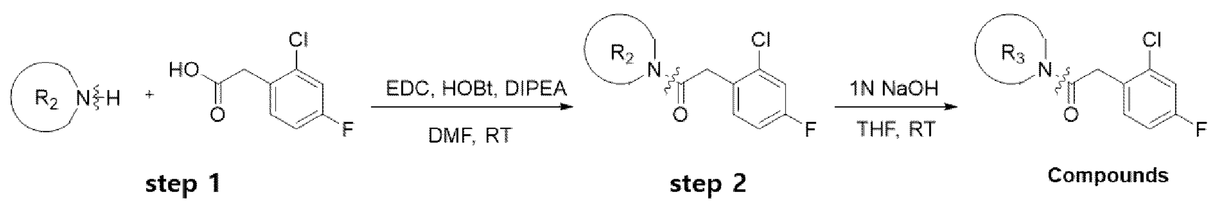

| Compound | $R_2$ | $R_3$ |
|---|---|---|
| BnH12001 | 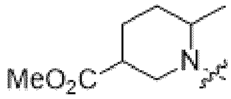 | 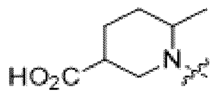 |
| BnH12004 | 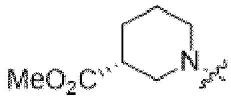 | 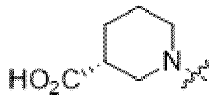 |
| BnH12005 | 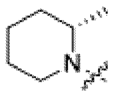 | - |
| BnH12012 | 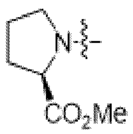 | 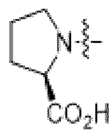 |
| BnH12013 | 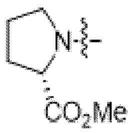 | 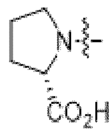 |

**(1) Synthesis of compound BnH12001(12-004)**

1-(2-(2-chloro-4-fluorophenyl)acetyl)-6-methylpiperidine-3-carboxylic acid

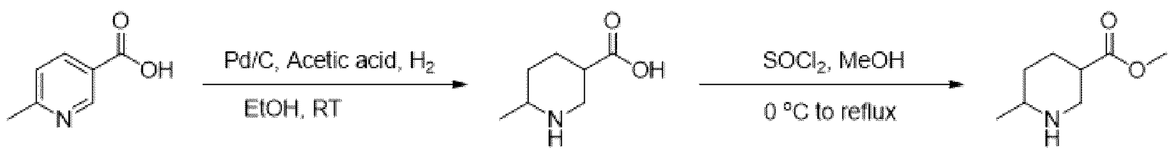


step 1 step 2 12-004

**Step 1:** Synthesis of 6-methylpiperidine-3-carboxylic acid (c)

To a solution of 6-methylnicotinic acid (500 mg, 3.65 mmol) in EtOH (6.63 mL) were added acetic acid (0.663 mL, 3.65 mmol) and Pd/C (272 mg, 2.55 mmol). The reaction mixture was degassed and purged with $H_2$ gas three times. The mixture was then stirred at room temperature for 3 days and at 50°C for 16 hours. After cooling to room temperature, the mixture was filtered through Celite and washed with MeOH (10.0 mL). The filtrate was concentrated under reduced pressure and used without further purification.

**Step 2:** Methyl 6-methylpiperidine-3-carboxylate (2-1-12-004)

To a solution of 6-methylpiperidine-3-carboxylic acid (crude) in MeOH (8.10 mL) was added $SOCl_2$ (0.798 mL, 10.9 mmol) dropwise at 0°C and refluxed at 70°C for 2 hours. The mixture was concentrated and the residue was dissolved in EtOAc (10.0 mL) and washed with saturated $NaHCO_3$ (10.0 mL). The organic layer was dried over $MgSO_4$, filtered, and concentrated under reduced pressure to afford methyl 6-methylpiperidine-3-carboxylate (180 mg, 31%, 2 step) as a brown oil. The desired compound was used without further purification.

**Step 3:** Synthesis of methyl 1-(2-(2-chloro-4-fluorophenyl)acetyl)-6-methylpiperidine-3-carboxylate

To a solution of 2-chloro-4-fluorophenylacetic acid (200 mg, 1.06 mmol) in DMF (3.53 mL) were added methyl 6-methylpiperidine-3-carboxylate (175 mg, 1.11 mmol), DIPEA (0.277 mL, 1.59 mmol), HOBt (172 mg, 1.27 mmol), and EDC·HCl (198 mg, 1.27 mmol). The mixture was stirred at room temperature for 3 hours and 30 minutes. The mixture was diluted with EtOAc (4.00 mL) and washed with saturated aqueous $NH_4Cl$ (4.00 mL) and brine (4.00 ml). The organic layer was dried over $MgSO_4$, filtered, and concentrated under reduced pressure. The residue was purified by column chromatography on $SiO_2$ (n-hexane:EtOAc = 3:1 to 2:1) to afford the desired compound (227 mg, 65%) as a colorless oil.

**Step 4:** Synthesis of Compound 12-004

To a solution of methyl 1-(2-(2-chloro-4-fluorophenyl)acetyl)-6-methylpiperidine-3-carboxylate (220 mg, 0.702 mmol) in THF (2.42 mL) was added 1 N NaOH (1.05 mL, 1.05 mmol). The mixture was stirred at room temperature for 2 hours. The mixture was diluted with $H_2O$ (3.00 mL) and washed twice with DCM (5.00 mL × 2). The aqueous layer was acidified with 1 N HCl until pH 1 to 2 at 0°C and extracted with EtOAc (5.00 mL). The organic layer was washed with brine (5.00 mL), dried over $MgSO_4$, filtered, and concentrated under reduced pressure. Et2O (5.00 mL) was added to the residue and stirred at room temperature for 1 hour. The precipitate was filtered and washed with Et2O (2.00 mL) to afford compound 12-004 (150 mg, 71%) as a white solid.

$^1H$ NMR (400 MHz, DMSO-d6) δ 12.48 (s, 1H) 7.43 - 7.22 (m, 2H), 7.21 - 7.10 (m, 1H), 4.66 (d, J = 29.7 Hz, 0.7H), 4.49 (d, J = 12.5 Hz, 0.3H), 4.27 (s, 0.3H), 4.03 (d, J = 7.2 Hz, 0.5H), 3.97 - 3.68 (m, 2.5H), 3.14 (t, J = 12.9 Hz, 0.5H), 2.71 - 2.57 (m, 1H), 2.34 (d, J = 8.9 Hz, 0.25H), 2.21 (s, 0.25H), 1.87 (t, J = 16.7 Hz, 2H), 1.77 - 1.50 (m, 2H), 1.41 - 1.34 (m, 0.5H), 1.27 - 1.00 (m, 3.5H).

**(2) Synthesis of compound BnH12004**

(R)-1-(2-(2-chloro-4-fluorophenyl)acetyl)piperidine-3-carboxylic acid

**Step 1:** Synthesis of methyl (R)-1-(2-(2-chloro-4-fluorophenyl)acetyl)piperidine-3-carboxylate

To a solution of 2-chloro-4-fluorophenylacetic acid (100 mg, 0.530 mmol) in DMF (1.77 mL) were added methyl (R)-piperidine-3-carboxylate (79.7 mg, 0.557 mmol), DIPEA (0.139 mL, 0.795 mmol), HOBt (86.0 mg, 0.636 mmol), and EDC·HCl (98.8 mg, 0.636 mmol). The mixture was stirred at room temperature for 3 hours and 30 minutes. The mixture was diluted with EtOAc (3.00 mL) and washed with saturated aqueous $NH_4Cl$ (3.00 mL) and brine (3.00 ml). The organic layer was dried over $MgSO_4$, filtered, and concentrated under reduced pressure. The residue was purified by column chromatography on $SiO_2$ (n-hexane:EtOAc = 3:1 to 2:1) to afford the desired compound (128 mg, 76%) as a colorless oil.

**Step 2:** Synthesis of compound BnH12004

To a solution of methyl (R)-1-(2-(2-chloro-4-fluorophenyl)acetyl)piperidine-3-carboxylate (128 mg, 0.408 mmol) in THF (1.41 mL) was added 1 N NaOH (0.612 mL, 0.612 mmol). The mixture was stirred at room temperature for 2 hours. The mixture was diluted with $H_2O$ (1.50 mL) and washed twice with DCM (3.00 mL × 2). The aqueous layer was acidified with 1 N HCl until pH 1 to 2 at 0°C and extracted with EtOAc (3.00 mL). The organic layer was washed with brine (3.00 mL), dried over $MgSO_4$, filtered, and concentrated under reduced pressure. The residue was purified by column chromatography on $SiO_2$ (DCM:MeOH= 95:5 to 90:10) to afford compound BnH12004 (83.0 mg, 67%) as a white solid.

$^1H$ NMR (400 MHz, DMSO-$d_6$) δ 7.39 (dt, *J* = 9.0, 3.1 Hz, 1H), 7.32 (ddd, *J* = 11.3, 8.6, 6.3 Hz, 1H), 7.16 (td, *J* = 8.5, 2.7 Hz, 1H), 4.42 - 4.31 (m, 0.5H), 3.87 - 3.76 (m, 4H), 3.53 - 3.41 (m, 2H), 3.12 - 3.02 (m, 1.5H), 2.78 (dd, *J* = 12.9, 10.6 Hz, 0.5H), 2.31 - 2.21 (m, 0.5H), 2.02 - 1.87 (m, 1H), 1.70 (d, *J* = 11.9 Hz, 1H), 1.57 (dt, *J* = 11.6, 6.0 Hz, 1H), 1.49 - 1.30 (m, 1H).

**(3) Synthesis of compound BnH12005**

(S)-2-(2-chloro-4-fluorophenyl)-1-(2-methylpiperidin-1-yl)ethan-1-one

**Step 1:** Synthesis of compound BnH12005

To a solution of 2-chloro-4-fluorophenylacetic acid (100 mg, 0.530 mmol) in DMF (1.77 mL) were added (S)-2-methylpiperidine (55.2 mg, 0.557 mmol), DIPEA (0.139 mL, 0.795 mmol), HOBt (86.0 mg, 0.636 mmol), and EDC·HCl (98.8 mg, 0.636 mmol). The mixture was stirred at room temperature for 3 hours and 30 minutes. The mixture was diluted with EtOAc (3.00 mL) and washed with saturated aqueous $NH_4Cl$ (3.00 mL) and brine (3.00 ml). The organic layer was dried over $MgSO_4$, filtered, and concentrated under reduced pressure. The residue was purified by column chromatography on $SiO_2$ (n-hexane:EtOAc = 3:1 to 2:1) to afford compound BnH12005 (112 mg, 78%) as a colorless oil.

$^1$H NMR (400 MHz, DMSO-$d_6$) δ 7.39 (dd, *J* = 8.9, 2.7 Hz, 1H), 7.33 (s, 1H), 7.16 (td, *J* = 8.5, 2.7 Hz, 1H), 4.70 (s, 0.5H), 4.25 (s, 0.5H), 3.77 (d, *J* = 39.4 Hz, 2.5H), 3.14 (d, *J* = 14.1 Hz, 0.5H), 2.71 - 2.60 (m, 0.5H), 1.68 - 1.45 (m, 5H), 1.38 - 1.04 (m, 4H).

**(4) Synthesis of compound BnH12012**

(2-(2-chloro-4-fluorophenyl)acetyl)-D-proline

**Step 1:** Synthesis of methyl (2-(2-chloro-4-fluorophenyl)acetyl)-D-prolinate

To a solution of 2-chloro-4-fluorophenylacetic acid (100 mg, 0.530 mmol) in DMF (1.77 mL) were added methyl D-prolinate (82.2 mg, 0.636 mmol), DIPEA (0.140 mL, 0.795 mmol), HOBt (86.0 mg, 0.636 mmol), and EDC·HCl (99.0 mg, 0.636 mmol). The mixture was stirred at room temperature for 3 hours and 30 minutes. The mixture was diluted with EtOAc (3.00 mL) and washed with saturated aqueous $NH_4Cl$ (3.00 mL) and brine (3.00 ml). The organic layer was dried over $MgSO_4$, filtered, and concentrated under reduced pressure. The residue was purified by column chromatography on $SiO_2$ (n-hexane:EtOAc = 2:1) to afford the desired compound (112 mg, 70%) as a colorless oil.

**Step 2:** Synthesis of compound BnH12012

To a solution of methyl (2-(2-chloro-4-fluorophenyl)acetyl)-D-prolinate (112 mg, 0.357 mmol) in THF (1.23 mL) was added 1 N NaOH (0.535 mL, 0.535 mmol). The mixture was stirred at room temperature for 2 hours. The mixture was diluted with $H_2O$ (2.00 mL) and washed twice with DCM (3.00 mL × 2). The aqueous layer was acidified with 1 N HCl until pH 1 to 2 at 0°C and extracted with EtOAc (3.00 mL). The organic layer was washed with brine (3.00 mL), dried over $MgSO_4$, filtered, and concentrated under reduced pressure to afford compound BnH12012 (24.0 mg, 22%) as a white solid.

$^1$H NMR (400 MHz, Chloroform-*d*) δ 7.31 (dd, *J* = 8.6, 5.9 Hz, 1H), 7.16 (dd, *J* = 8.4, 2.6 Hz, 1H), 6.99 (td, *J* = 8.3, 2.7 Hz, 1H), 4.68 - 4.62 (m, 1H), 3.80 (d, *J*= 2.8 Hz, 2H), 3.68 (ddd, *J* = 10.7, 7.4, 3.6 Hz, 1H), 3.63 - 3.55 (m, 1H), 2.55 - 2.47 (m, 1H), 2.20 - 1.96 (m, 3H).

**(5) Synthesis of compound BnH12013**

(2-(2-chloro-4-fluorophenyl)acetyl)-L-proline

**Step 1:** Synthesis of methyl (2-(2-chloro-4-fluorophenyl)acetyl)-L-prolinate

To a solution of 2-chloro-4-fluorophenylacetic acid (100 mg, 0.530 mmol) in DMF (1.77 mL) were added methyl L-prolinate (71.9 mg, 0.557 mmol), DIPEA (0.140 mL, 0.795 mmol), HOBt (86.0 mg, 0.636 mmol), and EDC·HCl (99.0 mg, 0.636 mmol). The mixture was stirred at room temperature for 3 hours and 30 minutes. The mixture was diluted with EtOAc (3.00 mL) and washed with saturated aqueous $NH_4Cl$ (3.00 mL) and brine (3.00 ml). The organic layer was dried over $MgSO_4$, filtered, and concentrated under reduced pressure. The residue was purified by column chromatography on $SiO_2$ (n-hexane:EtOAc = 2:1) to afford the desired compound (126 mg, 79%) as a colorless oil.

**Step 2:** Synthesis of compound BnH12013

To a solution of methyl (2-(2-chloro-4-fluorophenyl)acetyl)-L-prolinate (126 mg, 0.420 mmol) in THF (1.45 mL) was added 1 N NaOH (0.631 mL, 0.631 mmol). The mixture was stirred at room temperature for 2 hours. The mixture was

diluted with $H_2O$ (2.00 mL) and washed twice with DCM (3.00 mL × 2). The aqueous layer was acidified with 1 N HCl until pH 1 to 2 at 0°C and extracted with EtOAc (3.00 mL). The organic layer was washed with brine (3.00 mL), dried over $MgSO_4$, filtered, and concentrated under reduced pressure to afford compound BnH12013 (26.0 mg, 21%) as a white solid.

$^1H$ NMR (400 MHz, Chloroform-d) δ 7.30 (dd, *J* = 8.6, 5.9 Hz, 1H), 7.16 (dd, *J* = 8.4, 2.6 Hz, 1H), 7.00 (td, *J* = 8.3, 2.7 Hz, 1H), 4.68 - 4.62 (m, 1H), 3.80 (d, *J* = 2.7 Hz, 2H), 3.68 (td, *J* = 8.9, 7.6, 3.5 Hz, 1H), 3.63 - 3.55 (m, 1H), 2.57 - 2.46 (m, 1H), 2.20 - 1.90 (m, 3H).

**Preparation Example 3. Preparation of active compounds according to representative reaction scheme 4**

[Reaction scheme 4]

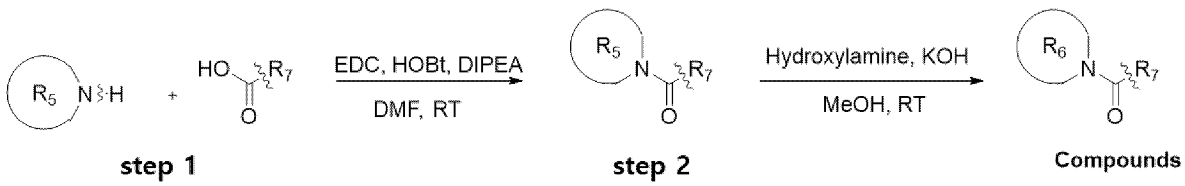


| Compound | $R_5$ | $R_6$ | $R_7$ |
|---|---|---|---|
| BnH12014 | 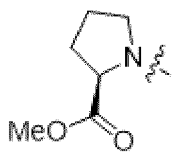 | 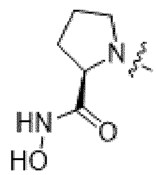 | 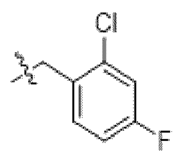 |
| BnH12016 | 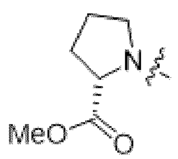 | 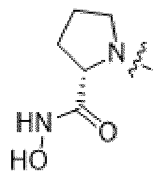 | 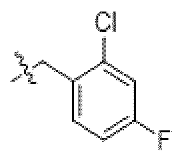 |
| BnH12009 | 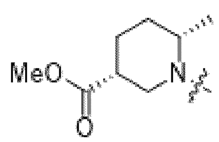 | 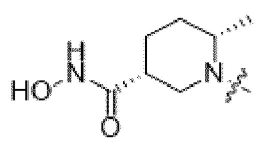 | 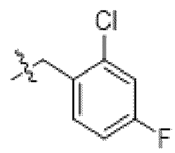 |
| BnH12018 | 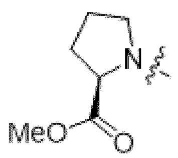 | 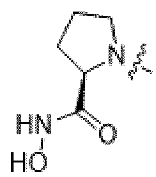 | 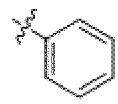 |
| BnH12019 | 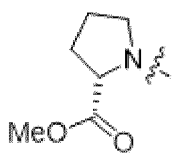 | 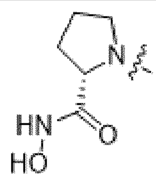 | 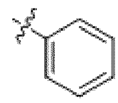 |

**(1) Synthesis of compound BnH12014**

(R)-1-(2-(2-chloro-4-fluorophenyl)acetyl)-N-hydroxypyrrolidine-2-carboxamide

**Step 1:** Synthesis of methyl (2-(2-chloro-4-fluorophenyl)acetyl)-D-prolinate

To a solution of 2-chloro-4-fluoroacetic acid (100 mg, 0.530 mmol) in DMF (1.77 mL) were added methyl D-prolinate (105 mg, 0.636 mmol), DIPEA (0.139 mL, 0.795 mmol), HOBt (86.0 mg, 0.636 mmol), and EDC·HCl (98.8 mg, 0.636 mmol). The mixture was stirred at room temperature for 3 hours and 30 minutes. The mixture was diluted with EtOAc (4.00 mL) and washed with saturated aqueous $NH_4Cl$ (4.00 mL) and brine (4.00 ml). The organic layer was dried over $MgSO_4$, filtered, and concentrated under reduced pressure. The residue was purified by column chromatography on $SiO_2$ (n-hexane:EtOAc = 2:1) to afford the desired compound (113 mg, 71%) as a colorless oil.

**Step 2:** Synthesis of compound BnH12014

To a solution of methyl (2-(2-chloro-4-fluorophenyl)acetyl)-D-prolinate (100 mg, 0.334 mmol) in MeOH (1.50 mL) and THF (1.50 mL) were added hydroxylamine hydrochloride (0.197 mL, 3.34 mmol) and KOH (37.4 mg, 0.667 mmol). The mixture was stirred at room temperature for 2 hours. The pH value was adjusted to pH 1 to 2 by adding 1 N HCl to the mixture, and the mixture was extracted with DCM (4.00 mL × 2). The combined organic layers were dried over $MgSO_4$, filtered, and concentrated under reduced pressure. The residue was purified by column chromatography on NH-$SiO_2$ (DCM:MeOH = 9:1) to afford compound BnH12014 (25.0 mg, 24%) as a white solid.

$^1H$ NMR (400 MHz, Chloroform-d) δ 9.92 (s, 1H), 7.34 (dd, J = 8.5, 6.0 Hz, 1H), 7.14 (dd, J = 8.5, 2.6 Hz, 1H), 6.99 (td, J = 8.3, 2.7 Hz, 1H), 4.52 (d, J = 7.8 Hz, 1H), 3.72 (s, 2H), 3.56 (dd, J = 17.2, 8.7 Hz, 2H), 2.42 (s, 1H), 2.22 - 2.09 (m, 1H), 2.08 - 1.90 (m, 2H).

**(2) Synthesis of compound BnH12016**

(S)-1-(2-(2-chloro-4-fluorophenyl)acetyl)-N-hydroxypyrrolidine-2-carboxamide

**Step 1:** Synthesis of methyl (2-(2-chloro-4-fluorophenyl)acetyl)-L-prolinate

To a solution of 2-chloro-4-fluoroacetic acid (100 mg, 0.530 mmol) in DMF (1.77 mL) were added methyl L-prolinate (105 mg, 0.636 mmol), DIPEA (0.139 mL, 0.795 mmol), HOBt (86.0 mg, 0.636 mmol), and EDC·HCl (98.8 mg, 0.636 mmol). The mixture was stirred at room temperature for 3 hours and 30 minutes. The mixture was diluted with EtOAc (4.00 mL) and washed with saturated aqueous $NH_4Cl$ (4.00 mL) and brine (4.00 ml). The organic layer was dried over $MgSO_4$, filtered, and concentrated under reduced pressure. The residue was purified by column chromatography on $SiO_2$ (n-hexane:EtOAc = 2:1) to afford the desired compound (128 mg, 80%) as a colorless oil.

**Step 2:** Synthesis of compound BnH12016

To a solution of methyl (2-(2-chloro-4-fluorophenyl)acetyl)-L-prolinate (128 mg, 0.427 mmol) in MeOH (1.50 mL) and THF (1.50 mL) were added hydroxylamine hydrochloride (59.4 mg, 0.854 mmol) and KOH (95.85 mg, 1.71 mmol). The mixture was stirred at room temperature for 17 hours. The pH value was adjusted to pH 1 to 2 by adding 1 N HCl to the mixture, and the mixture was extracted with DCM (4.00 mL × 2). The combined organic layers were dried over $MgSO_4$, filtered, and concentrated under reduced pressure. The residue was purified by column chromatography on NH-$SiO_2$ (DCM:MeOH = 9:1) to afford compound BnH12016 (19.0 mg, 14%) as a white solid.

$^1H$ NMR (400 MHz, Chloroform-d) δ 9.87 (s, 1H), 7.35 - 7.27 (m, 1H), 7.15 (dd, J = 8.4, 2.6 Hz, 1H), 6.99 (td, J = 8.3, 2.7 Hz, 1H), 4.54 (d, J = 8.1 Hz, 1H), 3.73 (d, J = 2.6 Hz, 2H), 3.66 - 3.49 (m, 2H), 2.46 (s, 1H), 2.17 (dd, J = 17.6, 8.5 Hz, 1H), 2.09 - 1.89 (m, 2H).

**(3) Synthesis of compound BnH12009**

(3R,6S)-1-(2-(2-chloro-4-fluorophenyl)acetyl)-N-hydroxy-6-methylpiperidine-3-carboxamide

**Step 1:** Synthesis of compound BnH12009

To a solution of compound BnH12001 enantiomer (3S,6R) (60.0 mg, 0.191 mmol) in THF (0.956 mL) was added CDI (46.5 mg, 0.287 mmol), and stirred at room temperature for 1 hour. To the mixture was added hydroxylamine

hydrochloride (39.9 mg, 0.574 mol), and the mixture was stirred at room temperature for 17 hours. The mixture was diluted with DCM (2.00 mL) and washed with 5% aqueous $KHSO_4$ (2.00 mL). The organic layer was dried over $MgSO_4$, filtered, and concentrated under reduced pressure. The residue was purified by column chromatography on NH-$SiO_2$ (DCM:MeOH = 10:1) to afford compound BnH12009 (32.0 mg, 50%) as a white solid.

$^1$H NMR (400 MHz, Chloroform-d) δ 9.58 (s, 1H), 7.24 (dd, J = 8.7, 6.1 Hz, 2H), 7.13 (dd, J = 8.4, 2.6 Hz, 1H), 6.96 (td, J = 8.2, 2.6 Hz, 1H), 4.54 (d, J = 13.4 Hz, 0.5H), 4.18 (s, 0.5H), 3.89 - 3.61 (m, 2H), 3.49 (s, 2H), 2.87 (t, J = 12.4 Hz, 0.5H), 2.07 (dd, J = 29.7, 12.3 Hz, 2H), 1.68 (d, J = 23.3 Hz, 3H), 1.36 - 1.18 (m, 4H).

**(4) Synthesis of compound BnH12018**

(R)-1-benzoyl-N-hydroxypyrrolidine-2-carboxamide

**Step 1:** Synthesis of methyl benzoyl-D-prolinate

To a solution of benzoic acid (100 mg, 0.819 mmol) in DMF (2.73 mL) were added methyl D-prolinate (163 mg, 0.983 mmol), DIPEA (0.215 mL, 1.23 mmol), HOBt (133 mg, 0.983 mmol), and EDC·HCl (153 mg, 0.983 mmol). The mixture was stirred at room temperature for 3 hours and 30 minutes. The mixture was diluted with EtOAc (4.00 mL) and washed with saturated aqueous $NH_4Cl$ (4.00 mL) and brine (4.00 ml). The organic layer was dried over $MgSO_4$, filtered, and concentrated under reduced pressure. The residue was purified by column chromatography on $SiO_2$ (n-hexane:E-tOAc = 2:1) to afford the desired compound (126 mg, 66%) as a colorless oil.

**Step 2:** Synthesis of compound BnH12018

To a solution of methyl benzoyl-D-prolinate (124 mg, 0.414 mmol) in MeOH (3.00 mL) and THF (3.00 mL) were added hydroxylamine hydrochloride (0.244 mL, 4.14 mmol) and KOH (46.4 mg, 0.827 mmol). The mixture was stirred at room temperature for 2 hours. The pH value was adjusted to pH 1 to 2 by adding 1 N HCl to the mixture, and the mixture was extracted with DCM (4.00 mL × 2). The combined organic layers were dried over $MgSO_4$, filtered, and concentrated under reduced pressure. The residue was purified by column chromatography on NH-$SiO_2$ (DCM:MeOH = 9:1) to afford compound BnH12018 (28.0 mg, 28%) as a white solid.

$^1$H NMR (400 MHz, Chloroform-d) δ 10.04 (s, 1H), 7.52 (d, J = 7.3 Hz, 2H), 7.44 (dq, J = 14.3, 7.3 Hz, 3H), 4.71 (s, 1H), 3.60 - 3.48 (m, 2H), 2.54 (d, J = 6.4 Hz, 1H), 2.12 (d, J = 13.3 Hz, 2H), 1.93 - 1.81 (m, 1H).

**(5) Synthesis of compound BnH12019**

(S)-1-Benzoyl-N-hydroxypyrrolidine-2-carboxamide

**Step 1:** Synthesis of methyl benzoyl-L-prolinate

To a solution of benzoic acid (100 mg, 0.819 mmol) in DMF (2.73 mL) were added methyl L-prolinate (163 mg, 0.983 mmol), DIPEA (0.215 mL, 1.23 mmol), HOBt (133 mg, 0.983 mmol), and EDC·HCl (153 mg, 0.983 mmol). The mixture was stirred at room temperature for 3 hours and 30 minutes. The mixture was diluted with EtOAc (4.00 mL) and washed with saturated aqueous $NH_4Cl$ (4.00 mL) and brine (4.00 ml). The organic layer was dried over $MgSO_4$, filtered, and concentrated under reduced pressure. The residue was purified by column chromatography on $SiO_2$ (n-hexane:E-tOAc = 2:1) to afford the desired compound (145 mg, 75%) as a colorless oil.

**Step 2:** Synthesis of compound BnH12019

To a solution of methyl benzoyl-L-prolinate (140 mg, 0.600 mmol) in MeOH (3.00 mL) and THF (3.00 mL) were added hydroxylamine hydrochloride (0.354 mL, 6.00 mmol) and KOH (67.4 mg, 1.20 mmol). The mixture was stirred at room temperature for 2 hours. The pH value was adjusted to pH 1 to 2 by adding 1 N HCl to the mixture, and the mixture was extracted with DCM (4.00 mL × 2). The combined organic layers were dried over $MgSO_4$, filtered, and concentrated under reduced pressure. The residue was purified by column chromatography on NH-$SiO_2$ (DCM:MeOH = 9:1) to afford compound BnH12019 (38.0 mg, 27%) as a white solid.

$^1$H NMR (400 MHz, Chloroform-d) δ 10.01 (s, 1H), 7.52 (d, J = 7.2 Hz, 2H), 7.44 (dq, J = 14.5, 7.2 Hz, 3H), 4.71 (t, J = 6.4 Hz, 1H), 3.55 (dt, J = 11.5, 6.2 Hz, 2H), 2.54 (d, J = 6.4 Hz, 1H), 2.10 (dp, J = 13.9, 7.1 Hz, 2H), 1.93 - 1.82 (m, 1H).

**Preparation Example 4. Preparation of active compounds according to representative reaction scheme 5**

[Reaction scheme 5]

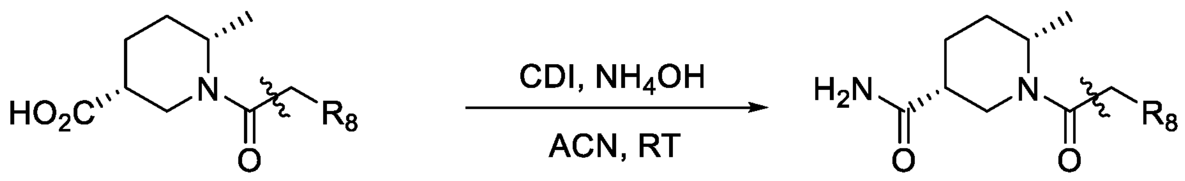


**Compounds** **Compounds**

| Compound | $R_5$ |
|---|---|
| BnH12022 | 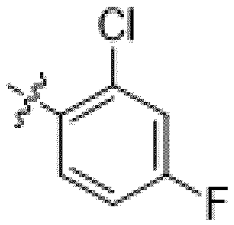 |

**(1) Synthesis of compound BnH12022**

(3R,6S)-1-(2-(2-chloro-4-fluorophenyl)acetyl)-6-methylpiperidine-3-carboxamide

**Step 1:** Synthesis of compound BnH12022

To a suspension of compound BnH12001 enantiomer (3S,6R) (100 mg, 0.319 mmol) in MeCN (3.43 mL) was added CDI (77.5 mg, 0.478 mmol), and stirred at room temperature for 20 minutes. To the mixture was added $NH_4OH$ (0.186 mL, 4.78 mmol), and the mixture was stirred at room temperature for 17 hours. The mixture was diluted with EtOAc (5.00 mL) and washed with saturated $NH_4Cl$ (4.00 mL), 10% citric acid (4.00 mL), and brine (4.00 mL). The organic layer was dried over $MgSO_4$, filtered, and concentrated. The residue was purified by column chromatography (5% MeOH in DCM) to afford compound BnH12022 (84 mg, 84%) as a white solid.

$^1H$ NMR (400 MHz, DMSO-d6) δ 7.43 - 7.26 (m, 3H), 7.16 (tt, J = 8.6, 2.3 Hz, 1H), 6.89 (d, J = 25.2 Hz, 1H), 4.69 (s, 0.5H), 4.32 (dd, J = 20.4, 8.7 Hz, 1H), 3.80 (ddd, J = 36.3, 31.6, 16.2 Hz, 2.5H), 3.19 - 3.08 (m, 0.5H), 2.65 (t, J = 12.6 Hz, 0.5H), 2.33 - 2.21 (m, 0.5H), 2.19 - 2.03 (m, 0.5H), 1.76 - 1.67 (m, 2H), 1.63 - 1.58 (m, 1H), 1.54 (s, 1H), 1.14 (dd, J = 61.2, 6.9 Hz, 3H).

**Preparation Example 5. Preparation of active compounds according to representative reaction scheme 6**

[Reaction scheme 6]

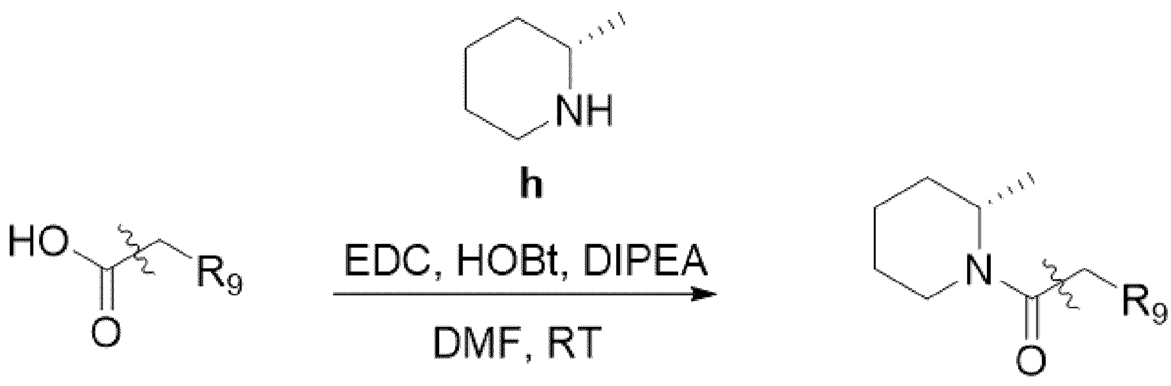


step 1

compounds

| Compound | $R_9$ |
|---|---|
| BnH12060 | 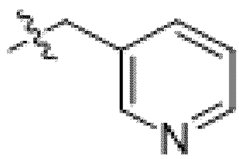 |
| BnH12061 | 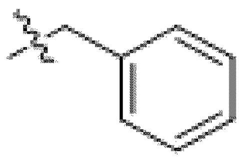 |

**(1) Synthesis of compound BnH12060**

(S)-1-(2-methylpiperidin-1-yl)-2-(pyridin-3-yl)ethan-1-one

**Step 1:** Synthesis of compound BnH12060

To a solution of 3-pyridylacetic acid (100 mg, 0.729 mmol) in DMF (2.43 mL) were added (S)-2-methylpiperidine (75.9 mg, 0.766 mmol), DIPEA (0.191 mL, 1.09 mmol), HOBt (118 mg, 0.875 mmol), and EDC·HCl (136 mg, 0.875 mmol). The mixture was stirred at room temperature for 2 hours. The mixture was diluted with EtOAc (5.00 mL) and washed with saturated aqueous $NH_4Cl$ (5.00 mL) and brine (5.00 mL). The organic layer was dried over $MgSO_4$, filtered, and concentrated under reduced pressure. The residue was purified by column chromatography on $SiO_2$ (0 to 5% MeOH in DCM) to afford compound BnH12060 (54.2 mg, 34%) as an orange oil.

$^1H$ NMR (400 MHz, Chloroform-d) δ 8.54-8.51 (dt, J = 6.3, 3.1 Hz, 2H), 7.69 (s, 1H), 7.33-7.30 (dd, J = 7.9, 4.8 Hz, 1H), 4.96 (s, 0.5H), 4.56-4.52 (d, J = 13.6 Hz, 0.5H), 4.19 (s, 0.5H), 3.80-3.73 (m, 2H), 3.67-3.63 (d, J = 14.1 Hz, 0.5H), 3.17-3.10 (t, J = 13.5 Hz, 0.5H), 2.75-2.68 (t, J = 13.4 Hz, 0.5H), 1.63-1.56 (d, J = 29.3 Hz, 5H), 1.36-1.28 (d, J = 34.4 Hz, 1H), 1.21-1.16 (d, J = 8.2 Hz, 3H).

**(2) Synthesis of compound BnH12061**

(S)-1-(2-methylpiperidin-1-yl)-2-phenylethan-1-one

**Step 1:** Synthesis of compound BnH12061

To a solution of 2-phenylacetic acid (100 mg, 0.734 mmol) in DMF (2.45 mL) were added (S)-2-methylpiperidine (76.5 mg, 0.771 mmol), DIPEA (0.192 mL, 1.10 mmol), HOBt (119 mg, 0.881 mmol), and EDC·HCl (137 mg, 0.881 mmol). The mixture was stirred at room temperature for 2 hours. The mixture was diluted with EtOAc (5.00 mL) and washed with

saturated aqueous $NH_4Cl$ (5.00 mL) and brine (5.00 mL). The organic layer was dried over $MgSO_4$, filtered, and concentrated under reduced pressure. The residue was purified by column chromatography on $SiO_2$ (n-hexane:EtOAc = 2:1) to afford compound BnH12061 (58.7 mg, 37%) as a colorless oil.

$^1H$ NMR (400 MHz, Chloroform-d) δ 7.35 - 7.23 (m, 5H), 5.01-4.97 (d, J = 8.0 Hz, 0.5H), 4.58-4.55 (d, J = 13.6 Hz, 0.5H), 4.17-4.13 (d, J = 9.5 Hz, 0.5H), 3.74 (s, 2H), 3.65-3.61 (d, J = 13.7 Hz, 0.5H), 3.06-2.99 (t, J = 13.5 Hz, 0.5H), 2.71-2.65 (t, J = 13.5 Hz, 0.5H), 1.61-1.44 (m, 6H), 1.16-1.09 (m, 3H).

**Preparation Example 6. Preparation of active compounds according to representative reaction scheme 7 (anhydrous form)**

[Reaction scheme 7]

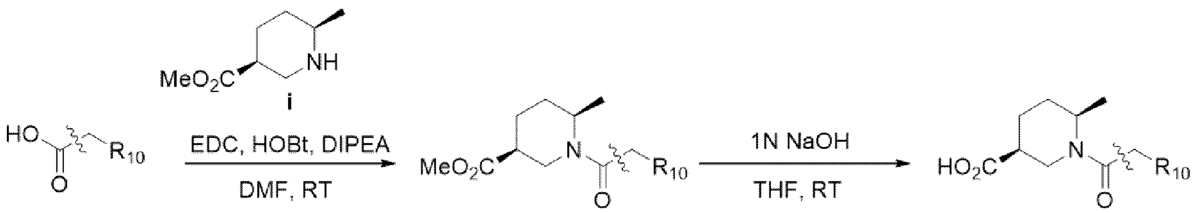


**step 1** **step 2** **Compounds**

| Compound | $R_{10}$ |
|---|---|
| BnH12062 | 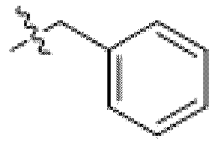 |
| BnH12063 | 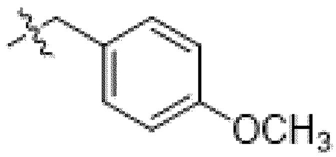 |
| BnH12068 | 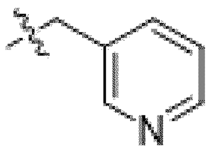 |

**(1) Synthesis of compound BnH12062**

(3S,6R)-6-methyl-1-(2-phenylacetyl)piperidine-3-carboxylic acid

**Step 1:** Synthesis of methyl (3S,6R)-6-methyl-1-(2-phenylacetyl)piperidine-3-carboxylate

To a solution of 2-phenylacetic acid (100 mg, 0.734 mmol) in DMF (2.45 mL) were added methyl (3S,6R)-methyl-6-methylpiperidine-3-carboxylate (anhydrous form) (121 mg, 0.771 mmol), DIPEA (0.192 mL, 1.10 mmol), HOBt (119 mg, 0.881 mmol), and EDC·HCl (137 mg, 0.881 mmol). The mixture was stirred at room temperature for 3 hours. The mixture was diluted with EtOAc (5.00 mL) and washed with saturated aqueous $NH_4Cl$ (5.00 mL) and brine (5.00 mL). The organic layer was dried over $MgSO_4$, filtered, and concentrated under reduced pressure. The residue was purified by column chromatography on $SiO_2$ (n-hexane:EtOAc = 3:1 to 2:1) to afford the desired compound (110 mg, 54%) as a colorless oil.

**Step 2:** Synthesis of compound BnH12062

To a solution of methyl (3S,6R)-6-methyl-1-(2-phenylacetyl)piperidine-3-carboxylate (105 mg, 0.381 mmol) in THF (1.32 mL) was added 1 NNaOH (0.572 mL, 0.572 mmol). The mixture was stirred at room temperature for 2 hours. The mixture was diluted with $H_2O$ (2.00 mL) and washed twice with DCM (3.00 mL × 2). The aqueous layer was acidified with 1 N HCl until pH 1 to 2 at 0°C and extracted with EtOAc (3.00 mL). The organic layer was washed with brine (3.00 mL), dried over $MgSO_4$, filtered, and concentrated under reduced pressure to afford compound BnH12062 (50.5 mg, 51%) as a white solid.

$^1$H NMR (400 MHz, DMSO-d6) δ 12.42 (s, 1H), 7.33-7.30 (t, J = 7.4 Hz, 2H), 7.25-7.19 (dd, J = 16.7, 7.8 Hz, 3H), 4.74 (s, 0.5H), 4.55-4.52 (d, J = 13.4 Hz, 0.5H), 4.24 (s, 0.5H), 3.90-3.86 (d, J = 13.8 Hz, 0.5H), 3.78-3.68 (t, J = 5.8 Hz, 2H), 2.99 (t, J = 12.8 Hz, 0.5H), 2.63-2.57 (d, J = 12.5 Hz, 0.5H), 2.17-2.08 (m, 1H), 1.79-1.76 (d, J = 12.9 Hz, 1H), 1.69-1.59 (m, 1H), 1.52-1.48 (d, J = 13.9 Hz, 2H), 1.05-1.03 (d, J = 6.8 Hz, 3H).

**(2) Synthesis of compound BnH12063**

(3S,6R)-1-(2-(4-methoxyphenyl)acetyl)-6-methylpiperidine-3-carboxylic acid

**Step 1:** Synthesis of methyl (3S,6R)-1-(2-(4-methoxyphenyl)acetyl)-6-methylpiperidine-3-carboxylate

To a solution of 2-(4-methoxyphenyl)acetic acid (100 mg, 0.602 mmol) in DMF (2.01 mL) were added methyl (3S,6R)-6-methylpiperidine-3-carboxylate (anhydrous form) (99.3 mg, 0.632 mmol), DIPEA (0.158 mL, 0.903 mmol), HOBt (97.6 mg, 0.722 mmol), and EDC·HCl (112 mg, 0.722 mmol). The mixture was stirred at room temperature for 1 hour and 30 minutes. The mixture was diluted with EtOAc (5.00 mL) and washed with saturated aqueous $NH_4Cl$ (5.00 mL) and brine (5.00 mL). The organic layer was dried over $MgSO_4$, filtered, and concentrated under reduced pressure. The residue was purified by column chromatography on $SiO_2$ (n-hexane:EtOAc = 2:1) to afford the desired compound (85.2 mg, 46%) as a colorless oil.

**Step 2:** Synthesis of compound BnH12063

To a solution of methyl (3S,6R)-1-(2-(4-methoxyphenyl)acetyl)-6-methylpiperidine-3-carboxylate (85.2 mg, 0.279 mmol) in THF (1.44 mL) was added 1 N NaOH (0.419 mL, 0.419 mmol). The mixture was stirred at room temperature for 2 hours. The mixture was diluted with $H_2O$ (2.00 mL) and washed twice with DCM (3.00 mL × 2). The aqueous layer was acidified with 1 N HCl until pH 1 to 2 at 0°C and extracted with EtOAc (3.00 mL). The organic layer was washed with brine (3.00 mL), dried over $MgSO_4$, filtered, and concentrated under reduced pressure to afford compound BnH12063 (65.1 mg, 80%) as a white solid.

$^1$H NMR (400 MHz, DMSO-d6) δ 12.42 (s, 1H), 7.16-7.10 (dd, J = 18.6, 8.1 Hz, 2H), 6.88-6.86 (d, J = 8.5 Hz, 2H), 4.73 (s, 0.5H), 4.54 - 4.50 (m, 0.5H), 4.23 (s, 0.5H), 3.89-3.86 (d, J = 14.4 Hz, 0.5H), 3.73 (s, 3H), 3.66-3.60 (m, 2H), 3.01-2.95 (t, J = 12.8 Hz, 0.5H), 2.62-2.59 (d, J = 12.4 Hz, 0.5H), 2.15 - 2.09 (m, 1H), 1.80-1.76 (d, J = 11.8 Hz, 1H), 1.69-1.63 (m, J = 13.4 Hz, 1H), 1.51-1.43 (m, J = 13.5 Hz, 2H), 1.03 (d, J = 6.9 Hz, 3H).

**(3) Synthesis of compound BnH12068**

Sodium (3S,6R)-6-methyl-1-(2-(pyridin-3-yl)acetyl)piperidine-3-carboxylate

**Step 1:** Synthesis of methyl (3S,6R)-6-methyl-1-(2-(pyridin-3-yl)acetyl)piperidine-3-carboxylate

To a solution of 2-(pyridin-3-yl)acetic acid (100 mg, 0.729 mmol) in DMF (2.43 mL) were added methyl (3S,6R)-6-methylpiperidine-3-carboxylate (anhydrous form) (120 mg, 0.766 mmol), DIPEA (0.191 mL, 1.09 mmol), HOBt (118 mg, 0.875 mmol), and EDC·HCl (136 mg, 0.875 mmol). The mixture was stirred at room temperature for 2 hours. The mixture was diluted with EtOAc (5.00 mL) and washed with saturated aqueous $NH_4Cl$ (5.00 mL) and brine (5.00 mL). The organic layer was dried over $MgSO_4$, filtered, and concentrated under reduced pressure. The residue was purified by column chromatography on $SiO_2$ (DCM: MeOH = 0 to 5%) to afford the desired compound (112 mg, 56%) as a pale yellow oil.

**Step 2:** Synthesis of compound BnH12068

To a solution of methyl (3S,6R)-6-methyl-1-(2-(pyridin-3-yl)acetyl)piperidine-3-carboxylate (112 mg, 0.405 mmol) in THF (2.10 mL) was added 1 N NaOH (0.608 mL, 0.608 mmol). The mixture was stirred at room temperature for 2 hours. The mixture was diluted with $H_2O$ (5.00 mL) and washed twice with DCM (5.00 mL × 2). The aqueous layer was

concentrated under reduced pressure. The residue was purified by column chromatography on C18-$SiO_2$ ($H_2O$:MeOH = 1:0 to 4:1) to afford compound BnH12068 (91.0 mg, 79%) as a pale blue solid.

$^1H$ NMR (400 MHz, DMSO-d6) δ 8.43-8.39 (m, 2H), 7.63-7.57 (dd, J = 15.5, 7.8 Hz, 1H), 7.34-7.30 (dt, J = 8.2, 4.4 Hz, 1H), 4.68 (s, 0.5H), 4.42-4.38 (d, J = 14.4 Hz, 0.5H), 4.20 (s, 0.5H), 3.81-3.68 (m, 2.5H), 2.99-2.92 (t, J = 12.8 Hz, 0.5H), 2.68 (s, 0.5H), 1.78-1.66 (dd, J = 18.2, 8.2 Hz, 2H), 1.56-1.43 (m, 3H), 1.11-1.02 (dd, J = 30.2, 6.9 Hz, 3H).

**Preparation Example 7. Preparation of active compounds by methods other than representative reaction scheme**

**(1) Synthesis of BnH12001**

1-(2-(2-chloro-4-fluorophenyl)acetyl)-6-methylpiperidine-3-carboxylic acid

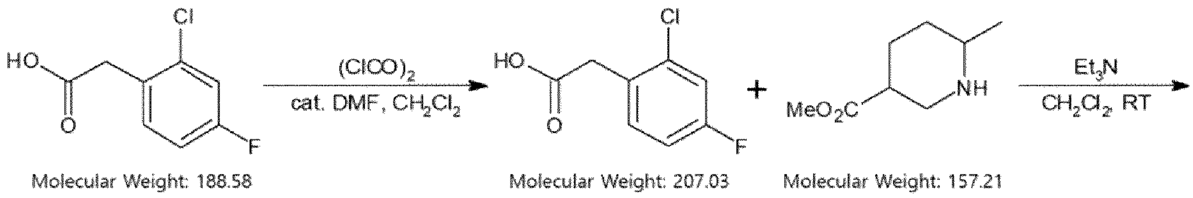


Compound 1 Compound 2 Compound 3

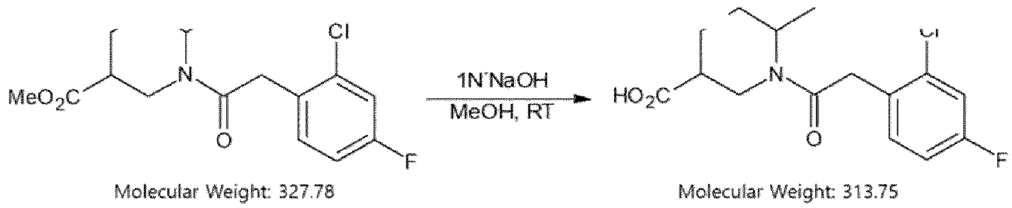


Compound 4 Target compound 5

**Step 1:** Compound 1 (200 mg, 1.06 mmol) was dissolved in 5 mL of dichloromethane (DCM), and oxalyl chloride (136 μL, 1.59 mmol) was added thereto dropwise for 5 min at 0°C, followed by the addition of dimethylformamide (DMF) (1 drop) together with a catalyst. After stirring at room temperature for 6 hours, the mixture was concentrated under reduced pressure to obtain 219 mg of compound 2. The reaction was carried out to the next step without further purification.

**Step 2:** Compound 3 (166 mg, 1.06 mmol) was dissolved in 10 mL of DCM, and triethyl amine (295 μL, 2.12 mmol) was added thereto at 0°C. Compound 2 (219 mg, 1.06 mmol) was dissolved in 5 mL of DCM, and added dropwise at 0°C, and stirred at room temperature for 2 hours. The reaction mixture was worked up with DCM and water, and then 131 mg of compound 4 was obtained (yield 38%) using a column filled with silica gel 60 (0.040 to 0.063 mm) (1.09385.9025, Merck).

**Step 3:** Compound 4 (131 mg, 400 μmol) was dissolved in 5 mL of tetrahydrofuran (THF)/methanol (1:1). 1 N-NaOH (800 μL, 800 μmol) was added thereto, and stirred at room temperature for 12 hours. After completion of the reaction, the pH was adjusted to 3 using 1 N-HCl, extracted with ethyl acetate, and concentrated under reduced pressure. From the obtained reaction mixture, 34 mg of the target compound 5 was obtained using prep TLC (yield 27%). The identity was confirmed by NMR spectrum: $^1H$ NMR (CDCl3, 400 MHz): δ 7.22 (1H, m), 7.08 (1H, m), 6.91 (1H, bs), 4.75 (1H, m), 3.86 (1H, m), 3.71 (2H, m), 3.01 (1H, m), 2.28 (1H, m), 1.91 (1H, bs), 1.74 (1H, m), 1.58 (1H, bs), 1.13 (3H, m).

**(2) Synthesis of BnH12001 enantiomer (3S,6R, 98%<)**

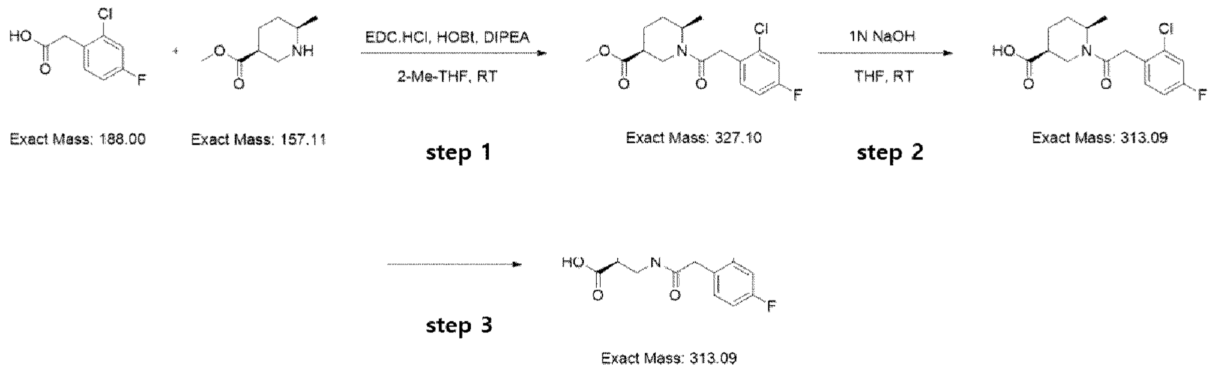


**Step 1: Preparation of methyl (3S,6R)-1-(2-(2-chloro-4-fluorophenyl)acetyl)-6-methylpiperidine-3-carboxylate**

2-(2-chloro-4-fluorophenyl)acetic acid (1.7 kg), (3S,6R)-methyl 6-methylpiperidine-3-carboxylate (1.05 eq.), DIPEA (1.5 eq.), HOBt (0.3 eq.), and EDC·HCl (1.2 eq.) were added to a 2-MeTHF (10 V) solution. The mixture was heated at 25°C to 35°C for 1.3 hours. The mixture was washed twice with $H_2O$ (5 V) to obtain methyl (3S,6R)-1-(2-(2-chloro-4-fluorophenyl)acetyl)-6-methylpiperidine-3-carboxylate with a purity of 99.6%.

**Step 2: Preparation of BnH12001 enantiomer (3S,6R, 98%<)**

Methyl (3S,6R)-1-(2-(2-chloro-4-fluorophenyl)acetyl)-6-methylpiperidine-3-carboxylate was scaled up to 2.953 kg. Methyl (3S,6R)-1-(2-(2-chloro-4-fluorophenyl)acetyl)-6-methylpiperidine-3-carboxylate was added to 2-MeTHF (10 V) together with 1 N NaOH (1.3 eq.). The mixture was heated at 25°C for 3 hours. Crystallization from 2-MeTHF/n-heptane gave 2.754 kg of (3S,6R)-1-(2-(2-chloro-4-fluorophenyl)acetyl)-6-methylpiperidine-3-carboxylic acid with less than 0.1% impurity.

**Step 3: Purification of BnH12001 enantiomer (3S,6R, 98%<)**

(3S,6R)-1-(2-(2-chloro-4-fluorophenyl)acetyl)-6-methylpiperidine-3-carboxylic acid (2.57 kg) was added to EA (5 V) and n-heptane (15 V). The mixture was heated from 0°C to 45°C to produce 2.404 kg of dried cake as a white solid.

**(3) Synthesis of compound BnH12010**

(3R,6S)-1-(2-chloro-4-fluorophenethyl)-6-methylpiperidine-3-carboxylic acid

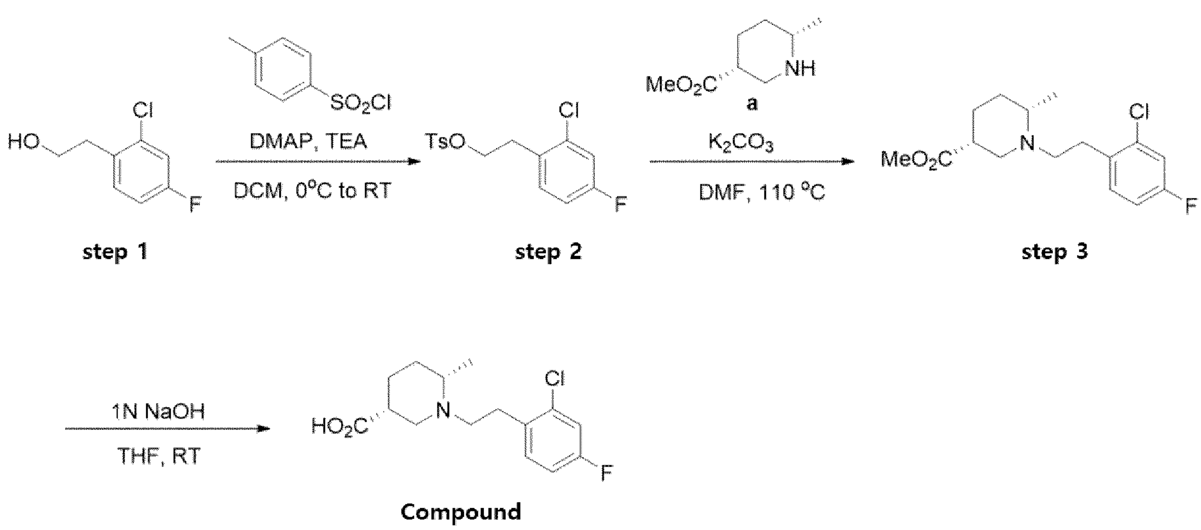

**Step 1:** 2-Chloro-4-fluorophenethyl 4-methylbenzenesulfonate (e)

To a solution of p-toluenesulfonyl chloride (240 mg, 2.21 mmol), DMAP (24.5 mg, 0.200 mmol), and TEA (0.503 mL, 3.61 mmol) in DCM (3.00 mL) was added 2-(2-chloro-4-fluorophenyl)ethanol (350 mg, 2.001 mmol) at 0°C, and stirred at room temperature for 17 hours. The mixture was diluted with EtOAc (5.00 mL) and washed with $H_2O$ (5.00 mL × 3). The organic layer was dried over $MgSO_4$, filtered, and concentrated under reduced pressure. The residue was purified by column chromatography (n-hexane:EtOAc = 10:1) to afford the desired compound (523 mg, 79%) as a colorless oil.

**Step 2:** Methyl (3R,6S)-1-(2-chloro-4-fluorophenethyl)-6-methylpiperidine-3-carboxylate (f)

To a solution of 2-chloro-4-fluorophenethyl 4-methylbenzenesulfonate (200 mg, 0.608 mmol) in DMF (3.00 mL) were added $K_2CO_3$ (252 mg, 1.83 mmol) and methyl (3R,6S)-rel-6-methyl-3-piperidinecarboxylate (76.5 mg, 0.487 mmol). The mixture was stirred at 110°C for 17 hours. The mixture was diluted with EtOAc (4.00 mL) and washed with $H_2O$ (4.00 mL × 3). The organic layer was dried over $MgSO_4$, filtered, and concentrated under reduced pressure. The residue was purified by column chromatography (n-hexane:EtOAc = 10:1) to afford the desired compound (63.0 mg, 33%) as a colorless oil.

**Step 3:** Synthesis of compound BnH12010

To a solution of methyl (3R,6S)-1-(2-chloro-4-fluorophenethyl)-6-methylpiperidine-3-carboxylate (60.0 mg, 0.191 mmol) in THF (0.659 mL) was added 1 NNaOH (0.287 mL, 0.287 mmol). The mixture was stirred at room temperature for 2 hours. The mixture was diluted with $H_2O$ (1.50 mL) and washed twice with DCM (2.00 mL × 2). The aqueous layer was acidified with 1 N HCl until pH 1 to 2 at 0°C and extracted with EtOAc (2.00 mL). The organic layer was washed with brine (2.00 mL), dried over $MgSO_4$, filtered, and concentrated under reduced pressure. Et2O (1.00 mL) was added to the residue and stirred at room temperature for 30 minutes. The precipitate was filtered and washed with $Et_2O$ (2.00 mL) to afford compound BnH12010 (42.0 mg, 73%) as a white solid.

$^1H$ NMR (400 MHz, Chloroform-d) δ 7.21 (dd, J = 8.5, 6.1 Hz, 1H), 7.12 (dd, J = 8.5, 2.7 Hz, 1H), 6.92 (td, J = 8.3, 2.7 Hz, 1H), 4.41 (s, 1H), 4.30 (t, J = 6.7 Hz, 2H), 4.21 (s, 1H), 3.06 (t, J = 6.7 Hz, 2H), 2.94 (dd, J = 13.7, 12.0 Hz, 1H), 2.39 (d, J = 13.1 Hz, 1H), 1.95 (t, J = 7.9 Hz, 1H), 1.84 - 1.52 (m, 3H), 1.12 (d, J = 6.9 Hz, 3H).

**(4) Synthesis of compound BnH12021**

Ethyl (3R,6S)-1-(2-(2-chloro-4-fluorophenyl)acetyl)-6-methylpiperidine-3-carboxylate

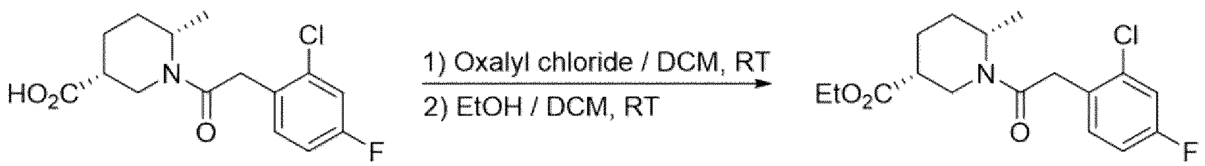


step 1

Compound

**Step 1:** Synthesis of ethyl (3R,6S)-1-(2-(2-chloro-4-fluorophenyl)acetyl)-6-methylpiperidine-3-carboxylate

To a solution of compound BnH12001 enantiomer (3S,6R) (70.0 mg, 0.223 mmol) in DCM (1.01 mL) was added catalytic amounts of DMF and oxalyl chloride (0.057 mL, 0.669 mmol). The mixture was stirred at room temperature for 2 hours and concentrated under reduced pressure. The residue was dissolved in DCM (1.01 mL) and EtOH (0.065 mL, 1.12 mmol) was added thereto. The mixture was stirred at room temperature for 1 hour and washed with saturated aqueous $NaHCO_3$ (2.00 mL). The organic layer was dried over $MgSO_4$, filtered, and concentrated under reduced pressure. The residue was purified by column chromatography on $SiO_2$ (n-hexane: EtOAc = 3:1) to afford compound BnH12021 (37.0 mg, 48%) as a colorless oil.

$^1H$ NMR (400 MHz, Chloroform-d) δ 7.14 (dd, J = 8.9, 2.6 Hz, 1H), 6.96 (td, J = 8.4, 2.7 Hz, 1H), 4.96 (s, 0.5H), 4.78 (d, J = 13.4 Hz, 0.5H), 4.15 (q, J = 7.2 Hz, 2.5H), 3.88 - 3.69 (m, 2.5H), 3.21 (t, J = 13.0 Hz, 0.5H), 2.79 (t, J = 12.9 Hz, 0.5H), 2.36 (d, J = 12.6 Hz, 0.5H), 2.23 (d, J = 11.2 Hz, 0.5H), 1.93 (d, J = 13.0 Hz, 1H), 1.78 (d, J = 14.8 Hz, 1H), 1.71 - 1.61 (m, 2H), 1.34 - 1.09 (m, 6H).

**(5) Synthesis of compound BnH12094**

1-(2-(2-chloro-4-fluorophenyl)acetyl)-6-ethylpiperidine-3-carboxylic acid

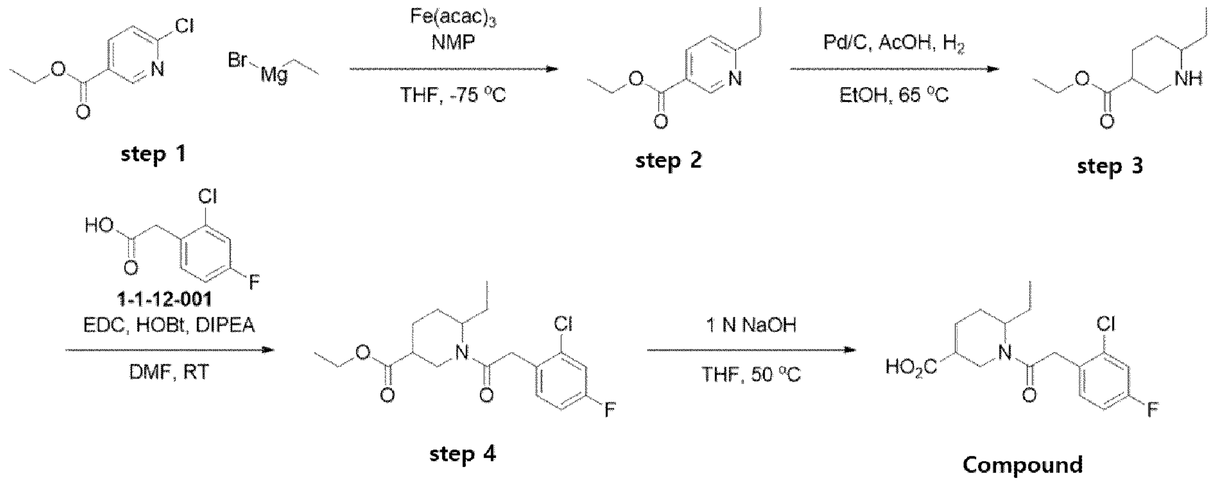


**Step 1:** Synthesis of ethyl 6-ethylnicotinate(o)

To a solution of ethyl 6-chloronicotinate (2.20 g, 11.9 mmol) in anhydrous THF (60.0 mL) were added $Fe(acac)_3$ (460 mg, 1.30 mmol) and NMP (1.60 mL, 16.6 mmol). The mixture was cooled to -78°C, and ethylmagnesium bromide was added dropwise. After stirring at -78°C for 1 hour, the reaction mixture was quenched with $H_2O$ (50.0 mL) at 0°C, and extracted with EtOAc (50.0 mL). The organic layer was dried over $Na_2SO_4$, filtered, and concentrated under reduced pressure. The residue was purified by column chromatography on $SiO_2$ (n-hexane:EtOAc = 1:0 to 9:1) to afford the desired compound (2.04 g, 96%) as a pale yellow oil.

**Step 2:** Ethyl 6-ethylpiperidine-3-carboxylate (p)

To a solution of ethyl 6-ethylnicotinate (2.04 g, 11.4 mmol) in EtOH (20.0 mL) were added acetic acid (2.07 mL, 11.4 mol) and Pd/C (848 mg, 7.97 mmol). The mixture was purged with $H_2$ gas three times and stirred for 24 hour at 65°C under $H_2$ gas. After cooling to room temperature, the reaction mixture was filtered through Celite and washed with MeOH (30.0 mL). The filtrate was concentrated under reduced pressure and used without further purification.

**Step 3:** Ethyl 1-(2-(2-chloro-4-fluorophenyl)acetyl)-6-ethylpiperidine-3-carboxylate (BnH12094-1)

To a solution of 2-(2-chloro-4-fluorophenyl)acetic acid (450 mg, 2.39 mmol) in DMF (8.00 mL) were added ethyl 6-ethylpiperidine-3-carboxylate (464 mg, 2.51 mmol), DIPEA (0.623 mL, 3.58 mmol), HOBt (387 mg, 2.86 mmol), and EDC·HCl (549 mg, 2.86 mmol). The mixture was stirred at room temperature for 3 hours. The reaction mixture was diluted with EtOAc (20.0 mL) and washed with saturated aqueous $NH_4Cl$ (20.0 mL) and brine (20.0 mL). The organic layer was dried over $Na_2SO_4$, filtered, and concentrated under reduced pressure. The residue was purified by column chromatography on $SiO_2$ (n-hexane:EtOAc = 1:0 to 2:1 and 0 to 3% MeOH in DCM) to afford the desired compound (121 mg, 24%, 2 step) as a colorless oil.

**Step 4:** Synthesis of compound BnH12094

To a solution of ethyl 1-(2-(2-chloro-4-fluorophenyl)acetyl)-6-ethylpiperidine-3-carboxylate (115 mg, 0.323 mmol) in THF (3.00 mL) was added 1 N NaOH (0.970 mL, 0.970 mmol). The mixture was stirred for 17 hours at 50°C. The reaction mixture was diluted with $H_2O$ (5.00 mL) and washed twice with DCM (5.00 mL × 2). The aqueous layer was acidified with 1 N HCl until pH 1 to 2 at 0°C and extracted with EtOAc (5.00 mL). The organic layer was dried over $Na_2SO_4$, filtered, and concentrated under reduced pressure. The residue was dissolved in EtOAc (2.00 mL), and n-hexane (10.0 mL) was added thereto. The resulting solid was filtered and washed with n-hexane (5.00 mL) to afford compound BnH12094 (82.0 mg, 77%) as a white solid.

$^1$H NMR (400 MHz, DMSO-d6) δ 12.43 (s, 1H), 7.42 - 7.23 (m, 2H), 7.17 (td, J = 8.5, 2.8 Hz, 1H), 4.54 - 4.47 (m, 1H), 3.96 - 3.70 (m, 2.5H), 3.25 - 3.21 (m, 0.5H), 3.07 (t, J = 12.9 Hz, 0.5H), 2.66 - 2.53 (m, 1H), 2.39 - 2.33 (m, 0.5H), 2.24 - 2.18 (m, 0.5H), 1.84 - 1.40 (m, 5.5H), 0.82 (dt, J = 42.2, 7.3 Hz, 3H).

**(6) Synthesis of compound BnH12096**

1-(2-(2-chloro-4-fluorophenyl)acetyl)-6-isopropylpiperidine-3-carboxylic acid

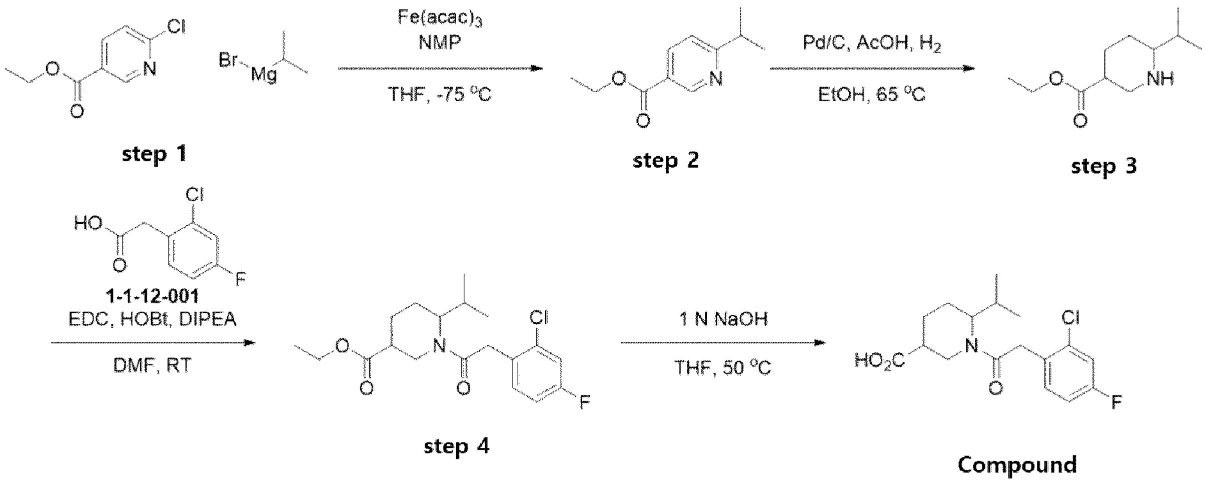


**Step 1:** Ethyl 6-isopropylnicotinate (r)

To a solution of ethyl 6-chloronicotinate (300 mg, 1.62 mmol) in anhydrous THF (8.00 mL) were added $Fe(acac)_3$ (62.8 mg, 0.178 mmol) and NMP (0.218 mL, 2.26 mmol). The mixture was cooled to -78°C, and isopropylmagnesium bromide was added dropwise. After stirring for 1 hour at -78°C, the reaction mixture was quenched with $H_2O$ (10.0 mL) at 0°C, and extracted with EtOAc (10.0 mL). The organic layer was dried over $Na_2SO_4$, filtered, and concentrated under reduced pressure. The residue was purified by column chromatography on $SiO_2$ (DCM: n-hexane = 1:1 and n-hexane: EtOAc = 9:1) to afford the desired compound (170 mg, 54%) as a light yellow oil.

**Step 2:** Ethyl 6-isopropylpiperidine-3-carboxylate (s)

To a solution of ethyl 6-isopropylnicotinate (165 mg, 0.854 mmol) in EtOH (2.00 mL) were added acetic acid (0.155 mL, 0.854 mol) and Pd/C (63.6 mg, 0.598 mmol). The mixture was purged with $H_2$ gas three times and stirred for 17 hours at 65°C under $H_2$ gas. After cooling to room temperature, the reaction mixture was filtered through Celite and washed with MeOH (5.00 mL). The filtrate was concentrated under reduced pressure and used without further purification.

**Step 3:** Ethyl 1-(2-(2-chloro-4-fluorophenyl)acetyl)-6-isopropylpiperidine-3-carboxylate (BnH12096-1)

To a solution of 2-(2-chloro-4-fluorophenyl)acetic acid (270 mg, 1.43 mmol) in DMF (5.00 mL) were added ethyl 6-isopropylpiperidine-3-carboxylate (300 mg, 1.51 mmol), DIPEA (0.375 mL, 2.15 mmol), HOBt (233 mg, 1.72 mmol), and EDC·HCl (330 mg, 1.72 mmol). The mixture was stirred at room temperature for 16 hours. The reaction mixture was diluted with EtOAc (15.0 mL) and washed with saturated aqueous $NH_4Cl$ (15.0 mL) and brine (15.0 mL). The organic layer was dried over $Na_2SO_4$, filtered, and concentrated under reduced pressure. The residue was purified by column chromatography on $SiO_2$ (n-hexane:EtOAc = 4:1 to 2:1) to afford the desired compound (176 mg, 54%, 2 step) as a pale yellow oil.

**Step 4:** Synthesis of compound BnH12096

To a solution of ethyl 1-(2-(2-chloro-4-fluorophenyl)acetyl)-6-isopropylpiperidine-3-carboxylate (176 mg, 0.476 mmol) in THF (1.64 mL) was added 1 N NaOH (0.714 mL, 0.714 mmol). The mixture was stirred for 4 hours at 50°C. The reaction mixture was diluted with $H_2O$ (3.00 mL) and washed twice with DCM (3.00 mL $\times$ 2). The aqueous layer was acidified with 1 N HCl until pH 1 to 2 at 0°C and extracted with EtOAc (3.00 mL). The organic layer was dried over $MgSO_4$, filtered, and concentrated under reduced pressure. $Et_2O$ (4.00 mL) was added to the residue and stirred at room temperature for 30 minutes. The precipitate was filtered and washed with $Et_2O$ (3.0 mL) to afford compound BnH12096 (102 mg, 63%) as a white solid.

$^1H$ NMR (400 MHz, DMSO-d6) $\delta$ 12.44 (s, 1H), 7.42 - 7.23 (m, 2H), 7.17 (td, J = 8.5, 2.8 Hz, 1H), 4.54 (dd, J = 13.2,

4.3 Hz, 0.5H), 4.23 - 4.13 (m, 0.5H), 4.01 - 3.67 (m, 2.5H), 3.57 (dd, J = 10.7, 4.5 Hz, 0.5H), 3.05 (dd, J = 14.1, 11.9 Hz, 0.5H), 2.45 - 2.31 (m, 0.5H), 2.30 - 1.96 (m, 1.5H), 1.87 (dd, J = 28.7, 12.7 Hz, 2H), 1.74 - 1.58 (m, 1H), 1.55 - 1.32 (m, 1H), 1.06 - 0.66 (m, 6H).

## Examples

The following biological assays were performed using the compounds obtained in the above preparation examples.

### Example 1. Cell culture and maintenance

Human undifferentiated neuroblastoma cell line SH-SY5Y and human embryonic kidney cell line (HEK)-293T were purchased from Korean Cell Line Bank (Korea) and Takara (Japan), respectively. Cells were grown in HyClone Minimal Essential Medium (MEM) and Dulbecco's Modified Eagle Medium (DMEM), respectively, supplemented with 10% (v/v) fetal bovine serum (FBS), penicillin (100 U/mL), and streptomycin (100 mg/mL). Cells were maintained in a humidified atmosphere of 95% air and 5% $CO_2$ at 37°C. Cells were passaged 10 times at maximum. Cells were dissociated using 0.05% trypsin-EDTA solution and the cells were seeded in new culture dishes for maintenance.

Meanwhile, primary cortical neuron cells were isolated from cerebral cortices of 18-day-old mouse fetuses, incubated in prep media (composition (mM): NaCl 107.5, $NaH_2PO_4$ 1, $NaHCO_3$ 33.5, glucose 11, HEPS 1%, penicillin-streptomycin 1%) containing 0.25% trypsin and DNase1 for 30 minutes, dissociated into single cells, and seeded. Primary cortical neuron cells were grown in Neurobasal medium supplemented with 1% (v/v) B27, 1% penicillin (100 U/mL), streptomycin (100 mg/mL), and 1% Glutamax. Cells were maintained in a humidified atmosphere of 95% air and 5% $CO_2$ at 37°C. Drug efficacy was evaluated after culturing primary cortical neuron cells for 14 days.

### Example 2. Cytotoxicity test

To examine the cytotoxicity of compounds described herein in human cell lines, SH-SY5Y and 293T cells were used. Approximately 8,000 cells (for 293T) and 15,000 cells (for SH-SY5Y) were plated per well of a 96-well plate (SPL Life Sciences Co., Ltd., Pocheon, Korea). After incubating at 37°C for 24 hours in a humidified atmosphere containing 5% $CO_2$, the culture medium was replaced by a series of concentrations of test compounds diluted in the corresponding culture medium. The time of co-incubation was 24 hours and six replicates were made for each measurement. Finally, 10 μL of CCK-8 reagent (Dojindo Laboratories, Rockville, MD) was added to each well, and after incubation at 37°C for 3 minutes, the OD at 450 nm was measured using a multifunction microplate reader (Tecan Infinite M200 Pro). These measurements were compared to that of a control and expressed as a percentage of the control to calculate cell viability. The $IC_{50}$ value was calculated using SPSS software. To do this, the absorbance of the blank wells with only growth medium is subtracted from the values or the wells with cells. The readout value of background (vehicle) was set as a 100% viability, and then the percent viability for each test solution was calculated.

$$\text{Cell viability (\%)} = [(\text{As-Ab}) / (\text{Ac-Ab})] \times 100$$

The data was presented as the mean value and standard error of the mean (S.E.M.). Statistical analysis was performed with the one-way ANOVA with Tukey's post hoc test (GraphPad Prism 5 software package, version 5.02, GraphPad Software Inc. USA). Significance was accepted at $p < 0.05$.

### Example 3. Measurement of expression levels of BDNF and/or GluN2B

#### Example 3.1. RT-PCR

RT-PCR was used to measure the amount of BDNF and GluN2B in response to compounds of the present disclosure. 293T cells were dissociated using 0.05% Trypsin-EDTA solution and seeded in 24 well or 6 well dishes for RNA work. After incubation at 37°C in a humidified atmosphere with 5% $CO_2$ for 24 hours, the culture medium was replaced by a medium containing a series of concentrations of tested compounds diluted with the DMEM without FBS. Time course experiments were performed. The same volume of DMSO was used for negative control.

RNA was isolated from the cells after the compound treatment. After removing growth media, 0.3 to 0.4 mL of TRIzol™ per $1 \times 10^5$ to $10^7$ cells were added directly to the culture dish to lyse the cells. After incubating for 10 minutes at 4°C, the sample was centrifuged for 10 minutes at 12,000 × g at 4°C. Total RNA precipitate formed a white gel-like pellet at the bottom of the tube. After discarding the supernatant, the pellet was resuspended in 1 mL of 75% ethanol per 1 mL of

TRIzol™ used for lysis. After vortexing the sample briefly, the sample was centrifuged for 5 minutes at 7500 × g at 4°C. The supernatant was discarded with a micropipettor and vacuum or air dried for 5 to 10 minutes. The pellet was resuspended in 20-50 μL of RNase-free water. RNA concentration was determined with the Thermo Scientific™ μDrop™ plate. RNA quality was determined by measuring A230/260 and A260/280 ratio.

On ice, the experimental RNA (up to 1 μg in 4 ul) and the oligo dT primer 1 ul (100 pmol) in Nuclease-Free Water were combined for a final volume of 5 μl per RT reaction. After closing each tube of RNA tightly, the tubes were placed into a 70°C for 5 minutes (in PCR machine). After taking the tubes out, the tubes were immediately chilled in ice-water for at least 5 minutes. Each tube was centrifuged for 10 seconds in a microcentrifuge to collect the condensate and maintain the original volume. The tubes were kept closed and on ice until the reverse transcription reaction mix was added. The reverse transcription reaction mix was prepared as follows.

| | |
|---|---|
| DW | 4.9 ul |
| 5x RT buffer | 4 ul |
| 25 mM $MgCl_2$ | 1.6 ul (final 2.6 mM) |
| 10 mM dNTP (2.5 mM each) | 3 ul (final 0.5 mM each dNTP) |
| RNasin (40 u/ul) | 0.5 ul (final 20 u) |
| RT | 1 ul |
| Total | 15 ul/tube |

Reverse transcription was performed using Improm-II Reverse Transcriptase (A3803) (Promega) and Recombinant RNasin Ribonuclease Inhibitor (N2515) (Promega) according to the manufacturer's instruction. 15 μl aliquots of the reverse transcription reaction mix were added to each reaction tube on ice and placed the tubes back in a PCR machine. PCR condition was as follows:

25°C, 5 min (annealing) → 42°C, 60 min (extension) → 70°C, 15 min (inactivation of RT) → holding at 4°C

The PCR product was analyzed by agarose gel electrophoresis. Housekeeping genes (ACTIN) were used as a PCR control. Replicate samples confirmed the validity of observed changes in RNA levels. Primer sequences used are as follows:

| | |
|---|---|
| h-GluN2B-Forward | CTTCCATTTACCCTGCCTCA |
| h-GluN2B-Reverse | CATTGCCTCTAGCTCCCTTG |
| h-BDNF-Forward | CATCCGAGGACAAGGTGGCTTG |
| h-BDNF-Reverse | GCCGAACTTTCTGGTCCTCATC |
| h-ActinB-Forward | CACCAACTGGGACGACAT |
| h-ActinB-Reverse | ACAGCCTGGATAGCAACG |

**Example 3.2. Western blotting**

The amount of GluN2B reacted with the compound of the present disclosure was measured using Western blot. The fetal cerebral cortex was extracted from mice at 17 days of gestation, dissociated into single cells, cultured for 13 days, and treated with BnH drugs for 24 hours. The drug-treated cells were subjected to protein lysate preparation using RIPA protein lysis buffer containing protease and phosphatase inhibitors. The protein concentration was determined using a BCA protein assay kit (Pierce Biotechnology) using bovine serum albumin as a standard. Equal amounts of protein (20 μg per lane) were separated by electrophoresis on SDS-polyacrylamide gels at 90 V for 2 hours, then transferred from the SDS-polyacrylamide gel to PVDF membrane at 100 V for 1 hour. Then, primary reaction with GluN2B antibody (ab65783, Abcam) was performed at 4°C for 1 day, washed three times at 10-minutes intervals with 1X PBT, and secondary reaction was performed with anti-mouse antibody (sc-2357, Santa Cruz) for 1 hour, followed by washing three times at 10-minutes intervals with 1X PBT. Western blotting was performed as described above to confirm GluN2B protein expression. Subsequently, each blot was re-probed using anti-β-actin antibody (sc-47778, Santa Cruz). Housekeeping protein β-actin was detected as a normalization control.

As a result, the protein changes of GluN2B were confirmed in various BnH12 series compounds as shown in Table 6.

Data analysis

The increase and decrease of BnH12 series were confirmed by setting the vehicle to 1 (N for each group was 3 or more).

**Example 4. Synaptic function test**

**Example 4.1. Os pump transplantation**

Eight weeks old male C57BL/6J mice were anesthetized with isoflurane during surgery. The mice were then be placed in a stereotaxic frame (RWD Life Science, China). The parietal and the frontal bones were exposed by a sagittal incision of the scalp at the midline from the level of the eyes to the occipital protuberance. A small burr hole was drilled after exposing the skull. The injection cannula was placed at the following coordinates: (Mouse: Bregma - 3.30, lateral -1.50, and ventral 0.7 mm). Sterile saline solution (+/- a compound of the present disclosure) was perfused at a constant flow rate of 0.5 μL/h through the injection cannula using a mini-Os pump (DURECT Corporation ALZET Osmotic Pumps, CA). Implantation was done in layer 4 of the barrel cortex ipsilateral. To estimate extent of the diffusion of the injected compound in layer 4 barrel cortex during pump injection, methylene blue was first injected to stain the diffusion area. On average methylene blue diffused to 2000.0 μm away from the injection site (the total area of the barrel cortex region is about 1500 μm). Based on this analysis, the present inventors recorded from barrel cortex area.

**Example 4.2. Oral administration of compounds**

2 mg of BnH12001 was dissolved in 800 ul of DMSO and prepared as a stock solution. For oral administration, BnH12001 DMSO stock:PEG400:Saline was mixed in a ratio of 5:55:40 and 200 μl/animal was administered. For the control group, a vehicle in which DMSO:PEG400:Saline was mixed in a 5:55:40 ratio was administered at 200ul/animal. In the single drug administration test, the compound was administered once a day for about 11 to 12 days at a dose of 1 mg/kg. Dose response test was performed by administering the compound once a day for about 11 to 12 days at doses of 0.03, 0.1, 0.3, 1, 3, and 10 mg/kg. 5 mice were used for the compound administration group and the control group, respectively.

Example 4.3. Intraperitoneal injection of compounds

The compound was diluted to 25 mg/kg using DMSO:PEG400:Saline(5:55:40). The compound was administered intraperitoneal at 10:00 a.m. daily (*i.p.* volume was 200 μl) for about 11 to 12 days. Control group was also administered by *i.p.* injection in the same manner using the vehicle (DMSO:PEG400:Saline(5:55:40)). 5 mice were used for the compound administration group and the control group, respectively.

**Example 4.4. Intravenous injection of compounds**

1 mg/ml of aducanumab or control antibody was used as a stock solution. For intravenous injection, the tail vein injection method was used. Aducanumab was administered at a dose of 5 mg/kg. Control antibody was administered using an antibody inactivated with aducanumab at a dose of 5 mg/kg. In the drug injection test, the compound was administered once a day for about 11 to 13 days. 5 mice were used for the compound administration group and the control group, respectively.

**Example 4.5. Preparation of thalamocortical (TC) or hippocampus slice**

For TC or hippocampus slice preparation, 9 to 10 weeks-old male C57BL/6J mice were anesthetized with isoflurane. Then, the brain was rapidly cooled via transcardial perfusion with ice-cold sucrose-artificial cerebrospinal fluid (aCSF). The brain was removed and placed in ice-cold sucrose aCSF. Paracoronal slices were prepared at an angle of 50° relative to the midline on a ramp at an angle of 10°. Then, slices were incubated in aCSF at 35°C for 30 minutes to recover. Slices were then incubated in aCSF at room temperature (23 to 25°C) for 1 to 4 hours before being placed in the recording chamber for experiments. The standard aCSF contains saturated with 95% $O_2$/5% $CO_2$. The brain of the mouse was removed and cut into 400 μm at a 50° angle (mouse at a 55° angle) to attach the cut portion to the vibratome disc to be the bottom. Brain slice was incubated in 35°C in aCSF solution for 30 minutes, or at RT in aCSF solution for 1 hour.

Composition of sucrose based aCSF

| Cutting aCSF (4°C) | | | | | | |
|---|---|---|---|---|---|---|
| Chemical | Sigma # | F.W | Conc (mM) | g/1L | g/2L | g/4L |
| Sucrose | S9378 | 342.3 | 195.5 | 66.931 | 133.862 | 267.725 |
| KCl | P9333 | 74.56 | 2.5 | 0.186 | 0.373 | 0.746 |
| $NaH_2PO_4$ | S8282 | 119.98 | 1 | 0.BnH120 | 0.240 | 0.480 |
| $NaHCO_3$ | S6297 | 84.01 | 32.5 | 2.730 | 5.461 | 10.921 |
| Glucose | G7528 | 180.16 | 11 | 1.982 | 3.964 | 7.927 |
| Na pyruvate | P5280 | 110.04 | 2 | 0.220 | 0.440 | 0.880 |
| Na L-ascorbate | A4034 | 198.11 | 1 | 0.198 | 0.396 | 0.792 |
| Daily addition to the cutting aCSF (4°C) | | | | | | |
| Stock solution | Conc (mM) | ml/600mL | ml/1L | ml/2L | ml/4L | ml/500ml |
| 1M $MgSO_4$ | 5 | 3 | 5 | 10 | 20 | 2.5 |
| 1M $CaCl_2$ | 0.5 | 0.3 | 0.5 | 1 | 2 | 0.25 |
| [702] Composition of recording aCSF (room temperature: RT) | | | | | | |

| Recording aCSF (22°C) | | | | | | |
|---|---|---|---|---|---|---|
| Chemical | Sigma # | F.W | Conc (mM) | g/1L | g/2L | g/4L |
| Sucrose | S7653 | 58.44 | 124 | 7.247 | 14.493 | 28.986 |
| KCl | P9333 | 74.56 | 2.5 | 0.186 | 0.373 | 0.746 |
| $NaH_2PO_4$ | S8282 | 119.98 | 1 | 0.BnH120 | 0.240 | 0.480 |
| $NaHCO_3$ | S6297 | 84.01 | 26.2 | 2.201 | 4.402 | 8.804 |
| Glucose | G7528 | 180.16 | 11 | 1.982 | 3.964 | 7.927 |
| Na pyruvate | P5280 | 110.04 | 2 | 0.220 | 0.440 | 0.880 |
| Na L-ascorbate | A4034 | 198.11 | 1 | 0.198 | 0.396 | 0.792 |
| Daily addition to the recording aCSF (4°C) | | | | | | |
| Stock solution | Conc (mM) | ml/600mL | ml/1L | ml/2L | ml/4L | ml/3.5L |
| 1M $MgSO_4$ | 1.3 | 0.65 | 1.3 | 2.6 | 5.2 | 4.55 |
| 1M $CaCl_2$ | 2.5 | 1.25 | 2.5 | 5 | 10 | 8.75 |

**Example 4.6. Electrophysiological experiment for barrel cortex**

All electrophysiological experiments were conducted at 27 to 29°C. For electrophysiological experiments, electrodes with 3.5-4.2 mΩ resistance were used and stimuli were applied to the ventral posteromedial nucleus (VPM) using a concentric bipolar electrode (FHC, Bowdoin, ME). The somatosensory cortex was identified by the presence of barrels under 4X magnification and differential interference contrast (DIC) optics, and by the ability to evoke short and constant latency field excitatory postsynaptic potentials (fEPSPs) by VPM stimulation. Whole-cell voltage-clamp recordings were made from stellate cells (SC) in layer IV of the somatosensory barrel cortex using infrared illumination and differential interference contrast (DIC) optics. The composition of whole cell recording solution is shown as follows:

The whole cell recording internal solution

| Cs methane sulfonate-based pipet solution | | | | |
|---|---|---|---|---|
| Chemical | Sigma # | F.W | Conc (mM) | g/100mL |
| Cs-me$SO_4$ | 19823 (A) | 228 | 125 | 2.8500 |
| NaCl | S-7653 | 58.44 | 8 | 0.0468 |
| Phosphocreatine di (Tris) | P-1937 | 453.4 | 5 | 0.2267 |
| MgATP | A-9187 | 507.2 | 4 | 0.2029 |
| $Na_2$GTP | G-8877 | 523.2 | 0.4 | 0.0209 |
| EGTA | E-4378 | 380.4 | 0.5 | 0.0190 |
| HEPES | H-3375 | 238.3 | 10 | 0.2383 |
| QX314-Cl | Q-150 | 289.85 | 5 | 0.1449 |
| pH=7.35 (with CsOH at RT), QX314-Cl (purchased from Alomone Labs)<br>mOsm = ~ 285 mOsm | | | | |

Whole cell recordings were made using a multiclamp 700A (Molecular devices, Sunnyvale, CA) digitized at 10 kHz and filtered at 2 kHz. Input resistance and series resistance were monitored continuously during recordings, as previously described (see Isaac, Crair, Nicoll, & Malenka, 1997, Silent synapses during development of thalamocortical inputs, DOI: 10.1016/s0896-6273(00)80267-6). TC EPSCs were evoked at 0.1 Hz by VB stimulation and were accepted as monosynaptic if they exhibited a short and constant latency that did not change with increasing stimulus intensity as previously described (see Feldman, Nicoll, Malenka, & Isaac, 1998, Synaptic plasticity at thalamocortical synapses in developing rat somatosensory cortex: LTP, LTD, and silent synapses, https://doi.org/10.1002/(SICI)1097-4695(199910)41:1<92::AID-NEU12>3.0.CO;2U; and see Isaac et al., 1997, Silent Synapses during Development of Thalamocortical Inputs, https://doi.org/10.1016/S08966273(00)80267-6).

For the minimal stimulation protocol (Potency), VB thalamic stimulation intensity was adjusted to find the lowest intensity that elicited a mixture of synaptic responses and failures. Failure rate was calculated as the number of failures/total number of trials. Potency was calculated as the mean EPSC peak amplitude excluding failures (see Chittajallu & Isaac, 2010, Emergence of cortical inhibition by coordinated sensory-driven plasticity at distinct synaptic loci, DOI: 10.1038/nn.2639; see Isaac et al., 1997, Silent Synapses during Development of Thalamocortical Inputs, https://doi.org/10.1016/S08966273(00)80267-6; and see Stevens & Wang, 1995, Facilitation and depression at single central synapses, DOI: 10.1016/08966273(95)90223-6).

The criteria for single-axon stimulation were all or no synaptic events and no change in the mean amplitude of the EPSC evoked by small increases in stimulus intensity, as previously reported (see Chittajallu and Isaac, 2010, Emergence of cortical inhibition by coordinated sensory-driven plasticity at distinct synaptic loci, DOI: 10.1038/nn.2639; see Gil et al., 1999, Efficacy of Thalamocortical and Intracortical Synaptic Connections: Quanta, Innervation, and Reliability, https://doi.org/10.1016/S0896-6273(00)80788-6). Measurement parameters are as follows:

Measurement parameters

| Measurement | Holding voltage | Record method |
|---|---|---|
| Minimal stimulation (Potency) | -70 mV | 50% on/off of 20 or 50 trials |
| GABA AMPA ratio | 0 mV/-70 mV | Average after 5 trials each |

Excitatory postsynaptic potential (EPSC) was recorded by the whole-cell patch clamp method using a patch clamp amplifier (molecular devices, USA). The measuring electrode was a borosilicate glass capillary, and a glass capillary having a resistance of 3.5 MΩ to 4.2 MΩ when the solution inside the electrode was filled was used. The brain slice in the chamber was placed on the upright microscope and fixed with an anchor to flow the recording aCSF solution at a rate of 5 to 10 mL/min by gravity. The electrode of the stimulator was placed on the thalamus fiber and connections in the S1 barrel cortex (layer 4) area and the cell in the area was found and recorded. After the whole cell patch, the membrane voltage was fixed at -70 mV (measure the AMPA current), and the stimulus was applied to check the EPSC, and the stimulator was manipulated to find the minimum stimulus intensity at which the current on/off becomes 50%. Amplitude

was measured with the trace that Recording and Current for 3 minutes and 20 seconds, or 8 minutes and 20 seconds (total 20 or 50 traces). For potency, the EPSC size was set to 50 to 100 pA at -70 mV and 5 traces were measured at 0 mV.

**Example 4.7. Electrophysiological experiment for hippocampus**

Field recordings were made with a concentric bipolar electrode positioned in the stratum radiatum of the CA1 region using an extracellular glass pipette (3.5 MΩ) filled with aCSF. Specifically, fEPSPs were evoked by Schaffer collateral (SC)/commissural fibers with a concentric bipolar electrode placed 200 to 300 μm from the recording pipette. Stimulation was delivered through a bipolar electrode (FHC, Bowdoin, ME, USA) placed in the Schaffer collateral-CA1 (SC). Te SC circuit was visualized using differential interference contrast (DIC) microscopy at 4X magnification and identified by the ability to evoke short and constant latency field excitatory postsynaptic potentials (fEPSPs) at CA1 synapses by SC input stimulation. In all fEPSP experiments, the test stimulation were measured prior to the beginning of all the experiments (30 μA) and a test-pulse stimulation strength that evokes 50% of the maximum fEPSP was used. Baseline synaptic responses was recorded for 30 minutes, and then long-term potentiation (LTP) was induced by high-frequency stimulation (HFS; 100 Hz, 4 trains, 1 s duration, 20 s inter-train interval). Recordings were made every 10 seconds for 1 hour using multiclamp 700A amplifier (Molecular Devices, San Jose, CA, USA) digitized at 10 kHz, and filtered at 2 kHz with Digidata 1440 and pClamp 10.0 software (Molecular Devise, San Jose, CA, USA). Measurement parameters are as follows:

Measurement parameters

| Measurement | Clamp mode | Record method |
|---|---|---|
| Input/Output Curve | Current clamp | Input (fiber volley-amplitude)/Output fEPSP (20 to 80% fEPSP slope) slope |
| Long-term potentiation | Current clamp | Time course analysis of the % EPSP slope before (20 min) and after (60 min) TBS with the value of 50% fEPSP |

Excitatory postsynaptic potential (EPSP) was recorded by the whole-cell patch clamp method using a patch clamp amplifier (molecular devices, USA). The measuring electrode was a borosilicate glass capillary, and a glass capillary having a resistance of 3.5 MΩ -4.2 MΩ when the solution inside the electrode was filled was used. The brain slice in the chamber was placed on the upright microscope and fixed with an anchor to flow the recording aCSF solution at a rate of 5 to 10 mL/min by gravity. The electrode of the stimulator was placed on the SC area and the connection in the CA1 area was found and recorded. As the stimulus intensity increased, the point was recorded until fEPSP did not increase or a pop spike occurred (I/O curve). Baseline synaptic responses were recorded for 20 minutes and then long-term potentiation (LTP) was induced by high-frequency stimulation (HFS; 100 Hz, 4 trains, 1 s duration, 20 s inter-train interval / TBS; 100 Hz, 10 trains, 50 ms duration, 200 ms inter-train interval). It was then recorded for 60 minutes (LTP).

**Example 4.8. Data analysis**

To perform potency analysis, the Clampfit program (Axon Instruments) was used. The baseline was set by specifying an interval between 0 and 100 ms. The trace was averaged by selecting 'On' current that comes out within 110 to 200 ms. The negative current amplitude (pA) of the average trace was checked.

Clampfit was used to analyze GABA/AMPA ratio. For AMPA current, the baseline was set by specifying an interval between 0 and 100 ms. Traces of the data within 110 to 200 ms was averaged. The negative current amplitude (pA) of the average trace was checked. For GABA current, the baseline was set by specifying an interval between 0 and 110 ms. Traces of the data within 110 to 300 ms was averaged. The positive current amplitude (pA) of the average trace was checked

Clampfit was used to calculate I/O curve. The baseline was set by specifying an interval between 0 and 6 ms. The traces recorded (in each of 5) from the same stimulus were averaged. The amplitude of fiber volley and the 20 to 80% rise slope of fEPSP were analyzed. For LTP, the baseline was set by specifying an interval between 0 and 6 ms. The 20 to 80% rise slope of fEPSP before and after LTP induction with HFS (or TBS) was analyzed.

**Example 4.9. Statistical analysis**

All data were presented as mean ± SEM, and n represents the number of cells used in each experiment. Typically, one slice was used from each animal; one recording was made from each slice. Unpaired Student's t test was used to compare mean values from the two groups and one-way ANOVA was used from three or more groups. p values < 0.05 were considered statistically significant. These statistical analyses were performed using Prism 9.0 (GraphPad Prism 9.1.2, GraphPad Software Inc. USA).

Dose response curve was drawn using Graphpad, Prism 9 according to the manufacturer's instructions.

**Example 4.10. Western blotting**

For preparation of thalamocortical (TC) slices, 9- to 10-week-old male C57BL/6J rodents (mice) were anesthetized with isoflurane. Then, the brains were rapidly cooled by transcardial perfusion with ice-cold sucrose-artificial cerebrospinal fluid (aCSF). The brains were removed and placed in ice-cold sucrose aCSF. Paracoronal slices were prepared at a 50° angle to the midline on a ramp at an angle of 10°. The slices were then incubated in aCSF for 30 minutes at 35°C to recover.

Subsequently, the slices were incubated in aCSF for 1 to 4 hours at room temperature (23 to 25°C) before being placed in the recording chamber for experiment. Standard aCSF contains saturated with 95% $O_2$/5% $CO_2$.

Composition of sucrose based aCSF

| Cutting aCSF (4°C) | | | | | | |
|---|---|---|---|---|---|---|
| Chemical | Sigma # | F.W | Conc (mM) | g/1L | g/2L | g/4L |
| Sucrose | S9378 | 342.3 | 195.5 | 66.931 | 133.862 | 267.725 |
| KCl | P9333 | 74.56 | 2.5 | 0.186 | 0.373 | 0.746 |
| $NaH_2PO_4$ | S8282 | 119.98 | 1 | 0.120 | 0.240 | 0.480 |
| $NaHCO_3$ | S6297 | 84.01 | 32.5 | 2.730 | 5.461 | 10.921 |
| Glucose | G7528 | 180.16 | 11 | 1.982 | 3.964 | 7.927 |
| Na pyruvate | P5280 | 110.04 | 2 | 0.220 | 0.440 | 0.880 |
| Na L-ascorbate | A4034 | 198.11 | 1 | 0.198 | 0.396 | 0.792 |
| Daily addition to the cutting aCSF (4°C) | | | | | | |
| Stock solution | Conc (mM) | ml/600mL | ml/1L | ml/2L | ml/4L | ml/500ml |
| 1M $MgSO_4$ | 5 | 3 | 5 | 10 | 20 | 2.5 |
| 1M $CaCl_2$ | 0.5 | 0.3 | 0.5 | 1 | 2 | 0.25 |

Composition of recording aCSF (room temperature: RT)

| Recording aCSF (22°C) | | | | | | |
|---|---|---|---|---|---|---|
| Chemical | Sigma # | F.W | Conc (mM) | g/1L | g/2L | g/4L |
| NaCl | S7653 | 58.44 | 124 | 7.247 | 14.493 | 28.986 |
| KCl | P9333 | 74.56 | 2.5 | 0.186 | 0.373 | 0.746 |
| $NaH_2PO_4$ | S8282 | 119.98 | 1 | 0.120 | 0.240 | 0.480 |
| $NaHCO_3$ | S6297 | 84.01 | 26.2 | 2.201 | 4.402 | 8.804 |
| Glucose | G7528 | 180.16 | 11 | 1.982 | 3.964 | 7.927 |
| Na pyruvate | P5280 | 110.04 | 2 | 0.220 | 0.440 | 0.880 |

(continued)

| Recording aCSF (22°C) | | | | | | |
|---|---|---|---|---|---|---|
| Chemical | Sigma # | F.W | Conc (mM) | g/1L | g/2L | g/4L |
| Na L-ascorbate | A4034 | 198.11 | 1 | 0.198 | 0.396 | 0.792 |
| Daily addition to the recording aCSF (4°C) | | | | | | |
| Stock solution | Conc (mM) | ml/600mL | ml/1L | ml/2L | ml/4L | ml/3.5L |
| 1M $MgSO_4$ | 1.3 | 0.65 | 1.3 | 2.6 | 5.2 | 4.55 |
| 1M $CaCl_2$ | 2.5 | 1.25 | 2.5 | 5 | 10 | 8.75 |

All electrophysiological experiments were conducted at 27 to 29°C. Rodent (mice) was perfused with flowing blood from a sucrose-based aCSF solution (4°C) using gravity. The brain of the rodent (mice) was removed and cut into 400 um at a 50° angle (mouse at a 55° angle) to attach the cut portion to the vibratome disc to be the bottom. Brain slice was incubated in Incubate aCSF solution at 35°C for 30 minutes or Incubate aCSF solution at RT for 1 hour. Cortical tissue from mice was prepared according to protocol above then was homogenized and lysed for protein extraction. The protein lysate was prepared from the tissue with NP40(FNN0021, Invitrogen) protein lysis buffer containing protease & phosphate inhibitor according to the manufacturer's instruction. Protein concentrations were determined using the BCA Protein Assay Kit (Pierce Biotechnology) with bovine serum albumin as a standard. Equal amounts of proteins (20 μg per lane) were separated by electrophoresis on 8% or 12% SDS-polyacrylamide gel, followed by blotting onto PVDF membranes (GE Healthcare). To block nonspecific binding, the membranes were incubated for 1 h at room temperature in 5% non-fat dry milk in PBST (150 mM NaCl, 50 mM Tris, pH 7.4, 0.05% Tween 20). The following antibodies were incubated overnight at 4°C in 2% blocking solution: Anti-HDAC4 (SAB4300413, Sigma-Aldrich), Anti-BDNF (ANT-010, Alomone Las), Anti-NMDAR2B (ab65783, abcam plc). Following the incubation with antibodies and several rinses in PBST, the membranes were incubated for 1 h with the mouse anti-rabbit IgG-HRP conjugated secondary anti-bodies (sc-2357, Santa Cruz). Each blot was subsequently re-probed with anti-β-actin antibody (sc-47778, Santa Cruz). Housekeeping protein β actin was detected as a normalization control. Antibodies used for methods described herein are as follows:

| | | |
|---|---|---|
| HDAC4 | 1st Ab | SIGMA (SAB4300413) |
| | 2nd Ab | Santa Cruz (sc 2357) |
| BDNF | 1st Ab | Alomone labs (ANT-010) |
| | 2nd Ab | Santa Cruz (sc 2357) |
| GluN2B | 1st Ab | Abcam (ab65783) |
| | 2nd Ab | Santa Cruz (sc 2357) |
| β-actin | 1st Ab | Santa Cruz (sc 47778) |
| | 2nd Ab | Santa Cruz (sc-020) |

**Example 5. Therapeutic efficacy in PTSD**

**Example 5.1. Animal obtained and management**

The animals were obtained from the DBL. Animals are obtained 7 days before the test start. Twenty male SD-rats aged about 7 weeks were used. Animals were maintained in conventional environmental conditions according to the 12/12 hours light-dark cycle in the colony room, with access to food and water *ad libitum.* The rats were acclimatized to their new housing cage over a period of 7 days. The animals were carried out for all tests. All experiments were performed in accordance with the Guide for the Care and Use of Laboratory Animals, and all efforts were made to minimize animal suffering. Weight was measured at 3-day intervals from Day 7.

**Example 5.2. Rat Modeling of PTSD**

This protocol involves two times exposure to foot shock without an opportunity of escape.

The Context A (Day -1)

The weight was measured three hours before the experiment. The temperature of the laboratory was maintained at 22°C. The animals were placed in the dark laboratory space for 10 minutes to adapt. The animals were each placed in the first room. Two minutes later, a door separating the two compartments was opened, and let the animals move freely on both sides. After 5 minutes, 15 times foot shocks of 1 mA/2 sec were applied at random time (90 to 270 seconds) intervals. After the test was completed, the animal was left in the box for one minute and moved to the colony room.

The Context B (Day 0)

The weight was measured three hours before the experiment. The temperature of the laboratory was maintained at 22°C. The animals were placed in the dark laboratory space for 10 minutes to adapt. The animal was placed in the first compartment with sandpapers attached. At this time, the door remained closed, and thus the animals were not allowed to cross to the second compartment. After 10 minutes, 3 times foot shocks of 2 mA/sec were applied to the animal. After the test was completed, the animal was left in the box for one minute and moved to the colony room.

Compound injection

The compound was diluted to 25 mg/kg using DMSO:PEG400: Saline (5:55:40). From Day 7 for 24 days the compound was administered intraperitoneal at 10:00 a.m. daily (*i.p.* volume was 200 μl). Control group was also administered by *i.p.* injection in the same manner using the vehicle (DMSO:PEG400:Saline (5:55:40)).

Freezing check

For the freezing check, 7 days after the initial foot shock protocol, the rats were placed in the first compartment with sandpaper of the apparatus for 5 minutes (no foot shock) and the freezing behavior was recorded using shutavoid_v1803 program (Panlab, Spain) according to the manufacturer's instruction. At this time, the door remained closed, like the context B. Freezing was defined as a state without skeletal muscle movement of the rat. The animal with a freezing time of 240 seconds or less were excluded. Excluded animals were sacrificed using $CO_2$.

Data analysis

In front of the clear acrylic plate of the electronic box, a camera was placed to check all animal movements. All groups were freezing checked every three days from Day 7. Each animal was film for 5 minutes. Based on the video, the freezing time of each animal was checked. The freezing time for Day 7 was set to 100%, and then the freezing time for each day was converted into a percentage.

**Example 6. Therapeutic efficacy in AD**

**Example 6.1. Animal obtained and management**

APP/PS1 mice, which is an animal model of Alzheimer's disease, were obtained from Taconic Bioscience. Animals are obtained 7 days before the test start. Twenty male SD-rats aged about 15 months were used. Animals were maintained in conventional environmental conditions according to the 12/12 hours light-dark cycle in the colony room, with access to food and water *ad libitum.* The mice were acclimatized to their new housing cage over a period of 7 days. The animals were carried out for all tests. All experiments were performed in accordance with the Guide for the Care and Use of Laboratory Animals, and all efforts were made to minimize animal suffering. Weight was measured at 3-day intervals from Day 7.

**Example 7. Selection of BnH12001**

**Example 7.1. *In vitro* cytotoxicity**

The cytotoxicity of the compound was evaluated by treating cells with BnH12001 (racemate) and measuring cell viability.

As a result of measuring cell viability in response to BnH12001 (0, 0.1, 1, 10, 100 μM) treatment in human cell lines 293T and SH-SY5Y, no significant change in cell viability was observed in all concentration ranges, confirming the cytotoxic safety of BnH12001 (FIGS. 1A and 1B).

### Example 7.2. Analysis of effect of *in vitro* HDAC4 reduction on gene expression

The BnH12 series drug according to the present disclosure was synthesized as a compound targeting HDAC4, which inhibits the expression of BDNF. Therefore, it was confirmed whether the expression of BDNF increased by the degradation of HDAC4 according to BnH12001 treatment. In addition, it is known that the increase in the expression of BDNF increases the expression of GluN2B, and the mRNA level of GluN2B was also measured together with BDNF to measure the efficacy of BnH12001 in degrading HDAC4.

When the mRNA levels of BDNF and GluN2B were measured 6 hours after treating 293T cells with BnH12001 (racemate), the expression of BDNF and GluN2B was significantly increased compared to the group that was not treated with the compound. BnH12001 effectively degrades HDAC4, and as a result, it was confirmed that BnH12001 exhibited the predicted efficacy very well *in vitro* by inducing an increase in the expression of BDNF and GluN2B (FIG. 2).

When BnH12001 (racemate) was applied to primary cultured cortical neurons using the same experimental procedure as above, the expression of HDAC4 protein was significantly suppressed, and the expression of BDNF and GluN2B was significantly increased (FIG. 3).

### Example 7.3. Analysis of efficacy in enhancing *in vitro* synaptic plasticity

After the administration of BnH12001 (racemate), the potency and GABA/AMPA ratio were measured in the barrel cortex to confirm the compound efficacy, and it was attempted to confirm the effective concentration through the dose response curve of the BnH12001.

To this end, after 8-week-old C57BL/6J mice were grouped into a control group (vehicle administration group) and an experimental group (BnH12001 administration group) by 5 mice each, and after Os pump injection surgery, BnH12001 was directly injected into layer 4 of the barrel cortex at a total dose of 3 μM continuously for 2 weeks using an Os pump (FIGS. 4A and 4B).

Upon the compound administration of the compound BnH12001, as confirmed by the measurement of the GABA/AMPA ratio in the mouse barrel cortex (layer 4), it was confirmed that while the balance of excitation/inhibition in the mouse barrel cortex was maintained (FIG. 4C), the excitatory TC synaptic potency (TC potency) was significantly increased (FIG. 4D). Since an increase in the TC potency indicates that synaptic plasticity is increased, the above results show that the administration of BnH12001 exhibits an effect of increasing the TC synaptic potency in the sensory cortex, while maintaining the balance of excitation/inhibition, which indicates that the BnH12001 is effective in improving cognitive ability.

8-week-old C57BL/6J mice were orally administrated at a dose of 1 mg/kg. In the control group and the compound administration group, the experiments were conducted with 5 mice, respectively. As a result, it was confirmed that the administration of BnH12001 could increase the TC synaptic potency in the sensory cortex while maintaining the balance of excitation/inhibition (FIGS. 5A and 5B). Meanwhile, changes in the TC EPSC potency in the mouse barrel cortex (layer) in response to the administration of BnH12001 at each dose (0.03, 0.1, 0.3, 1, 3, and 10 mg/kg) were observed. For each concentration of the control group and the compound administration group, the experiments were conducted with 5 mice. As a result, it was confirmed that EC50=0.3023 for BnH12001 (FIG. 5C).

### Example 7.4. Analysis of efficacy of enhancing *ex vivo* synaptic plasticity in Alzheimer's animal model

Using APP/PS1 mice (Taconic Bioscience) (15 months old) as Alzheimer's animal model, it was attempted to confirm the efficacy of administering BnH12001 enantiomer (3 S,6R, 98%<) alone or in combination with aducanumab (Creative Biolabs). Here, an isotype antibody was used as a control antibody. The administration schedule was as shown in FIG. 6A (for intravenous administration, see Example 4.4). For each administration group, five mice were used.

As a result of observing the changes in slope of the field excitatory postsynaptic potential (fEPSP) in the CA1 region of the Schaffer collateral circuit in the hippocampus, it was confirmed that BnH12001 enantiomer (3S,6R, 98%<) alone, aducanumab alone, and the combination of BnH12001 enantiomer (3S,6R, 98%<) and aducanumab all increased synaptic efficacy (FIG. 6B). In addition, as a result of the LTP measurement of fEPSP in the CA1 region of Schaffer's collateral circuit in the hippocampus, it was also confirmed that the synaptic long-tern potentiation (LTP) of the excitatory SC pathway was also significantly increased (FIG. 6B). Since increases in fEPSP slope and LTP indicate that synaptic plasticity is increased, it was found from these results that the administration of BnH12001 enantiomer (3 S,6R, 98%<) alone and in combination with aducanumab increased the SC pathway synaptic potency in the hippocampus, which indicates that the administration of BnH12001 enantiomer (3 S,6R, 98%<) alone and in combination with aducanumab is effective in treating Alzheimer's disease.

Meanwhile, after administering BnH12001 enantiomer (3S,6R, 98%<) alone or in combination with aducanumab to APP/PS1 mice, it was attempted to confirm the compound efficacy for Alzheimer's disease by measuring the potency and GABA/AMPA ratio in the sensory cerebral cortex, and in particular, it was attempted to compare the effects of the single

administration and combinational administration of BnH12001 enantiomer (3S,6R, 98%<) and aducanumab.

When BnH12001 enantiomer (3S,6R, 98%<) was administered alone or together with aducanumab to APP/PS1 mice, such administration increased the excitatory TC synaptic potency (TC potency) (left diagram in FIG. 6C), while maintaining the balance of excitation/inhibition (right diagram in FIG. 6C) as confirmed by the measurement of the GABA/AMPA ratio. Since an increase in TC potency indicates that synaptic plasticity is increased, it was found from these results that the administration of BnH12001 enantiomer (3S,6R, 98%<) alone or in combination with aducanumab increased the TC synaptic potency in the sensory cortex, while maintaining the balance of excitation/inhibition, which indicates that the administration of BnH12001 enantiomer (3S,6R, 98%<) alone or in combination with aducanumab is effective in treating Alzheimer's disease. In this case, a significantly excellent effect was exhibited when BnH12001 enantiomer (3S,6R, 98%<) and aducanumab were co-administered compared to when BnH12001 enantiomer (3S,6R, 98%<) and aducanumab were administered alone, respectively.

**Example 8. Surface plasmon resonance assay**

Compounds of the present disclosure were selected for their ability to interact with HDAC4. To conduct the test, surface plasmon resonance assay was used. The samples were prepared as follows and loaded onto Biacore T200.

| Type | Sample | Stock | Solvent | M.W. |
|---|---|---|---|---|
| Ligand 1 | HDAC4 | 20 ug | 25 mM Tris-HCl, pH 7.3, 100 mM glycine, 10% glycerol | 118.9 kDa |
| Analyte 1 | BnHR12001 (racemate) | 3.6 mg | DMSO | 313.75 Da |

The samples were immobilized under the following conditions. The protein stock was diluted with 10 mM sodium acetate. 1 M ethanolamine (pH 8.5) was used as a blocking agent.

| Chip No | Ligand | Conc. | Immobilization Buffer | Immobilization level |
|---|---|---|---|---|
| #1 | HDAC4 | 50 ug/mL | Sodium acetate, pH 5.0 | 5815.5 RU |

FIG. 7 illustrates the binding kinetics of BnH12001 to HDAC4.

**Example 9. Effect of compounds on HDAC catalytic activities**

The compound (racemate) was resuspended in 100 mM DMSO and tested in a 10-dose $IC_{50}$ with 3-fold serial dilution starting at 100 $\mu$M against HDACs 1-9, and 11. As reference compounds, Trichostatin A (TSA) and TMP269 were tested in a 10-dose $IC_{50}$ with 3-fold serial dilution starting at 10 $\mu$M or 1 $\mu$M.

10 $\mu$M Fluorogenic peptide from p53 residues 379-382 (RHKK(Ac)AMC) was used for the substrate for HDAC 1, 2, 3, and 6. 50 $\mu$M Fluorogenic HDAC Class2a Substrate (Trifluoroacetyl Lysine) was used for the substrate for HDAC 4, 5, 7, 9, and 11. 100 $\mu$M Fluorogenic peptide from p53 residues 379-382 (RHK(Ac)K(Ac)AMC) was used for the substrate for HDAC8.

$IC_{50}$ values were calculated using the GraphPad Prism 4 program based on a sigmoidal dose-response equation and the blank (DMSO) value concentration was entered as 1.00E-12 M for curve fitting. Curve fits were performed for compound data sets that displayed enzyme activity less than 65% at the highest concentration of compound tested.

The experiments were performed in duplicates. BnH12001 did not show any inhibitory effect on HDAC 1 to 9, and 11's catalytic activities.

**Example 10. Effects on expressions of BDNF and GluN2B, and thalamocortical EPSC potency**

When HDAC4 protein expression was suppressed by transfecting lentiviral HDAC4 shRNA, BDNF and GluN2B expression was significantly increased in SH-SY5Y cells (FIGS. 8A and 8B). Similarly, BnH12001 (racemate) (1 $\mu$M) augmented BDNF and GluN2B expression by application for 6 and 24 hours in SH-SY5Y cells (FIGS. 9A and 9B). BnH12001 significantly suppressed HDAC4 protein expression by oral and intraperitoneal administration, respectively, in mice barrel cortex and this inhibitory effect was maintained at least for 9 days (FIGS. 10A and 10B).

The present inventors investigated the effect of BnH12001 on thalamocortical EPSC potency and involvement of BDNF-TrkB signaling in it. As shown in the figure (A1 to B of FIG. 11A), TC EPSC potency was significantly increased by applying BDNF to the mice barrel cortex using Alzet osmotic pump without alteration of IPSC/EPSC ratio. This BDNF-induced augmentation of TC EPSC potency is almost completely prevented by cyclotraxin B (1 $\mu$M) application (A1 to B in

FIG. 11A). Similarly, orally-applied BnH12001 (3 μM) significantly augmented TC EPSC potency in the layer 4 barrel cortex (A1 to B in FIG. 11B) and this increasing effect was nearly completed hindered by cyclotraxin B (1 μM) application (C1 to D in FIG. 11B).

**Example 11. Confirmation of efficacy of BnH12001 enantiomer**

**Example 11.1. Analysis of efficacy of enhancing *ex vivo* synaptic plasticity**

After the oral administration of BnH12001 enantiomer (3R,6S, 98%<) and BnH12001 enantiomer (3S,6R, 98% <), the potency and GABA/AMPA ratio were measured in the barrel cortex to confirm the efficacy of these compounds.

Each of BnH12001 (racemate), BnH12001 enantiomer (3R,6S, 98%<), and BnH12001 enantiomer (3S,6R, 98% <) was orally administered to 8-week-old C57BL/6J mice at a dose of 1 mg/kg. In the control group (vehicle) and the compound administration group, the experiments were conducted with 5 mice, respectively. As a result, it was confirmed that both the racemate and the enantiomer could increase the TC synaptic potency in the sensory cortex while maintaining the balance of excitation/inhibition, and the both enantiomers showed better efficacy than the racemate, and in particular, the BnH12001 enantiomer (3S,6R, 98%<) showed the highest efficacy (FIGS. 12A and 12B).

**Example 11.2. Effects on expressions of BDNF and GluN2B, and thalamocortical EPSC potency**

BnH12001 enantiomer (3S,6R, 98%<) (1 μM) increased BDNF and GluN2B expression in cultured primary cortical neurons (DIV 14) by 24-hour treatment (FIG. 13A).

In addition, the BnH12001 enantiomer (3S,6R, 98%<) was orally administered at a dose of 1 mg/kg to 8-week-old C57BL/6J mice, and the mice were sacrificed 2 days later to observe changes in the expression of HDAC4, BDNF, and GluN2B. As a result, it was confirmed that when the BnH12001 enantiomer (3S,6R, 98%<) was administered, HDAC4 was effectively degraded and the expression of BDNF and GluN2B significantly increased, compared to the group (vehicle) that was not treated with the compound. Therefore, it was confirmed that the BnH12001 enantiomer (3S,6R, 98%<) exhibited excellent efficacy even *in vivo* (FIG. 13B).

Meanwhile, the BnH12001 enantiomer (3S,6R, 98%<) was orally administered at a dose of 1 mg/kg to 13-month-old C57BL/6J mice, and the mice were sacrificed 2 days later to observe changes in the expression of GluN2B. As a result, it was confirmed that when the BnH12001 enantiomer (3S,6R, 98%<) was administered, the expression of GluN2B significantly increased compared to the group (vehicle) that was not treated with the compound (FIG. 13C).

**Example 12. *In vitro* cytotoxicity of various BnH12 series compounds**

By treating cells with various BnH12 series compounds and measuring cell viability using the experimental procedure described in Example 2, the cytotoxicity of the compounds was evaluated. As a result, the cells showed a cell viability of 85% or higher for almost all compounds at a concentration of 10 μM, and showed a cell viability of 80% or higher even when the concentration was increased to 100 μM.

[Table 5]

| Compound name | Cell viability test results (CCK) | |
|---|---|---|
| | 10 uM | 100 uM |
| BnH12001(3S,6R, 98%<) | 99% < | 95% < |
| BnH12004 | 95% < | 85% < |
| BnH12005 | 99% < | 90% < |
| BnH12006 | 99% < | 95% < |
| BnH12007 | 95% < | 95% < |
| BnH12008 | 95% < | 90% < |
| BnH12009 | 95% < | 90% < |
| BnH12011 | 95% < | 95% < |
| BnH12012 | 95% < | 90% < |
| BnH12013 | 99% < | 99% < |
| BnH12014 | 99% < | 90% < |
| BnH12016 | 99% < | 90% < |
| BnH12018 | 90% < | 90% < |
| BnH12019 | 99% < | 90% < |
| BnH12020 | 95% < | 95% < |
| BnH12021 | 90% < | 80% < |

| | | |
|---|---|---|
| BnH12022 | 99% < | 99% < |
| BnH12023 | 95% < | 80% < |
| BnH12024 | 99% < | 99% < |
| BnH12025 | 90% < | 80% < |
| BnH12026 | 99% < | 99% < |
| BnH12028 | 90% < | 80% < |
| BnH12029 | 99% < | 80% < |
| BnH12030 | 95% < | 85% < |
| BnH12031 | 90% < | 75% < |
| BnH12032 | 99% < | 95% < |
| BnH12033 | 90% < | 65% < |
| BnH12034 | 90% < | 85% < |
| BnH12035 | 90% < | 85% < |
| BnH12038 | 85% < | 85% < |
| BnH12039 | 95% < | 90% < |
| BnH12040 | 85% < | 80% < |
| BnH12041 | 90% < | 70% < |
| BnH12060 | 99% < | 99% < |
| BnH12061 | 95% < | 95% < |
| BnH12062 | 85% < | 75% < |

| | | |
|---|---|---|
| BnH12063 | 95% < | 95% < |
| BnH12065 | 95% < | 95% < |
| BnH12068 | 95% < | 95% < |
| BnH12069 | 80% < | 80% < |
| BnH12070 | 99% < | 90% < |
| BnH12073 | 85% < | 85% < |
| BnH12074 | 85% < | 80% < |
| BnH12075 | 99% < | 99% < |
| BnH12076 | 95% < | 95% < |
| BnH12079 | 99% < | 90% < |
| BnH12094 | 80% < | 70% < |
| BnH12096 | 70% < | 65% < |

As a result of measuring cell viability depending on the compound concentration (10 or 100 μM) in 293T cell line, most compounds showed no significant change in cell viability in both concentration ranges.

**Example 13. Confirmation of efficacy of various BnH12 series compounds**

Using the experimental procedure described in Example 3, it was determined whether various BnH12 series compounds increase GluN2B expression, and the results are shown in Table 6. These *in vitro* experimental results are expressed as relative values to the group (vehicle) that was not treated with the compound, with the effect of the group set to 1.

[Table 6]

| Compound name | Western blot results | |
|---|---|---|
| | Mean | Standard Error |
| BnH12001(3S,6R, 98%<) | 1.548 | 0.039 |
| BnH12005 | 1.206 | 0.151 |
| BnH12006 | 1.238 | 0.134 |
| BnH12007 | 1.311 | 0.177 |
| BnH12009 | 1.248 | 0.115 |
| BnH12010 | 1.074 | 0.146 |
| BnH12016 | 1.311 | 0.04 |
| BnH12018 | 1.381 | 0.029 |
| BnH12025 | 1.582 | 0.181 |
| BnH12038 | 1.811 | 0.26 |
| BnH12039 | 1.103 | 0.115 |
| BnH12060 | 1.469 | 0.109 |
| BnH12061 | 1.688 | 0.2598 |
| BnH12063 | 1.652 | 0.097 |
| BnH12065 | 1.326 | 0.02251 |
| BnH12068 | 1.286 | 0.1548 |

| | | |
|---|---|---|
| BnH12069 | 1.993 | 0.2935 |
| BnH12070 | 1.302 | 0.1805 |
| BnH12073 | 2.013 | 0.117 |
| BnH12074 | 2.146 | 0.175 |
| BnH12079 | 1.062 | 0.04795 |
| BnH12096 | 2.848 | 0.008825 |

As can be seen from Table 6, the representative compounds of the present disclosure showed superior effects compared to the vehicle. In particular, BnH12001 enantiomer (3S,6R, 98%<), BnH12018, BnH12025, BnH12038, BnH12060, BnH12061, BnH12063, BnH12069, BnH12073, BnH12074, and BnH12096 showed excellent effects.

## Claims

**1.** A compound represented by Formula (1), a stereoisomer thereof, or a pharmaceutically acceptable salt thereof:

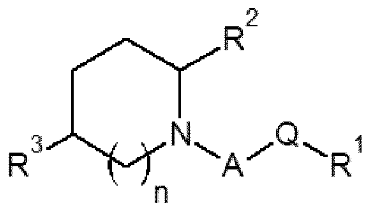

(1)

in the formula,

$R^1$ is $C_{1-6}$ alkyl, $C_{3-12}$ cycloalkyl, aryl, or heteroaryl, wherein at least one hydrogen atom in these groups is unsubstituted or each independently substituted with a halogen atom, $C_{1-6}$ alkyl, $C_{1-6}$ alkoxy, nitro, amino optionally substituted with at least one $C_{1-6}$ alkyl, or hydroxy,
A is C=O or a bond,
Q is $-(CR^4R^5)_m-$ or a bond, wherein $R^4$ and $R^5$ are each independently hydrogen or $C_{1-6}$ alkyl, and m is an integer from 1 to 6,
$R^2$ is hydrogen, $C_{1-6}$ alkyl, or aryl,
$R^3$ is hydrogen, carboxyl, N-hydroxy carboxamide, or carboxamide, wherein at least one hydrogen in these groups is unsubstituted or substituted with $C_{1-6}$ alkyl, and
n is 0 or 1.

**2.** The compound of claim 1, a stereoisomer thereof, or a pharmaceutically acceptable salt thereof, wherein $R^1$ is $C_{1-6}$ alkyl, $C_{3-12}$ cycloalkyl, phenyl, pyridinyl, pyridazinyl, pyrimidinyl, pyrazinyl, pyrrole, furanyl, or thiophenyl, wherein at least one hydrogen atom in these groups is unsubstituted or each independently substituted with a halogen atom, $C_{1-6}$ alkyl, $C_{1-6}$ alkoxy, nitro, amino optionally substituted with at least one $C_{1-6}$ alkyl, or hydroxy.

**3.** The compound of claim 1, a stereoisomer thereof, or a pharmaceutically acceptable salt thereof, wherein $R^1$ is methyl, cyclohexyl, phenyl, 4-methoxyphenyl, 4-(tert-butyl)phenyl, 2-chlorophenyl, 3-chlorophenyl, 4-chlorophenyl, 4-fluorophenyl, 4-bromophenyl, 3,4-dichlorophenyl, 2-chloro-4-fluorophenyl, p-tolyl, m-tolyl, pyridin-4-yl, thiophen-2-yl, furan-2-yl, pyridin-3-yl, pyridin-2-yl, pyrimidin-5-yl, pyrimidin-2-yl, 4-nitrophenyl, 4-aminophenyl, 3-nitrophenyl, 3-aminophenyl, 4-methoxy-pyridin-3-yl, 6-methoxy-pyridin-3-yl, 4-(dimethylamino)phenyl, or 4-hydroxy-phenyl.

**4.** The compound of claim 1, a stereoisomer thereof, or a pharmaceutically acceptable salt thereof, wherein $R^2$ is H or $C_{1-6}$ alkyl.

**5.** The compound of claim 1, a stereoisomer thereof, or a pharmaceutically acceptable salt thereof, wherein $R^3$ is H, carboxyl, methyl carboxyl, ethyl carboxyl, N-hydroxy carboxamide, carboxamide, or sodium carboxylate.

**6.** A compound represented by Formula (2-A) or (2-B), a stereoisomer thereof, or a pharmaceutically acceptable salt thereof:

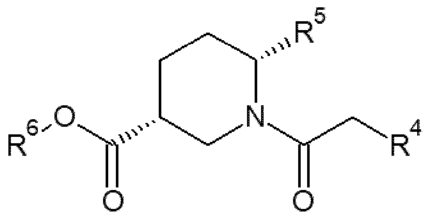 (2-A) or 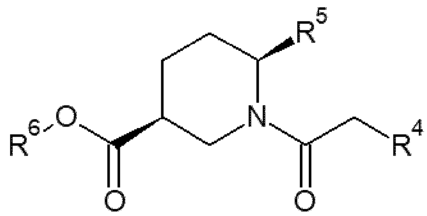 (2-B)

in the formula,

$R^4$ is $C_{1-6}$ alkyl, $C_{3-12}$ cycloalkyl, phenyl, pyridinyl, pyridazinyl, pyrimidinyl, pyrazinyl, pyrrole, furanyl, or thiophenyl, wherein at least one hydrogen atom in these groups is unsubstituted or each independently substituted with a halogen atom, $C_{1-6}$ alkyl, $C_{1-6}$ alkoxy, nitro, amino optionally substituted with at least one $C_{1-6}$ alkyl, or hydroxy, and
$R^5$ and $R^6$ are each independently hydrogen or $C_{1-6}$ alkyl.

**7.** A compound represented by Formula (4), a stereoisomer thereof, or a pharmaceutically acceptable salt thereof:

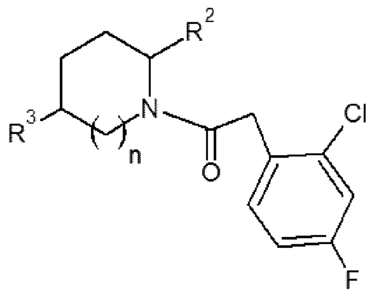 (4)

in the formula,

$R^2$ is hydrogen, $C_{1-6}$ alkyl, or aryl,
$R^3$ is hydrogen, carboxyl, N-hydroxycarboxamide, or carboxamide, wherein at least one hydrogen in these groups is unsubstituted or substituted with $C_{1-6}$ alkyl, and
n is 0 or 1.

**8.** A compound represented by Formula (5), a stereoisomer thereof, or a pharmaceutically acceptable salt thereof:

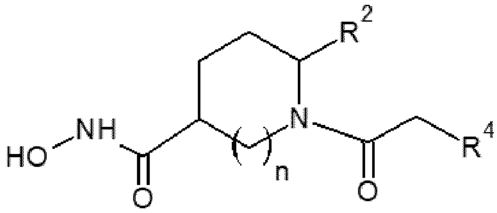 (5)

in the formula,

$R^2$ is hydrogen, $C_{1-6}$ alkyl, or aryl,
$R^4$is phenyl or phenyl substituted with at least one halogen, and
n is 0 or 1.

**9.** The compound of claim 1, a stereoisomer thereof, or a pharmaceutically acceptable salt thereof, wherein the compound is selected from the following compounds:

| Name | Structure | Name | Structure |
|---|---|---|---|
| BnH12001 | 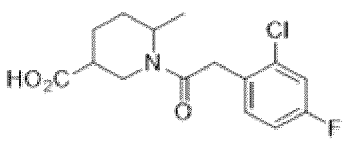 | BnH12001 enantiomer(3R,6S, 98%<) | 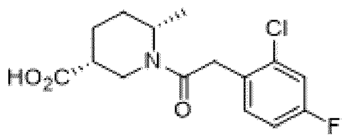 |
| BnH12001 enantiomer (3S,6R, 98%<) | 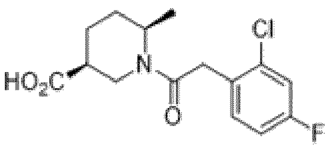 | BnH12004 | 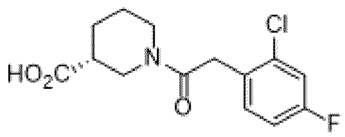 |
| BnH12005 | 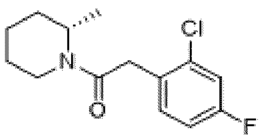 | BnH12006 | 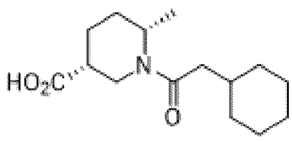 |
| BnH12007 | 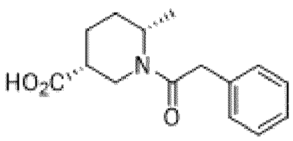 | BnH12008 | 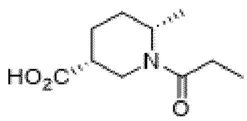 |
| BnH12009 | 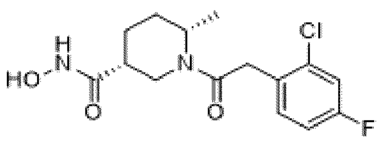 | BnH12010 | 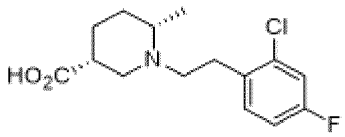 |
| BnH12011 | 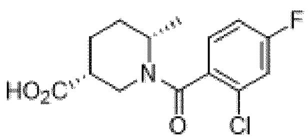 | BnH12012 | 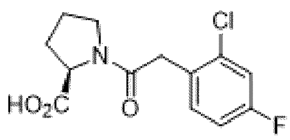 |
| BnH12013 | 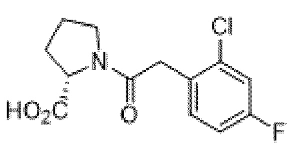 | BnH12014 | 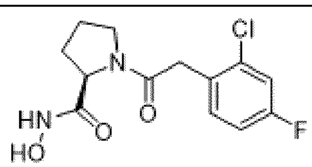 |

| | | | |
|---|---|---|---|
| BnH12016 | 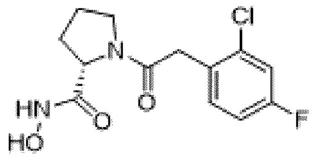 | BnH12018 | 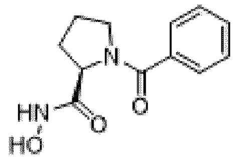 |
| BnH12019 | 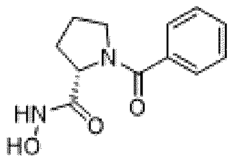 | BnH12020 | 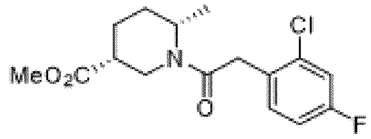 |
| BnH12021 | 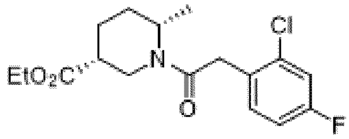 | BnH12022 | 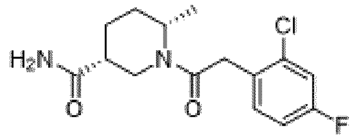 |
| BnH12023 | 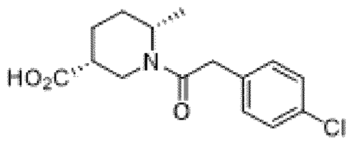 | BnH12024 | 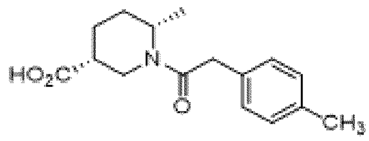 |
| BnH12025 | 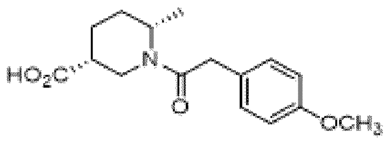 | BnH12026 | 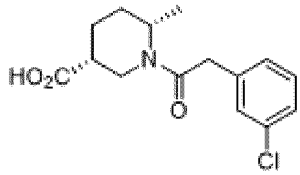 |
| BnH12027 | 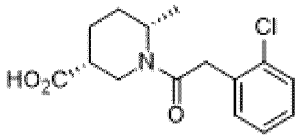 | BnH12028 | 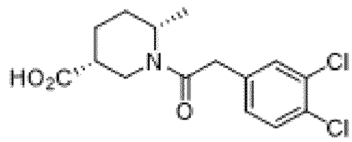 |
| BnH12029 | 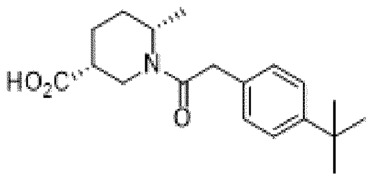 | BnH12030 | 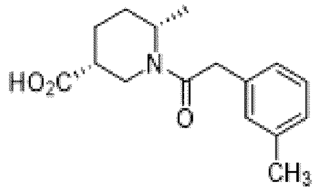 |
| BnH12031 | 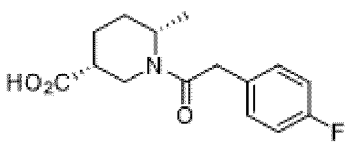 | BnH12032 | 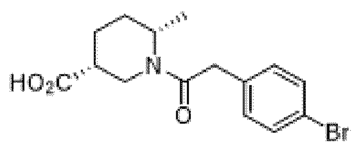 |
| BnH12033 | 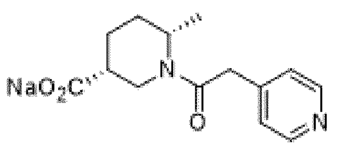 | BnH12034 | 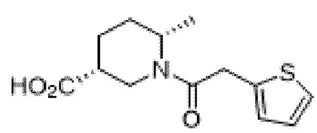 |

| | | | |
|---|---|---|---|
| BnH12035 | 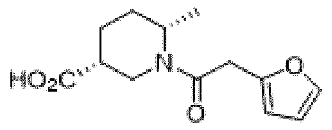 | BnH12038 | 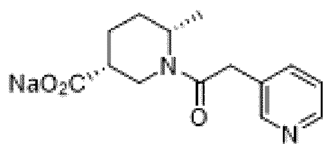 |
| BnH12039 | 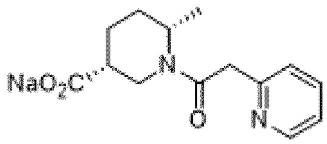 | BnH12040 | 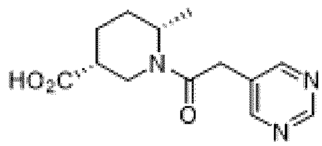 |
| BnH12041 | 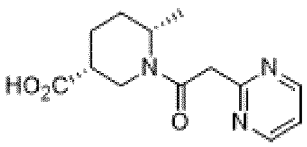 | BnH12060 | 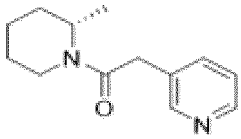 |
| BnH12061 | 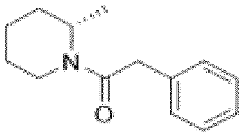 | BnH12062 | 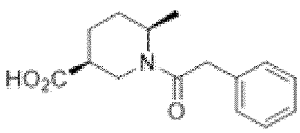 |
| BnH12063 | 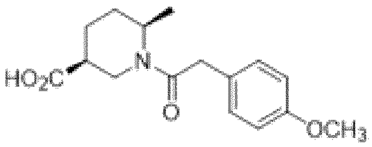 | BnH12065 | 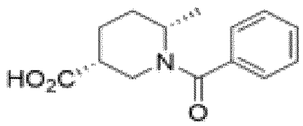 |
| BnH12068 | 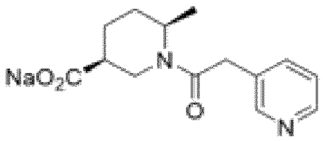 | BnH12069 | 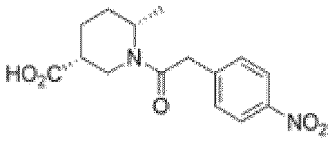 |
| BnH12070 | 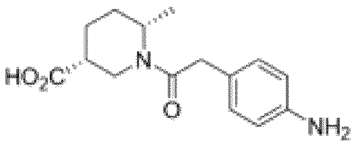 | BnH12073 | 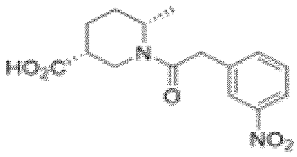 |
| BnH12074 | 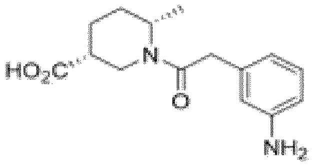 | BnH12075 | 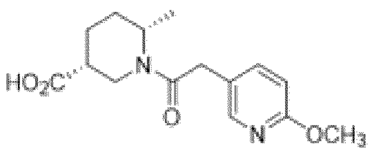 |
| BnH12076 | 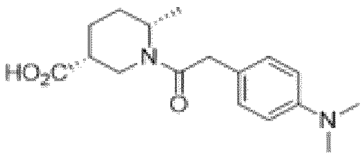 | BnH12079 | 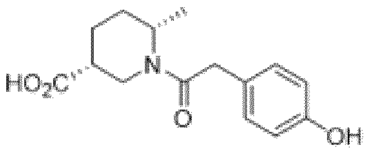 |
| BnH12094 | 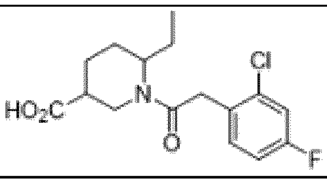 | BnH12096 | 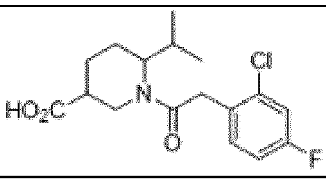 |

**10.** The compound of claim 9, wherein the compound is BnH12001 or an enantiomer thereof.

**11.** The compound of claim 1, a stereoisomer thereof, or a pharmaceutically acceptable salt thereof, wherein the stereoisomer is an optical isomer.

**12.** The compound of claim 1, a stereoisomer thereof, or a pharmaceutically acceptable salt thereof, wherein the compound is an inhibitor of HDAC.

**13.** The compound of claim 12, a stereoisomer thereof, or a pharmaceutically acceptable salt thereof, wherein the HDAC is HDAC4.

**14.** The compound of claim 1, a stereoisomer thereof, or a pharmaceutically acceptable salt thereof, wherein the compound increases synaptic plasticity.

**15.** A pharmaceutical composition comprising, as an active ingredient, the compound of any one of claims 1 to 14, a stereoisomer thereof, or a pharmaceutically acceptable salt thereof.

**16.** The pharmaceutical composition of claim 15, wherein the composition is for prevention or treatment of a neurological disorder or a neurodegenerative disorder.

**17.** The pharmaceutical composition of claim 16, wherein the compound, a stereoisomer thereof, or a pharmaceutically acceptable salt thereof is delivered to the brain at a concentration of about 0.1 to about 3 μM.

**18.** The pharmaceutical composition of claim 16, wherein the disorder is Alzheimer's disease.

**19.** The pharmaceutical composition of claim 16, wherein the disorder is PTSD

**20.** The pharmaceutical composition of claim 15, further comprising a second therapeutic agent.

**21.** The pharmaceutical composition of claim 20, wherein the second therapeutic agent is aducanumab.

**22.** A method for preventing or treating a neurological disorder or a neurodegenerative disorder, comprising administering, to a subject in need thereof, the compound according to any one of claims 1 to 14, a stereoisomer thereof, or a pharmaceutically acceptable salt thereof.

[FIG. 1A]

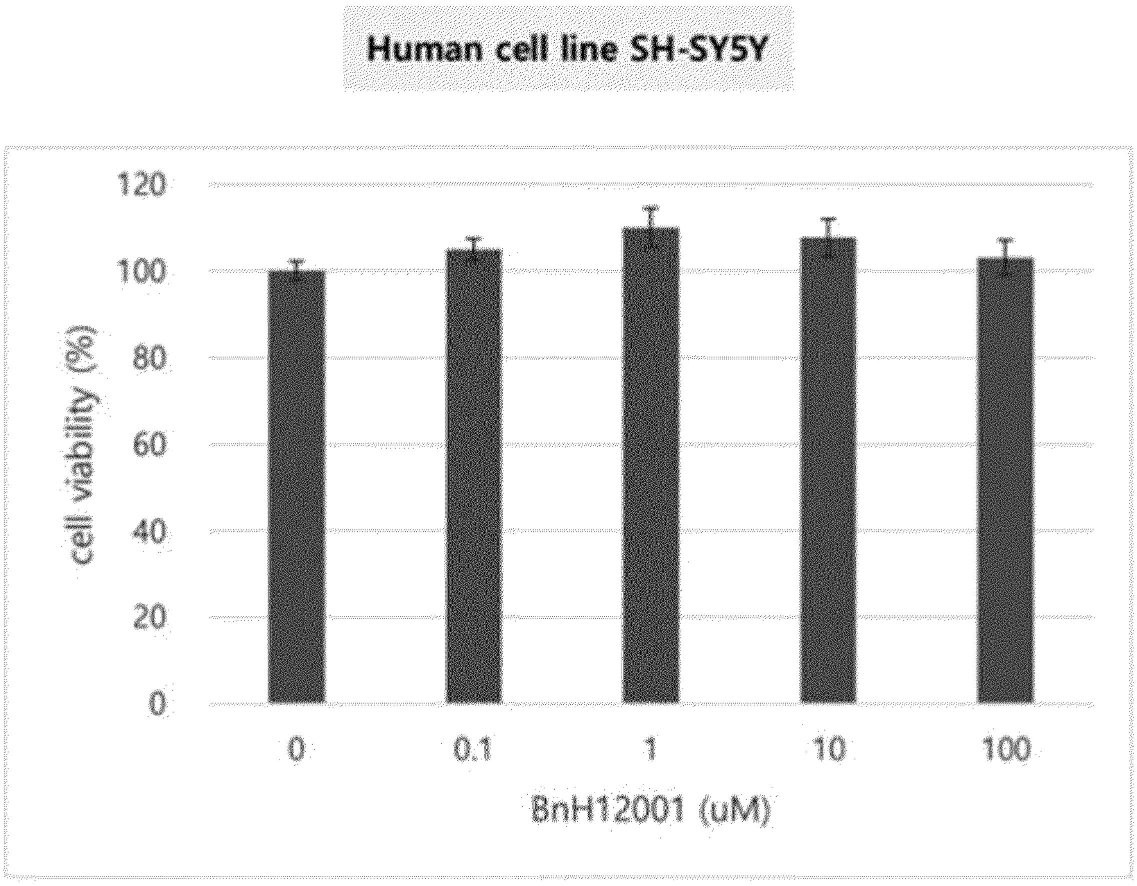
Human cell line SH-SY5Y
120
100
80
60
40
20
0
cell viability (%)
0
0.1
1
10
100
BnH12001 (uM)

[FIG. 1B]

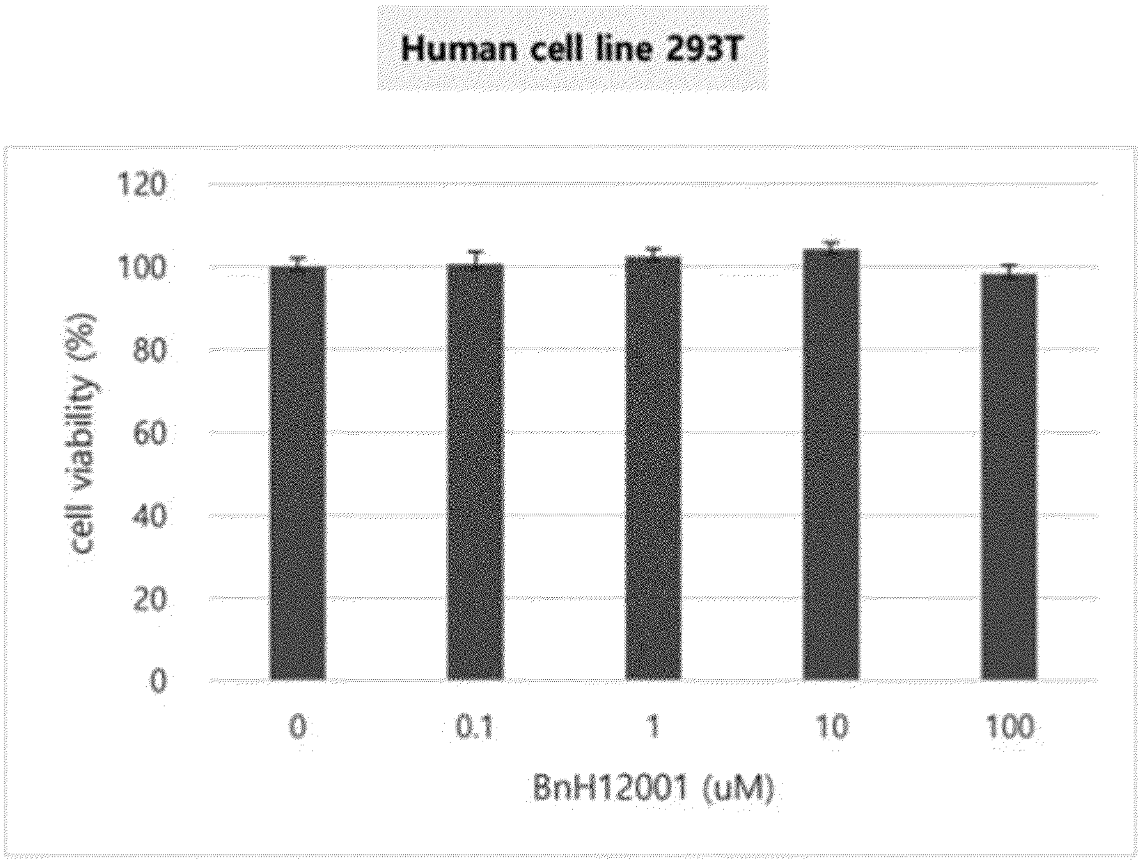
Human cell line 293T
120
100
80
60
40
20
0
cell viability (%)
0
0.1
1
10
100
BnH12001 (uM)

[FIG. 2]

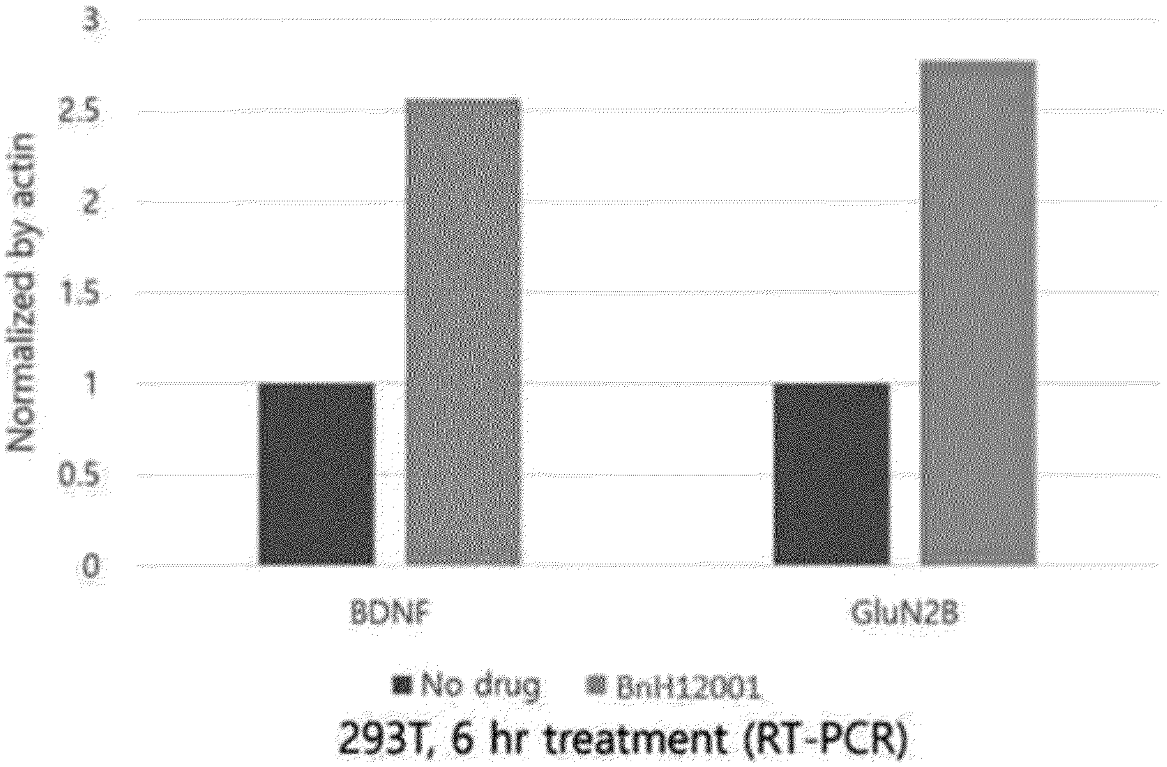
3
2.5
2
1.5
1
0.5
0
Normalized by actin
BDNF
GluN2B
No drug
BnH12001
293T, 6 hr treatment (RT-PCR)

[FIG. 3A]

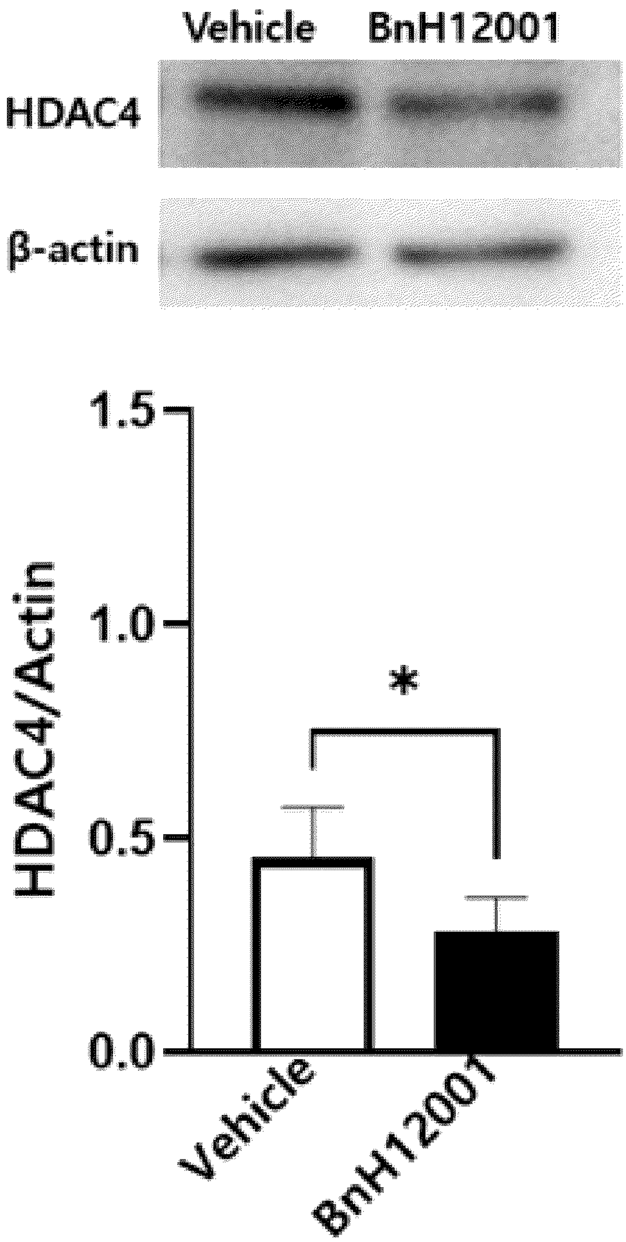
Vehicle
BnH12001
HDAC4
β-actin
1.5
1.0
0.5
0.0
HDAC4/Actin
*
Vehicle
BnH12001

[FIG. 3B]

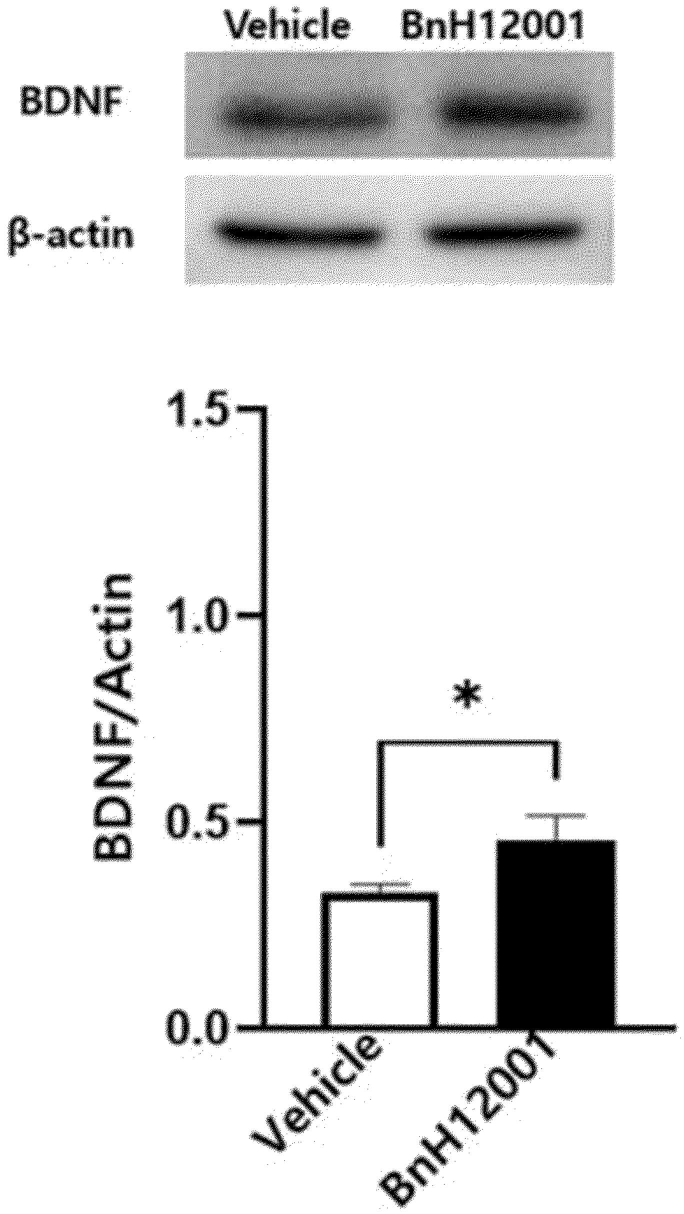
Vehicle
BnH12001
BDNF
β-actin
1.5
1.0
0.5
0.0
BDNF/Actin
*
Vehicle
BnH12001

[FIG. 3C]

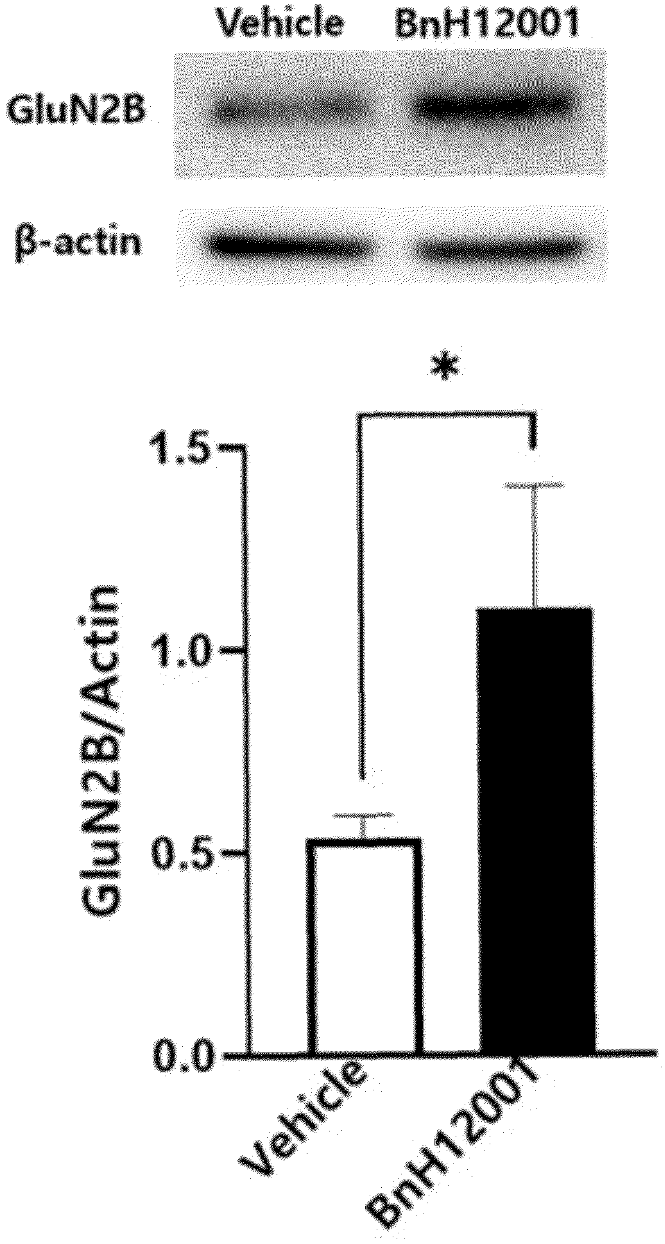
Vehicle
BnH12001
GluN2B
β-actin
*
1.5
1.0
0.5
0.0
GluN2B/Actin
Vehicle
BnH12001

[FIG. 4A]

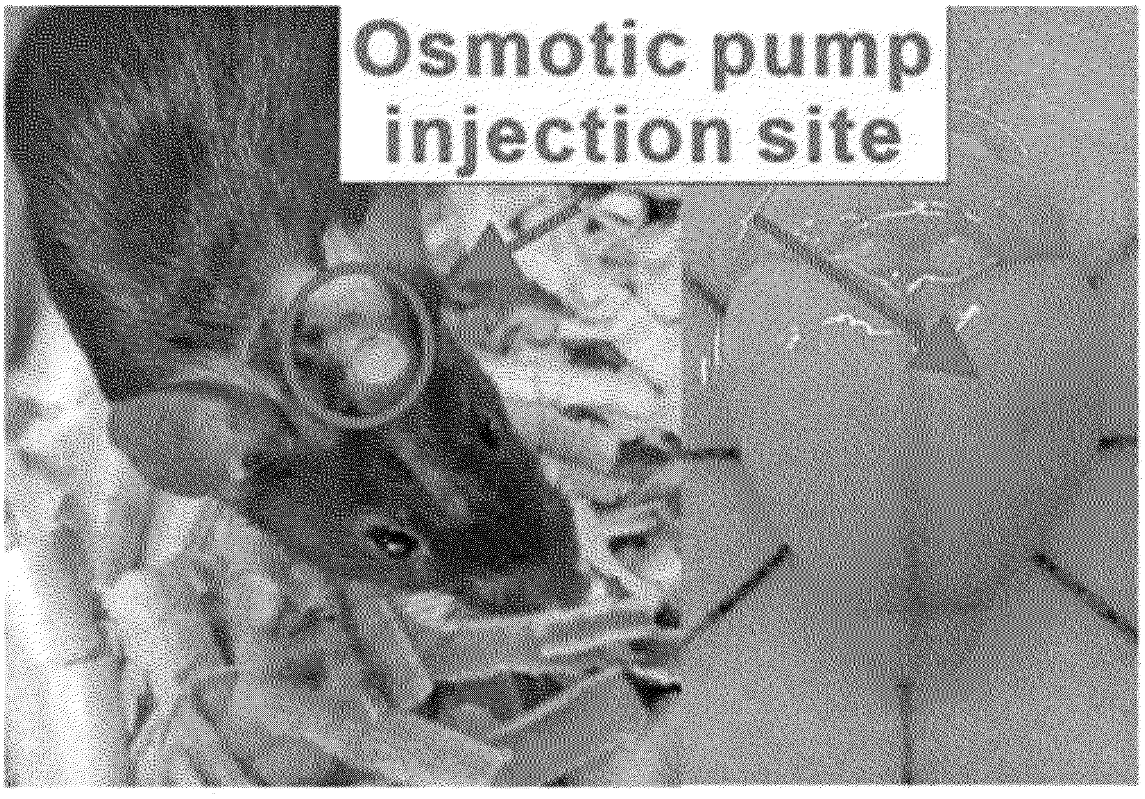

[FIG. 4B]

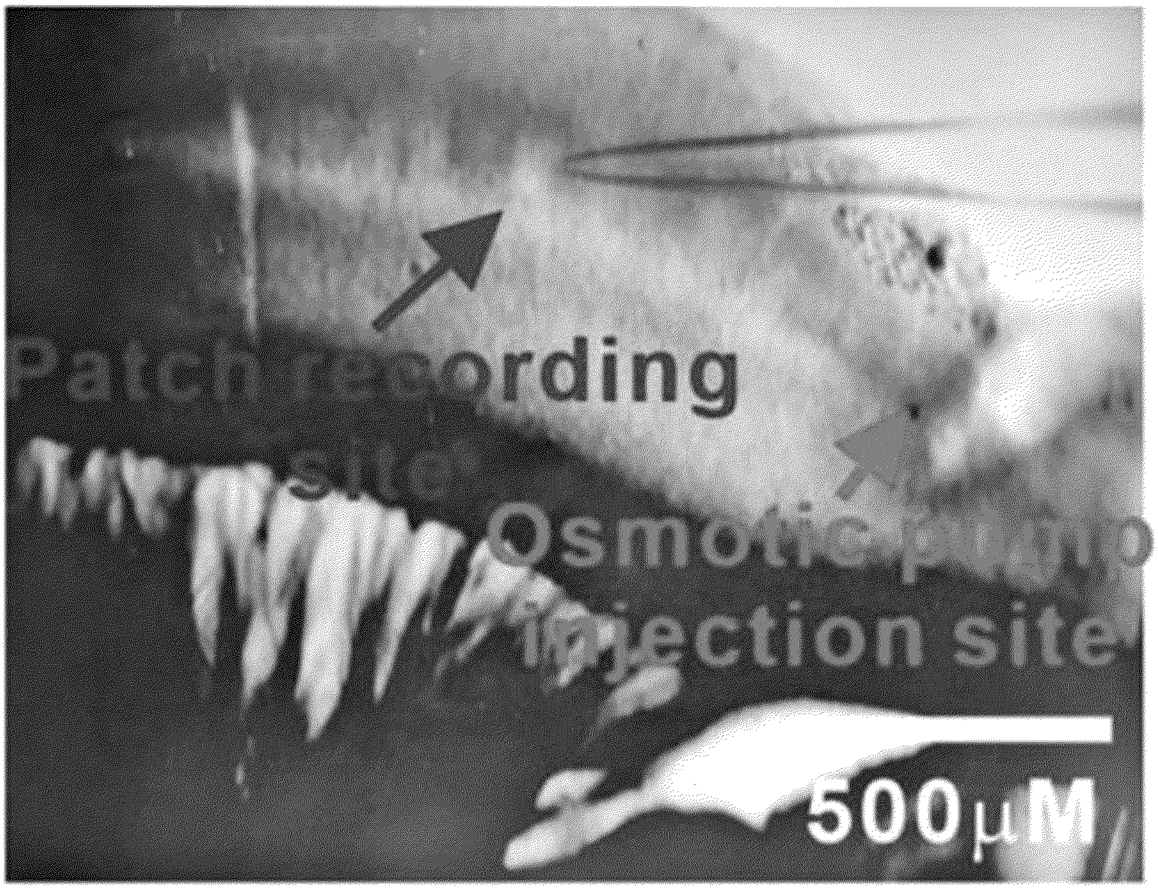

[FIG. 4C]

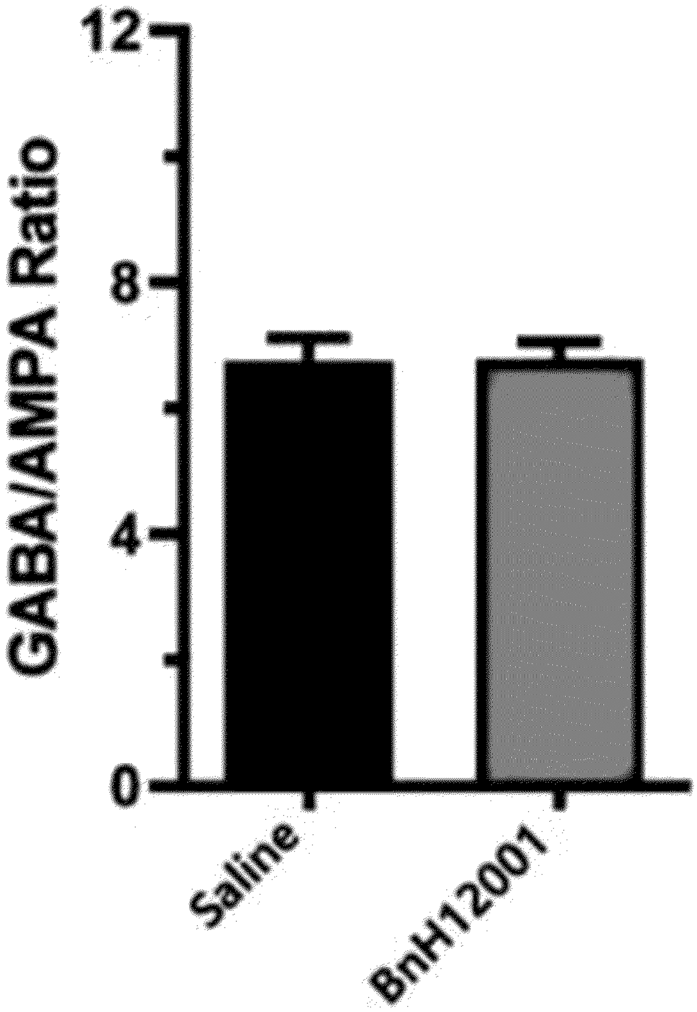

[FIG. 4D]

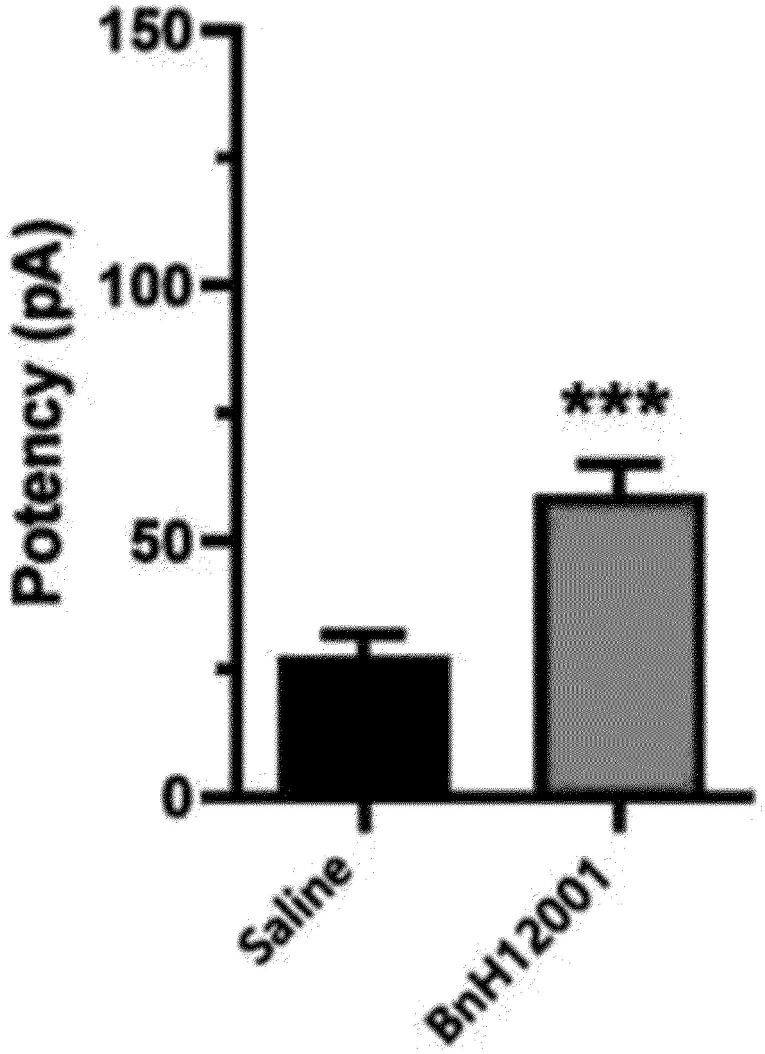

[FIG. 5A]

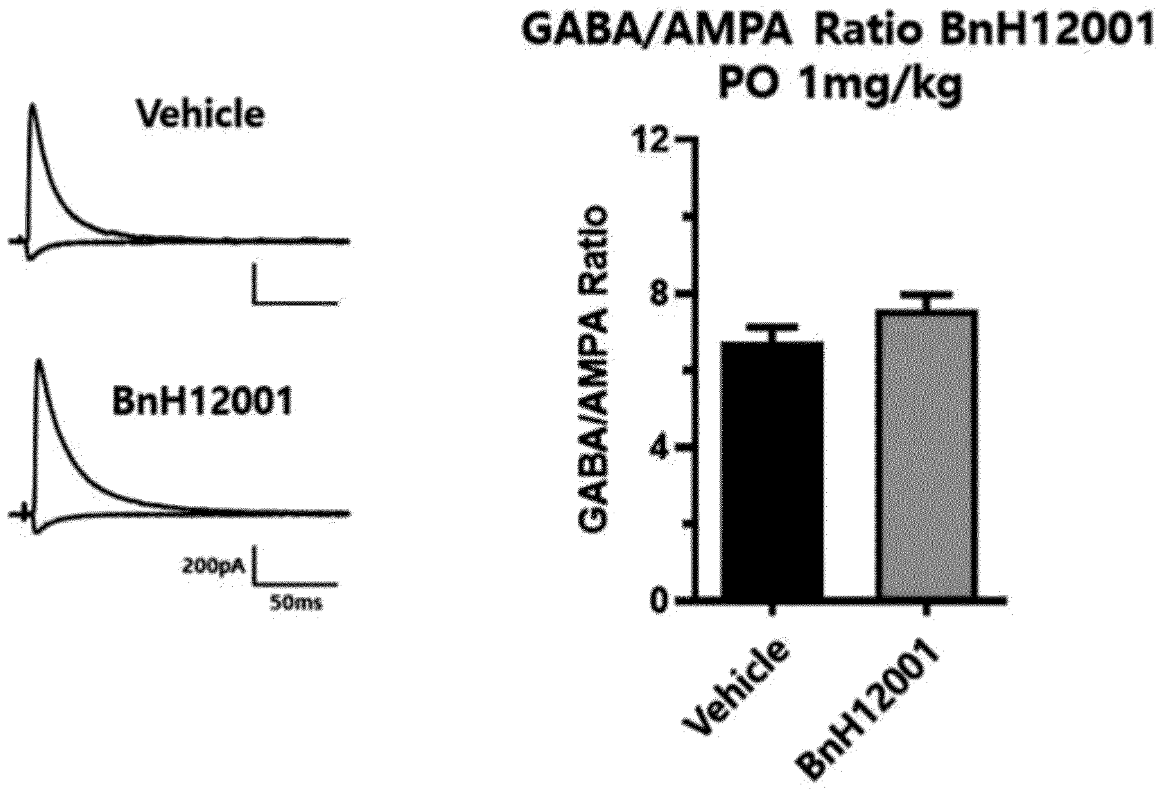
Vehicle
BnH12001
200pA
50ms
GABA/AMPA Ratio BnH12001
PO 1mg/kg
GABA/AMPA Ratio
12
8
4
0
Vehicle
BnH12001

[FIG. 5B]

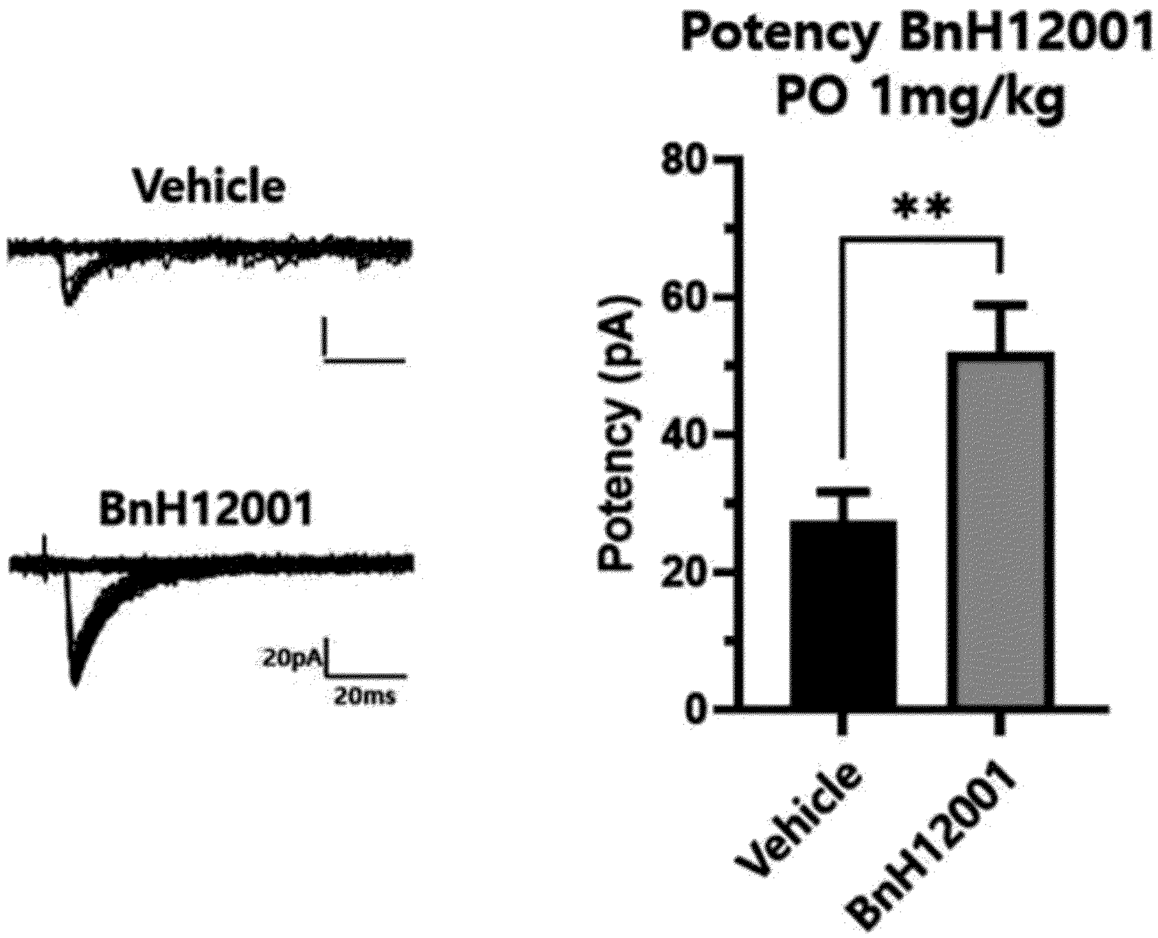
Vehicle
BnH12001
20pA
20ms
Potency BnH12001
PO 1mg/kg
Potency (pA)
80
60
40
20
0
**
Vehicle
BnH12001

[FIG. 5C]

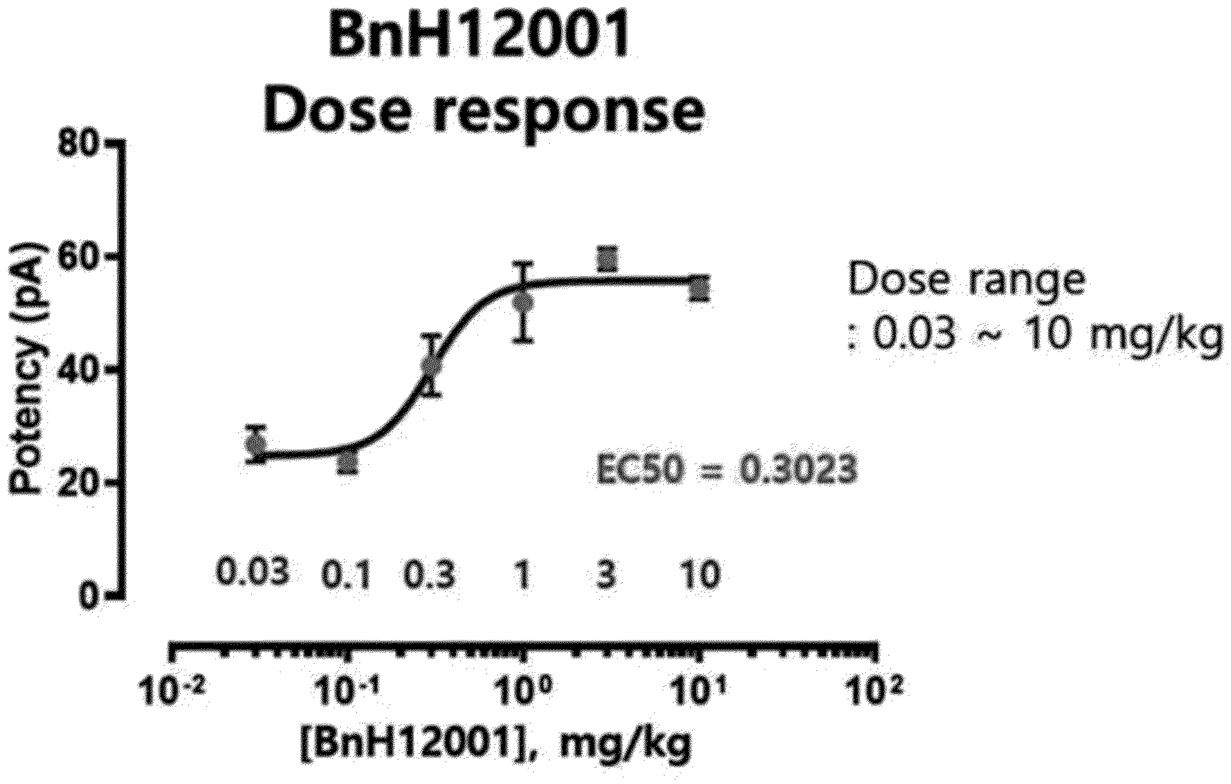
BnH12001
Dose response
Potency (pA)
80
60
40
20
0
0.03
0.1
0.3
1
3
10
EC50 = 0.3023
Dose range
: 0.03 ~ 10 mg/kg
10-2
10-1
100
101
102
[BnH12001], mg/kg

[FIG. 6A]

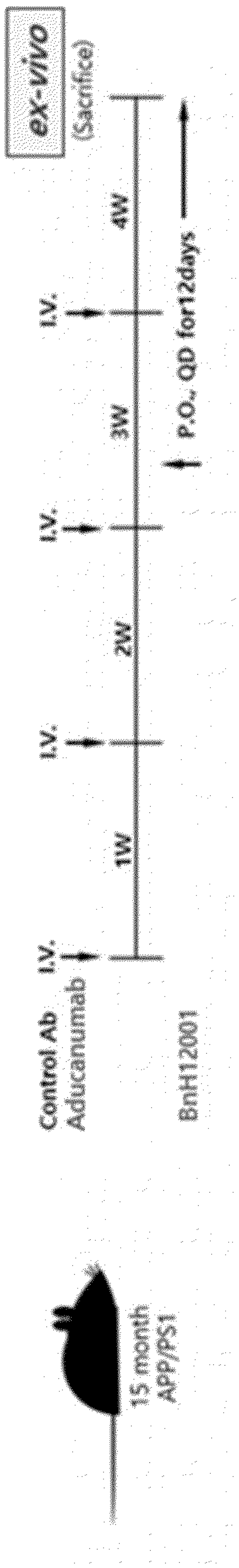
15 month
APP/PS1
Control Ab
Aducanumab
BnH12001
I.V.
1W
I.V.
2W
I.V.
3W
I.V.
4W
P.O., QD for12days
ex-vivo
(Sacrifice)

[FIG. 6B]

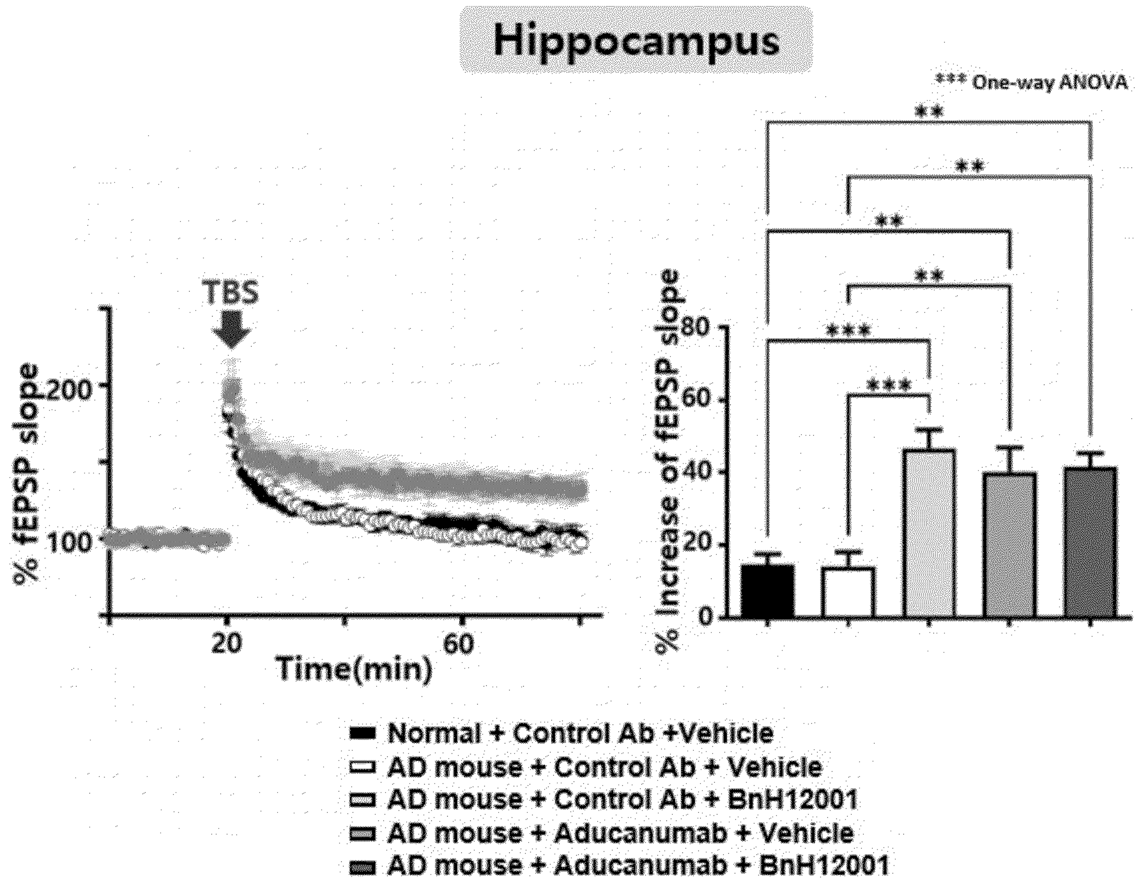
Hippocampus
*** One-way ANOVA
**
**
**
**
***
***
TBS
% fEPSP slope
200
100
20
60
Time(min)
% Increase of fEPSP slope
80
60
40
20
0
Normal + Control Ab +Vehicle
AD mouse + Control Ab + Vehicle
AD mouse + Control Ab + BnH12001
AD mouse + Aducanumab + Vehicle
AD mouse + Aducanumab + BnH12001

[FIG. 6C]

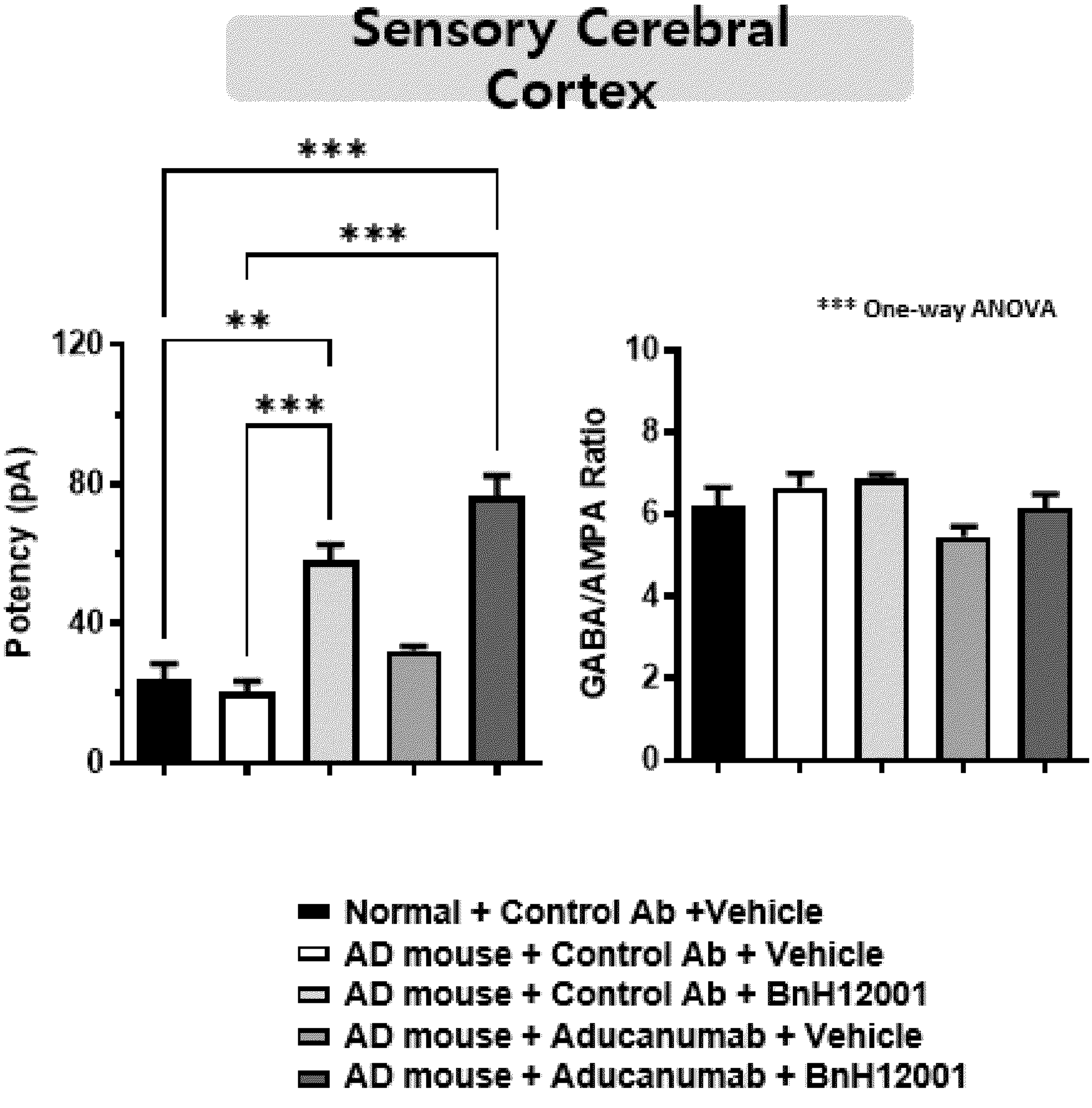
Sensory Cerebral Cortex
***
***
**
***
*** One-way ANOVA
Potency (pA)
0
40
80
120
GABA/AMPA Ratio
0
2
4
6
8
10
Normal + Control Ab +Vehicle
AD mouse + Control Ab + Vehicle
AD mouse + Control Ab + BnH12001
AD mouse + Aducanumab + Vehicle
AD mouse + Aducanumab + BnH12001

[FIG. 7]

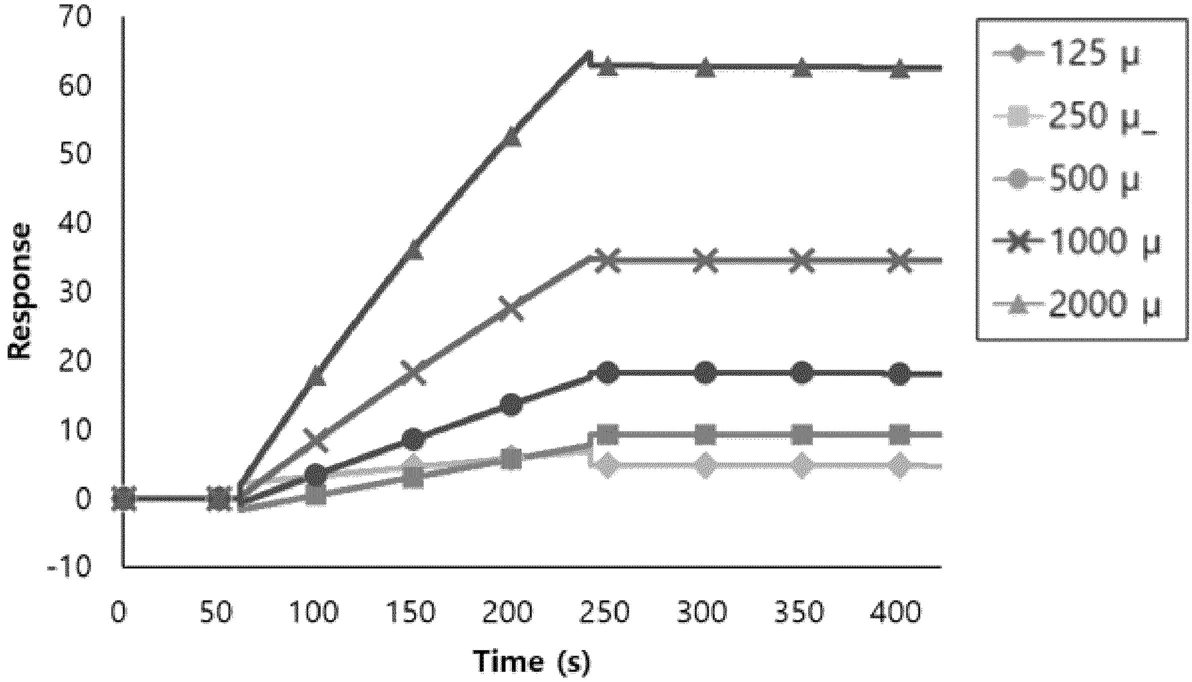
70
60
50
40
30
20
10
0
-10
Response
0
50
100
150
200
250
300
350
400
Time (s)
125 μ
250 μ_
500 μ
1000 μ
2000 μ

[FIG. 8A]

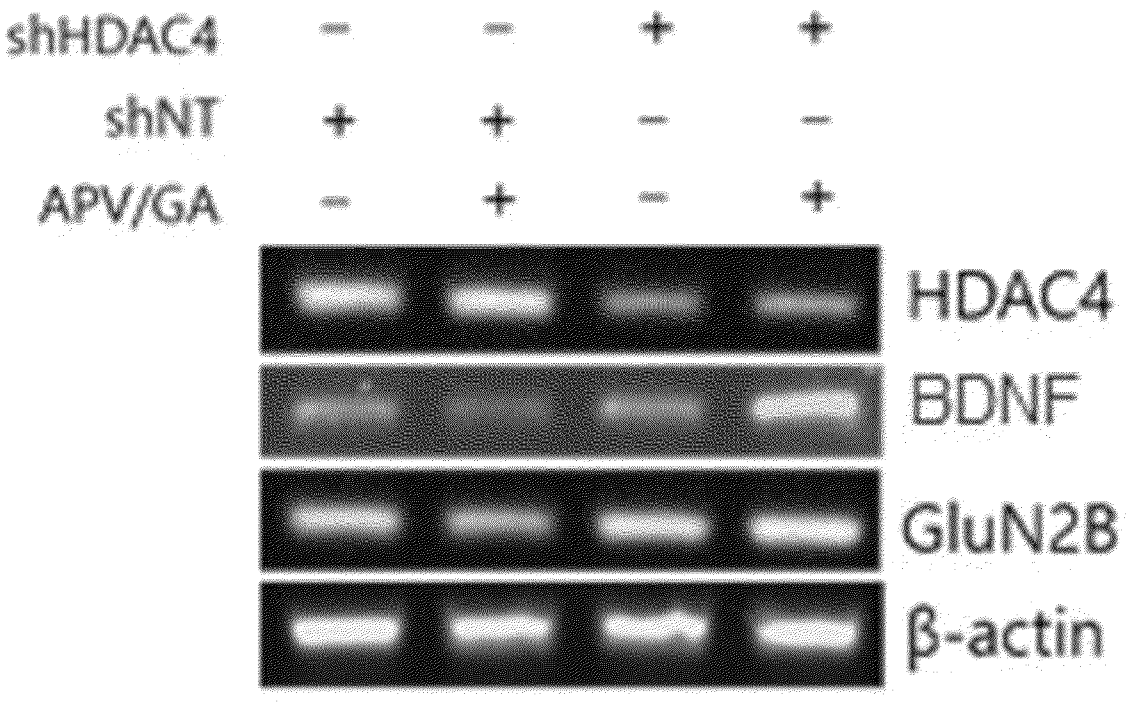

[FIG. 8B]

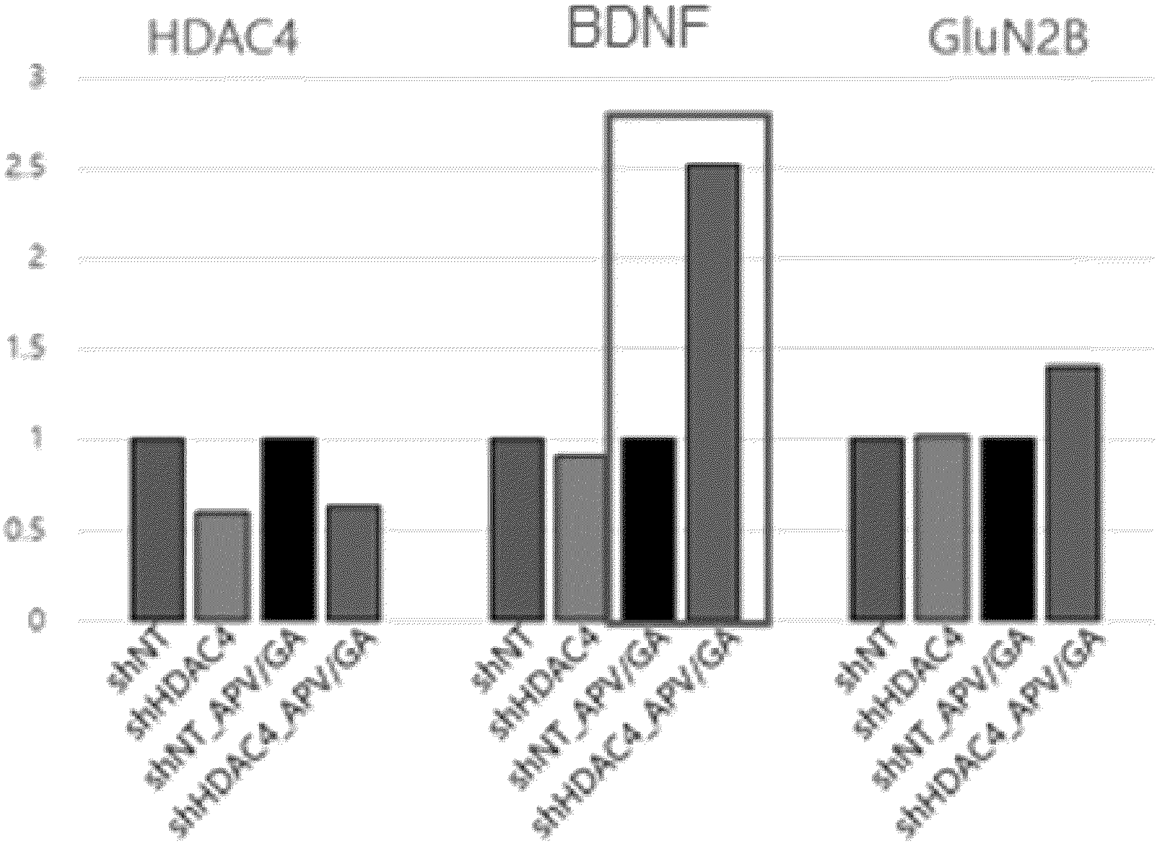
HDAC4
BDNF
GluN2B
3
2.5
2
1.5
1
0.5
0
shNT
shHDAC4
shNT_APV/GA
shHDAC4_APV/GA
shNT
shHDAC4
shNT_APV/GA
shHDAC4_APV/GA
shNT
shHDAC4
shNT_APV/GA
shHDAC4_APV/GA

[FIG. 9A]

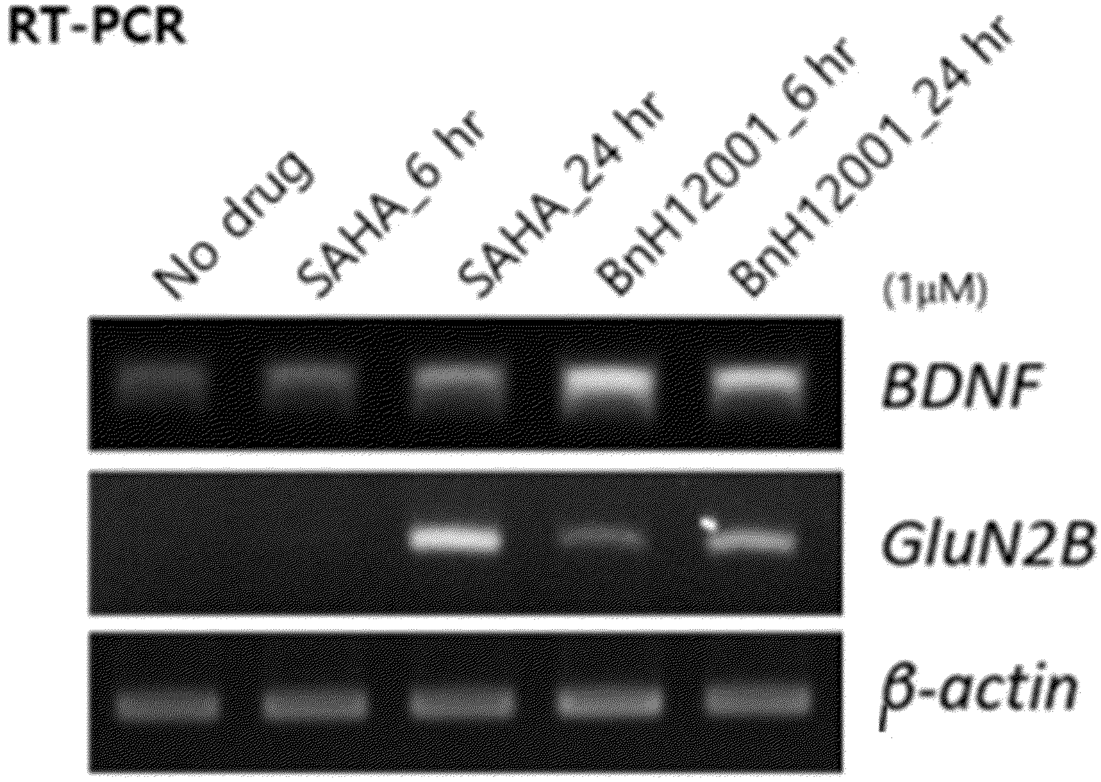
RT-PCR
No drug
SAHA_6 hr
SAHA_24 hr
BnH12001_6 hr
BnH12001_24 hr
(1μM)
BDNF
GluN2B
β-actin

[FIG. 9B]

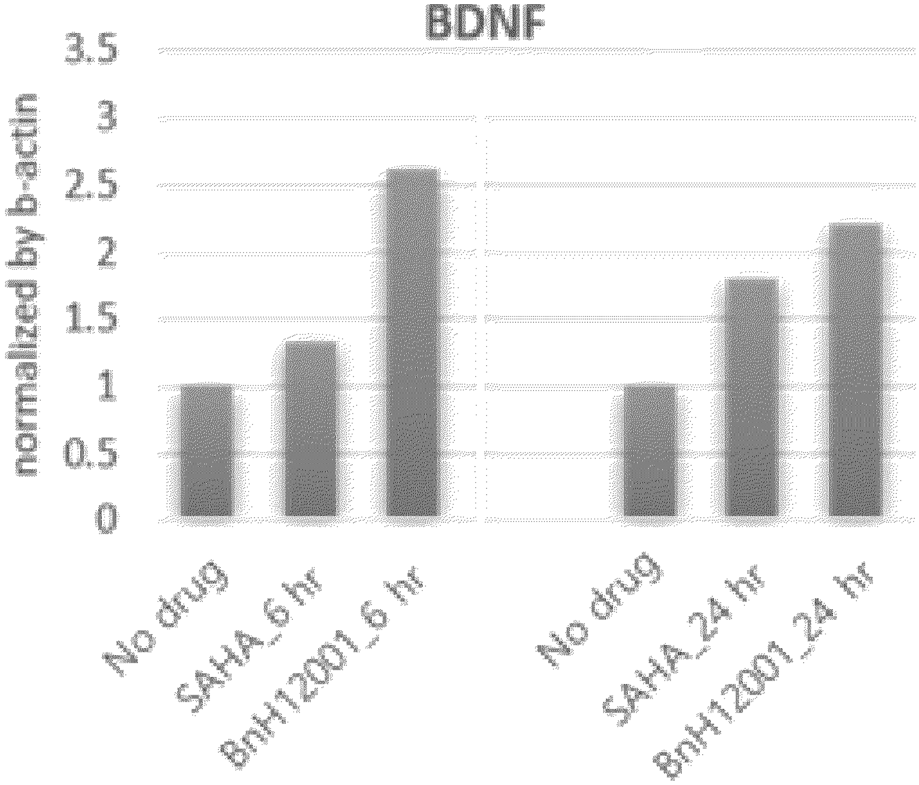
BDNF
normalized by b-actin
3.5
3
2.5
2
1.5
1
0.5
0
No drug
SAHA_6 hr
BnH12001_6 hr
No drug
SAHA_24 hr
BnH12001_24 hr

[FIG. 9C]

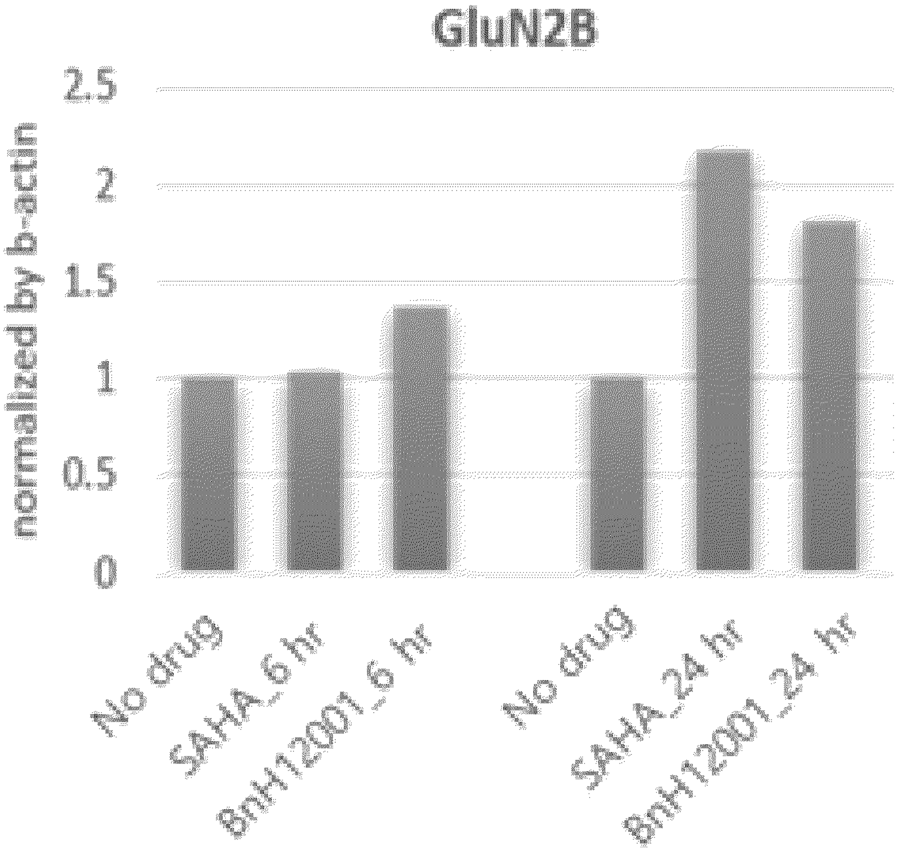
GluN2B
normalized by b-actin
2.5
2
1.5
1
0.5
0
No drug
SAHA_6 hr
BnH12001_6 hr
No drug
SAHA_24 hr
BnH12001_24 hr

[FIG. 10A]

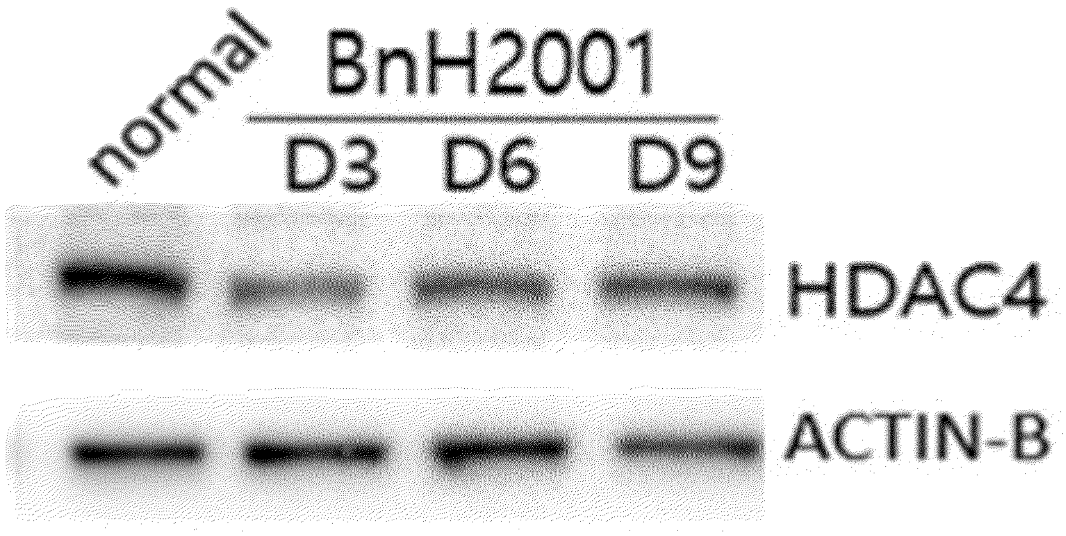

[FIG. 10B]

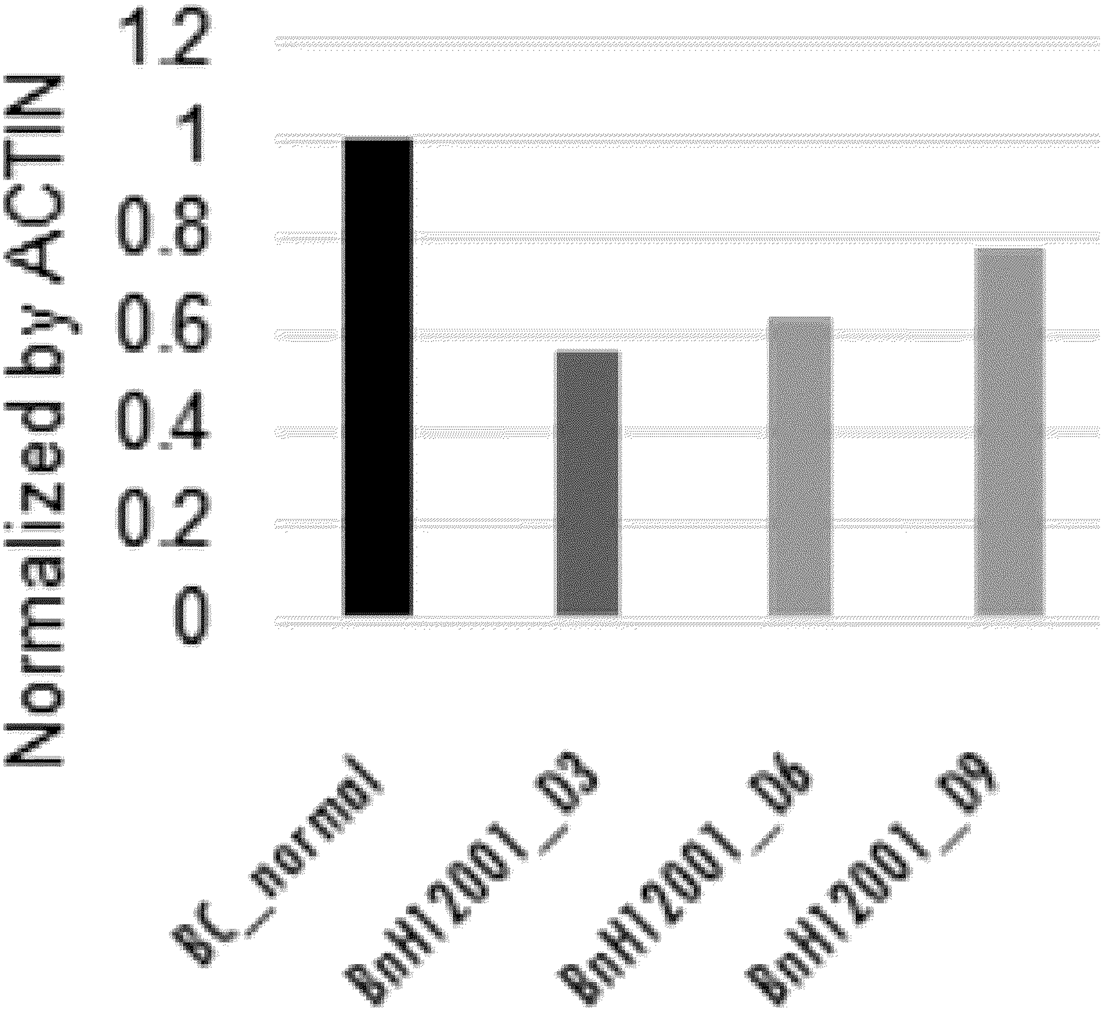
Normalized by ACTIN
1.2
1
0.8
0.6
0.4
0.2
0
BC_normal
BnHI2001_03
BnHI2001_06
BnHI2001_09

[FIG. 11A]

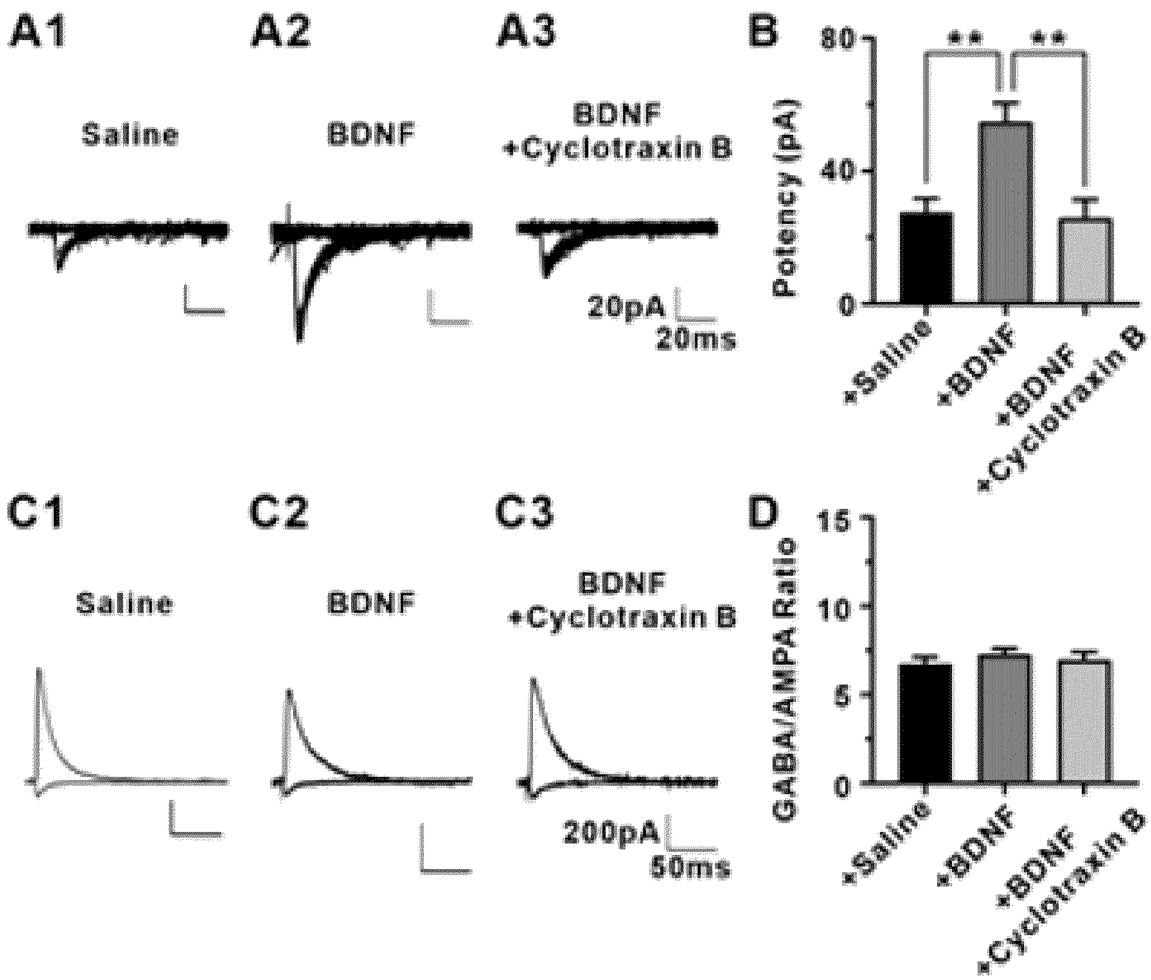
A1
A2
A3
B
Saline
BDNF
BDNF
+Cyclotraxin B
20pA
20ms
Potency (pA)
80
40
0
**
**
+Saline
+BDNF
+BDNF
+Cyclotraxin B
C1
C2
C3
D
Saline
BDNF
BDNF
+Cyclotraxin B
200pA
50ms
GABA/AMPA Ratio
15
10
5
0
+Saline
+BDNF
+BDNF
+Cyclotraxin B

[FIG. 11B]

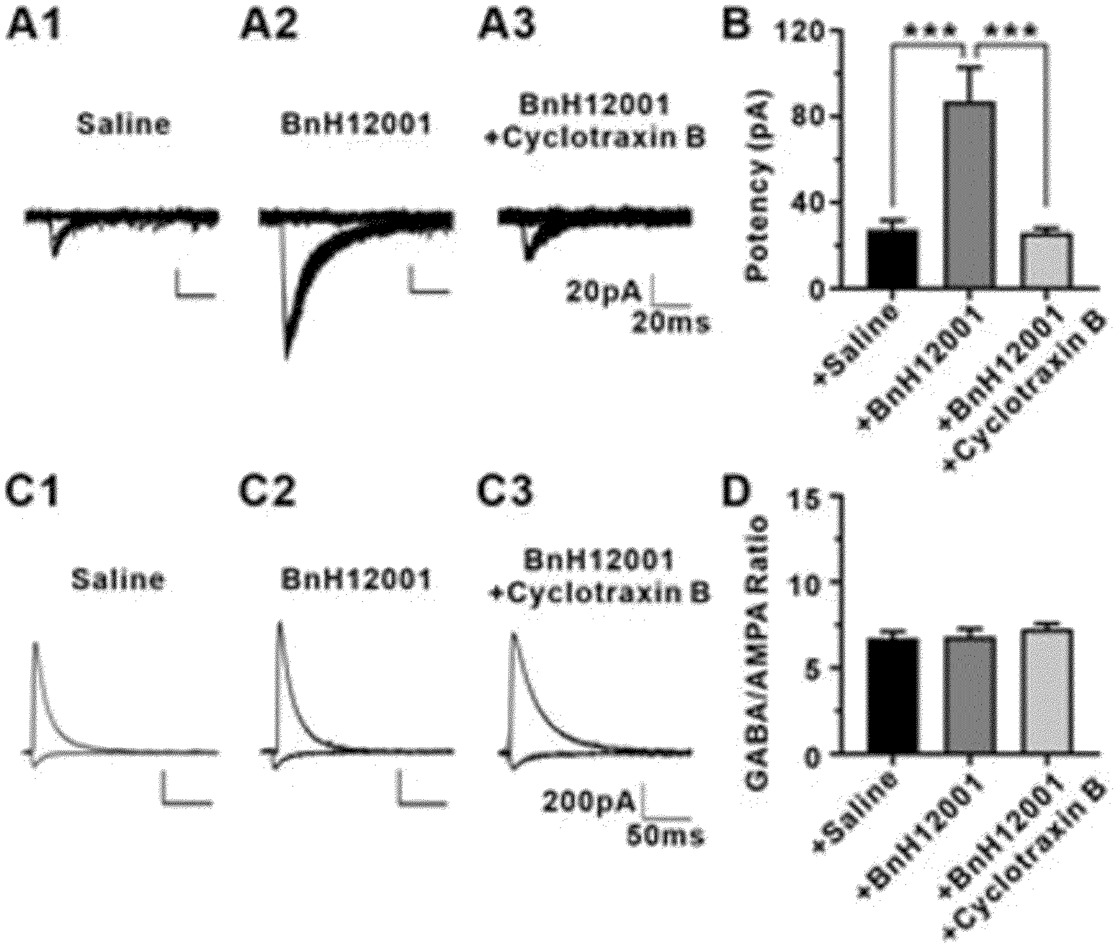
A1
A2
A3
B
Saline
BnH12001
BnH12001
+Cyclotraxin B
20pA
20ms
Potency (pA)
120
80
40
0
***
***
+Saline
+BnH12001
+BnH12001
+Cyclotraxin B
C1
C2
C3
D
Saline
BnH12001
BnH12001
+Cyclotraxin B
200pA
50ms
GABA/AMPA Ratio
15
10
5
0
+Saline
+BnH12001
+BnH12001
+Cyclotraxin B

[FIG. 12A]

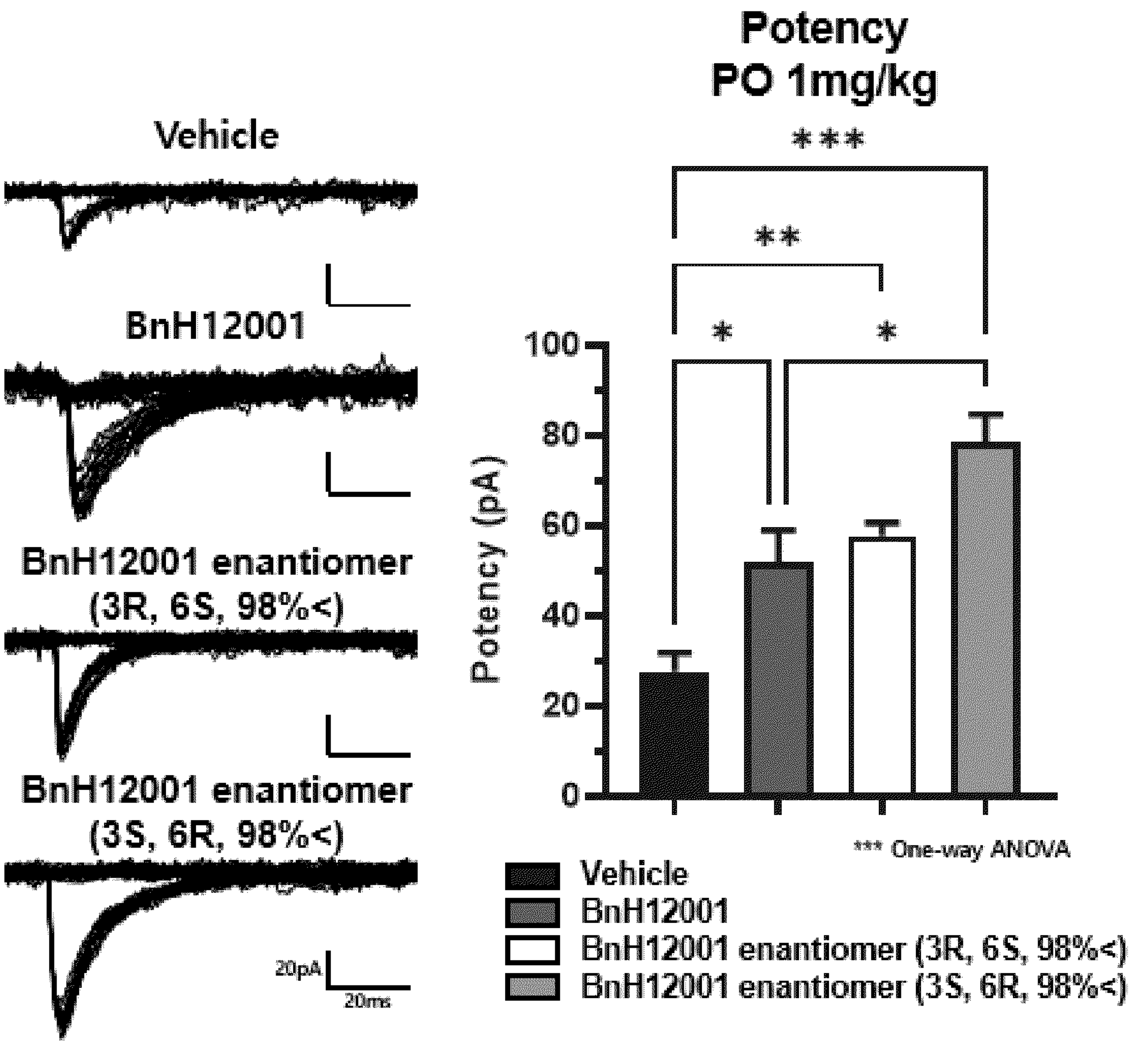
Vehicle
BnH12001
BnH12001 enantiomer (3R, 6S, 98%<)
BnH12001 enantiomer (3S, 6R, 98%<)
20pA
20ms
Potency
PO 1mg/kg
***
**
*
*
Potency (pA)
100
80
60
40
20
0
*** One-way ANOVA
Vehicle
BnH12001
BnH12001 enantiomer (3R, 6S, 98%<)
BnH12001 enantiomer (3S, 6R, 98%<)

[FIG. 12B]

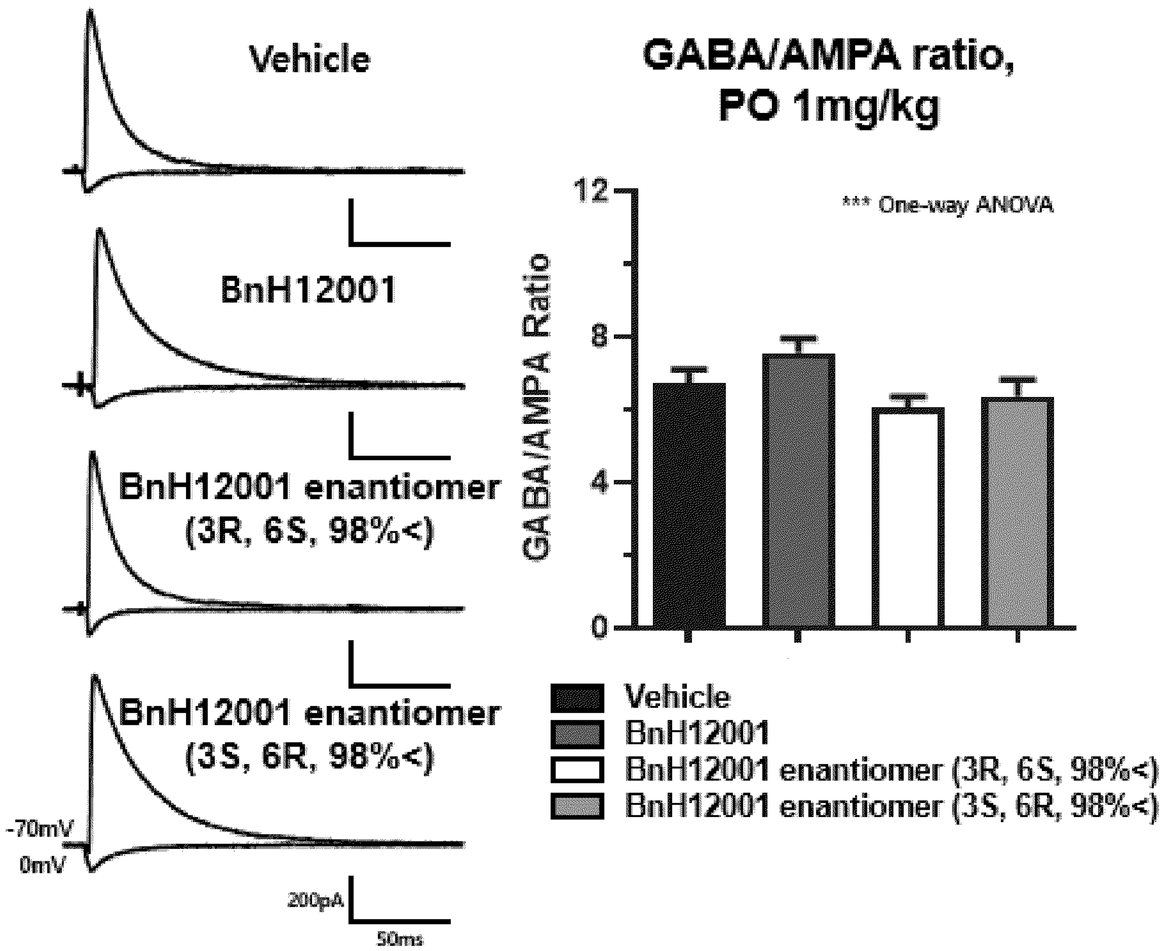
Vehicle
BnH12001
BnH12001 enantiomer (3R, 6S, 98%<)
BnH12001 enantiomer (3S, 6R, 98%<)
-70mV
0mV
200pA
50ms
GABA/AMPA ratio, PO 1mg/kg
*** One-way ANOVA
GABA/AMPA Ratio
12
8
4
0
Vehicle
BnH12001
BnH12001 enantiomer (3R, 6S, 98%<)
BnH12001 enantiomer (3S, 6R, 98%<)

[FIG. 13A]

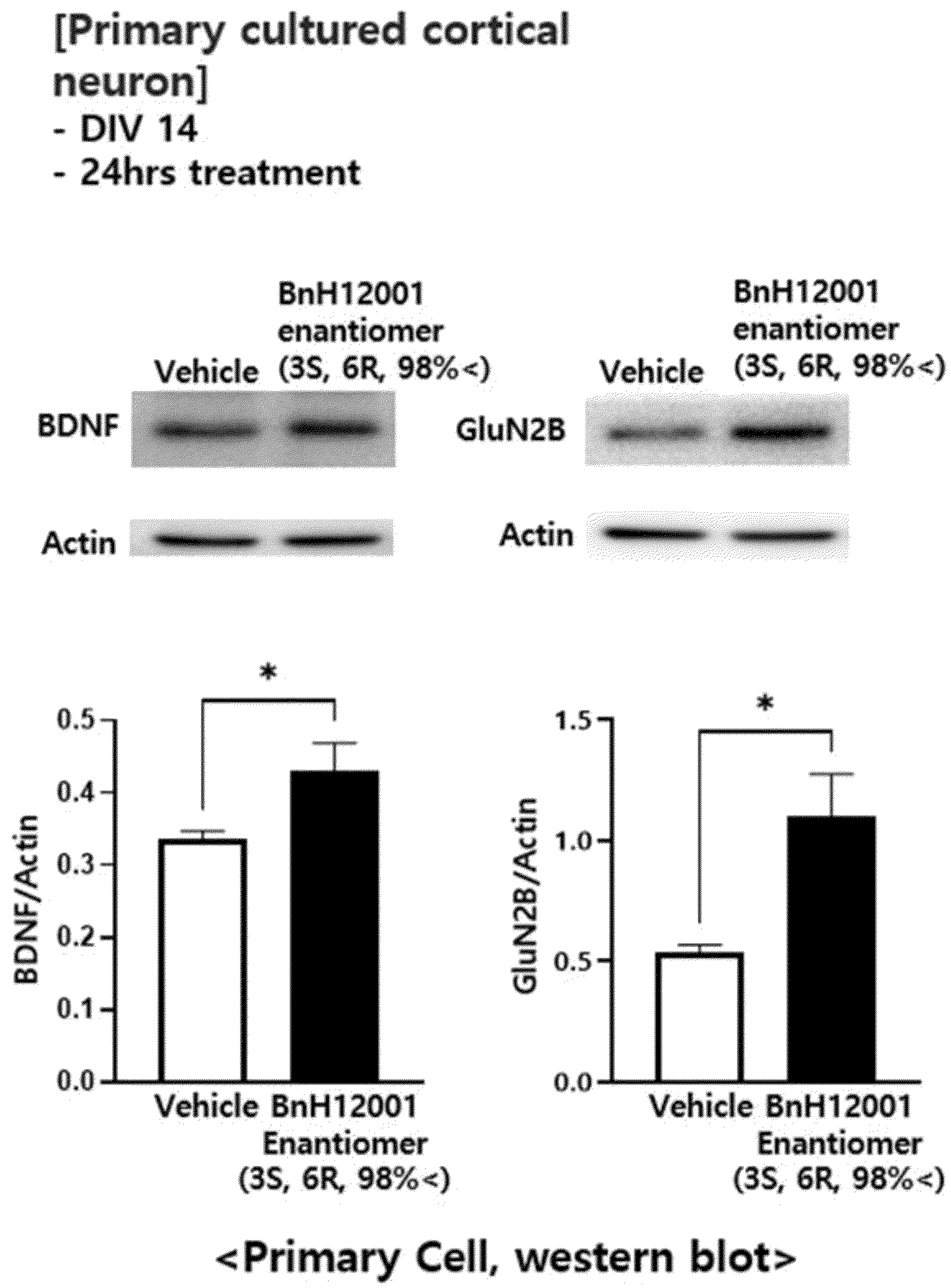
[Primary cultured cortical neuron]
- DIV 14
- 24hrs treatment
Vehicle
BnH12001 enantiomer (3S, 6R, 98%<)
BDNF
Actin
Vehicle
BnH12001 enantiomer (3S, 6R, 98%<)
GluN2B
Actin
*
BDNF/Actin
0.0
0.1
0.2
0.3
0.4
0.5
Vehicle
BnH12001 Enantiomer (3S, 6R, 98%<)
*
GluN2B/Actin
0.0
0.5
1.0
1.5
Vehicle
BnH12001 Enantiomer (3S, 6R, 98%<)
<Primary Cell, western blot>

[FIG. 13B]

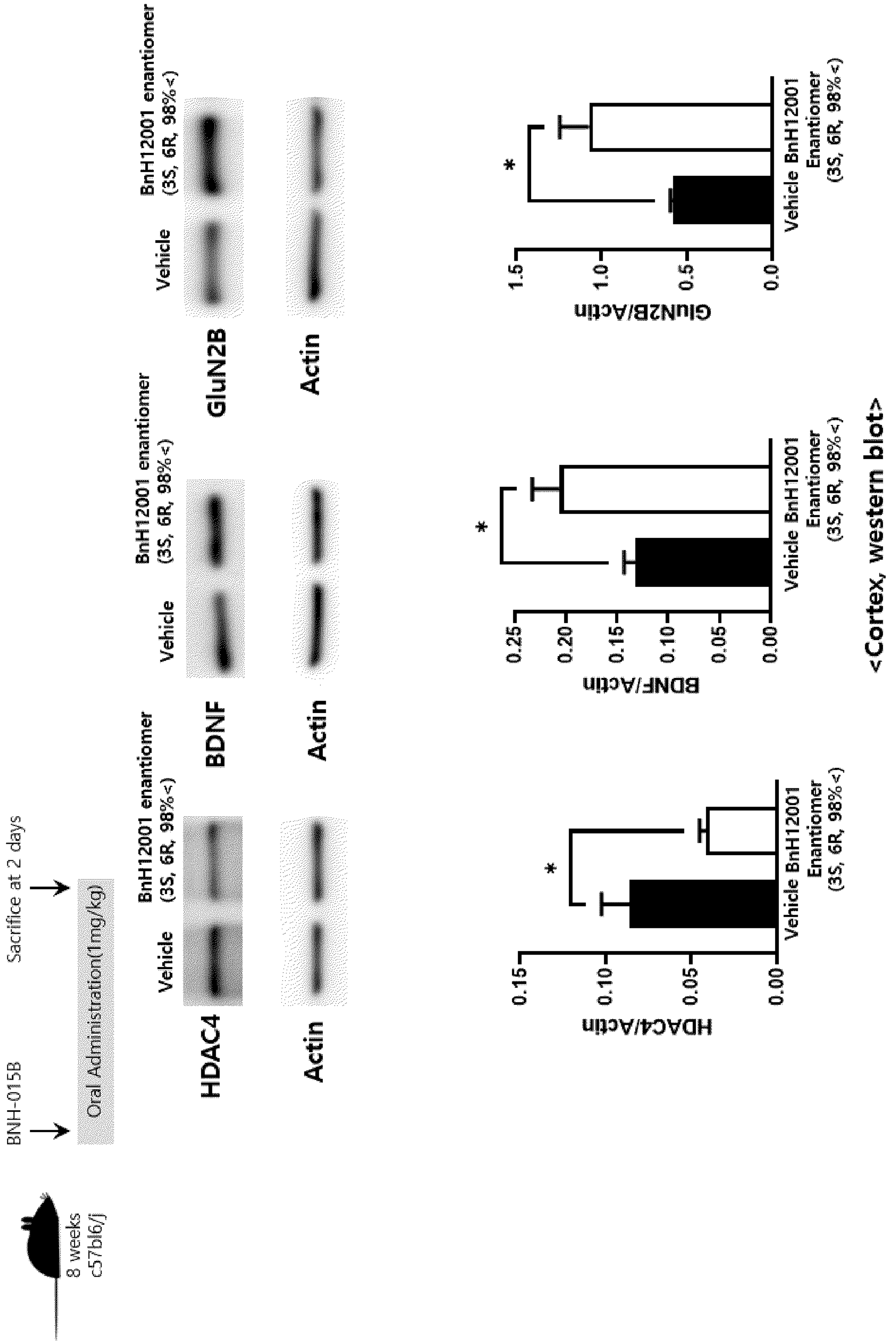
8 weeks
c57bl6/j
BNH-015B
Sacrifice at 2 days
Oral Administration(1mg/kg)
HDAC4
BDNF
GluN2B
Actin
Vehicle
BnH12001 enantiomer
(3S, 6R, 98%<)
HDAC4/Actin
BDNF/Actin
GluN2B/Actin
Vehicle BnH12001
Enantiomer
(3S, 6R, 98%<)
*
<Cortex, western blot>

[FIG. 13C]

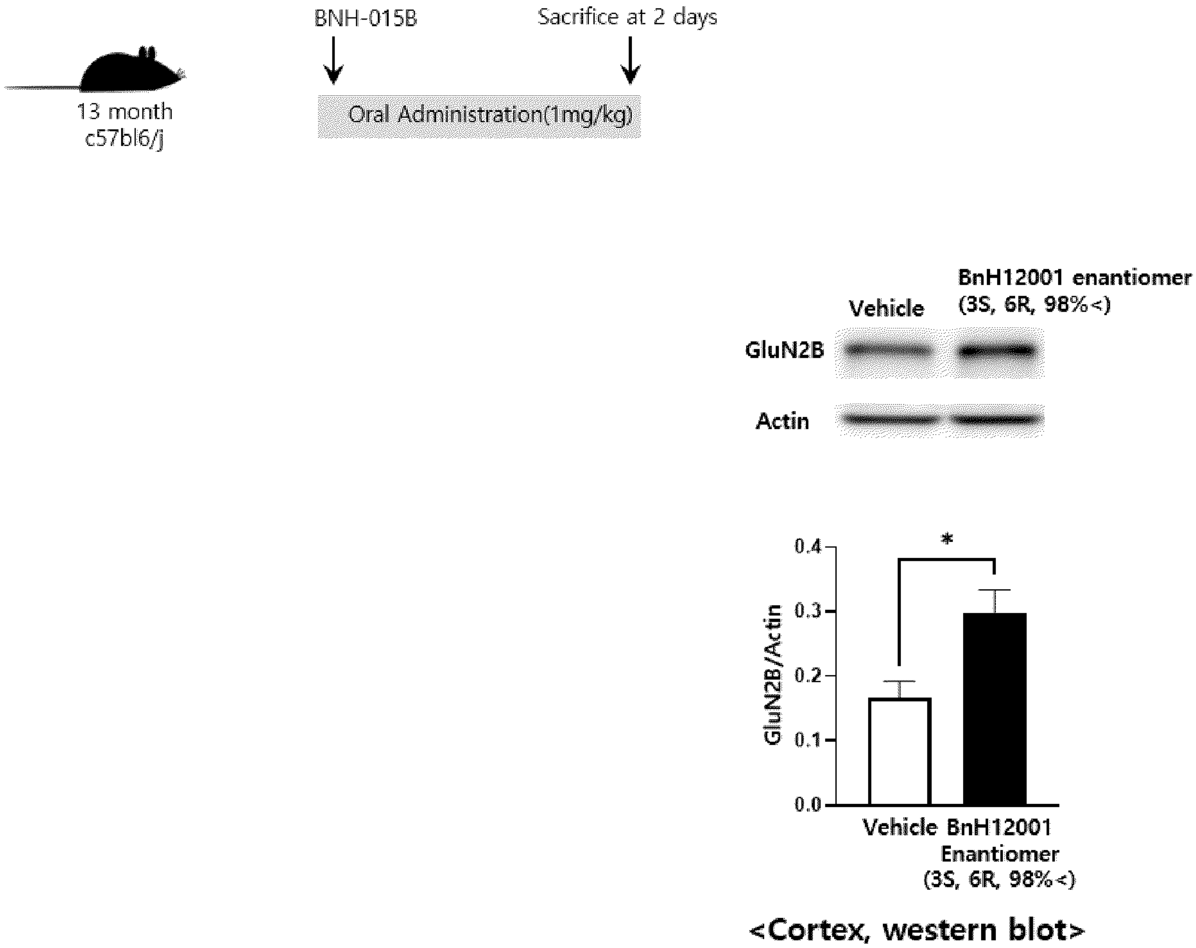
BNH-015B
Sacrifice at 2 days
13 month
c57bl6/j
Oral Administration(1mg/kg)
Vehicle
BnH12001 enantiomer
(3S, 6R, 98%<)
GluN2B
Actin
GluN2B/Actin
0.4
0.3
0.2
0.1
0.0
*
Vehicle
BnH12001
Enantiomer
(3S, 6R, 98%<)
<Cortex, western blot>

**INTERNATIONAL SEARCH REPORT**

International application No. **PCT/KR2023/004138**

**A. CLASSIFICATION OF SUBJECT MATTER**

**C07D 211/60**(2006.01)i; **C07D 211/16**(2006.01)i; **C07D 207/16**(2006.01)i; **C07D 207/08**(2006.01)i; **C07D 401/06**(2006.01)i; **C07D 409/06**(2006.01)i; **C07D 405/06**(2006.01)i; **A61K 31/445**(2006.01)i; **A61K 31/40**(2006.01)i; **A61P 25/00**(2006.01)i

According to International Patent Classification (IPC) or to both national classification and IPC

**B. FIELDS SEARCHED**

Minimum documentation searched (classification system followed by classification symbols)

C07D 211/60(2006.01); C07D 279/12(2006.01); C07D 401/12(2006.01); C07D 403/12(2006.01); C07D 405/14(2006.01); C07D 471/04(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (registry, caplus), Google & keywords: 질소 함유 헤테로고리(nitrogen-containing heterocycle), 시냅스 가소성(synaptic plasticity), 신경퇴행성 질환(neurodegenerative disease), HDAC

**C. DOCUMENTS CONSIDERED TO BE RELEVANT**

| Category* | Citation of document, with indication, where appropriate, of the relevant passages | Relevant to claim No. |
|---|---|---|
| X<br>A | WO 2016-029146 A1 (UNIVERSITY OF WASHINGTON) 25 February 2016 (2016-02-25)<br>See paragraph [0217], scheme 23. | 1-6,11-14<br>7-10,15-21 |
| X | Chemical Abstract Compound. STNext. RN 757961-21-0. 07 October 2014.<br>See the compounds. | 1-5,11-14 |
| A | US 2004-0072802 A1 (DUAN, J. et al.) 15 April 2004 (2004-04-15)<br>See entire document. | 1-21 |
| A | GROTJAHN, D. B. et al. Oxidative addition of the C–O bond of amino acid esters to Rh (I) forming chelating acyl complexes. Inorganica chimica acta. 2004, vol. 357, no. 10, pp. 3047-3056.<br>See entire document. | 1-21 |
| A | WO 01-70734 A2 (DUPONT PHARMACEUTICALS COMPANY) 27 September 2001 (2001-09-27)<br>See entire document. | 1-21 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 July 2023** | **18 July 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2023/004138**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed.
   - b. ☐ furnished subsequent to the international filing date for the purposes of international search (Rule 13*ter*.1(a)),
     - ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/KR2023/004138**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **22**
because they relate to subject matter not required to be searched by this Authority, namely:

Claim 22 pertains to a method for treatment of the human body by surgery or therapy, and thus pertains to a subject matter on which the International Searching Authority is not required to carry out an international search under the provisions of PCT Article 17(2)(a)(i) and PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/KR2023/004138**

| Patent document cited in search report | | | Publication date (day/month/year) | Patent family member(s) | | | Publication date (day/month/year) |
|---|---|---|---|---|---|---|---|
| WO | 2016-029146 | A1 | 25 February 2016 | CN | 205034082 | U | 17 February 2016 |
| | | | | US | 10913736 | B2 | 09 February 2021 |
| | | | | US | 2017-0275279 | A1 | 28 September 2017 |
| US | 2004-0072802 | A1 | 15 April 2004 | None | | | |
| WO | 01-70734 | A2 | 27 September 2001 | AR | 035174 | A1 | 05 May 2004 |
| | | | | AT | 260272 | T | 15 March 2004 |
| | | | | AU | 2001-50850 | A1 | 03 October 2001 |
| | | | | BR | 0109469 | A | 29 April 2003 |
| | | | | CA | 2400168 | A1 | 27 September 2001 |
| | | | | CN | 1420881 | A | 28 May 2003 |
| | | | | DE | 60102137 | T2 | 21 October 2004 |
| | | | | EP | 1263756 | A2 | 11 December 2002 |
| | | | | EP | 1263756 | B1 | 25 February 2004 |
| | | | | ES | 2215893 | T3 | 16 October 2004 |
| | | | | HK | 1049334 | A1 | 09 May 2003 |
| | | | | HK | 1049334 | B | 16 July 2004 |
| | | | | IL | 151018 | A | 12 February 2003 |
| | | | | JP | 2003-528097 | A | 24 September 2003 |
| | | | | NZ | 521245 | A | 30 April 2004 |
| | | | | US | 2002-0013341 | A1 | 31 January 2002 |
| | | | | US | 6495565 | B2 | 17 December 2002 |
| | | | | WO | 01-70734 | A3 | 14 March 2002 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2006008517 A


### Non-patent literature cited in the description


- **YU et al.** *Neuron.*, 2012, vol. 74, 731-42
- **PETRUS et al.** *Neuron.*, 2014, vol. 81, 664-73
- **GAO et al.** *J Neurosci.*, 2014, vol. 34, 10770-9
- **VERDIN** ; **OTT**. *Nat Rev Mol Cell Biol*, 2015, vol. 16, 258-264
- **GROZINGER et al.** *Proc. Natl. Acad. Sci. USA*, 1999, vol. 96, 4868-4873
- **DARCY et al.** *J. Comp. Neurol.*, 2010, vol. 518, 722-740
- **SANDO et al.** *Cell*, 2012, vol. 151, 821-834
- **TAKASE et al.** *PloS ONE*, 2013, vol. 8, e58473
- **BOLGER** ; **YAO**. *J Neurosci. 2005*, 2005, vol. 25, 9544-9553
- **MIELCAREK et al.** *PLoS Biol.*, 2013, vol. 11, e1001717
- **SAILAJA et al.** *Proc. Natl. Acad. Sci. USA*, 2012, vol. 109, E3687-E3695
- **BOLGER** ; **YAO**. *J. Neurosci.*, 2005, vol. 25, 9544-9553
- **LI et al.** *Nat. Med.*, 2012, vol. 18, 783-790
- **YANG et al.** *FEBS Lett.*, 2011, vol. 585, 761-766
- **SARKAR et al.** *Neuropsychopharmacology*, 2014, vol. 39, 2221-2232
- **FITZSIMONS et al.** *PloS ONE*, 2013, vol. 8, e83903
- **MAIN et al.** *Front. Mol. Neurosci.*, 2021, vol. 14, 616-642
- **YEH et al.** *Brain Res.*, 2013, vol. 1504, 16-24
- **WHITEHOUSE et al.** *Neuropathol. Appl. Neurobiol*, 2014, vol. 41, 245257
- **TAKAHASHI-FUJIGASAKI** ; **FUJIGASAKI**. *Neuropathol. Appl. Neurobiol.*, 2006, vol. 32, 562-566
- **NARDONE et al.** *Transl. Psychiatry*, 2014, vol. 4, e433
- **GERVAIN et al.** *Front Syst Neurosci.*, 2013, vol. 7, 102
- **BARONCELLI L et al.** *J Neurosci.*, 2016, vol. 36, 3430-40
- **BAGHERI et al.** *Int. J. Mol. Sci.*, 2019, vol. 20, 1109
- **HOCKLY et al.** *J Biol. Chem.*, 2003, vol. 278, 16059-16072
- **GARDIAN et al.** *J. Biol. Chem.*, 2005, vol. 280, 556-563
- **ZIELONKA et al.** *Front. Physiol.*, 2014, vol. 5, 380
- **MIELCAREK et al.** *PLoS Genet.*, 2014, vol. 10, e1004550
- **MIELCAREK et al.** *PloS ONE*, 2014, vol. 9, e108961
- **ZIELONKA et al.** *Exp. Clin. Cardiol.*, 2014, vol. 20, 25472554
- **MIELCAREK et al.** *PloS ONE*, 2011, vol. 6, e27746
- **KOPPEL** ; **TIMMUSK**. *Neuropharmacology*, 2013, vol. 75, 106-11
- **MEGHAN E. et al.** *Bone.*, 2011, vol. 48, 1117-1126
- Diagnostic and Statistical Manual of Mental Disorders. American Psychiatric Association, 1994
- **GREENE et al.** Protective Groups in Organic Synthesis. John Wiley and Sons, 1991
- **CITROME**. *Psychopharmacol. Bull.*, 2003, vol. 37 (2), 74-88
- **JOHANNESSEN**. *CNS Drug Rev.*, 2003, vol. 9, 199-216
- **TSANKOVA et al.** *Nat. Neurosci.*, 2006, vol. 9, 519-525
- **FRAY et al.** CANTAB battery: proposed utility in neurotoxicology.. *Neurotoxicol Teratol*, 1996, vol. 18 (4), 499-504
- **DEHAENE et al.** Reward-dependent learning in neuronal networks for planning and decision making. *Brain Res.*, 2000, vol. 126, 21729
- **IVERSON et al.** Interpreting change on the WAIS-III/WMS-I11 in clinical samples. *Arch Clin Neuropsychol.*, 2001, vol. 16 (2), 183-91
- **WEAVER et al.** Mild memory impairment in healthy older adults is distinct from normal aging.. *Cogn.*, 2006, vol. 60 (2), 146-55
- **LATTAL et al.** *Behav. Neurosci.*, 2007, vol. 121 (5), 1125-1131

- Cognitive-Behavioral Therapies. Guilford Publications, Inc., 2002
- **JUDITH S.S. BECK**. The new Handbook of Cognitive Therapy: Basics and Beyond. Guilford Publications, Inc., 1995
- **ISAAC** ; **CRAIR** ; **NICOLL** ; **MALENKA**. *Silent synapses during development of thalamocortical inputs*, 1997
- **FELDMAN** ; **NICOLL** ; **MALENKA** ; **ISAAC**. *Synaptic plasticity at thalamocortical synapses in developing rat somatosensory cortex: LTP, LTD, and silent synapses*, 1998, https://doi.org/10.1002/(SICI)1097-4695(199910)41:1<92::AID-NEU12>3.0.CO;2U
- **ISAAC et al.** *Silent Synapses during Development of Thalamocortical Inputs*, 1997, https://doi.org/10.1016/S08966273(00)80267-6
- **CHITTAJALLU** ; **ISAAC**. *Emergence of cortical inhibition by coordinated sensory-driven plasticity at distinct synaptic loci*, 2010
- **STEVENS** ; **WANG**. *Facilitation and depression at single central synapses*, 1995
- **GIL et al.** *Efficacy of Thalamocortical and Intracortical Synaptic Connections: Quanta, Innervation, and Reliability*, 1999, https://doi.org/10.1016/S0896-6273(00)80788-6